(19) Europäisches Patentamt
European Patent Office
Office européen des brevets

(11) EP 1 541 563 A1

(12) # EUROPEAN PATENT APPLICATION
published in accordance with Art. 158(3) EPC

(43) Date of publication:
**15.06.2005 Bulletin 2005/24**

(21) Application number: **03764137.0**

(22) Date of filing: **08.07.2003**

(51) Int Cl.[7]: **C07D 241/22**, A61K 31/4965, A61K 31/497, A61K 31/498, A61K 31/506, A61P 1/04, A61P 1/16, A61P 1/18, A61P 3/10, A61P 9/10, A61P 11/00, A61P 11/06

(86) International application number:
**PCT/JP2003/008654**

(87) International publication number:
**WO 2004/007472 (22.01.2004 Gazette 2004/04)**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **10.07.2002 JP 2002200879**

(71) Applicant: **ONO PHARMACEUTICAL CO., LTD.**
**Osaka-shi, Osaka 541-8526 (JP)**

(72) Inventors:
- **HABASHITA, Hiromu**
  **c/o Ono Pharmaceutical Co., Ltd.**
  **Mishima-gun, Osaka 618-8585 (JP)**
- **KOKUBO, Masaya**
  **c/o Ono Pharmaceutical Co., Ltd.**
  **Mishima-gun, Osaka 618-8585 (JP)**
- **SHIBAYAMA, Shiro**
  **c/o Ono Pharmaceutical Co., Ltd.**
  **Tsukuba-shi, Ibaraki 300-4247 (JP)**
- **TADA, Hideaki c/o Ono Pharmaceutical Co., Ltd.**
  **Tsukuba-shi, Ibaraki 300-4247 (JP)**
- **SAGAWA, Kenji**
  **c/o Ono Pharmaceutical Co., Ltd.**
  **Mishima-gun, Osaka 618-8585 (JP)**

(74) Representative: **Henkel, Feiler & Hänzel**
**Möhlstrasse 37**
**81675 München (DE)**

(54) **CCR4 ANTAGONIST AND MEDICINAL USE THEREOF**

(57) A compound of formula (I) or a salt thereof, and medical use thereof (the symbols in the formula are as described in the specification).

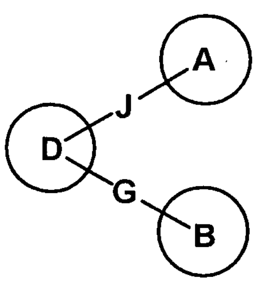

(I)

The compound of formula (I) has CCR4 antagonistic activity, and therefore is useful as a preventive and/or therapeutic agent for diseases associated with CCR4, i.e., CCR4-mediated diseases such as inflammatory and/or allergic diseases [e.g., systemic inflammatory response syndrome (SIRS), anaphylaxis or anaphylaxis-like reaction, allergic vasculitis, reject reaction for graft organ, hepatitis, nephritis, nephrosis, pancreatitis, rhinitis, arthritis, inflammatory ocular diseases, inflammatory bowel diseases, diseases in cerebro and/or circulatory system, respiratory diseases, dermatic diseases, autoimmune diseases, *etc.*], and the like.

EP 1 541 563 A1

## Description

TECHNICAL FIELD

The present invention relates to a compound having CCR4 antagonistic activity which is useful as a medicament, a method for producing the same, and use thereof.

BACKGROUND ART

Chemokine is known as a basic protein having endogenous leukocyte chemotactic and activating activities and strong heparin-binding abilities. At present, it is considered that chemokine is related to not only the control of infiltration of specific leukocyte at the time of inflammations and immune responses but also the development and homing of lymphocyte under physiological conditions and migration of hemocyte precursor cells and somatic cells.

Differentiation, proliferation and cell death of hemocytes are controlled by various types of cytokine. In the living body, inflammations are found locally and differentiation, maturation and the like of lymphocytes are carried out at certain specific sites. That is, various necessary cells migrate into certain specified sites and accumulate therein to cause a series of inflammations and immune responses. Accordingly, migration of cells is also an indispensable phenomenon to lead to the immune system in addition to differentiation, proliferation and death of cells.

Migration of hemocytes in the living body starts firstly in the development stage by the shift of hematopoiesis started in the AGM region into permanent hematopoiesis in bone marrow via fetal liver. Furthermore, precursor cells of T cells and thymus dendritic cells migrate from the fetal liver into the bone marrow and then into the thymus gland and cytodifferentiate under thymus environment. The T cell which is subjected to clone selection migrates into secondary lymphoid tissues and takes part in an immune response in the periphery. The Langerhans's cell of the skin activated and differentiated by capturing an antigen migrates into the T cell region of a topical lymph node and activates naive T cell therein as a dendritic cell. The memory T cell again performs its homing again into the lymph node via lymphatic and blood vessels. Also, B cell, T cell in the intestinal epithelium, γδ T cell, NKT cell and dendritic cell migrate from bone marrow without passing through the thymus gland and differentiate to take part in an immune response.

Chemokine is deeply related to the migration of these various cells. For example, CCR4 which is a receptor for MDC and TARC is expressed in Th2 cell (see *J. Immunol.*, 161, 5111 (1998)), and is known to play an important role in the migration of Th2 cell into topical sites where immune and inflammatory responses related to the Th2 cell is induced. In a mouse OVA-induced airway hypersensitivity model, an anti-MDC antibody suppressed the number of eosinophils accumulated in the lung interstitium, and suppressed airway hypersensitivity (see *J. Immunol.*, 163, 403 (1999)). In a mouse OVA-induced airway hypersensitivity model, anti-TARC antibody suppressed infiltration of eosinophils and lymphocytes into the airway as well as airway hypersensitivity (see *J. Immunol*, 166, 2055 (2001)). In the investigation with Nc/Nga mouse, it was recognized that amounts of TARC and MDC have increased in the atopic dermatitis-like lesion site (see *J. Clin. Invest.*, 104, 1097 (1999)). For CCR4 relation to human malignant neoplasm such as leukemia, cancer and cancer metastasis, *etc.*), and the like], infections [for example, viral diseases (e.g., acquired immunodeficiency syndrome, SARS, *etc.*), and the like], and the like.

On the other hand, it was described that a compound of formula (X):

$$J^X—M^X \quad (X)$$

wherein $J^X$ represents an aromatic moiety; and $M^X$ represents a moiety interacting with a G protein-coupled receptor. It was also described that as a more specific compound, a compound of formula (X-I):

$$A^X—L^{1X}—B^X—L^{2X}—E^X \quad (X\text{-}1)$$

wherein $A^X$ represents alkyl, aryl or heteroaryl which may be substituted, *etc.*; $L^{1X}$ represents O, S, CHOH, $O(CH_2)_{nX}$, *etc.;* nX represents 0, 1, 2 or 3; $B^X$ represents an 5- to 7-membered aromatic ring which may be substituted and may have 0 to 3 hetero atoms; $L^{2X}$ represents $CH_2C$=O, NHC=0, OC=O, *etc.;* and $E^X$ represents a moiety interacting with a G protein-coupled receptor, with the proviso that the definitions of each symbol are partially excerpted, binds to a G protein-coupled receptor (e.g., WO00/46203).

Furthermore, it was described that a compound of formula (Y):

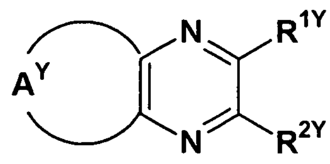

(Y)

wherein $A^Y$ represents

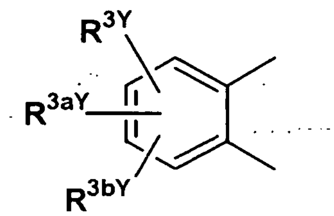

,

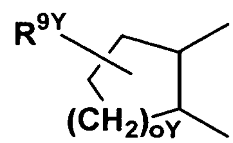

*etc.*; $R^{3Y}$, $R^{3aY}$ and $R^{3bY}$ each independently represents hydrogen, alkyl, *etc.;* oY represents 1 or 2; $R^{9Y}$ represents hydrogen or alkyl; $R^{1Y}$ represents alkoxy, halogen, pathologic conditions, the number of CCR4 positive memory-T lymphocyte in peripheral blood increased depending on severity of dermatitis (see *J. Allergy Clin. Immunol.*, 107, 353 (2001)), and the amount of TARC in the serum was also correlated to the severity in the atopic dermatitis patients (see *J. Allergy Clin. Immunol.*, 107, 535 (2001)). The amount of TARC in the serum and the induced sputum also increased in the asthma patients (see *Allergy*, 57, 173 (2002)). MDC concentration in the blood was high in Th2 diseases such as atopic dermatitis and Sezary syndrome (see *Eur. J. Immunol.*, 30, 201 (2000)).

There have been many reports suggesting correlation with other inflammatory diseases than allergic diseases, and CCR4 positive cell was accumulated selectively in the affected site of Lupus nephritis (see *Arthritis Rheum.,* 46, 735 (2002)). Expression of TARC and MDC was high in the affected site of Crohn's disease (see *Eur. Cytokine Netw.*, 12, 468 (2001)). CCR4 expression rose in the peripheral blood CD4 positive cells of systemic lupus erythematodes patients as compared with healthy persons (see *J. Leuko., Biol.*, 70, 749 (2001)).

Furthermore, it has been known that chemokine play various roles in immune responses in addition to the migration of various cells. In the investigation with CCR4 deficient mouse, it was recognized that lethality by high dose LPS shock reduced as compared with wild type, and further, amounts of TNF$\alpha$, IL-1$\beta$ and MIP-1$\alpha$ also reduced in the blood after administration of LPS. Furthermore, in a rat fulminant hepatitis model (P.acnes + LPS), an anti-TARC antibody suppressed increase of the amount of ALT in the blood and increase of the expression amounts of TNF$\alpha$ and FasL in the liver and also improved lethality of the rats (see *J. Clin. Invest.*, 102, 1933 (1998)). It was shown that CCR4 contributes to the binding of activated T cells and dendritic cells (see *J. Immunol.*, 167, 4791 (2001)). Furthermore, TARC and MDC caused platelet aggregation mediated by CCR4 (see *Thrombosis Research,* 101, 279 (2001)), which is one of various physiological activities of chemokines and chemokine receptors.

Based on the above, chemokines and chemokine receptors are greatly related to the control of inflammation and/or immune responses through a mechanism in which they are expressed at certain specified periods in variously specific cells and its effector cells are accumulated in a region where chemokine is produced.

As described above, CCR4 antagonists have TNF$\alpha$ regulatory activity and inhibitory activity for the functions of the effector cells in addition to CCR4 antagonistic activity. Therefore, it is considered to use CCR4 antagonist as a preventive and/or therapeutic agent for inflammatory and/or allergic diseases [for example, systemic inflammatory response syndrome (SIRS), anaphylaxis or anaphylactoid reaction, allergic vasculitis, transplant rejection reaction, hepatitis, nephritis, nephropathy, pancreatitis, rhinitis, arthritis, inflammatory ocular diseases (e.g., conjunctivitis, *etc.*), inflammatory bowel diseases (e.g., ulcerative colitis, Crohn's disease, eosinophilic gastroenteropathy, *etc.*), diseases in cerebro and/or circulatory system (e.g., arteriosclerosis, thrombosis, ischemic/reperfusion disorders, restenosis, infarction, *etc.*), respiratory diseases (e.g., acute respiratory distress syndrome (ARDS), asthma, allergic bronchopulmonary aspergillosis, *etc.*), dermatosis (e.g., dermatitis such as atopic dermatitis, psoriasis, contact dermatitis, eczema, urticaria and pruritus, and the like), autoimmune diseases (e.g., multiple sclerosis, chronic articular rheumatism, systemic lupus erythematodes, Type I diabetes mellitus, glomerular nephritis, Sjoegren's syndrome, *etc.*), and the like], metabolism and/or endocrine system diseases (e.g., diabetes mellitus, *etc.*), cancer diseases [for example,

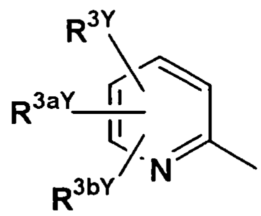 , 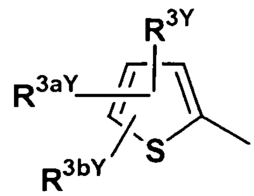

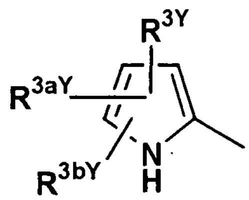 , 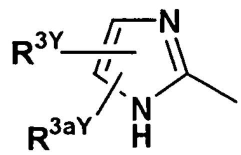

*etc.;* $R^{2Y}$ represents $CF_3$, $-NR^{10Y}R^{11Y}$, *etc.*; $R^{10Y}$ represents hydrogen, alkyl or aralkyl; $R^{11Y}$ represents

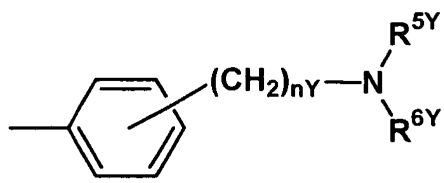

*etc.*; nY represents 0 or 1; $R^{5Y}$ and $R^{6Y}$ each independently represents hydrogen, alkyl, cycloalkyl, *etc.*, with the proviso that the definitions of each symbol are partially excerpted, is useful as an IL-8 receptor (CXCR1 and CXCR2) agonist (e.g., WO99/42463).

Furthermore, so far, several low molecular compounds have been reported to have CCR4 antagonistic activity (e.g., WO02/30357, WO02/30358 and WO02/94264).

However, until now, no pyrazine derivatives having CCR4 antagonistic activity have been reported.

DISCLOSURE OF THE INVENTION

A preventive and/or therapeutic agent for asthma, atopic dermatitis and the like which is useful as a medicament, and development of a compound having excellent oral absorption and safe CCR4 antagonistic activity is desired.

The present inventors have made extensive studies to find a compound having CCR4 antagonistic activity, and as a result, have found that the object is achieved by the compound of the present invention of formula (I), and then have completed the present invention.

Namely, the present invention relates to the followings:

1. A compound of formula (I):

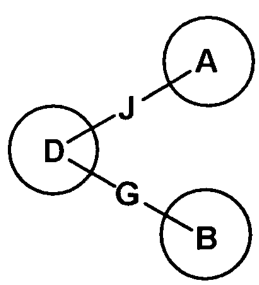

(I)

wherein ring A, ring B, and ring D each independently represents a cyclic group which may be substituted;
J represents a bond or a spacer having 1 to 8 atoms in its main chain; and
G represents a bond or a spacer having 1 to 4 atoms in its main chain;
or a salt thereof.
2. The compound according to the above 1, wherein

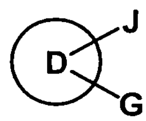

is

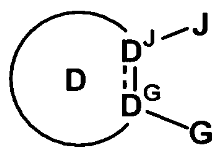

wherein $D^J$ and $D^G$ each independently represents a carbon atom or a nitrogen atom; and ---- represents a single bond or a double bond, and
when ---- represents a double bond, $D^J$ and $D^G$ each represents a carbon atom.
3. The compound according to the above 2, wherein ring D is a carbocyclic ring which may be substituted.
4. The compound according to the above 2, wherein ring D is a heterocyclic ring which may be substituted.
5. The compound according to the above 4, wherein the heterocyclic ring is a 3- to 15-membered monocyclic, bicyclic or tricyclic heterocyclic ring having 1 to 4 nitrogen atoms, 1 or 2 oxygen atoms and/or 1 or 2 sulfur atoms as a hetero atom(s).
6. The compound according to the above 2, wherein

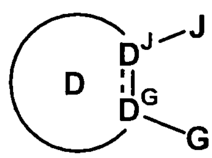

is

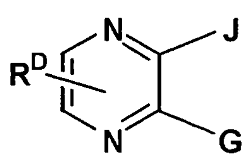

or

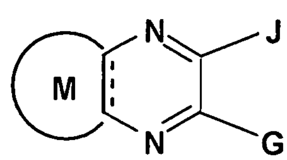

wherein $R^D$ represents a substituent of ring D; and M represents a 3- to 11-membered monocyclic or bicyclic cyclic group which may be substituted.
7. The compound according to the above 6, wherein

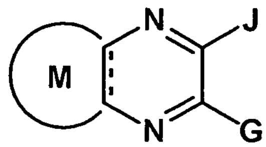

is

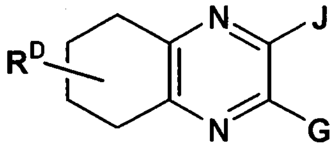, 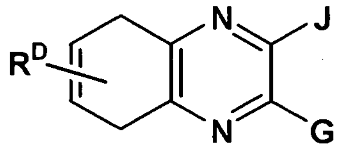, 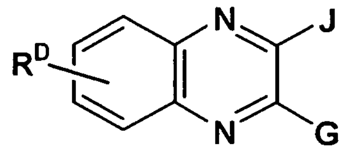、

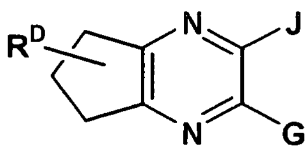, 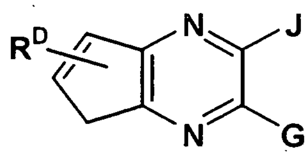 or 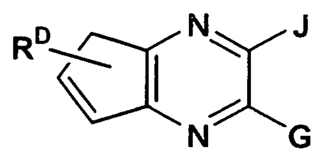

wherein $R^D$ has the same meaning as described in the above 6.

8. The compound according to the above 1, wherein ring A is a carbocyclic ring which may be substituted.

9. The compound according to the above 1, wherein ring A is a heterocyclic ring which may be substituted.

10. The compound according to the above 8, wherein the carbocyclic ring is a C3-15 monocyclic, bicyclic or tricyclic carbocyclic ring.

11. The compound according to the above 9, wherein the heterocyclic ring is a 3- to 15-membered monocyclic, bicyclic or tricyclic heterocyclic ring having 1 to 4 nitrogen atoms, 1 or 2 oxygen atoms and/or 1 or 2 sulfur atoms as a hetero atom(s).

12. The compound according to the above 10, wherein the carbocyclic ring is a benzene ring or a naphthalene ring.

13. The compound according to the above 11 wherein the heterocyclic ring is a pyridine ring, a pyrazole ring, a dioxaindane ring or a benzodioxane ring.

14. The compound according to the above 1, wherein ring B is a carbocyclic ring which may be substituted.

15. The compound according to the above 1, wherein ring B is a heterocyclic ring which may be substituted.

16. The compound according to the above 14, wherein the carbocyclic ring is a C3-15 monocyclic, bicyclic or tricyclic carbocyclic ring.

17. The compound according to the above 15, wherein the heterocyclic ring is a 3- to 15-membered monocyclic, bicyclic or tricyclic heterocyclic ring having 1 to 4 nitrogen atoms, 1 or 2 oxygen atoms and/or 1 or 2 sulfur atoms as a hetero atom(s).

18. The compound according to the above 16, wherein the carbocyclic ring is a C3-8 monocyclic carbocyclic ring.

19. The compound according to the above 17, wherein the heterocyclic ring is a 3- to 8-membered monocyclic heterocyclic ring having 1 to 4 nitrogen atoms, 1 or 2 oxygen atoms and/or 1 or 2 sulfur atoms as a hetero atom(s).

20. The compound according to the above 18, wherein the carbocyclic ring is a benzene ring.

21. The compound according to the above 19, wherein the heterocyclic ring is a pyridine ring or a thiophene ring.

22. The compound according to the above 1, wherein J is a spacer having 1 to 8 atoms in its main chain and containing at least one oxygen atom,

23. The compound according to the above 22, wherein the oxygen atom binds to ring D.

24. The compound according to the above 22, wherein J is

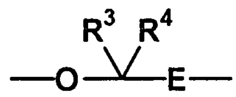

wherein $R^3$ and $R^4$ each independently represents hydrogen or C1-8 alkyl; and E represents a bond or a spacer having 1 to 6 atoms in its main chain.

25. The compound according to the above 24, wherein $R^3$ and $R^4$ each independently represents hydrogen or methyl.

26. The compound according to the above 24, wherein E is a bond,

27. The compound according to the above 24, wherein E is a spacer having 1 to 6 atoms in its main chain.

28. The compound according to the above 27, wherein E is C1-4 alkylene or C1-3 alkyleneoxy.

29. The compound according to the above 28, wherein E is methylene or methylenoxy.

30. The compound according to the above 1, wherein G is a spacer having 1 to 4 atoms in its main chain and containing at least one nitrogen atom.

31. The compound according to the above 30, wherein G is $-NR^{T1}-$, $-NR^{T1}-SO_2-$, $-NR^{T1}-CO-$, $-NR^{T1}-CO-NR^{T2}-$, $-NR^{T1}-SO_2-NR^{T2}-$, $-NR^{T1}-COO-$, $-NR^{T1}-O-$, $-NR^{T1}-NR^{T2}-$, $-NR^{T1}-W-$, $-SO_2-NR^{T1}-$, $-CO-NR^{T1}-$, $-OCO-NR^{T1}-$, $-O-NR^{T1}-$ or $W-NR^{T1}-$, wherein W represents a bivalent C1-3 aliphatic hydrocarbon group which may be substituted; $R^{T1}$ and $R^{T2}$ each independently represents hydrogen, C1-8 alkyl which may be substituted, C2-8 alkenyl which may be substituted, C2-8 alkynyl which may be substituted or a 3- to 8-membered cyclic group which may be substituted.

32. The compound according to the above 31, wherein G is $-NH-SO_2-$.

33. The compound according to the above 1, wherein the compound is a compound of formula (A):

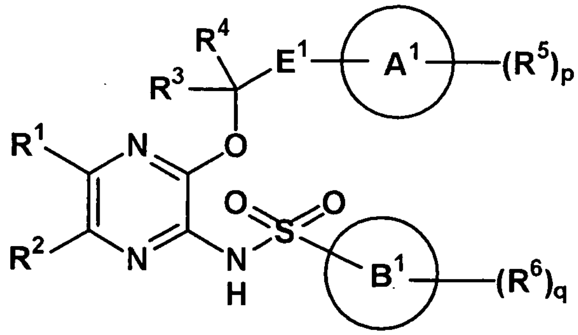

(A)

wherein $R^1$ and $R^2$ each independently represents (1) hydrogen, (2) C1-8 alkyl, (3) C2-8 alkenyl, (4) C2-8 alkynyl, (5) halogen, (6) cyano, (7) nitro, (8) $-CONR^7R^8$, (9) $-COOR^9$, (10) Cyc1 or (11) C1-8 alkyl substituted with 1 to 5 groups selected from (a) $-CONR^7R^8$, (b) $-COOR^9$, (c) $-OR^{10}$, (d) $-NR^{11}R^{12}$, (e) halogen, and (f) Cyc1; or

$R^1$ and $R^2$ are taken together to represent C3-4 alkylene, $-CH=CH-CH_2-$, $-CH_2-CH=CH-$, $-CH=CH-CH=CH-$ or $-CH=CH-CH_2-CH_2-$, wherein the carbocyclic ring to be formed may bc substituted with C1-8 alkyl, C2-8 alkenyl, C2-8 alkynyl, C1-8 alkoxy, halogen, cyano, nitro or hydroxyl, wherein $R^7$ and $R^8$ each independently represents (1) hydrogen, (2) C1-8 alkyl, (3) C2-8 alkenyl, (4) C2-8 alkynyl, (5) Cyc2, (6) $-OR^{13}$ or (7) C1-8 alkyl, C2-8 alkenyl or C2-8 alkynyl substituted with 1 to 5 groups selected from (a) $-OR^{13}$, (b) $-NR^{14}R^{15}$, (c) $-NR^{16}COR^{17}$, (d) halogen, (e) $CF_3$, and (f) Cyc2; or

$R^7$ and $R^8$ are taken together with the adjacent nitrogen atom to represent a 3- to 8-membered monocyclic heterocyclic ring having at least one nitrogen atom as a hetero atom(s) and 0 to 3 nitrogen atoms, 0 to 1 oxygen atom and/or 0 to 1 sulfur atom as an other hetero atom(s),

wherein the heterocyclic ring may be substituted with (a) C1-8 alkyl, (b) halogen, (c) hydroxyl, or (d) C1-8 alkyl substituted with hydroxyl;

$R^{13}$ to $R^{17}$ each independently represents (1) hydrogen, (2) C1-8 alkyl, (3) C2-8 alkenyl, (4) C2-8 alkynyl, (5) Cyc1, or (6) C1-8 alkyl, C2-8 alkenyl or C2-8 alkynyl substituted with Cyc1;

$R^9$ to $R^{12}$ each independently represents (1) hydrogen, (2) C1-8 alkyl, (3) C2-8 alkenyl, (4) C2-8 alkynyl, (5) Cyc1, or (6) C1-8 alkyl, C2-8 alkenyl or C2-8 alkynyl substituted with Cycl;

Cyc1 represents a C3-15 monocyclic, bicyclic or tricyclic carbocyclic ring or a 3- to 15-membered monocyclic,

bicyclic or tricyclic heterocyclic ring having 1 to 4 nitrogen atoms, 1 or 2 oxygen atoms and/or 1 or 2 sulfur atoms as a hetero atom(s), wherein Cyc1 may be substituted with 1 to 5 of $R^{18}$;

$R^{18}$ represents (1) C1-8 alkyl, (2) C2-8 alkenyl, (3) C2-8 alkynyl, (4) halogen, (5) cyano, (6) nitro, (7) trifluoromethyl, (8) trifluoromethoxy, (9) -$OR^{19}$, (10) -$SR^{20}$, (11) -$NR^{21}R^{22}$, (12) -$COR^{23}$, (13) -$COOR^{24}$, (14) -$NR^{25}COR^{26}$, (15) -$CONR^{27}R^{28}$, (16) Cyc2, or (17) C1-8 alkyl, C2-8 alkenyl or C2-8 alkynyl substituted with 1 to 5 groups selected from (a) halogen, (b) cyano, (c) nitro, (d) trifluoromethyl, (e) trifluoromethoxy, (f) -$OR^{19}$, (g) -$SR^{20}$, (h) -$NR^{21}R^{22}$, (i) -$COR^{23}$, (j) -$COOR^{24}$, (k) -$NR^{25}COR^{26}$, (1) -$CONR^{27}R^{28}$, and (m) Cyc2;

$R^{19}$ to $R^{28}$ each independently represents (1) hydrogen, (2) C1-8 alkyl, (3) C2-8 alkenyl, (4) C2-8 alkynyl, (5) Cyc2, or (6) C1-8 alkyl, C2-8 alkenyl or C2-8 alkynyl substituted with Cyc2;

Cyc2 represents a C3-8 monocyclic carbocyclic ring or a 3- to 8-membered monocyclic heterocyclic ring having 1 to 4 nitrogen atoms, 1 or 2 oxygen atoms and/or 1 or 2 sulfur atoms as a hetero atom(s), wherein Cyc2 may be substituted with 1 to 5 of $R^{29}$;

$R^{29}$ represents (1) C1-8 alkyl, (2) C2-8 alkenyl, (3) C2-8 alkynyl, (4) halogen, (5) cyano, (6) nitro, (7) hydroxyl, (8) trifluoromethyl, (9) trifluoromethoxy, or (10) -$OR^{100}$;

$R^{100}$ represents C1-8 alkyl.;

$R^3$ and $R^4$ each independently represents hydrogen or C 1-8 alkyl;

E' represents a bond or C1-6 alkylene, wherein a carbon atom in the alkylene group may be substituted with oxygen, sulfur, or -$NR^{30}$-;

$R^{30}$ represents (1) C1-8 alkyl, (2) C2-8 alkenyl, (3) C2-8 alkynyl, (4) phenyl, or (5) C1-8 alkyl substituted with phenyl;

ring $A^1$ represents a C3-15 monocyclic, bicyclic or tricyclic carbocyclic ring or a 3- to 15-membered monocyclic, bicyclic or tricyclic heterocyclic ring having 1 to 4 nitrogen atoms, 1 or 2 oxygen atoms and/or 1 or 2 sulfur atoms as a hetero atom(s);

$R^5$ represents (1) C1-8 alkyl, (2) C2-8 alkenyl, (3) C2-8 alkynyl, (4) halogen, (5) cyano, (6) nitro, (7) trifluoromethyl, (8) trifluoromethoxy, (9) -$OR^{31}$, (10) -$NR^{32}R^{33}$, (11) -$NR^{34}COR^{35}$, (12) Cyc3, or (13) C1-8 alkyl, C2-8 alkenyl or C2-8 alkynyl substituted with 1 to 5 groups selected from (a) halogen, (b) cyano, (c) nitro, (d) trifluoromethyl, (e) trifluoromethoxy, (f) -$OR^{31}$, (g) - $NR^{32}COR^{33}$, (h) -$NR^{34}COR^{35}$, and (i) Cyc3;

$R^{31}$ to $R^{35}$ each independently represents (1) hydrogen, (2) C1-8 alkyl, (3) C2-8 alkenyl, (4) C2-8 alkynyl, (5) Cyc3, or (6) C1-8 alkyl, C2-8 alkenyl or C2-8 alkynyl substituted with 1 to 5 groups selected from (a) Cyc3, (b) -$OR^{36}$ and (c) -$NR^{37}R^{38}$;

$R^{36}$ to $R^{38}$ each independently represents (1) hydrogen, (2) C1-8 alkyl, (3) -$OR^{39}$, or (4) -$NR^{40}R^{41}$;

$R^{39}$ to $R^{41}$ each independently represents hydrogen or C 1-8 alkyl;

Cyc3 represents a C3-8 monocyclic carbocyclic ring or a 3- to 8-membered monocyclic heterocyclic ring having 1 to 4 nitrogen atoms, 1 or 2 oxygen atoms and/or 1 or 2 sulfur atoms as a hetero atom(s);

ring $B^1$ represents a C3-15 monocyclic, bicyclic or tricyclic carbocyclic ring or a 3- to 15-membered monocyclic, bicyclic or tricyclic heterocyclic ring having 1 to 4 nitrogen atoms, 1 or 2 oxygen atoms and/or 1 or 2 sulfur atoms as a hetero atom(s);

$R^6$ represents (1) C1-8 alkyl, (2) C2-8 alkenyl, (3) C2-8alkynyl, (4) halogen, (5) cyano, (6) nitro, (7) trifluoromethyl, (8) trifluoromethoxy, (9) -$OR^{42}$, (10) -$NR^{43}R^{44}$, (11) -$SR^{101}$, (12) -$SO_2R^{102}$,
(13) -$COR^{103}$, (14) -$COOR^{104}$; (15) Cyc2, or (16) C1-8 alkyl, C2-8 alkenyl or C2-8 alkynyl substituted with 1 to 5 groups selected from (a) -$COOR^{104}$, (b) -$NR^{105}COR^{106}$, and (c) Cyc2;

$R^{42}$ to $R^{44}$ and $R^{101}$ to $R^{106}$ each independently represents (1) hydrogen, (2) C1-8 alkyl, (3) Cyc2,
or (4) -$COR^{107}$, or (5) C1-8 alkyl substituted with 1 to 5 halogen atoms;

$R^{107}$ represents C1-8 alkyl; and

p and q each independently represents 0 or an integer of 1 to 5.

34. A prodrug for the compound according to the above 1.

35. A pharmaceutical composition which comprises the compound of formula (I):

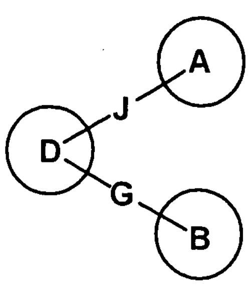

(I)

wherein ring A, ring B, and ring D each independently represents a cyclic group which may be substituted; J represents a bond or a spacer having 1 to 8 atoms in its main chain; and G represents a bond or a spacer having 1 to 4 atoms in its main chain;
or a salt thereof.
36. The pharmaceutical composition according to the above 35, which is a chemokine receptor antagonist.
37. The pharmaceutical composition according to the above 36, wherein the chemokine receptor is CCR4.
38. The pharmaceutical composition according to the above 37, which is a preventive and/or therapeutic agent for CCR4-mediated diseases.
39. The pharmaceutical composition according to the above 38, wherein the CCR4-mediated diseases are inflammatory and/or allergic diseases, metabolism and/or endocrine system diseases, cancer diseases or infections.
40. The pharmaceutical composition according to the above 39, wherein the CCR4-mediated diseases are inflammatory and/or allergic diseases.
41. The pharmaceutical composition according to the above 40, wherein the inflammatory and/or allergic diseases are respiratory diseases or dermatosis.
42. The pharmaceutical composition according to the above 41, wherein the respiratory diseases are asthma.
43. The pharmaceutical composition according to the above 41, wherein the dermatosis is atopic dermatitis.
44. A method for preventing and/or treating CCR4-mediated diseases in a mammal, which comprises administering to a mammal an effective amount of the compound according to the above 1 or a salt thereof.
45. Use of the compound according to the above 1 or a salt thereof for the manufacture of a preventive and/or therapeutic agent for CCR4-mediated diseases.
46. A pharmaceutical composition which comprises: a preventive and/or therapeutic agent for CCR4-mediated diseases, which comprises the compound according to the above 1 or a salt thereof as an active ingredient; and one or at least two medicaments selected from a bronchodilator drug, a steroid drug, a non-steroidal antiinflammatory drug, a leukotriene receptor antagonist, a phosphodiesterase inhibitor, an immunosuppressant, an antiallergic drug, a mediator-release inhibitor, an antihistamine drug, a metabolism promoter and/or a chemokine inhibitor.
47. The pharmaceutical composition according to the above 35, which is an inhibitor of effector cell function.
48. The pharmaceutical composition according to the above 47, which is an inhibitor of cell migration function.
49. The pharmaceutical composition according to the above 35, which is a TNF$\alpha$ regulator.

In the present specification, the "cyclic group" in the "cyclic group which may be substituted" represented by ring A, ring B and ring D includes, for example, a carbocyclic ring, a heterocyclic ring and the like.
The carbocyclic ring includes, for example, a "C3-15 monocyclic, bicyclic or tricyclic carbocyclic ring", and the like. The "C3-15 monocyclic, bicyclic or tricyclic carbocyclic ring" includes a C3-15 monocyclic, bicyclic or tricyclic unsaturated carbocyclic ring, or partially or completely saturated one thereof, a spiro-bound bicyclic carbocyclic ring and a crosslinked bicyclic carbocyclic ring.
The "C3-15 monocyclic, bicyclic or tricyclic unsaturated carbocyclic ring, or partially or completely saturated one thereof" includes, for example, cyclopropane, cyclobutane, cyclopentane, cyclohexane, cycloheptane, cyclooctane, cyclononane, cyclodecane, cycloundecane, cyclododecane, cyclotridecane, cyclotetradecane, cyclopentadecane, cyclopentene, cyclohexene, cycloheptene, cyclooctene, cyclopentadiene, cyclohexadiene, cycloheptadiene, cyclooctadiene, benzene, pentalene, perhydropentalene, azulene, perhydroazulene, indene, perhydroindene, indane, naphthalene, dihydronaphthalene, tetrahydronaphthalene, perhydronaphthalene, heptalene, perhydroheptalene, biphenylene, as-indacene, s-indeacene, acenaphthylene, acenaphthene, fluorene, phenalene, phenanthrene, anthracene rings, and the like. The "spiro-bound bicyclic carbocyclic ring" includes, for example, spiro[4.4]nonane, spiro[4.5]decane, spiro[5.5]undecane rings, and the like. The "crosslinked bicyclic carbocyclic ring" includes, for example, bicyclo[2.2.1]heptane, bicyclo[2.2.1]hepta-2-ene, bicyclo[3.1.1]heptane, bicyclo[3.1.1]hepta-2-ene, bicyclo[3.2.1]octane, bicyclo[2.2.2]octane, bicyclo[2.2.2]octa-2-ene, adamantane, noradamantane rings, and the like. Among these, a "C3-15 monocyclic, bicyclic or tricyclic aromatic carbocyclic ring" includes, for example, benzene, azulene, naphthalene, phenanthrene, anthracene rings, and the like.
The heterocyclic ring includes, for example, a "3- to 15-membered monocyclic, bicyclic or tricyclic heterocyclic ring having 1 to 4 nitrogen atoms, 1 or 2 oxygen atoms and/or 1 or 2 sulfur atoms as a hetero atom(s)", and the like. Herein, the "3- to 15-membered monocyclic, bicyclic or tricyclic heterocyclic ring having 1 to 4 nitrogen atoms, 1 or 2 oxygen atoms and/or 1 or 2 sulfur atoms as a hetero atom(s)" includes 3- to 15-membered monocyclic, bicyclic or tricyclic unsaturated heterocyclic ring having 1 to 4 nitrogen atoms, 1 or 2 oxygen atoms and/or 1 or 2 sulfur atoms as a hetero atom(s), or partially or completely saturated one thereof, a spiro-bound bicyclic heterocyclic ring and a crosslinked bicyclic heterocyclic ring.
The "3- to 15-membered monocyclic, bicyclic or tricyclic unsaturated heterocyclic ring having 1 to 4 nitrogen atoms, 1 or 2 oxygen atoms and/or 1 or 2 sulfur atoms as a hetero atom(s), or partially or completely saturated one

thereof" includes, for example, pyrrole, imidazole, triazole, tetrazole, pyrazole, pyridine, pyrazine, pyrimidine, pyridazine, azepine, diazepine, furan, pyran, oxepine, thiophene, thiopyran, thiepine, oxazole, isoxazole, thiazole, isothiazole, furazan, oxadiazole, oxazine, oxadiazine, oxazepine, oxadiazepine, thiadiazole, thiazine, thiadiazine, thiazepine, thiadiazepine, indole, isoindole, indolizine, benzofuran, isobenzofuran, benzothiophene, isobenzothiophene, dithianaphthalene, indazole, quinoline, isoquinoline, quinolizine, purine, phthalazine, pteridine, naphthyridine, quinoxaline, quinazoline, cinnoline, benzoxazole, benzothiazole, benzimidazole, chromene, benzoxepine, benzoxazepine, benzoxadiazepine, benzothiepine, benzothiazepine, benzothiadiazepine, benzoazepine, benzodiazepine, benzofurazan, benzothiadiazole, benzotriazole, carbazole, β-carboline, acridine, phenazine, dibenzofuran, xanthene, dibenzothiophene, phenothiazine, phenoxazine, phenoxathiin, thianthrene, phenanthridine, phenanthroline, perimidine, aziridine, azetidine, pyrroline, pyrrolidine, imidazoline, imidazolidine, triazoline, triazolidine, tetrazoline, tetrazolidine, pyrazoline, pyrazolidine, dihydropyridine, tetrahydropyridine, piperidine, dihydropyrazine, tetrahydropyrazine, piperazine, dihydropyrimidine, tetrahydropyrimidine, perhydropyrimidine, dihydropyridazine, tetrahydropyridazine, perhydropyridazine, dihydroazepine, tetrahydroazepine, perhydroazepine, dihydrodiazepine, tetrahydrodiazepine, perhydrodiazepine, oxirane, oxetane, dihydrofuran, tetrahydrofuran, dihydropyran, tetrahydropyran, dihydroxepine, tetrahydroxepine, perhydroxepine, thiirane, thietane, dihydrothiophene, tetrahydrothiophene, dihydrothiopyran, tetrahydrothiopyran, dihydrothiepin, tetrahydrothiepin, perhydrothiepin, dihydroxazole, tetrahydroxazole (oxazolidine), dihydroisoxazole, tetrahydroisoxazole (isoxazolidine), dihydrothiazole, tetrahydrothiazole (thiazolidine), dihydroisothiazole, tetrahydroisothiazole (isothiazolidine), dihydrofurazan, tetrahydrofurazan, dihydroxadiazole, tetrahydroxadiazole (oxadiazolidine), dihydroxazine, tetrahydroxazine, dihydroxadiazine, tetrahydroxadiazine, dihydroxazepine, tetrahydroxazepine, perhydroxazepine, dihydroxadiazepine, tetrahydroxadiazepine, perhydroxadiazepine, dihydrothiadiazole, tetrahydrothiadiazole (thiadiazolidine), dihydrothiazine, tetrahydrothiazine, dihydrothiadiazine, tetrahydrothiadiazine, dihydrothiazepine, tetrahydrothiazepine, perhydrothiazepine, dihydrothiadiazepine, tetrahydrothiadiazepine, perhydrothiadiazepine, morpholine, thiomorpholine, oxathiane, indoline, isoindoline, dihydrobenzofuran, perhydrobenzofuran, dihydroisobenzofuran, perhydroisobenzofuran, dihydrobenzothiophene, perhydrobenzothiophene, dihydroisobenzothiophene, perhydroisobenzothiophene, dihydroindazole, perhydroindazole, dihydroquinoline, tetrahydroquinoline, perhydroquinoline, dihydroisoquinoline, tetrahydroisoquinoline, perhydroisoquinoline, dihydrophthalazine, tetrahydrophthalazine, perhydrophthalazine, dihydronaphthyridine, tetrahydronaphthyridine, perhydronaphthyridine, dihydroquinoxaline, tetrahydroquinoxaline, perhydroquinoxaline, dihydroquinazoline, tetrahydroquinazoline, perhydroquinazoline, dihydrocinnoline, tetrahydrocinnoline, perhydrocinnoline, benzoxathiane, dihydrobenzoxazine, dihydrobenzothiazine, pyrazinomorpholine, dihydrobenzoxazole, perhydrobenzoxazole, dihydrobenzothiazole, perhydrobenzothiazole, dihydrobenzimidazole, perhydrobenzimidazole, dihydrobenzazepine, tetrahydrobenzazepine, dihydrobenzodiazepine, tetrahydrobenzodiazepine, benzodioxepane, dihydrobenzoxazepine, tetrahydrobenzoxazepine, dihydrocarbazole, tetrahydrocarbazole, perhydrocarbazole, dihydroacridine, tetrahydroacridine, perhydroacridine, dihydrodibenzofuran, dihydrodibenzothiophene, tetrahydrodibenzofuran, tetrahydrodibenzothiophene, perhydrodibenzofuran, perhydrodibenzothiophene, dioxolane, dioxane, dithiolane, dithiane, dioxaindane, benzodioxane, chroman, benzodithiolane, benzodithiane, 6,7-dihydro-5H-cyclopenta[b]pyrazine, 5H-cyclopenta[b]pyrazine, imidazo[2,1-b][1,3]thiazole rings, and the like. The "spiro-bound bicyclic heterocyclic ring" includes, for example, azaspiro[4.4]nonane, oxazaspiro[4.4]nonane, dioxaspiro[4.4]nonane, azaspiro[4.5]decane, thiaspiro[4.5]decane, dithiaspiro[4.5]decane, dioxaspiro[4.5]decane, oxazaspiro[4.5]decane, azaspiro[5.5]undecane, oxaspiro[5.5]undecane, dioxaspiro[5.5]undecane rings, and the like. The "crosslinked bicyclic heterocyclic ring" includes, for example, azabicyclo[2.2.1]heptane, oxabicyclo[2.2.1]heptane, azabicyclo[3.1.1]heptane, azabicyclo[3.2.1]octane, oxabicyclo[3.2.1]octane, azabicyclo[2.2.2]octane, diazabicyclo[2.2.2]octane rings, and the like.

Among these, the "3- to 15-membered monocyclic, bicyclic or tricyclic aromatic heterocyclic ring having 1 to 4 nitrogen atoms, 1 or 2 oxygen atoms and/or I or 2 sulfur atoms as a hetero atom(s)" includes, for example, pyrrole, imidazole, triazole, tetrazole, pyrazole, pyridine, pyrazine, pyrimidine, pyridazine, furan, thiophene, oxazole, isoxazole, thiazole, isothiazole, furazan, oxadiazole, thiadiazole, indole, isoindole, benzofuran, isobenzofuran, benzothiophene, isobenzothiophene, indazole, quinoline, isoquinoline, purine, phthalazine, pteridine, naphthyridine, quinoxaline, quinazoline, cinnoline, benzoxazole, benzothiazole, benzimidazole, benzofurazan, benzothiadiazole, benzotriazole, carbazole, β-carboline, acridine, phenazine, dibenzofuran, dibenzothiophene, phenanthridine, phenanthroline, perimidine rings, and the like.

In the present specification, the "substituent" in the "cyclic group which may be substituted" represented by ring A and ring B is not particularly limited, so long as it is a substituent. The "substituent" includes, for example, substituents as exemplified below.

The "substituent" in the above-described "cyclic group which may be substituted" includes, for example, (1) a substituent selected from the following Group I, (2) a substituent selected from the following Group II, (3) 3- to 15-membered cyclic group which may be substituted, (4) carbamoyl which may be substituted, (5) an aliphatic hydrocarbon group which may be substituted, and the like. The substituents may be substituted in the number of 1 to 10, preferably 1 to 5, more preferably 1 to 3, at a substitutable position.

<Group I>

(1) halogen (chlorine, bromine, fluorine, and iodine), (2) cyano, (3) nitro, (4) trifluoromethyl, (5) trifluoromethoxy, (6) oxo and (7) thioxo.

<Group II>

(1) -$OR^{a1}$, (2) -$NR^{a1}R^{a2}$, (3) -$NR^{a1}COR^{a2}$, (4) -$COOR^{a1}$, (5) -$SR^{a1}$, (6) -$SOR^{a1}$, (7) -$SO_2R^{a1}$ and (8) -$COR^{a1}$, wherein $R^{a1}$ and $R^{a2}$ each independently represents (a) hydrogen, (b) an aliphatic hydrocarbon group which may be substituted, or (c) a 3- or 15-membered cyclic group which may be substituted. If plural substituents are selected, plural $R^{a1}$ or plural $R^{a2}$ are the same or different.

Herein, the "aliphatic hydrocarbon group" in the "aliphatic hydrocarbon group which may be substituted" represented by $R^{a1}$ and $R^{a2}$ includes, for example, a "straight or branched aliphatic hydrocarbon group", and the like. The "straight or branched aliphatic hydrocarbon group" includes, for example, a "C1-8 aliphatic hydrocarbon group", and the like. The "C1-8 aliphatic hydrocarbon group" includes, for example, C1-8 alkyl, C2-8 alkenyl, C2-8 alkynyl, and the like.

The C1-8alkyl includes, for example, methyl, ethyl, propyl, isopropyl, butyl, *sec*-butyl, *tert*-butyl, pentyl, hexyl, heptyl, octyl, isomers thereof, and the like.

The C2-8 alkenyl includes, for example, vinyl, propenyl, butenyl, pentenyl, hexenyl, heptenyl, octenyl, butadienyl, pentadienyl, hexadienyl, heptadienyl, octadienyl, hexatrienyl, heptatrienyl, octatrienyl, isomers thereof, and the like.

The C2-8 alkynyl includes, for example, ethynyl, propynyl, butynyl, pentynyl, hexynyl, heptynyl, octynyl, butadiynyl, pentadiynyl, hexadiynyl, heptadiynyl, octadiynyl, hexatriynyl, heptatriynyl, octatriynyl, isomers thereof, and the like.

The "substitutent" in the "aliphatic hydrocarbon group which may be substituted" represented by $R^{a1}$ and $R^{a2}$ includes, for example, (1) a substituent selected from the above-described Group I, (2) a substituent selected from the following Group III, (3) a 3- to 15-membered cyclic group which may be substituted, and the like. The substituents may be substituted in the number of 1 to 8, preferably 1 to 5, at a substitutable position.

<Group III>

(1) -$OR^{b1}$ and (2) -$NR^{b1}R^{b2}$,
wherein $R^{b1}$ and $R^{b2}$ each independently represents (a) hydrogen, (b) hydroxyl, (c) cyano, (d) C1-8 alkyl (which has the same meaning as described in the above), (e) C1-8 alkoxy (e.g., methoxy, ethoxy, propoxy, butoxy, pentyloxy, hexyloxy, heptyloxy, octyloxy, isomers thereof, *etc.),* (f) monosubstituted or disubstituted C1-8 alkylamino (e.g., methylamino, ethylamino, propylamino, dimethylamino, diethylamino, *etc.),* (g) C1-8 alkyl substituted with the "monosubstituted or disubstituted C1-8 alkylamino" (the C1-8 alkyl has the same meaning as described in the above), and (h) C1-8 alkoxy substituted with the "monosubstituted or disubstituted C1-8 alkylamino " (the C1-8 alkoxy has the same meaning as described in the above). If plural substituents are selected, plural $R^{b1}$ or plural $R^{b2}$ are the same or different.

In the present specification, the "3- or 15-membered cyclic group" in the "3- or 15-membered cyclic group which may be substituted" includes, for example, the above-described "C3-15 monocyclic, bicyclic or tricyclic carbocyclic ring", the above-described "3- to 15-membered monocyclic, bicyclic or tricyclic heterocyclic ring having 1 to 4 nitrogen atoms, 1 or 2 oxygen atoms and/or 1 or 2 sulfur atoms as a hetero atom(s)", and the like.

The "substituent" in the "3- or 15-membered cyclic group which may be substituted" includes, for example, (1) a substituent selected from the above-described Group I, (2) an aliphatic hydrocarbon group which may be substituted, (3) a substituent selected from the following Group IV, (4) a 3- to 8-membered cyclic group which may be substituted, and the like. The substituents may be substituted in the number of 1 to 10, preferably 1 to 5, more preferably 1 to 3, at a substitutable position. The "aliphatic hydrocarbon group" in the "aliphatic hydrocarbon group which may be substituted" as used herein includes, for example, the above-described "C1-8 aliphatic hydrocarbon group", and the like. The "substitutent" in the "aliphatic hydrocarbon group which may be substituted" includes, for example, (1) a substituent selected from the above-described Group I, (2) a substituent selected from the following Group IV; (3) 3- to 8-membered cyclic group which may be substituted, and the like. The substituents may be substituted in the number of 1 to 8, preferably 1 to 5, at a substitutable position.

<Group IV>

(1) -$OR^{c1}$, (2) -$SR^{c1}$, (3) -$NR^{c1}R^{c2}$, (4) -$COR^{c1}$, (5) -$COOR^{c1}$, (6) -$NR^{c1}COR^{c2}$, (7) -$CONR^{c1}R^{c2}$, (8) -$SOR^{c1}$ and (9) a -$SO_2R^{c1}$,

wherein $R^{c1}$ and $R^{c2}$ each independently represents (a) hydrogen, (b) a 3- to 8-membered cyclic group which may be substituted, or (c) an aliphatic hydrocarbon group which may be substituted with a 3- to 8-membered cyclic group which may be substituted. If plural substituents are selected, plural $R^{c1}$ or plural $R^{c2}$ are the same or different.

The "aliphatic hydrocarbon group" in the "aliphatic hydrocarbon group which may be substituted with a 3- to 8-membered cyclic group which may be substituted" represented by $R^{c1}$ or $R^{c2}$ as used herein includes, for example, the above-described "C1-8 aliphatic hydrocarbon group", and the like.

In the present specification, "3- to 8-membered cyclic group" in the "3- to 8-membered cyclic group which may be substituted" includes, for example, a "C3-8 monocyclic carbocyclic ring", a "3- to 8-membered monocyclic heterocyclic ring", and the like. The "C3-8 monocyclic carbocyclic ring" as used herein include a C3-8 monocyclic unsaturated carbocyclic ring, or partially or completely saturated one thereof.

The "C3-8 monocyclic unsaturated carbocyclic ring, or partially or completely saturated one thereof" includes, for example, cyclopropane, cyclobutane, cyclopentane, cyclohexane, cycloheptane, cyclooctane, cyclopentene, cyclohexene, cycloheptene, cyclooctene, cyclopentadiene, cyclohexadiene, cycloheptadiene, cyclooctadiene, benzene rings, and the like. Among these, "C3-8 monocyclic aromatic carbocyclic ring" includes, for example, a benzene ring, and the like.

The "3- to 8-membered monocyclic heterocyclic ring" includes, for example, a "3-to 8-membered monocyclic heterocyclic ring having 1 to 4 nitrogen atoms, 1 or 2 oxygen atoms and/or 1 or 2 sulfur atoms as a hetero atom(s)", and the like. The "3- to 8-membered monocyclic heterocyclic ring having 1 to 4 nitrogen atoms, 1 or 2 oxygen atoms and/or 1 or 2 sulfur atoms as a hetero atom(s)" as used herein includes, for example, a 3- to 8-membered monocyclic unsaturated heterocyclic ring having 1 to 4 nitrogen atoms, 1 or 2 oxygen atoms and/or 1 or 2 sulfur atoms as a hetero atom(s), or partially or completely saturated one thereof.

The "3- to 8-membered monocyclic unsaturated heterocyclic ring having 1 to 4 nitrogen atoms, 1 or 2 oxygen atoms and/or 1 or 2 sulfur atoms as a hetero atom(s), or partially or completely saturated one thereof" includes, for example, pyrrole, imidazole, triazole, tetrazole, pyrazole, pyridine, pyrazine, pyrimidine, pyridazine, azepine, diazepine, furan, pyran, oxepine, thiophene, thiopyran, thiepine, oxazole, isoxazole, thiazole, isothiazole, furazan, oxadiazole, oxazine, oxadiazine, oxazepine, oxadiazepine, thiadiazole, thiazine, thiadiazine, thiazepine, thiadiazepine, aziridine, azetidine, pyrroline, pyrrolidine, imidazoline, imidazolidine, triazoline, triazolidine, tetrazoline, tetrazolidine, pyrazoline, pyrazolidine, dihydropyridine, tetrahydropyridine, piperidine, dihydropyrazine, tetrahydropyrazine, piperazine, dihydropyrimidine, tetrahydropyrimidine, perhydropyrimidine, dihydropyridazine, tetrahydropyridazine, perhydropyridazine, dihydroazepine, tetrahydroazepine, perhydroazepine, dihydrodiazepine, tetrahydrodiazepine, perhydrodiazepine, oxirane, oxetane, dihydrofuran, tetrahydrofuran, dihydropyran, tetrahydropyran, dihydroxepine, tetrahydroxepine, perhydroxepine, thiirane, thietane, dihydrothiophene, tetrahydrothiophene, dihydrothiopyran, tetrahydrothiopyran, dihydrothiepin, tetrahydrothiepin, perhydrothiepin, dihydroxazole, tetrahydroxazole (oxazolidine), dihydroisoxazole, tetrahydroisoxazole (isoxazolidine), dihydrothiazole, tetrahydrothiazole (thiazolidine), dihydroisothiazole, tetrahydroisothiazole (isothiazolidine), dihydrofurazan, tetrahydrofurazan, dihydroxadiazole, tetrahydroxadiazole (oxadiazolidine), dihydroxazine, tetrahydroxazine, dihydroxadiazine, tetrahydroxadiazine, dihydroxazepine, tetrahydroxazepine, perhydroxazepine, dihydroxadiazepine, tetrahydroxadiazepine, perhydroxadiazepine, dihydrothiadiazole, tetrahydrothiadiazole (thiadiazolidine), dihydrothiazine, tetrahydrothiazine, dihydrothiadiazine, tetrahydrothiadiazine, dihydrothiazepine, tetrahydrothiazepine, perhydrothiazepine, dihydrothiadiazepine, tetrahydrothiadiazepine, perhydrothiadiazepine, morpholine, thiomorpholine, oxathiane, dioxolane, dioxane, dithiolane, dithiane rings, and the like. Among these, the "3- to 8-membered monocyclic aromatic heterocyclic ring having 1 to 4 nitrogen atoms, 1 or 2 oxygen atoms and/or 1 or 2 sulfur atoms as a hetero atom(s)" includes, for example, pyrrole, imidazole, triazole, tetrazole, pyrazole, pyridine, pyrazine, pyrimidine, pyridazine, furan, thiophene, oxazole, isoxazole, thiazole, isothiazole, furazan, oxadiazole, thiadiazole rings, and the like.

The "substituent" in the "3- to 8-membered cyclic group which may be substituted" includes, for example, (1) a substituent selected from the above-described Group I, (2) C1-8 alkyl (which has the same meaning as described in the above), (3) C2-8 alkenyl (which has the same meaning as described in the above), (4) C2-8 alkynyl (which has the same meaning as described in the above), (5) hydroxyl, and the like. The substituents may be substituted in the number of 1 to 8, preferably 1 to 5, at a substitutable position.

The "carbamoyl which may be substituted" as the "substituent" in the "cyclic group which may be substituted" represented by ring A and ring B includes, for example, *N*-monosubstituted carbamoyl and *N*,*N*-disubstituted carbamoyl, in addition to unsubstituted carbamoyl. The "*N*-monosubstituted carbamoyl" means carbamoyl having one substituent on the nitrogen atom, and the "*N*,*N*-disubstituted carbamoyl " means carbamoyl having two substituents on the nitrogen atom. The substituent of the carbamoyl includes, for example, (1) the above-described "3- to 15-membered cyclic group which may be substituted", (2) an aliphatic hydrocarbon group which may be substituted, (3) an optionally protected hydroxyl, and the like. These substituents may be substituted in the number of 1 to 2 at a substitutable position. The substituent of the carbamoyl in the "*N*,*N*-disubstituted carbamoyl", together with the nitrogen atom to which they are bound, form (4) 3- to 8-membered nitrogen-containing heterocyclic ring which may be substituted.

The "optionally protected hydroxyl" as used herein includes, for example, protected hydroxyl, in addition to hydroxyl. The "protective group" in the "protected hydroxyl" includes, for example, (1) C1-8 alkyl which may be substituted (wherein the C1-8 alkyl has the same meaning as described in the above), (2) C2-8 alkenyl which may be substituted (wherein the C2-8 alkenyl has the same meaning as described in the above), (3) C2-8 alkynyl which may be substituted (wherein the C2-8 alkynyl has the same meaning as described in the above), (4) the above-described "3- to 15-membered cyclic group which may be substituted", and the like. The "substituent" in the "C1-8 alkyl which may be substituted", the "C2-8 alkenyl which maybe substituted " and the "C2-8 alkynyl which may be substituted" as used herein includes, for example (1) the above-described "3- to 15-membered cyclic group which may be substituted", (2) a substituent selected from the above-described Group I, and the like. The substituents may be substituted in the number of 1 to 8, preferably 1 to 5, at a substitutable position.

The "aliphatic hydrocarbon group" in the "aliphatic hydrocarbon group which may be substituted" as the substituent of the "*N*-monosubstituted carbamoyl" and the "*N*,*N*-disubstituted carbamoyl" includes, for example, the above-described "C1-8 aliphatic hydrocarbon group", and the like. The "substituent" in the "aliphatic hydrocarbon group which may be substituted" as used herein includes, for example, (1) a substituent selected from the above-described Group I, (2) the above-described " 3- to 15-membered cyclic group which may be substituted", (3) a substituent selected from the above-described Group II, and the like. The substituents may be substituted in the number of 1 to 8, preferably 1 to 5, at a substitutable position.

In the present specification, the "3- to 8-membered nitrogen-containing heterocyclic ring" in the "3- to 8-membered nitrogen-containing heterocyclic ring which may be substituted" includes, for example, the "3- to 8-membered monocyclic heterocyclic ring having at least one nitrogen atom as a hetero atom(s) and 0 to 3 nitrogen atoms, 0 to 1 oxygen atom and/or 0 to 1 sulfur atom as an other hetero atom(s)", and the like. The "3- to 8-membered monocyclic heterocyclic ring having at least one nitrogen atom as a hetero atom(s) and 0 to 3 nitrogen atoms, 0 to 1 oxygen atom and/or 0 to 1 sulfur atom as an other hetero atom(s)" as used herein includes, for example 3- to 8-membered monocyclic unsaturated heterocyclic ring having at least one nitrogen atom as a hetero atom(s) and 0 to 3 nitrogen atoms, 0 to 1 oxygen atom and/or 0 to 1 sulfur atom as an other hetero atom(s), or partially or completely saturated one thereof. The "3- to 8-membered monocyclic unsaturated heterocyclic ring having at least one nitrogen atom as a hetero atom(s) and 0 to 3 nitrogen atoms, 0 to 1 oxygen atom and/or 0 to 1 sulfur atom as an other hetero atom(s), or partially or completely saturated one thereof" includes, for example, pyrrole, imidazole, triazole, tetrazole, pyrazole, aziridine, azetidine, pyrroline, pyrrolidine, imidazoline, imidazolidine, triazoline, triazolidine, tetrazoline, tetrazolidine, pyrazoline, pyrazolidine, dihydropyridine, tetrahydropyridine, piperidine, dihydropyrazine, tetrahydropyrazine, piperazine, dihydropyrimidine, tetrahydropyrimidine, perhydropyrimidine, dihydropyridazine, tetrahydropyridazine, perhydropyridazine, dihydroazepine, tetrahydroazepine, perhydroazepine, dihydrodiazepine, tetrahydrodiazepine, perhydrodiazepine, dihydroxazole, tetrahydroxazole (oxazolidine), dihydroisoxazole, tetrahydroisoxazole (isoxazolidine), dihydrothiazole, tetrahydrothiazole (thiazolidine), dihydroisothiazole, tetrahydroisothiazole (isothiazolidine), dihydrofurazan, tetrahydrofurazan, dihydroxadiazole, tetrahydroxadiazole (oxadiazolidine), dihydroxazine, tetrahydroxazine, dihydroxadiazine, tetrahydroxadiazine, dihydroxazepine, tetrahydroxazepine, perhydroxazepine, dihydroxadiazepine, tetrahydroxadiazepine, perhydroxadiazepine, dihydrothiadiazole, tetrahydrothiadiazole (thiadiazolidine), dihydrothiazine, tetrahydrothiazine, dihydrothiadiazine, tetrahydrothiadiazine, dihydrothiazepine, tetrahydrothiazepine, perhydrothiazepine, dihydrothiadiazepine, tetrahydrothiadiazepine, perhydrothiadiazepine, morpholine, thiomorpholine rings, and the like. Among these, the "3- to 8-membered monocyclic aromatic heterocyclic ring having at least one nitrogen atom as a hetero atom(s) and 0 to 3 nitrogen atoms, 0 to 1 oxygen atom and/or 0 to 1 sulfur atom as an other hetero atom(s)" includes, for example, pyrrole, imidazole, triazole, tetrazole, pyrazole, pyridine, pyrazine, pyrimidine, pyridazine, furan, thiophene, oxazole, isoxazole, thiazole, isothiazole, furazan, oxadiazole, thiadiazole rings, and the like.

The "substituent" in the "3- to 8-membered nitrogen-containing heterocyclic ring which may be substituted" includes, for example, (1) a substituent selected from the above-described Group I, (2) hydroxyl, (3) C 1-8 alkyl (wherein the C 1-8 alkyl has the same meaning as described in the above) which may be substituted with 1 to 8 hydroxyl groups, and the like. The substituents may be substituted in the number of 1 to 8, preferably 1 to 5, at a substitutable position.

The "aliphatic hydrocarbon group" in the "aliphatic hydrocarbon group which may be substituted" as the "substituent" in the "cyclic group which may be substituted" represented by ring A and ring B includes, for example, the above-described "C1-8 aliphatic hydrocarbon group", and the like. The "substituent" in the "aliphatic hydrocarbon group which may be substituted" as used herein includes, for example, (1) a substituent selected from the above-described Group I, (2) the above-described "3- to 15-membered cyclic group which may be substituted", (3) the above-described "carbamoyl which may be substituted", (4) a substituent selected from the above-described Group II, and the like. The substituents may be substituted in the number of 1 to 8, preferably 1 to 5, at a substitutable position.

The "substituent" in the "cyclic group which may be substituted" represented by ring D is not particularly limited, so long as it is a substituent. The substituent includes, for example, the substituents represented by ring $R^D$, and the like.

The "substituent of ring D" represented by ring $R^D$ includes, for example, the above-described substituents

exemplified as the "substituent" in "cyclic ring which may be substituted" represented by A and B, and the like. The substituents may be substituted in the number of 1 to 10, preferably 1 to 5, more preferably 1 to 3, at a substitutable position.

The "spacer having 1 to 4 atoms in its main chain" represented by G means a space in which 1 to 4 atoms exist successively in its main chain. The "atomic number in its main chain" is counted such that the atoms of the main chain are minimized. For example, the atomic number in 1,2-cyclopentylene is counted as 2, and in 1,3-cyclopentylene as 3.

The "spacer having 1 to 4 atoms in its main chain" includes, for example, a bivalent group having 1 to 4 successive atoms in its main chain and consisting of 1 to 4 groups selected from -O-, -S-, -CO-, -SO-, $-SO_2-$, a nitrogen atom which may be substituted, a bivalent C1-4 aliphatic hydrocarbon group which may be substituted, a bivalent C3-8 monocyclic carbocyclic group which may be substituted, and a bivalent 3- to 8-membered monocyclic heterocyclic group which may be substituted, and the like. The "nitrogen atom which may be substituted" as used herein represents -NH-, and further a group in which the hydrogen atom in the "-NH-" is substituted with (1) C1-8 alkyl which may be substituted (the C1-8 alkyl has the same meaning as described in the above), (2) C2-8 alkenyl which may be substituted (the C2-8 alkenyl has the same meaning as described in the above), (3) C2-8 alkynyl which may be substituted (the C2-8 alkynyl has the same meaning as described in the above), (4) the above-described "3- to 8-membered cyclic group which may be substituted", or the like. The "substituent" in the "C1-8 alkyl which may be substituted", the "C2-8 alkenyl which may be substituted" and the "C2-8 alkynyl which may be substituted" as the "substituent" of the "nitrogen which may be substituted" as use herein includes, for example, (a) hydroxyl, (b) the above-described "3- to 8-membered cyclic ring which may be substituted", and the like. The substituents may be substituted in the number of 1 to 8, preferably 1 to 5, at a substitutable position.

The "bivalent C1-4 aliphatic hydrocarbon group" in the "bivalent C1-4 aliphatic hydrocarbon group which may be substituted" includes, for example, C1-4 alkylene (e.g., $-CH_2-$, $-(CH_2)_2-$, $-(CH_2)_3-$, $-(CH_2)_4-$, *etc.*), $C_{2-4}$ alkenylene (e.g., -CH=CH-, $-CH_2-CH=CH-$, $-CH=CH-CH_2-$, $-(CH_2)_2-CH=CH-$, $-CH=CH-(CH_2)_2-$, $-CH_2-CH=CH-CH_2-$, *etc.*), $C_{2-4}$ alkynylene (e.g., $-C\equiv C-$, $-CH_2-C\equiv C-$, $-C\equiv C-CH_2-$, $-(CH_2)_2-C\equiv C-$, $-C\equiv C-(CH_2)_2-$, $-CH_2-C\equiv C-CH_2-$, *etc.*), and the like. The "substituent" in the "a bivalent C1-4 aliphatic hydrocarbon group which may be substituted" includes, for example, (1) C1-8 alkyl (which has the same meaning as described in the above), (2) C1-8 alkoxy (which has the same meaning as described in the above), (3) halogen (which has the same meaning as described in the above), (4) hydroxyl, (5) oxo, (6) thioxo, (7) cyano, (8) $=N-OR^n$, wherein $R^n$ represents hydrogen or has the same meaning as the "substituent" in the "nitrogen which may be substituted", and the like. The substituents may be substituted in the number of 1 to 5, preferably 1 to 2, at a substitutable position.

In addition, the "bivalent C3-8 monocyclic carbocyclic group" in the "bivalent C3-8 monocyclic carbocyclic group which may be substituted" includes, for example, a bivalent group made by removing any two hydrogen atoms from the rings exemplified as the "C3-8 monocyclic carbocyclic ring", and the like. The "substituent" in the "bivalent C3-8 monocyclic carbocyclic ring which may be substituted" includes, for example, those exemplified as the "substituent" in the above-described "3- to 8-membered cyclic group which may be substituted", and the like. The substituents may be substituted in the number of 1 to 8, preferably 1 to 5, at a substitutable position.

The "bivalent 3- to 8-membered monocyclic heterocyclic group" in the "bivalent 3-to 8-membered monocyclic heterocyclic group which may be substituted" includes, for example, a bivalent group made by removing any two hydrogen atoms from the rings exemplified as the above-described "3- to 8-membered monocyclic heterocyclic ring", and the like. The "substituent" in the "bivalent 3- to 8-membered monocyclic heterocyclic group which may be substituted" includes, for example, those exemplified as the substituent in the above-described "3- to 8-membered cyclic group which may be substituted", and the like. The substituents may be substituted in the number of 1 to 8, preferably 1 to 5, at a substitutable position.

The "spacer having 1 to 4 atoms in its main chain and containing at least one nitrogen atom" represents a bivalent group containing at least one the above-described "nitrogen atom which may be substituted" among the groups, in the "spacer having 1 to 4 atoms in its main chain". If the "nitrogen atom which may be substituted" is contained in two or more, the subsitutents of each nitrogen atom are the same or different. The "spacer having 1 to 4 atoms in its main chain and containing at least one nitrogen atom" preferably includes, for example, $-NR^{T1}-$, $-NR^{T1}-SO_2-$, $-NR^{T1}-CO-$, $-NR^{T1}-CO-NR^{T2}-$, $-NR^{T1}-SO_2-NR^{T2}-$, $-NR^{T1}-COO-$, $-NR^{T1}-O-$, $-NR^{T1}-NR^{T2}-$, $-NR^{T1}-W-$, $- SO_2-NR^{T1}-$, $-CO-NR^{T1}-$, $-OCO-NR^{T1}-$, $-O-NR^{T1}-$, $-W-NR^{T1}-$, wherein W represents the "bivalent C1-3 aliphatic hydrocarbon group which may be substituted", and $R^{T1}$ and $R^{T2}$ each independently represents hydrogen or has the same meaning as the substituents in the above-described "nitrogen atom which may be substituted", and the like. The "bivalent C1-3 aliphatic hydrocarbon group" in the "bivalent C1-3 aliphatic hydrocarbon group which may be substituted" represented by W as used herein represents C1-3 alkylene (e.g., $-CH_2-$, $-(CH_2)_2-$, $-(CH_2)_3-$, *etc.*), $C_{2-3}$ alkenylene (e.g., -CH=CH-, $-CH_2-CH=CH-$, $-CH=CH-CH_2-$, *etc.*) and $C_{2-3}$ alkynylene (e.g., $-C\equiv C-$, $-CH_2-C\equiv C-$, $-C\equiv C-CH_2-$, *etc.*), among the groups exemplified as the above-described "bivalent C1-4 aliphatic hydrocarbon group which may be substituted". Furthermore, the "substituent" in the "bivalent C1-3 aliphatic hydrocarbon group which may be substituted" has the

same meaning as the substituent in the above-described "bivalent C1-4 aliphatic hydrocarbon group which may be substituted".

The "spacer having 1 to 8 atoms in its main chain" represented by J means a spacer in which 1 to 8 atoms exist successively in its main chain. The "atomic number in its main chain" as used herein is counted such that the atoms of the main chain are minimized as in the "spacer having 1 to 4 atoms in its main chain". For example, the atomic number in 1,4-phenylene is counted as 4, and in 1,3-phenylene as 3.

The "spacer having 1 to 8 atoms in its main chain" includes, for example, a bivalent group having 1 to 8 successive atoms in its main chain and containing 1 to 8 groups selected from -O-, -S-, -CO-, -SO-, $-SO_2-$, the above-described "nitrogen atom which may be substituted", a bivalent C1-8 aliphatic hydrocarbon group which may be substituted, the above-described "bivalent C3-8 monocyclic carbocyclic group which may be substituted", and the above-described "bivalent 3- to 8-membered monocyclic heterocyclic group which may be substituted", and the like.

The "bivalent C1-8 aliphatic hydrocarbon group" in the "bivalent C1-8 aliphatic hydrocarbon group which may be substituted" includes, for example, C1-8 alkylene (e.g., methylene, ethylene, trimethylene, tetramethylene, pentamethylene, hexamethylene, heptamethylene, octamethylene, *etc.*), C2-8 alkenylene (e.g., ethenylene, propenylene, butenylene, butadiene, pentenylene, pentadienylene, hexenylene, hexadienylene, heptenylene, heptadienylene, octenylene, octadienylene, *etc.*), C2-8 alkynylene (e.g., ethynylene, propynylene, butynylene, butadiynylene, pentynylene, pentadiynylene, hexynylene, hexadiynylene, heptynylene, heptadiynylene, octynylene, octadiynylene, *etc.*), and the like. The "substituent" in the " bivalent C1-8 aliphatic hydrocarbon group which may be substituted" includes, for example, those exemplified as the "substituent" in the above-described "bivalent C1-4 aliphatic hydrocarbon group which may be substituted", and the like. The substituents may be substituted in the number of 1 to 10, preferably 1 to 5, more preferably 1 to 3, at a substitutable position.

The "spacer having 1 to 8 atoms in its main chain and containing at least one oxygen atom" represents a bivalent group containing at least one -O- among the groups in the above-described "spacer having 1 to 8 atoms in its main chain". The "spacer having 1 to 8 atoms in its main chain and containing at least one oxygen atom" is preferably those in which an oxygen atom is bound to ring D.

The "spacer having 1 to 6 atoms in its main chain" represented by E includes those in which 1 to 6 atoms exist successively in its main chain among the above-described "spacer having 1 to 8 atoms in its main chain". The "spacer having 1 to 6 atoms in its main chain" represented by E is preferably, for example, the "C 1-6 alkylene which may be substituted", the "C1-5 alkyleneoxy which may be substituted", and the like. The "substituent" in the "C1-6 alkylene which may be substituted" and the "C1-5 alkyleneoxy which may be substituted" as used herein includes, for example, those exemplified as the "substituent" in the above-described "bivalent C1-4 aliphatic hydrocarbon group which may be substituted", and the like. The substituents may be substituted in the number of 1 to 8, preferably 1 to 5, more preferably 1 to 3, at a substitutable position.

The C1-6 alkylene includes, for example, methylene, ethylene, trimethylene, tetramethylene, pentamethylene, hexamethylene, isomers thereof, and the like.

The C1-5 alkyleneoxy includes, for example, methylenoxy, ethyleneoxy, trimethylenoxy, tetramethylenoxy, pentamethylenoxy, isomers thereof, and the like.

The C1-4 alkylene includes, for example, methylene, ethylene, trimethylene, tetramethylene, isomers thereof, and the like.

The C1-3 alkyleneoxy includes, for example, methylenoxy, ethyleneoxy, trimethylenoxy, isomers thereof, and the like.

The "3- to 11-membered monocyclic or bicyclic cyclic group" in the "3- to 11-membered monocyclic or bicyclic cyclic group which may be substituted" represented by M includes, for example, a "3- to 11-membered monocyclic or bicyclic carbocyclic ring", a "3- to 11-membered monocyclic or bicyclic heterocyclic ring containing 1 or 2 nitrogen atoms, 1 or 2 oxygen atoms and/or 1 or 2 sulfur atoms as a hetero atom(s)", and the like. The "3- to 11-membered monocyclic or bicyclic carbocyclic ring" as used herein includes a C3-11 monocyclic or bicyclic unsaturated carbocyclic ring, or partially or completely saturated one thereof, a spiro-bound bicyclic carbocyclic ring and a crosslinked bicyclic carbocyclic ring. The "C3-11 monocyclic or bicyclic unsaturated carbocyclic ring, or partially or completely saturated one thereof" includes, for example, cyclopropane, cyclobutane, cyclopentane, cyclohexane, cycloheptane, cyclooctane, cyclononane, cyclodecane, cycloundecane, cyclopentene, cyclohexene, cycloheptene, cyclooctene, cyclopentadiene, cyclohexadiene, cycloheptadiene, cyclooctadiene, benzene, pentalene, perhydropentalene, azulene, perhydroazulene, indene, perhydroindene, indane, naphthalene, dihydronaphthalene, tetrahydronaphthalene, perhydronaphthalene rings, and the like. The "spiro-bound bicyclic carbocyclic ring" includes, for example, spiro[4.4]nonane, spiro[4.5]decane, spiro[5.5]undecane rings, and the like. The "crosslinked bicyclic carbocyclic ring" includes, for example, bicyclo[2.2.1]heptane, bicyclo[2.2.1]hepta-2-ene, bicyclo[3.1.1]heptane, bicyclo[3.1.1]hepta-2-ene, bicyclo[2.2.2]octane, bicyclo[2.2.2]octa-2-ene, adamantane, noradamantane rings, and the like. Among these, a "C3-11 monocyclic or bicyclic aromatic carbocyclic ring" includes, for example, benzene, azulene, naphthalene rings, and the like.

The "3- to 11-membered monocyclic or bicyclic heterocyclic ring containing 1 or 2 nitrogen atoms, 1 or 2

oxygen atoms and/or 1 or 2 sulfur atoms as a hetero atom(s)" includes a 3- to 11-membered monocyclic or bicyclic unsaturated heterocyclic ring containing 1 or 2 nitrogen atoms, 1 or 2 oxygen atoms and/or 1 or 2 sulfur atoms as a hetero atom(s), or partially or completely saturated one thereof, a spiro-bound bicyclic heterocyclic ring and a crosslinked bicyclic heterocyclic ring. The "3- to 11-membered monocyclic or bicyclic unsaturated heterocyclic ring containing 1 or 2 nitrogen atoms, 1 or 2 oxygen atoms and/or 1 or 2 sulfur atoms as a hetero atom(s), or partially or completely saturated one thereof" includes, for example, pyrrole, imidazole, pyrazole, pyridine, pyrazine, pyrimidine, pyridazine, azepine, diazepine, furan, pyran, oxepine, thiophene, thiopyran, thiepine, oxazole, isoxazole, thiazole, isothiazole, furazan, oxazine, oxazepine, oxadiazepine, thiazine, thiazepine, thiadiazepine, indole, isoindole, indolizine, benzofuran, isobenzofuran, benzothiophene, isobenzothiophene, dithianaphthalene, indazole, quinoline, isoquinoline, quinolizine, phthalazine, haphthyridine, quinoxaline, quinazoline, cinnoline, benzoxazole, benzothiazole, benzimidazole, chromene, benzoxepine, benzoxazepine, benzoxadiazepine, benzothiepine, benzothiazepine, benzothiadiazepine, benzoazepine, benzodiazepine, benzofurazan, benzothiadiazole, aziridine, azetidine, pyrroline, pyrrolidine, imidazoline, imidazolidine, pyrazoline, pyrazolidine, dihydropyridine, tetrahydropyridine, piperidine, dihydropyrazine, tetrahydropyrazine, piperazine, dihydropyrimidine, tetrahydropyrimidine, perhydropyrimidine, dihydropyridazine, tetrahydropyridazine, perhydropyridazine, dihydroazepine, tetrahydroazepine, perhydroazepine, dihydrodiazepine, tetrahydrodiazepine, perhydrodiazepine, oxirane, oxetane, dihydrofuran, tetrahydrofuran, dihydropyran, tetrahydropyran, dihydroxepine, tetrahydroxepine, perhydroxepine, thiirane, thietane, dihydrothiophene, tetrahydrothiophene, dihydrothiopyran, tetrahydrothiopyran, dihydrothiepin, tetrahydrothiepin, perhydrothiepin, dihydroxazole, tetrahydroxazole (oxazolidine), dihydroisoxazole, tetrahydroisoxazole (isoxazolidine), dihydrothiazole, tetrahydrothiazole (thiazolidine), dihydroisothiazole, tetrahydroisothiazole (isothiazolidine), dihydrofurazan, tetrahydrofurazan, dihydroxazine, tetrahydroxazine, dihydroxazepine, tetrahydroxazepine, perhydroxazepine, dihydroxadiazepine, tetrahydroxadiazepine, perhydroxadiazepine, dihydrothiazine, tetrahydrothiazine, dihydrothiazepine, tetrahydrothiazepine, perhydrothiazepine, dihydrothiadiazepine, tetrahydrothiadiazepine, perhydrothiadiazepine, morpholine, thiomorpholine, oxathiane, indoline, isoindoline, dihydrobenzofuran, perhydrobenzofuran, dihydroisobenzofuran, perhydroisobenzofuran, dihydrobenzothiophene, perhydrobenzothiophene, dihydroisobenzothiophene, perhydroisobenzothiophene, dihydroindazole, perhydroindazole, dihydroquinoline, tetrahydroquinoline, perhydroquinoline, dihydroisoquinoline, tetrahydroisoquinoline, perhydroisoquinoline, dihydrophthalazine, tetrahydrophthalazine, perhydrophthalazine, dihydronaphthyridine, tetrahydronaphthyridine, perhydronaphthyridine, dihydroquinoxaline, tetrahydroquinoxaline, perhydroquinoxaline, dihydroquinazoline, tetrahydroquinazoline, perhydroquinazoline, dihydrocinnoline, tetrahydrocinnoline, perhydrocinnoline, benzoxathiane, dihydrobenzoxazine, dihydrobenzothiazine, dihydrobenzoxazole, perhydrobenzoxazole, dihydrobenzothiazole, perhydrobenzothiazole, dihydrobenzimidazole, perhydrobenzimidazole, dihydrobenzazepine, tetrahydrobenzazepine, dihydrobenzodiazepine, tetrahydrobenzodiazepine, benzodioxepane, dihydrobenzoxazepine, tetrahydrobenzoxazepine, dioxolane, dioxane, dithiolane, dithiane, dioxaindane, benzodioxane, chroman, benzodithiolane, benzodithiane rings, and the like. The "spiro-bound bicyclic heterocyclic ring" includes, for example, azaspiro[4.4]nonane, oxazaspiro[4.4]nonane, dioxaspiro[4.4]nonane, azaspiro[4.5]decane, thiaspiro[4.5]decane, dithiaspiro[4.5]decane, dioxaspiro[4.5]decane, oxazaspiro[4.5]decane, azaspiro[5.5]undecane, oxaspiro[5.5]undecane, dioxaspiro[5.5] undecane rings, and the like. The "crosslinked bicyclic heterocyclic ring" includes, for example, azabicyclo[2.2.1]heptane, oxabicyclo[2.2.1]heptane, azabicydo[3.1.1]heptane, azabicyclo[3.2.1]octane, oxabicyclo[3.2.1]octane, azabicyclo[2.2.2]octane, diazabicyclo[2.2.2]octane rings, and the like. Among these, the "3- to 11-membered monocyclic or bicyclic aromatic heterocyclic ring containing 1 or 2 nitrogen atoms, 1 or 2 oxygen atoms and/or 1 or 2 sulfur atoms as a hetero atom(s)" includes, for example, pyrrole, imidazole, pyrazole, pyridine, pyrazine, pyrimidine, pyridazine, furan, thiophene, oxazole, isoxazole, thiazole, isothiazole, furazan, indole, isoindole, benzofuran, isobenzofuran, benzothiophene, isobenzothiophene, indazole, quinolihe, isoquinoline, phthalazine, naphthyridine, quinoxaline, quinazoline, cinnoline, benzoxazole, benzothiazole, benzimidazole, benzofurazan, benzothiadiazole rings, and the like.

The "substituent" in the "3- to 11-membered monocyclic or bicyclic cyclic group which may be substituted" represented by M as used herein includes, for example, the above-described substituents represented by $R^D$. The substituents may be substituted in the number of 1 to 10, preferably 1 to 5, more preferably 1 to 3, at a substitutable position.

In formula (A), the "3- to 8-membered monocyclic heterocyclic ring having at least one nitrogen atom as a hetero atom(s) and 0 to 3 nitrogen atoms, 0 to 1 oxygen atom and/or 0 to 1 sulfur atom as an other hetero atom(s)" includes, for example a 3- to 8-membered monocyclic heterocyclic aryl having at least one nitrogen atom as a hetero atom(s) and 0 to 3 nitrogen atoms, 0 to 1 oxygen atom and/or 0 to 1 sulfur atom as an other hetero atom(s), or partially or completely saturated one thereof. Examples include pyrrole, imidazole, triazole, tetrazole, pyrazole, aziridine, azetidine, pyrroline, pyrrolidine, imidazoline, imidazolidine, triazoline, triazolidine, tetrazoline, tetrazolidine, pyrazoline, pyrazolidine, dihydropyridine, tetrahydropyridine, piperidine, dihydropyrazine, tetrahydropyrazine, piperazine, dihydropyrimidine, tetrahydropyrimidine, perhydropyrimidine, dihydropyridazine, tetrahydropyridazine, perhydropyridazine, dihydroazepine, tetrahydroazepine, perhydroazepine, dihydrodiazepine, tetrahydrodiazepine, perhydrodiazepine, dihydroxazole, tetrahydroxazole (oxazolidine), dihydroisoxazole, tetrahydroisoxazole (isoxazolidine), dihydrothiazole, tetrahydrothiazole (thiazolidine), dihydroisothiazole, tetrahydroisothiazole (isothiazolidine), dihydrofurazan, tetrahydrofurazan, dihydroxadiazole, tetrahydroxadiazole (oxadiazolidine), dihydroxazine, tetrahydroxazine, dihydroxadiazine, tetrahydroxadiazine, dihydroxazepine, tetrahydroxazepine, perhydroxazepine, dihydroxadiazepine, tetrahydroxadiazepine, perhydroxadiazepine, dihydrothiadiazole, tetrahydrothiadiazole (thiadiazolidine), dihydrothiazine, tetrahydrothiazine, dihydrothiadiazine, tetrahydrothiadiazine, dihydrothiazepine, tetrahydrothiazepine, perhydrothiazepine, dihydrothiadiazepine, tetrahydrothiadiazepine, perhydrothiadiazepine, morpholine, thiomorpholine rings, and the like.

In formula (A), the "C3-15 monocyclic, bicyclic or tricyclic carbocyclic ring" includes a C3-15 monocyclic, bicyclic or tricyclic carbocyclic aryl, or partially or completely one thereof. Examples includes cyclopropane, cyclobutane, cyclopentane, cyclohexane, cycloheptane, cyclooctane, cyclononane, cyclodecane, cycloundecane, cyclododecane, cyclotridecane, cyclotetradecane, cyclopentadecane, cyclopentene, cyclohexene, cycloheptene, cyclooctene, cyclopentadiene, cyclohexadiene, cycloheptadiene, cyclooctadiene, benzene, pentalene, perhydropentalene, azulene, perhydroazulene, indene, perhydroindene, indane, naphthalene, dihydronaphthalene, tetrahydronaphthalene, perhydronaphthalene, heptalene, perhydroheptalene, biphenylene, as-indacene, s-indacene, acenaphthylene, acenaphthene, fluorene, phenalene, phenanthrene, anthracene rings, and the like.

In formula (A), the "3- to 15-membered monocyclic, bicyclic or tricyclic heterocyclic ring having 1 to 4 nitrogen atoms, 1 or 2 oxygen atoms and/or 1 or 2 sulfur atoms as a hetero atom(s)" includes a 3- to 15-membered monocyclic, bicyclic or tricyclic heterocyclic aryl containing hetero atoms selected 1 to 4 nitrogen atoms, 1 or 2 oxygen atoms and/or 1 or 2 sulfur atoms as a hetero atom(s), or partially or completely saturated one thereof.

The 3- to 15-membered monocyclic, bicyclic or tricyclic heterocyclic aryl having 1 to 4 nitrogen atoms, 1 or 2 oxygen atoms and/or 1 or 2 sulfur atoms as a hetero atom(s) includes, for example, pyrrole, imidazole, triazole, tetrazole, pyrazole, pyridine, pyrazine, pyrimidine, pyridazine, azepine, diazepine, furan, pyran, oxepine, thiophene, thiopyran, thiepine, oxazole, isoxazole, thiazole, isothiazole, furazan, oxadiazole, oxazine, oxadiazine, oxazepine, oxadiazepine, thiadiazole, thiazine, thiadiazine, thiazepine, thiadiazepine, indole, isoindole, indolizine, benzofuran, isobenzofuran, benzothiophene, isobenzothiophene, dithianaphthalene, indazole, quinoline, isoquinoline, quinolizine, purine, phthalazine, pteridine, naphthyridine, quinoxaline, quinazoline, cinnoline, benzoxazole, benzothiazole, benzimidazole, chromene, benzoxepine, benzoxazepine, benzoxadiazepine, benzothiepine, benzothiazepine, benzothiadiazepine, benzoazepine, benzodiazepine, benzofurazan, benzothiadiazole, benzotriazole, carbazole, β-carboline, acridine, phenazine, dibenzofuran, xanthene, dibenzothiophene, phenothiazine, phenoxazine, phenoxathiin, thianthrene, phenanthridine, phenanthroline, perimidine rings, and the like. The partially or completely saturated 3- to 15-membered monocyclic, bicyclic or tricyclic heterocyclic aryl having 1 to 4 nitrogen atoms, 1 or 2 oxygen atoms and/or 1 or 2 sulfur atoms as a hetero atom(s) includes, for example, aziridine, azetidine, pyrroline, pyrrolidine, imidazoline, imidazolidine, triazoline, triazolidine, tetrazoline, tetrazolidine, pyrazoline, pyrazolidine, dihydropyridine, tetrahydropyridine, piperidine, dihydropyrazine, tetralrydropyrazine, piperazine, dihydropyrimidine, tetrahydropyrimidine, perhydropyrimidine, dihydropyridazine, tetrahydropyridazine, perhydropyridazine, dihydroazepine, tetrahydroazepine, perhydroazepine, dihydrodiazepine, tetrahydrodiazepine, perhydrodiazepine, oxirane, oxetane, dihydrofuran, tetrahydrofuran, dihydropyran, tetrahydropyran, dihydroxepine, tetrahydroxepine, perhydroxepine, thiirane, thietane, dihydrothiophene, tetrahydrothiophene, dihydrothiopyran, tetrahydrothiopyran, dihydrothiepin, tetrahydrothiepin, perhydrothiepin, dihydroxazole, tetrahydroxazole (oxazolidine), dihydroisoxazole, tetrahydroisoxazole (isoxazolidine), dihydrothiazole, tetrahydrothiazole (thiazolidine), dihydroisothiazole, tetrahydroisothiazole (isothiazolidine), dihydrofurazan, tetrahydrofurazan, dihydroxadiazole, tetrahydroxadiazole (oxadiazolidine), dihydroxazine, tetrahydroxazine, dihydroxadiazine, tetrahydroxadiazine, dihydroxazepine, tetrahydroxazepine, perhydroxazepine, dihydroxadiazepine, tetrahydroxadiazepine, perhydroxadiazepine, dihydrothiadiazole, tetrahydrothiadiazole (thiadiazolidine), dihydrothiazine, tetrahydrothiazine, dihydrothiadiazine, tetrahydrothiadiazine, dihydrothiazepine, tetrahydrothiazepine, perhydrothiazepine, dihydrothiadiazepine, tetrahydrothiadiazepine, perhydrothiadiazepine, morpholine, thiomorpholine, oxathiane, indoline, isoindoline, dihydrobenzofuran, perhydrobenzofuran, dihydroisobenzofuran, perhydroisobenzofuran, dihydrobenzothiophene, perhydrodibenzothiophene, dihydroindazole, perhydroindazole, dihydroquinoline, tetrahydroquinoline, perhydroquinoline, dihydroisoquinoline, tetrahydroisoquinoline, perhydroisoquinoline, dihydrophthalazine, tetrahydrophthalazine, perhydrophthalazine, dihydronaphthyridine, tetrahydronaphthyridine, perhydronaphthyridine, dihydroquinoxaline, tetrahydroquinoxaline, perhydroquinoxaline, dihydroquinazoline, tetrahydroquinazoline, perhydroquinazoline, dihydrocinnoline, tetrahydrocinnoline, perhydrocinnoline, benzoxathiane, dihydrobenzoxazine, dihydrobenzothiazine, pyrazinomorpholine, dihydrobenzoxazole, perhydrobenzoxazole, dihydrobenzothiazole, perhydrobenzothiazole, dihydrobenzimidazole, perhydrobenzimidazole, dihydrobenzazepine, tetrahydrobenzazepine, dihydrobenzodiazepine, tetrahydrobenzodiazepine, benzodioxepane, dihydrobenzoxazepine, tetrahydrobenzoxazepine, dihydrocarbazole, tetrahydrocarbazole, perhydrocarbazole, dihydroacridine, tetrahydroacridine, perhydroacridine, dihydrodibenzofuran, dihydrodibenzothiophene, tetrahydrodibenzofuran, tetrahydrodibenzothiophene, perhydrodibenzofuran, perhydrodibenzothiophene, dioxolane, dioxane, dithiolane, dithiane, dioxaindane, benzodioxane, chroman, benzodithiolane, benzodithiane rings, and the like.

In formula (A), the C3-8 monocyclic carbocyclic ring includes C3-8 monocyclic carbocyclic aryl, or partially or

completely saturated one thereof. Examples include cyclopropane, cyclobutane, cyclopentane, cyclohexane, cycloheptane, cyclooctane, cyclopentene, cyclohexene, cycloheptene, cyclooctene, cyclopentadiene, cyclohexadiene, cycloheptadiene, cyclooctadiene, benzene rings, and the like.

In formula (A), the 3- to 8-membered monocyclic heterocyclic ring having 1 to 4 nitrogen atoms, 1 or 2 oxygen atoms and/or 1 or 2 sulfur atoms as a hetero atom(s) includes, for example, the 3- to 8-membered monocyclic heterocyclic aryl having 1 to 4 nitrogen atoms, 1 or 2 oxygen atoms and/or 1 or 2 sulfur atoms as a hetero atom(s) or partially or completely saturated one thereof. The 3- to 15-membered monocyclic, bicyclic or tricyclic heterocyclic aryl having 1 to 4 nitrogen atoms, 1 or 2 oxygen atoms and/or 12 sulfur atoms as a hetero atom(s) includes, for example, pyrrole, imidazole, triazole, tetrazole, pyrazole, pyridine, pyrazine, pyrimidine, pyridazine, azepine, diazepine, furan, pyran, oxepine, thiophene, thiopyran, thiepine, oxazole, isoxazole, thiazole, isothiazole, furazan, oxadiazole, oxazine, oxadiazine, oxazepine, oxadiazepine, thiadiazole, thiazine, thiadiazine, thiazepine, thiadiazepine rings, and the like. Furthermore, the partially or completely saturated 3- to 8-membered monocyclic heterocyclic aryl having 1 to 4 nitrogen atoms, 1 or 2 oxygen atoms and/or 1 or 2 sulfur atoms as a hetero atom(s) includes, for example, aziridine, azetidine, pyrroline, pyrrolidine, imidazoline, imidazolidine, triazoline, triazolidine, tetrazoline, tetrazolidine, pyrazoline, pyrazolidine, dihydropyridine, tetrahydropyridine, piperidine, dihydropyrazine, tetrahydropyrazine, piperazine, dihydropyrimidine, tetrahydropyrimidine, perhydropyrimidine, dihydropyridazine, tetrahydropyridazine, perhydropyridazine, dihydroazepine, tetrahydroazepine, perhydroazepine, dihydrodiazepine, tetrahydrodiazepine, perhydrodiazepine, oxirane, oxetane, dihydrofuran, tetrahydrofuran, dihydropyran, tetrahydropyran, dihydroxepine, tetrahydroxepine, perhydroxepine, thiirane, thietane, dihydrothiophene, tetrahydrothiophene, dihydrothiopyran, tetrahydrothiopyran, dihydrothiepin, tetrahydrothiepin, perhydrothiepin, dihydroxazole, tetrahydroxazole (oxazolidine), dihydroisoxazole, tetrahydroisoxazole (isoxazolidine), dihydrothiazole, tetrahydrothiazole (thiazolidine), dihydroisothiazole, tetrahydroisothiazole (isothiazolidine), dihydrofurazan, tetrahydrofurazan, dihydroxadiazole, tetrahydroxadiazole (oxadiazolidine), dihydroxazine, tetrahydroxazine, dihydroxadiazine, tetrahydroxadiazine, dihydroxazepine, tetrahydroxazepine, perhydroxazepine, dihydroxadiazepine, tetrahydroxadiazepine, perhydroxadiazepine, dihydrothiadiazole, tetrahydrothiadiazole (thiadiazolidine), dihydrothiazine, tetrahydrothiazine, dihydrothiadiazine, tetrahydrothiadiazine, dihydrothiazepine, tetrahydrothiazepine, perhydrothiazepine, dihydrothiadiazepine, tetrahydrothiadiazepine, perhydrothiadiazepine, morpholine, thiomorpholine, oxathiane, dioxolane, dioxane, dithiolane, dithiane rings, and the like.

The C3-4 alkylene includes, for example, trimethylene, tetramethylene and isomers thereof.

In the present specification, the effector cells include all T cells except naive T cells. The naive T cells mean T cells which receive no antigen stimulation. The effector cells include, for example, RA negative and/or RO positive T cells. The "RA negative and/or RO positive T cells" include, for example, Th1 cells, Th2 cells, cytotoxic T-lymphocytes (CTL), central memory T cells (TCM), effector memory T cell (TEM), and the like. RA and RO mean cell surface antigens. The term "negative" means that surface antigen can not be detected, and the term "positive" means that surface antigen can be detected. A method used to detect the surface antigen includes all the methods of detecting a surface antigen known so far. For example, it includes techniques used for the skilled persons in the art to detect proteins (e.g., flow cytometry (FACS), immunostaining, Western blot, fluorescent antibody method, *etc*.) or equivalent techniques thereof. TCM and TEM are those defined in literatures (*Nature.* 1999 Oct. 14; 401 (6754): 708-12.). In the present invention, effector cells are preferably effects cells which express CCR4, i.e., CCR4 positive effector cells.

In the present specification, effector cell functions include all the effector cell functions related to CCR4. The effector cell functions related to CCR4 include, for example, cell migration, permeation increase to blood vessel wall, tissue infiltration, tissue accumulation, release of humoral factor, expression of cell surface antigen.

In the present specification, TNF$\alpha$ regulating activity mean activity of regulating TNF$\alpha$ amount in the living body, preferably reducing TNF$\alpha$ amount in the tissue or the blood, more specifically, reducing TNF$\alpha$ amount in the tissue or the blood in various diseases known to increase TNF$\alpha$ amount in the tissue or the blood.

Unless otherwise specified, the present invention includes all isomers. For example, alkyl, alkenyl, alkynyl, alkoxy, alkylthio, alkylene, alkenylene, alkynylene, *etc.* include straight or branched ones. In addition, the present invention also include isomers on double bond, ring, fused ring (*E-, Z-,* cis-, trans-isomer), isomers generated from asymmetric carbon atoms (*R*-, *S*-isomer, $\alpha$-, $\beta$-configuration, enantiomer, diastereomer), optically active isomers (*D-, L-, d-, l*-isomer), polar compounds generated by chromatographic separation (more polar compound, less polar compound), equilibrium compounds, rotational isomers, mixtures thereof at any ratios and racemic mixtures.

Salts

Salts of the compound of formula (I) include all non-toxic salts and pharmacologically acceptable salts. The pharmacologically acceptable salts are preferably non-toxic and water-soluble salts. Suitable salts of the compound of formula (I) include, for example, salts of alkali metals (potassium or sodium, lithium, *etc*.), salts of alkaline earth metals (calcium or magnesium, *etc*.), ammonium salts (tetramethylammonium salts, tetrabutylammonium salts, *etc*.), salts with organic amines (triethylamine, methylamine, dimethylamine, cyclopentylamine, benzylamine, phenethylamine, piperidine, monoethanolamine, diethanolamine, tris(hydroxymethyl)methylamine, lysine, arginine or *N*-methyl-*D*-glucamine, *etc*.) and acid addition salts [salts of inorganic acids (hydrochloride, hydrobromide, hydroiodide, sulfate, phosphate, nitrate, *etc*.), salts of organic acids (acetate, trifluoroacetate, lactate, tartrate, oxalate, fumarate, maleate, benzoate, citrate, methanesulfonate, ethanesulfonate, benzenesulfonate, toluenesulfonate, isethionate, glucuronate, gluconate, *etc*.), and the like]. Salts of the compound of the present invention also include solvates, or solvates of the above-described alkali (alkaline earth) metal salts, ammonium salts, organic amine salts, and acid addition salts of the compound of the present invention, and the like. The solvates are preferably non-toxic and water-soluble. The appropriate solvates include, for example, solvates such as water, alcohol solvents (ethanol, *etc*.), and the like. The compound of the present invention may be converted into non-toxic and pharmaceutically acceptable salts by a known method.

Furthermore, the salts also include quaternary ammonium salts. The quaternary ammonium salts of the compound of formula (I) mean compounds where a nitrogen atom of the compound of formula (I) is quatemized by $R^0$ ($R^0$ represents C1-8 alkyl or C1-8 alkyl substituted with phenyl).

Furthermore, the salts also include *N*-oxides. The compound of the present invention can be converted to *N*-oxide by any known method. *N*-oxides are the compounds where nitrogen of the compound of formula (I) is oxidized.

The prodrug for the compound of formula (I) means a compound which is converted to the compound of formula (I) by reaction with an enzyme, a gastric acid, or the like, in the living body. Examples of the prodrug for the compound of formula (I) include a compound wherein amino of the compound of formula (I) is substituted with acyl, alkyl, phosphoric acid, or the like (e.g., a compound wherein amino of the compound of formula (I) is substituted with eicosanyl, alanyl, pentylaminocarbonyl, (5-methyl-2-oxo-1,3-dioxolen-4-yl)methoxycarbonyl, tetrahydrofuranyl, pyrrolidylmethyl, pivaloyloxymethyl, acetoxymethy, *tert*-butyl, *etc*.); a compound wherein hydroxyl of the compound of formula (I) is substituted with acyl, alkyl, phosphoric acid, boric acid, or the like (e.g., a compound wherein hydroxyl of the compound of formula (I) is modified with acetyl, palmitoyl, propanoyl, pivaloyl, succinyl, fumaryl, alanyl, dimethylaminomethylcarbonyl, *etc*.); a compound wherein carboxyl of the compound of formula (I) is modified with ester, amide, or the like (e.g., a compound wherein carboxyl of the compound of formula (I) is modified with ethyl ester, phenyl ester, carboxymethyl ester, dimethylaminomethyl ester, pivaloyloxymethyl ester, ethoxycarbonyloxyethyl ester, phthalidyl ester, (5-methyl-2-oxo-1,3-dioxolen-4-yl) methyl ester, cyclohexyloxycarbonylethyl ester, methyl amide, *etc*.), and the like. These compounds may be prepared by per se known method. In addition, the prodrug for the compound of formula (I) may hydrate or non-hydrate. In addition, the prodrug for the compound of formula (I) may be a compound which is converted into the compound of formula (I) under the physiological conditions as described in *Pharmaceutical Research and Development,* Vol.7 "Molecular Design", pages 163-198 published in 1990 by Hirokawa Publishing Co. In addition, compound (I) may be labeled with an isotope (e.g., $^{3}H$, $^{14}C$, $^{35}S$, $^{125}I$, *etc.*) and the like.

In formula (I) of the present invention, any of each definition represented by ring A, ring B, ring D, J, G, $R^D$, $R^n$ and E is preferred. In the following, preferred groups, and preferred rings will be listed. The symbols used herein have the same meanings as described above.

The "cyclic group" in the "cyclic group which may be substituted" represented by ring A is preferably, for example, a carbocyclic ring, a heterocyclic ring, or the like; more preferably, for example, the "C3-15 monocyclic, bicyclic or tricyclic carbocyclic ring", "3- to 15-membered monocyclic, bicyclic or tricyclic heterocyclic ring having 1 to 4 nitrogen atoms, 1 or 2 oxygen atoms and/or 1 or 2 sulfur atoms as a hetero atom(s)", or the like; especially preferably, for example, benzene, naphthalene, pyridine, pyrazole, dioxaindane, benzodioxane, cyclopropane, cycloheptane, cyclohexane, furan, thiophene tetrahydrofuran, piperidine, morpholine, pyridin-1-oxide, 1-methylpyridinium rings, or the like; and most preferably, benzene, naphthalene, pyridine, pyrazole, dioxaindane, benzodioxane rings, or the like.

The "substituent" in the "cyclic group which may be substituted" represented by ring A is preferably, for example, halogen, trifluoromethyl, an aliphatic hydrocarbon group which may be substituted, $-OR^{a1}$, $-NR^{a1}R^{a2}$, an 3- to 15-membered cyclic group which may be substituted or the like; more preferably, for example, halogen, C1-8 alkyl which may be substituted, hydroxyl, amino, -O-(C1-8 alkyl) which may be substituted with $-NR^{b1}R^{b2}$, or the like; especially preferably, for example, halogen, C1-4 alkyl which may be substituted, hydroxyl, amino, -O-(C1-4 alkyl) which may be substituted with $-NR^{b1}R^{b2}$, or the like; and most preferably, for example, fluorine, chlorine, methyl, hydroxyl, methoxy, 2-dimethylaminoethyloxy, amino, or the like.

The "cyclic group" in the "cyclic group which may be substituted" represented by ring B is preferably, for example, a carbocyclic ring, a heterocyclic ring, or the like; and more preferably, for example, the "C3-15 monocyclic, bicyclic or tricyclic carbocyclic ring", the "3-to 15-membered monocyclic, bicyclic or tricyclic heterocyclic ring having 1 to 4 nitrogen atoms, 1 or 2 oxygen atoms and/or 1 or 2 sulfur atoms as a hetero atom(s)", or the like. Examples include benzene, pyridine, thiophene, naphthalene, pyrrole, pyrazole, isoxazole, thiazole, benzothiadiazole, benzothiophene, imidazole, benzofurazan, furan, benzopyran rings, and the like. The "C3-15 monocyclic, bicyclic or tricyclic carbocyclic ring" or the "3- to 15-membered monocyclic, bicyclic or tricyclic heterocyclic ring having 1 to 4 nitrogen atoms, 1 or 2 oxygen atoms and/or 1 or 2 sulfur atoms as a hetero atom(s)" is especially preferably, for example, the "C3-8 monocyclic carbocyclic ring" or the "3- to 8-membered monocyclic heterocyclic ring having 1 to 4 nitrogen atoms, 1 or 2 oxygen atoms and/or 1 or 2 sulfur atoms as a hetero atom(s)", or the like. Among these, preferred examples include the "C3-8

monocyclic aromatic carbocyclic ring", the "3- to 8-membered monocyclic aromatic heterocyclic ring having 1 to 4 nitrogen atoms, 1 or 2 oxygen atoms and/or 1 or 2 sulfur atoms as a hetero atom(s)", and the like. Specific examples include benzene, pyridine, thiophene, furan, pyrrole, pyrazole, isoxazole, thiazole rings, and the like, and especially preferred examples include benzene, pyridine, thiophene rings, and the like.

The "substituent" in the "cyclic group which may be substituted" represented by ring B is preferably, for example, a substituent selected from the above-described Group I, an aliphatic hydrocarbon group which may be substituted, a substituent selected from the above-described Group II, an carbamoyl which may be substituted, or the like; more preferably, for example, an aliphatic hydrocarbon group which may be substituted, halogen, nitro, trifluoromethyl, trifluoromethoxy, $-OR^{a1}$, $-NR^{a1}R^{a2}$, $-SO_2R^{a1}$, $-SR^{a1}$, $-COOR^{a1}$, $-COR^{a1}$ or the like; especially preferably, for example, C1-8 alkyl, halogen, nitro, trifluoromethyl, or the like; and most preferably, for example, methyl, fluorine, chlorine, bromine, nitro, trifluoromethyl, or the like.

The "cyclic group" in the "cyclic group which may be substituted" represented by ring D is preferably, for example, a carbocyclic ring, a heterocyclic ring, or the like; more preferably, for example, the "3- to 15-membered monocyclic, bicyclic or tricyclic heterocyclic ring having 1 to 4 nitrogen atoms, 1 or 2 oxygen atoms and/or 1 or 2 sulfur atoms as a hetero atom(s)", or the like; and especially preferably, for example, a 3- to 10-membered monocyclic or bicyclic heterocyclic ring having 1 to 4 nitrogen atoms, 1 or 2 oxygen atoms and/or 1 or 2 sulfur atoms as a hetero atom(s), or the like. The "3- to 10-membered monocyclic or bicyclic heterocyclic ring having 1 to 4 nitrogen atoms, 1 or 2 oxygen atoms and/or 1 or 2 sulfur atoms as a hetero atom(s)" is preferably, for example, "3- to 10-membered monocyclic or bicyclic heterocyclic ring having 1 to 4 nitrogen atoms as a hetero atom(s)", or the like; more preferably, for example, "3- to 10-membered monocyclic or bicyclic heterocyclic ring containing 1 or 2 nitrogen atoms as a hetero atom(s)", or the like; especially preferably, for example,

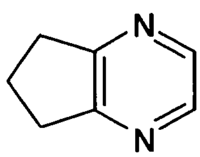 , 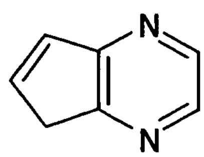 , 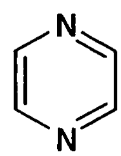 ,

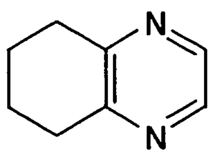 , 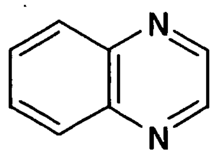 or 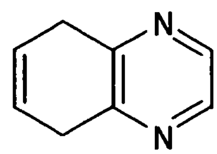

or the like; and most preferably, for example,

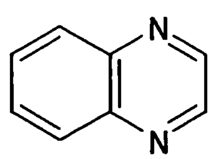 or 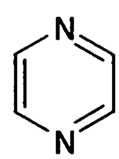

or the like.

The "substituent" in the "cyclic group which may be substituted" represented by ring D is preferably, for example, an aliphatic hydrocarbon group which may be substituted, halogen, cyano, trifluoromethyl, $-COOR^{a1}$, an 3- to 15-membered cyclic group which may be substituted, or the like; more preferably, for example, C1-8 alkyl, halogen, trifluoromethyl, a C3-10 monocyclic or bicyclic carbocyclic ring which may be substituted, or the like; and especially preferably, for example, methyl, chlorine, bromine, trifluoromethyl, a benzene ring which may be substituted, or the like.

G is preferably, for example, a spacer having 1 to 4 atoms in its main chain, or the like; more preferably, for example, a spacer having 1 to 4 atoms in its main chain and containing at least one nitrogen, or the like; and especially preferably, for example, $-NR^{T1}-$, $-NR^{T1}-SO_2-$, $-NR^{T1}-CO-$, $-NR^{T1}-CO-NR^{T2}-$, $-NR^{T1}-SO_2-NR^{T2}-$, $-NR^{T1}-COO-$, $-NR^{T1}-O-$, $-NR^{T1}-NR^{T2}-$, $-NR^{T1}-W-$,- $SO_2-NR^{T1}-$, $-CO-NR^{T1}-$, $-OCO-NR^{T1}-$, $-O-NR^{T1}-$, $-W-NR^{T1}-$, or the like. Among these, $-NR^{T1}-SO_2-$ (wherein the nitrogen is bound to ring D, and the sulfur atom is bound to ring B), especially, for example, $-NH-SO_2-$ (wherein the nitrogen is bound to ring D, and the sulfur is bound to ring B), is preferred.

J is preferably, for example, a spacer having 1 to 8 atoms in its main chain, or the like; more preferably, for example, a spacer having 1 to 8 atoms in its main chain and containing at least one oxygen atom, or the like; and especially preferably, for example, those in which the oxygen atom is bound to ring D. More specifically, J is preferably, for example,

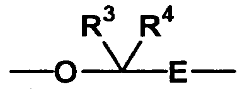

wherein all symbols have the same meanings as described above, and the like. Herein, $R^3$ and $R^4$ are each preferably, for example, hydrogen, methyl, or the like. E is preferably, for example, a bond, C1-6 alkylene, C1-5 alkyleneoxy, or the like; more preferably, for example, a bond, C1-4 alkylene, C1-3 alkyleneoxy, or the like; and especially preferably, for example, a bond, methylene, methylenoxy, or the like. As for E, more preferred is one that the oxygen in C1-5 alkyleneoxy which is described as a preferred group, C1-3 alkyleneoxy which is described as a more preferred group or methylenoxy which is described as most preferred group, is bound to ring A.

In the present invention, preferred is the compound of formula (I) comprising a combination of the above-described preferred groups and preferred rings. Especially, the compound of formula (A) is preferred.

In formula (A), any of each definition represented by $R^1$, $R^2$, $R^3$, $R^4$, $R^5$, $R^6$, $E^1$, ring $A^1$, ring $B^1$, p and q is preferred. In the following, preferred groups, and preferred rings will be listed. The symbols used herein have the same meanings as described above.

Ring $A^1$ is preferably, for example, "C3-10 monocyclic or bicyclic carbocyclic ring", "3- to 10-membered monocyclic or bicyclic heterocyclic ring having 1 to 4 nitrogen atoms, 1 or 2 oxygen atoms and/or 1 or 2 sulfur atoms as a hetero atom(s)", or the like; more preferably, for example, "C3-10 monocyclic or bicyclic carbocyclic aryl", "3- to 10-membered monocyclic or bicyclic heterocyclic aryl having 1 to 4 nitrogen atoms, 1 or 2 oxygen atoms and/or 1 or 2 sulfur atoms as a hetero atom(s) or partially or completely saturated one thereof", or the like; and especially preferably, for example, benzene, naphthalene, pyridine, pyrazole, dioxaindane, benzodioxane rings, or the like.

$R^5$ is preferably, for example, halogen, C1-8 alkyl, $-OR^{31}$, $-NR^{32}R^{33}$, or the like; more preferably, for example, halogen, C1-4 alkyl, hydroxyl, C1-4 alkoxy, C1-4 alkyloxy substituted with $-NR^{37}R^{38}$, amino, or the like; and especially preferably, for example, chlorine, methyl, hydroxyl, methoxy, 2-dimethylaminoethyloxy, amino, or the like.

p is preferably 0, 1 or 2.

Ring $B^1$ is preferably, for example, "C3-8 monocyclic carbocyclic ring", "3- to 8-membered monocyclic heterocyclic ring having 1 to 4 nitrogen atoms, 1 or 2 oxygen atoms and/or 1 or 2 sulfur atoms as a hetero atom(s)", or the like; more preferably, for example, "C3-8 monocyclic carbocyclic aryl", "3- to 8-membered monocyclic heterocyclic aryl having 1 to 4 nitrogen atoms, 1 or 2 oxygen atoms and/or 1 or 2 sulfur atoms as a hetero atom(s)", or the like; and especially preferably, for example, benzene, pyridine, thiophene, or the like.

$R^6$ is preferably, for example, the other substituents than Cyc2 among the above-described substituents as $R^6$; more preferably, for example, C1-8 alkyl, halogen, or the like; especially preferably, for example, C1-4 alkyl, halogen, or the like, and most preferably, for example, methyl, fluorine, chlorine or bromine, or the like.

q is preferably 0, 1 or 2.

$R^1$ and $R^2$ are each preferably, for example, hydrogen, C1-8 alkyl, halogen, trifluoromethyl, cyano, Cyc1, or the like; more preferably, for example, hydrogen, C1-4 alkyl, halogen, trifluoromethyl, cyano or "C3-15 monocyclic, bicyclic or tricyclic carbocyclic ring", or the like; and especially preferably, for example, hydrogen, methyl, chlorine or bromine, trifluoromethyl, cyano, a benzene ring, or the like. Furthermore, $R^1$ and $R^2$ also preferably form -CH=CH-CH=CH-, together with each other.

Cyc1 is preferably, for example, "C3-8 monocyclic carbocyclic ring", "3- to 8-membered monocyclic heterocyclic ring having 1 to 4 nitrogen atoms, 1 or 2 oxygen atoms and/or 1 or 2 sulfur atoms as a hetero atom(s)", or the like; and more preferably, for example, cyclopropane, cyclobutane, cyclopentane, cyclohexane, benzene, imidazole, pyridine, piperidine, morpholine, or the like.

$R^{18}$ as a substituent of Cyc1 is preferably, for example, $-NR^{21}R^{22}$, $-COR^{23}$, or the like; and especially preferably, for example, $-NH_2$ or -CHO, or the like.

$R^3$ and $R^4$ are each preferably, for example, hydrogen, methyl, or the like.

$E^1$ is preferably, for example, a single bond, C1-4 alkylene, C1-3 alkyleneoxy; and especially preferably, for example, a single bond, methylene, methylenoxy, or the like.

The compound of the present invention of formula (I), especially, the compound of the present invention of formula (A) is preferably the compound of formula (A) comprising a combination of the above-described preferred groups and preferred rings among the present compound of formula (A).

Examples of the preferred compound of the present invention include the compounds described in Examples

or salts thereof.

The compound of the present invention of formula (I), especially, the compound of the present invention of formula (A) is preferably, for example, a pyrazine derivative or a salt thereof of formula (I-1):

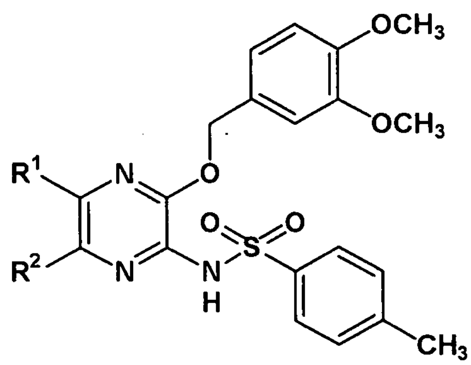

(I − 1)

wherein $R^1$ and $R^2$ have the same meanings as described above,

formula (I-2):

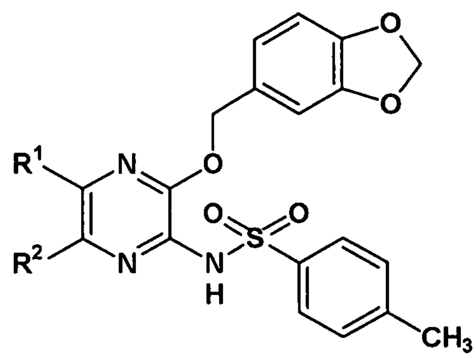

(I − 2)

wherein $R^1$ and $R^2$ have the same meanings as described above,

formula (I-3):

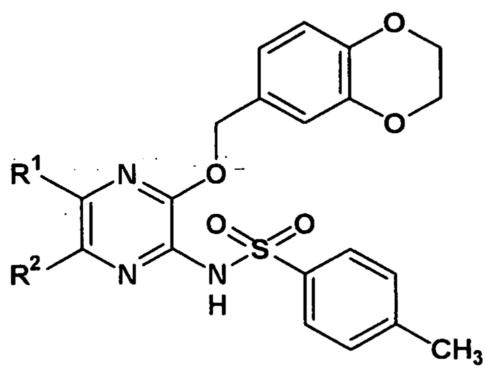

(I − 3)

wherein $R^1$ and $R^2$ have the same meanings as described above,

formula (I-4):

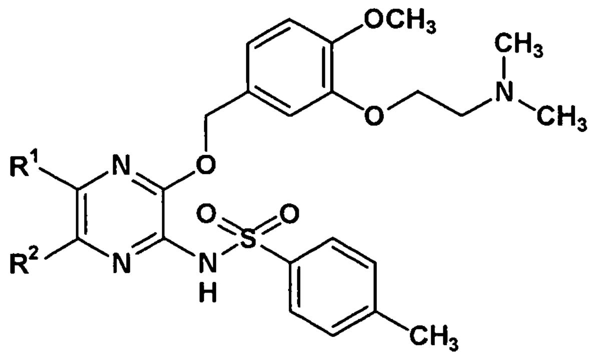

(I - 4)

wherein $R^1$ and $R^2$ have the same meanings as described above,

formula (I-5):

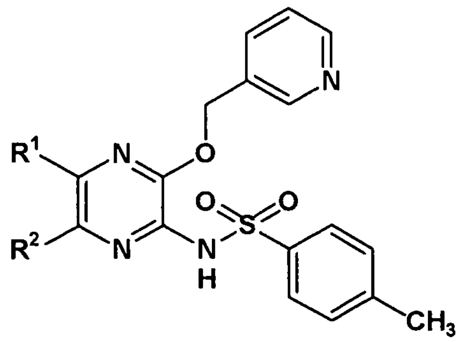

(I - 5)

wherein $R^1$ and $R^2$ have the same meanings as described above,

formula (I-6):

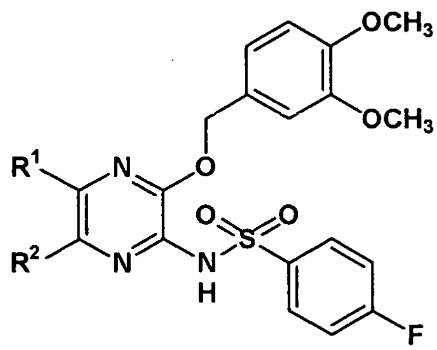

(I - 6)

wherein $R^1$ and $R^2$ have the same meanings as described above,

formula (I-7):

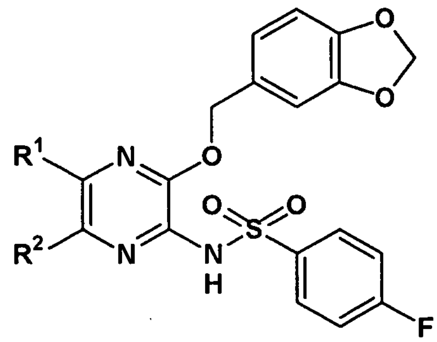

(I − 7)

wherein $R^1$ and $R^2$ have the same meanings as described above,

formula (I-8):

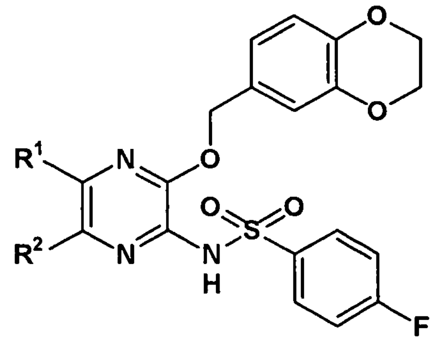

(I − 8)

wherein $R^1$ and $R^2$ have the same meanings as described above,

formula (I-9):

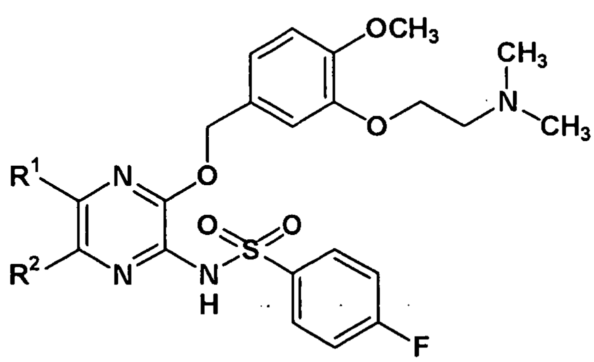

(I − 9)

wherein $R^1$ and $R^2$ have the same meanings as described above,

formula (I-10):

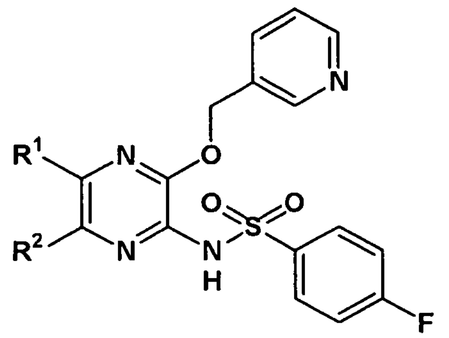

(I-10)

wherein $R^1$ and $R^2$ have the same meanings as described above,

formula (I-11):

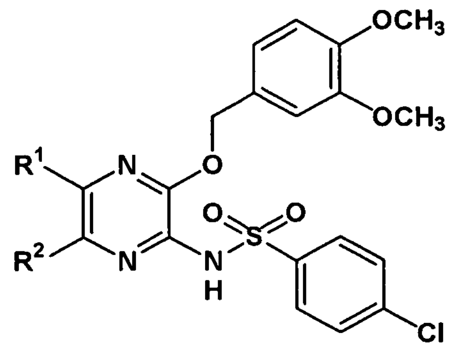

(I-11)

wherein $R^1$ and $R^2$ have the same meanings as described above,

formula (I-12):

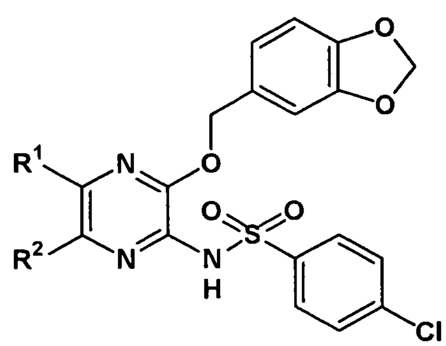

(I-12)

wherein $R^1$ and $R^2$ have the same meanings as described above,

formula (I-13):

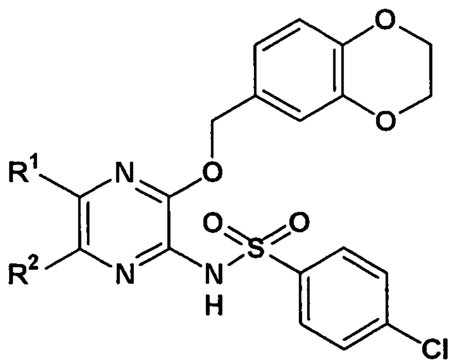

(I－13)

wherein $R^1$ and $R^2$ have the same meanings as described above,

formula (I-14):

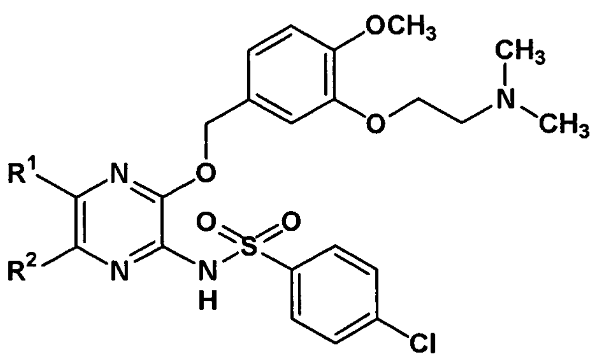

(I－14)

wherein $R^1$ and $R^2$ have the same meanings as described above,

formula (I-15):

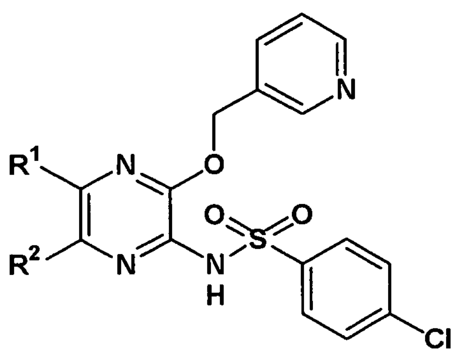

(I－15)

wherein $R^1$ and $R^2$ have the same meanings as described above,

formula (I-16):

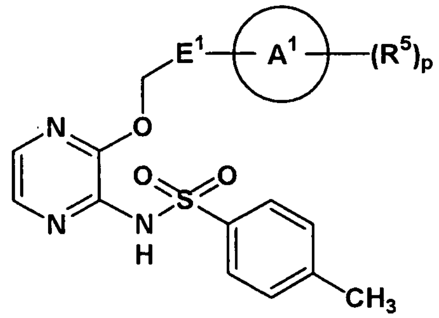

(I-16)

wherein $E^1$, ring $A^1$, $R^5$ and p have the same meanings as described above,

formula (I-17):

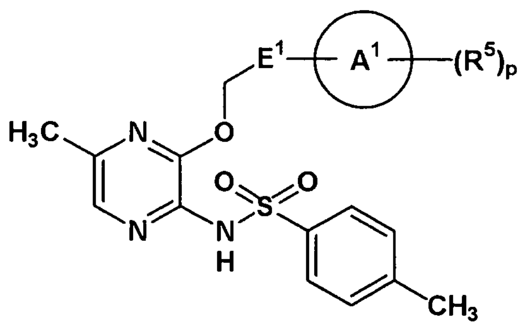

(I-17)

wherein $E^1$, ring $A^1$, $R^5$ and p have the same meanings as described above,

formula (I-18):

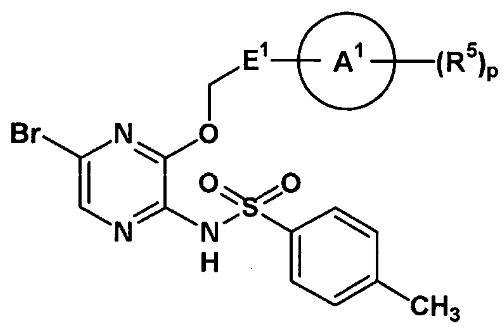

(I-18)

wherein $E^1$, ring $A^1$, $R^5$ and p have the same meanings as described above,

formula (I-19):

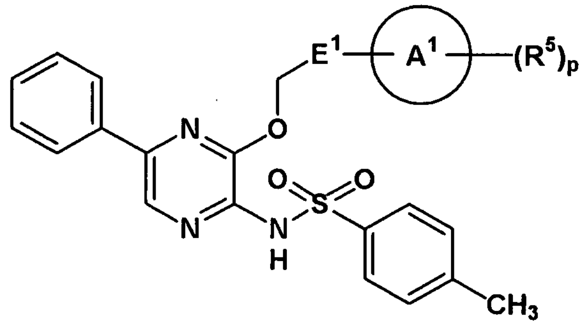

(I－19)

wherein $E^1$, ring $A^1$, $R^5$ and p have the same meanings as described above,

formula (I-20):

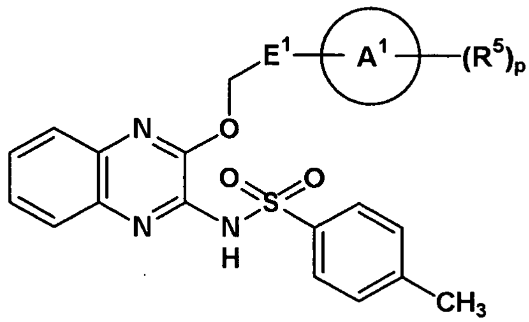

(I－20)

wherein $E^1$, ring $A^1$, $R^5$ and p have the same meanings as described above,

formula (I-21):

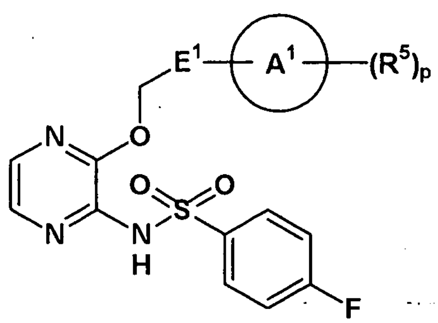

(I－21)

wherein $E^1$, ring $A^1$, $R^5$ and p have the same meanings as described above,

formula (I-22):

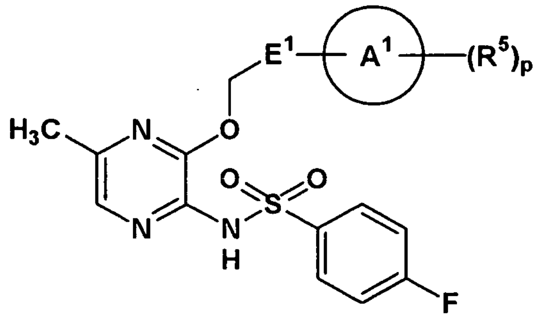

(I－22)

wherein $E^1$, ring $A^1$, $R^5$ and p have the same meanings as described above,

formula (I-23):

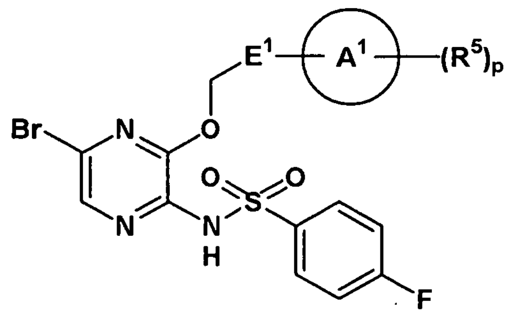

(I－23)

wherein $E^1$, ring $A^1$, $R^5$ and p have the same meanings as described above,

formula (I-24):

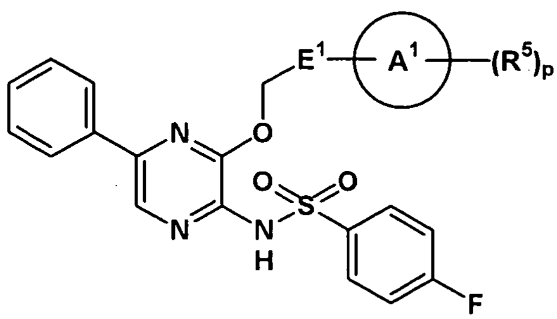

(I－24)

wherein $E^1$, ring $A^1$, $R^5$ and p have the same meanings as described above,

formula (I-25):

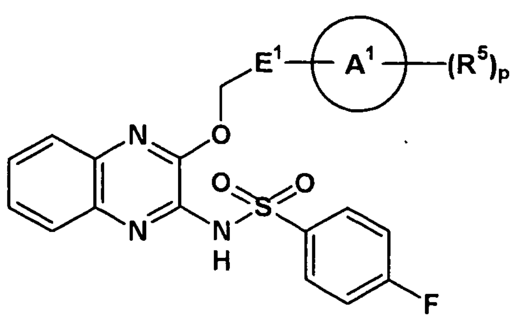

(I－25)

wherein $E^1$, ring $A^1$, $R^5$ and p have the same meanings as described above,

formula (I-26):

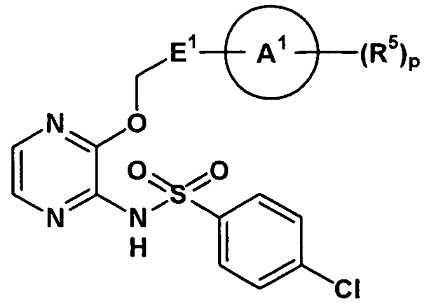

(I－26)

wherein $E^1$, ring $A^1$, $R^5$ and p have the same meanings as described above,

formula (I-27):

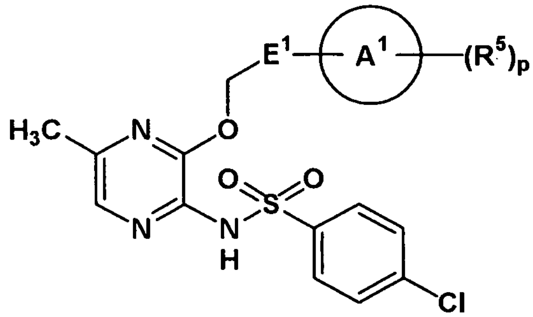

(I－27)

wherein $E^1$, ring $A^1$, $R^5$ and p have the same meanings as described above,

formula (I-28):

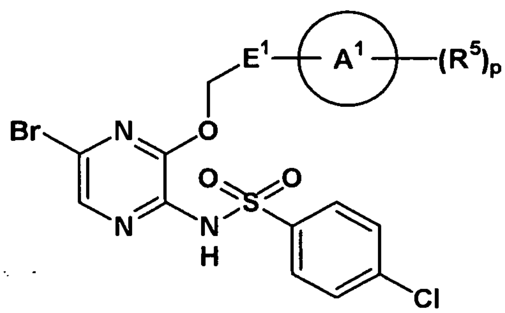

(I－28)

wherein $E^1$, ring $A^1$, $R^5$ and p have the same meanings as described above,

formula (I-29):

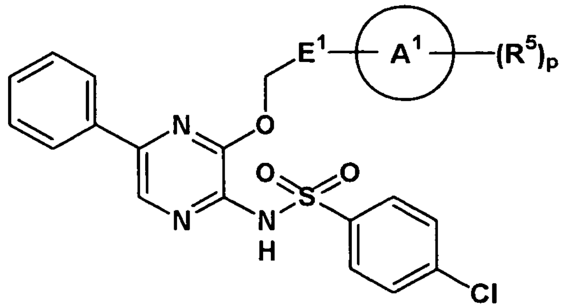

(I－29)

wherein $E^1$, ring $A^1$, $R^5$ and p have the same meanings as described above, and

formula (I-30):

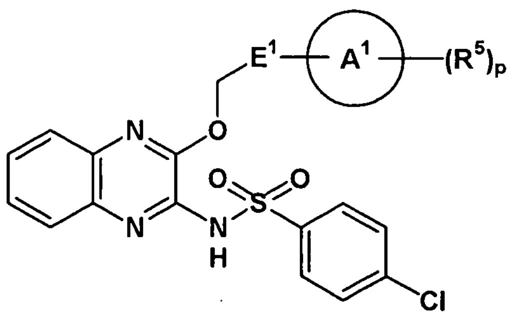

(I－30)

wherein $E^1$, ring $A^1$, $R^5$ and p have the same meanings as described above.

Examples of the compound of the present invention include the compounds shown in the following Tables 1 to 30, the compounds described in Examples, and salts thereof.

Table 1

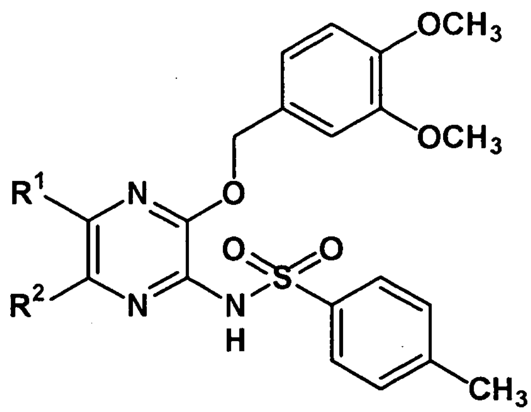

(I－1)

| No. | $R^1$ | $R^2$ | No. | $R^1$ | $R^2$ |
|---|---|---|---|---|---|
| 1 | H | H | 11 | H | $CH_3$ |
| 2 | $CH_3$ | H | 12 | H | $OCH_3$ |
| 3 | $OCH_3$ | H | 13 | H | Cl |
| 4 | Cl | H | 14 | H | Br |
| 5 | Br | H | 15 | H | Ph |
| 6 | Ph | H | 16 | H | CN |
| 7 | CN | H | 17 | H | $NO_2$ |
| 8 | $NO_2$ | H | 18 | H | COOH |
| 9 | COOH | H | 19 | $-(CH_2)_4-$ | |
| 10 | $-(CH_2)_3-$ | | 20 | -CH=CH-CH=CH- | |

Table 2

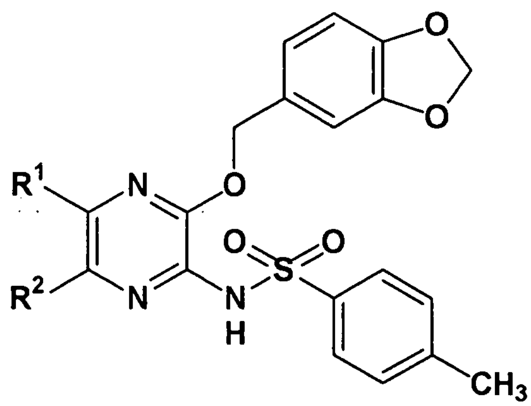

(I − 2)

| No. | $R^1$ | $R^2$ | No. | $R^1$ | $R^2$ |
|---|---|---|---|---|---|
| 1 | H | H | 11 | H | $CH_3$ |
| 2 | $CH_3$ | H | 12 | H | $OCH_3$ |
| 3 | $OCH_3$ | H | 13 | H | Cl |
| 4 | Cl | H | 14 | H | Br |
| 5 | Br | H | 15 | H | Ph |
| 6 | Ph | H | 16 | H | CN |
| 7 | CN | H | 17 | H | $NO_2$ |
| 8 | $NO_2$ | H | 18 | H | COOH |
| 9 | COOH | H | 19 | $-(CH_2)_4-$ | |
| 10 | $-(CH_2)_3-$ | | 20 | -CH=CH-CH=CH- | |

Table 3

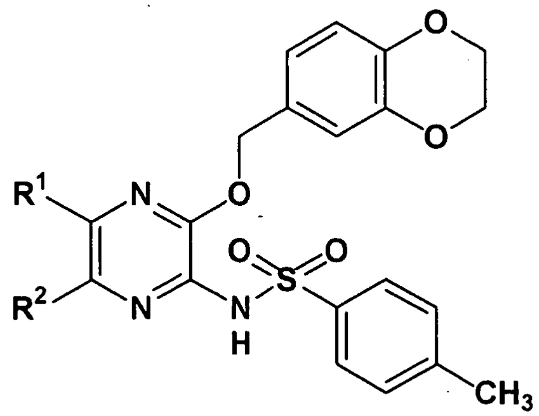

(I − 3)

| No. | $R^1$ | $R^2$ | No. | $R^1$ | $R^2$ |
|---|---|---|---|---|---|
| 1 | H | H | 11 | H | $CH_3$ |
| 2 | $CH_3$ | H | 12 | H | $OCH_3$ |
| 3 | $OCH_3$ | H | 13 | H | Cl |
| 4 | Cl | H | 14 | H | Br |
| 5 | Br | H | 15 | H | Ph |
| 6 | Ph | H | 16 | H | CN |
| 7 | CN | H | 17 | H | $NO_2$ |
| 8 | $NO_2$ | H | 18 | H | COOH |
| 9 | COOH | H | 19 | $-(CH_2)_4-$ | |
| 10 | $-(CH_2)_3-$ | | 20 | -CH=CH-CH=CH- | |

Table 4

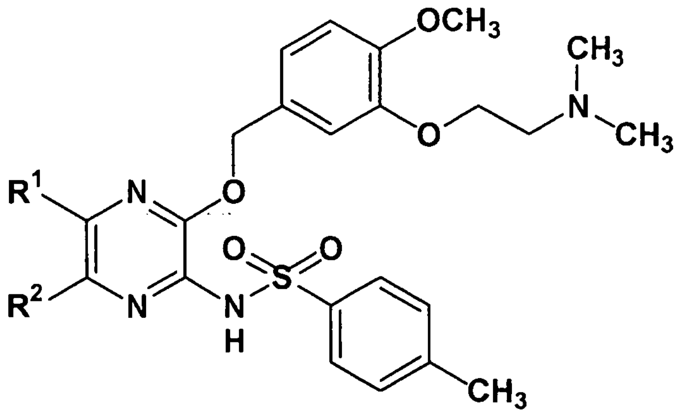

(I－4)...

| No. | $R^1$ | $R^2$ | No. | $R^1$ | $R^2$ |
|---|---|---|---|---|---|
| 1 | H | H | 11 | H | $CH_3$ |
| 2 | $CH_3$ | H | 12 | H | $OCH_3$ |
| 3 | $OCH_3$ | H | 13 | H | Cl |
| 4 | Cl | H | 14 | H | Br |
| 5 | Br | H | 15 | H | Ph |
| 6 | Ph | H | 16 | H | CN |
| 7 | CN | H | 17 | H | $NO_2$ |
| 8 | $NO_2$ | H | 18 | H | COOH |
| 9 | COOH | H | 19 | $-(CH_2)_4-$ | |
| 10 | $-(CH_2)_3-$ | | 20 | -CH=CH-CH=CH- | |

Table 5

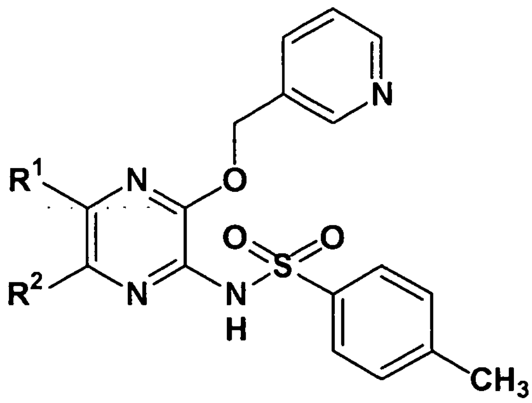

(I－5)

| No. | $R^1$ | $R^2$ | No. | $R^1$ | $R^2$ |
|---|---|---|---|---|---|
| 1 | H | H | 11 | H | $CH_3$ |
| 2 | $CH_3$ | H | 12 | H | $OCH_3$ |
| 3 | $OCH_3$ | H | 13 | H | Cl |
| 4 | Cl | H | 14 | H | Br |
| 5 | Br | H | 15 | H | Ph |
| 6 | Ph | H | 16 | H | CN |
| 7 | CN | H | 17 | H | $NO_2$ |
| 8 | $NO_2$ | H | 18 | H | COOH |
| 9 | COOH | H | 19 | $-(CH_2)_4-$ | |
| 10 | $-(CH_2)_3-$ | | 20 | -CH=CH-CH=CH- | |

Table 6

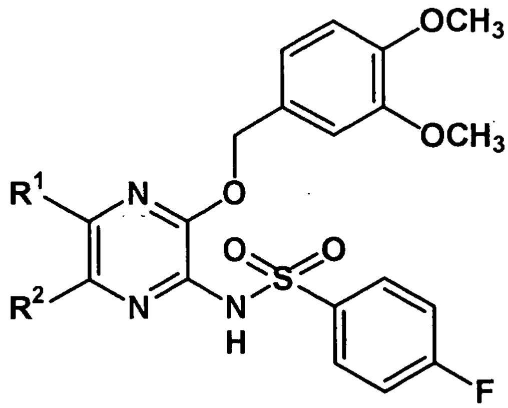

(I − 6)

| No. | $R^1$ | $R^2$ | No. | $R^1$ | $R^2$ |
|---|---|---|---|---|---|
| 1 | H | H | 11 | H | $CH_3$ |
| 2 | $CH_3$ | H | 12 | H | $OCH_3$ |
| 3 | $OCH_3$ | H | 13 | H | Cl |
| 4 | Cl | H | 14 | H | Br |
| 5 | Br | H | 15 | H | Ph |
| 6 | Ph | H | 16 | H | CN |
| 7 | CN | H | 17 | H | $NO_2$ |
| 8 | $NO_2$ | H | 18 | H | COOH |
| 9 | COOH | H | 19 | $-(CH_2)_4-$ | |
| 10 | $-(CH_2)_3-$ | | 20 | -CH=CH-CH=CH- | |

Table 7

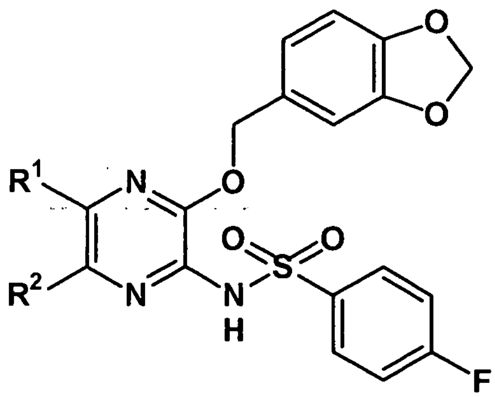

(I − 7)

| No. | $R^1$ | $R^2$ | No. | $R^1$ | $R^2$ |
|---|---|---|---|---|---|
| 1 | H | H | 11 | H | $CH_3$ |
| 2 | $CH_3$ | H | 12 | H | $OCH_3$ |
| 3 | $OCH_3$ | H | 13 | H | Cl |
| 4 | Cl | H | 14 | H | Br |
| 5 | Br | H | 15 | H | Ph |
| 6 | Ph | H | 16 | H | CN |
| 7 | CN | H | 17 | H | $NO_2$ |
| 8 | $NO_2$ | H | 18 | H | COOH |
| 9 | COOH | H | 19 | $-(CH_2)_4-$ | |
| 10 | $-(CH_2)_3-$ | | 20 | -CH=CH-CH=CH- | |

Table 8

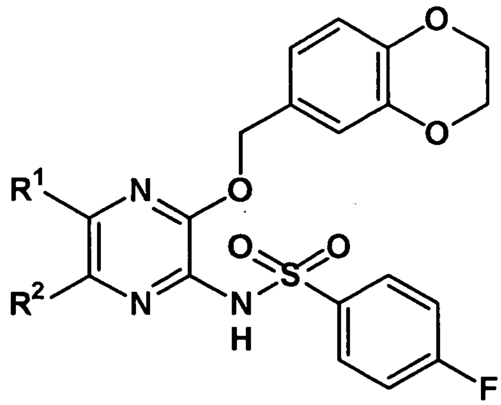

(I－8)

| No. | $R^1$ | $R^2$ | No. | $R^1$ | $R^2$ |
|---|---|---|---|---|---|
| 1 | H | H | 11 | H | $CH_3$ |
| 2 | $CH_3$ | H | 12 | H | $OCH_3$ |
| 3 | $OCH_3$ | H | 13 | H | Cl |
| 4 | Cl | H | 14 | H | Br |
| 5 | Br | H | 15 | H | Ph |
| 6 | Ph | H | 16 | H | CN |
| 7 | CN | H | 17 | H | $NO_2$ |
| 8 | $NO_2$ | H | 18 | H | COOH |
| 9 | COOH | H | 19 | $-(CH_2)_4-$ | |
| 10 | $-(CH_2)_3-$ | | 20 | -CH=CH-CH=CH- | |

Table 9

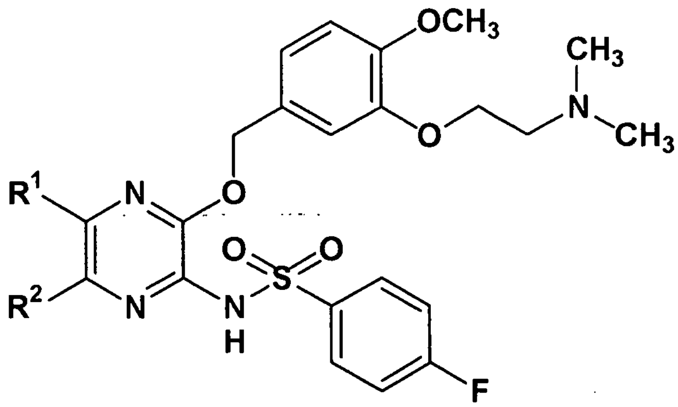

(I - 9)

| No. | $R^1$ | $R^2$ | No. | $R^1$ | $R^2$ |
|---|---|---|---|---|---|
| 1 | H | H | 11 | H | $CH_3$ |
| 2 | $CH_3$ | H | 12 | H | $OCH_3$ |
| 3 | $OCH_3$ | H | 13 | H | Cl |
| 4 | Cl | H | 14 | H | Br |
| 5 | Br | H | 15 | H | Ph |
| 6 | Ph | H | 16 | H | CN |
| 7 | CN | H | 17 | H | $NO_2$ |
| 8 | $NO_2$ | H | 18 | H | COOH |
| 9 | COOH | H | 19 | $-(CH_2)_4-$ | |
| 10 | $-(CH_2)_3-$ | | 20 | -CH=CH-CH=CH- | |

Table 10

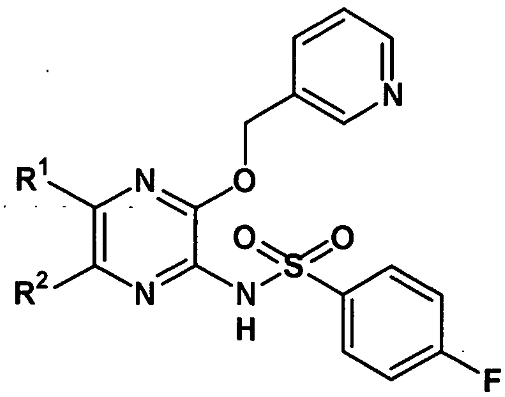

(I－1 0)

| No. | $R^1$ | $R^2$ | No. | $R^1$ | $R^2$ |
|---|---|---|---|---|---|
| 1 | H | H | 11 | H | $CH_3$ |
| 2 | $CH_3$ | H | 12 | H | $OCH_3$ |
| 3 | $OCH_3$ | H | 13 | H | Cl |
| 4 | Cl | H | 14 | H | Br |
| 5 | Br | H | 15 | H | Ph |
| 6 | Ph | H | 16 | H | CN |
| 7 | CN | H | 17 | H | $NO_2$ |
| 8 | $NO_2$ | H | 18 | H | COOH |
| 9 | COOH | H | 19 | $-(CH_2)_4-$ | |
| 10 | $-(CH_2)_3-$ | | 20 | -CH=CH-CH=CH- | |

Table 11

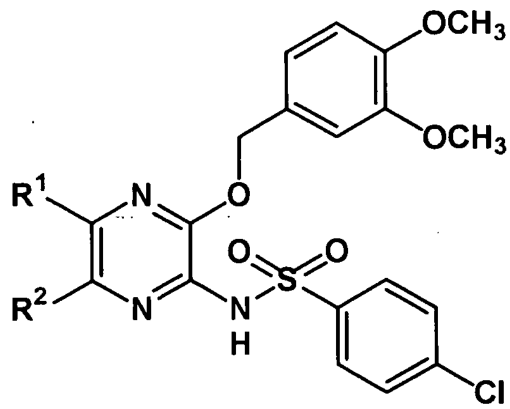

(I－11)

| No. | $R^1$ | $R^2$ | No. | $R^1$ | $R^2$ |
|---|---|---|---|---|---|
| 1 | H | H | 11 | H | $CH_3$ |
| 2 | $CH_3$ | H | 12 | H | $OCH_3$ |
| 3 | $OCH_3$ | H | 13 | H | Cl |
| 4 | Cl | H | 14 | H | Br |
| 5 | Br | H | 15 | H | Ph |
| 6 | Ph | H | 16 | H | CN |
| 7 | CN | H | 17 | H | $NO_2$ |
| 8 | $NO_2$ | H | 18 | H | COOH |
| 9 | COOH | H | 19 | $-(CH_2)_4-$ | |
| 10 | $-(CH_2)_3-$ | | 20 | -CH=CH-CH=CH- | |

Table 12

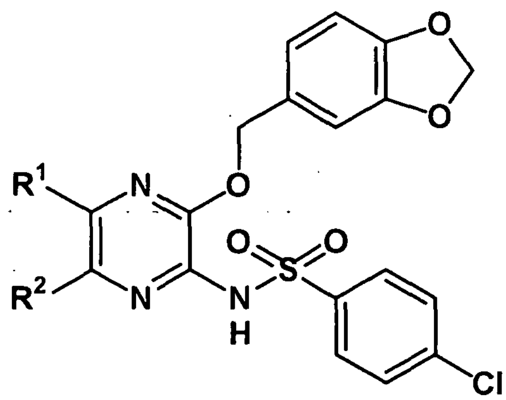

(I－1 2)

| No. | $R^1$ | $R^2$ | No. | $R^1$ | $R^2$ |
|---|---|---|---|---|---|
| 1 | H | H | 11 | H | $CH_3$ |
| 2 | $CH_3$ | H | 12 | H | $OCH_3$ |
| 3 | $OCH_3$ | H | 13 | H | Cl |
| 4 | Cl | H | 14 | H | Br |
| 5 | Br | H | 15 | H | Ph |
| 6 | Ph | H | 16 | H | CN |
| 7 | CN | H | 17 | H | $NO_2$ |
| 8 | $NO_2$ | H | 18 | H | COOH |
| 9 | COOH | H | 19 | $-(CH_2)_4-$ | |
| 10 | $-(CH_2)_3-$ | | 20 | -CH=CH-CH=CH- | |

Table 13

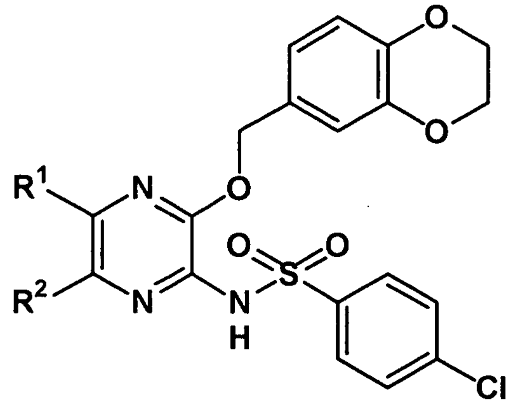

(I－13)

| No. | $R^1$ | $R^2$ | No. | $R^1$ | $R^2$ |
|---|---|---|---|---|---|
| 1 | H | H | 11 | H | $CH_3$ |
| 2 | $CH_3$ | H | 12 | H | $OCH_3$ |
| 3 | $OCH_3$ | H | 13 | H | Cl |
| 4 | Cl | H | 14 | H | Br |
| 5 | Br | H | 15 | H | Ph |
| 6 | Ph | H | 16 | H | CN |
| 7 | CN | H | 17 | H | $NO_2$ |
| 8 | $NO_2$ | H | 18 | H | COOH |
| 9 | COOH | H | 19 | $-(CH_2)_4-$ | |
| 10 | $-(CH_2)_3-$ | | 20 | -CH=CH-CH=CH- | |

Table 14

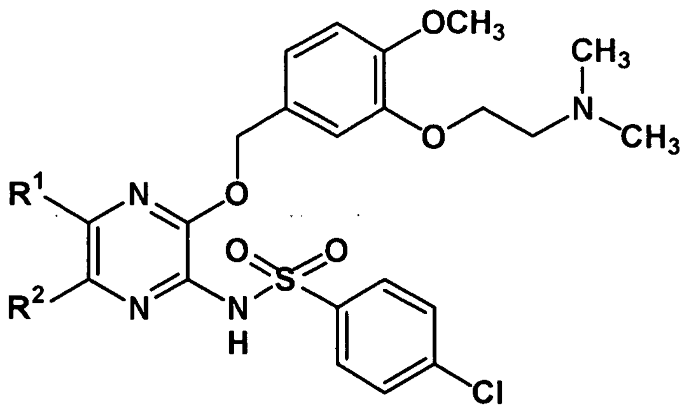

(I-14)

| No. | $R^1$ | $R^2$ | No. | $R^1$ | $R^2$ |
|---|---|---|---|---|---|
| 1 | H | H | 11 | H | $CH_3$ |
| 2 | $CH_3$ | H | 12 | H | $OCH_3$ |
| 3 | $OCH_3$ | H | 13 | H | Cl |
| 4 | Cl | H | 14 | H | Br |
| 5 | Br | H | 15 | H | Ph |
| 6 | Ph | H | 16 | H | CN |
| 7 | CN | H | 17 | H | $NO_2$ |
| 8 | $NO_2$ | H | 18 | H | COOH |
| 9 | COOH | H | 19 | $-(CH_2)_4-$ | |
| 10 | $-(CH_2)_3-$ | | 20 | -CH=CH-CH=CH- | |

Table 15

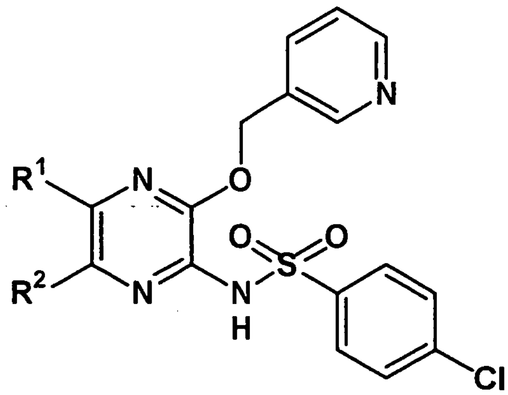

(I－15)

| No. | $R^1$ | $R^2$ | No. | $R^1$ | $R^2$ |
|---|---|---|---|---|---|
| 1 | H | H | 11 | H | $CH_3$ |
| 2 | $CH_3$ | H | 12 | H | $OCH_3$ |
| 3 | $OCH_3$ | H | 13 | H | Cl |
| 4 | Cl | H | 14 | H | Br |
| 5 | Br | H | 15 | H | Ph |
| 6 | Ph | H | 16 | H | CN |
| 7 | CN | H | 17 | H | $NO_2$ |
| 8 | $NO_2$ | H | 18 | H | COOH |
| 9 | COOH | H | 19 | $-(CH_2)_4-$ | |
| 10 | $-(CH_2)_3-$ | | 20 | -CH=CH-CH=CH- | |

Table 16

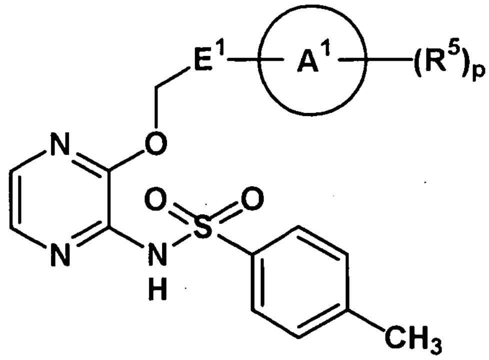

(I－16)

| No. | 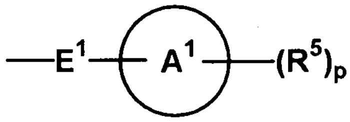 | No. | 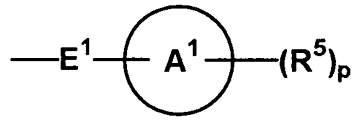 |
|---|---|---|---|
| 1 | 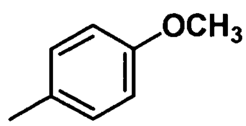 | 8 | 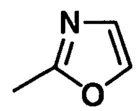 |
| 2 | 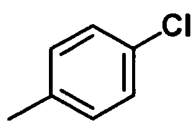 | 9 | 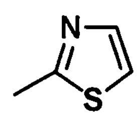 |
| 3 | 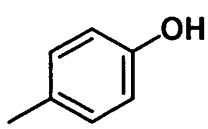 | 10 | 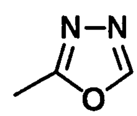 |
| 4 | 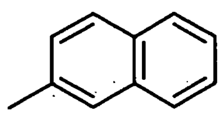 | 11 | 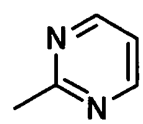 |
| 5 | 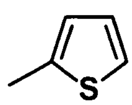 | 12 | 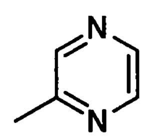 |
| 6 | 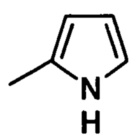 | 13 | 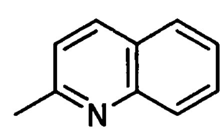 |
| 7 | 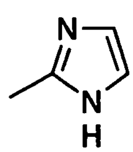 | 14 | 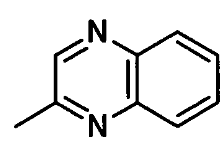 |

Table 17

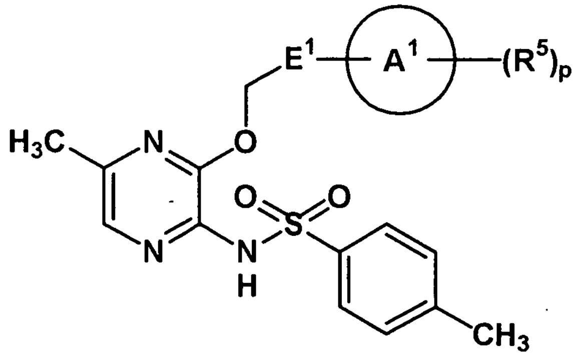

(I－17)

| No. | 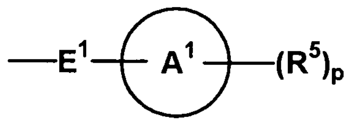 | No. | 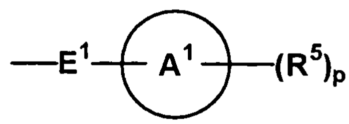 |
|---|---|---|---|
| 1 | 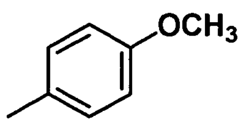 | 8 | 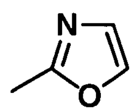 |
| 2 | 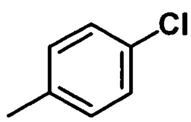 | 9 | 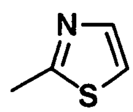 |
| 3 | 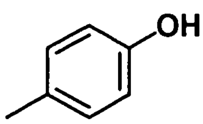 | 10 | 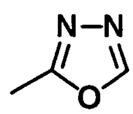 |
| 4 | 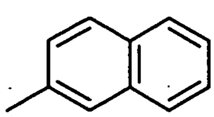 | 11 | 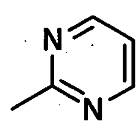 |
| 5 | 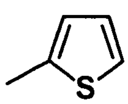 | 12 | 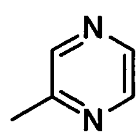 |
| 6 | 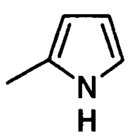 | 13 | 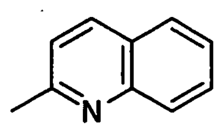 |
| 7 | 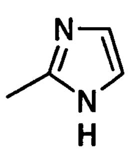 | 14 | 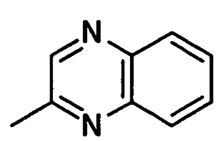 |

Table 18

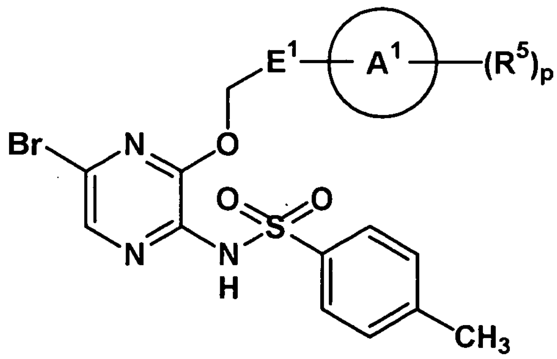

(I－18)

| No. | 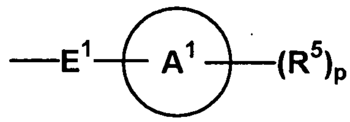 | No. | 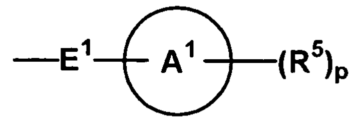 |
|---|---|---|---|
| 1 | 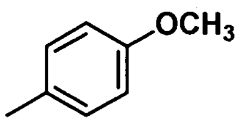 | 8 | 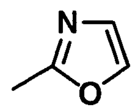 |
| 2 | 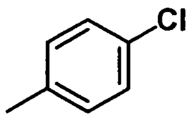 | 9 | 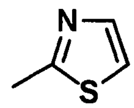 |
| 3 | 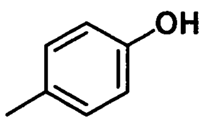 | 10 | 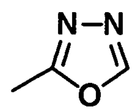 |
| 4 | 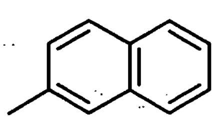 | 11 | 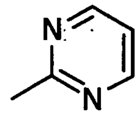 |
| 5 | 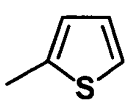 | 12 | 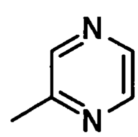 |
| 6 | 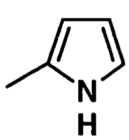 | 13 | 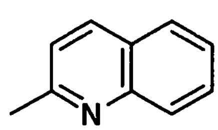 |
| 7 | 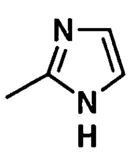 | 14 | 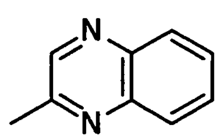 |

Table 19

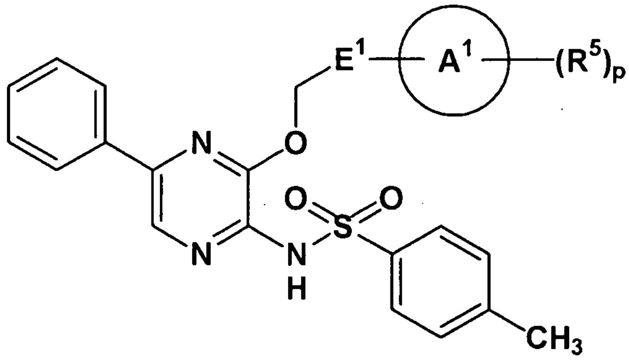

(I－1 9)

| No. | 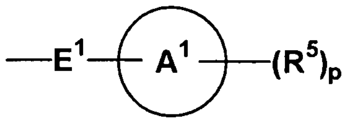 | No. | 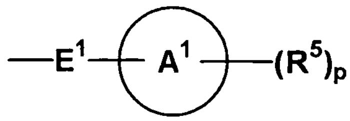 |
|---|---|---|---|
| 1 | 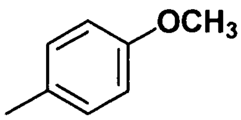 | 8 | 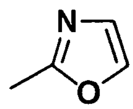 |
| 2 | 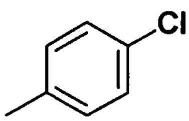 | 9 | 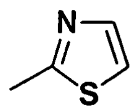 |
| 3 | 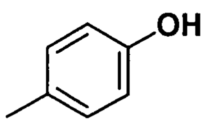 | 10 | 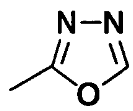 |
| 4 | 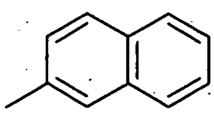 | 11 | 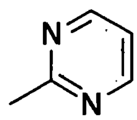 |
| 5 | 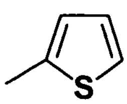 | 12 | 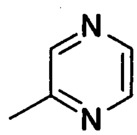 |
| 6 | 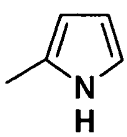 | 13 | 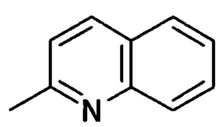 |
| 7 | 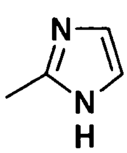 | 14 | 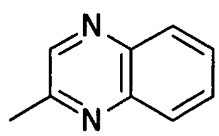 |

Table 20

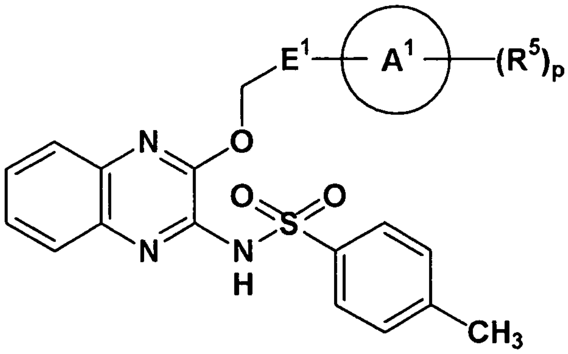

(I－２０)

| No. | 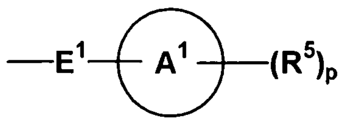 | No. | 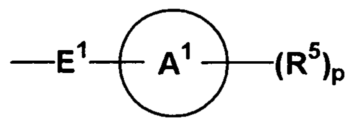 |
|---|---|---|---|
| 1 | 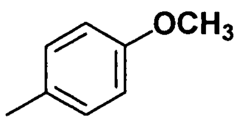 | 8 | 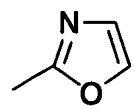 |
| 2 | 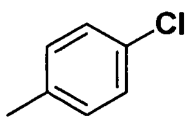 | 9 | 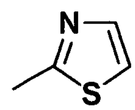 |
| 3 | 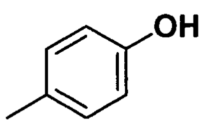 | 10 | 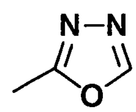 |
| 4 | 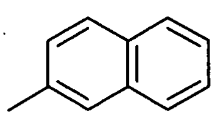 | 11 | 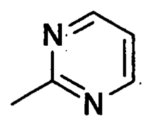 |
| 5 | 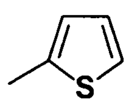 | 12 | 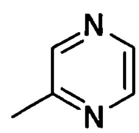 |
| 6 | 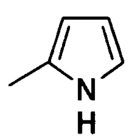 | 13 | 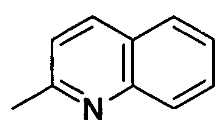 |
| 7 | 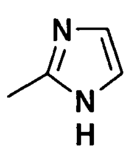 | 14 | 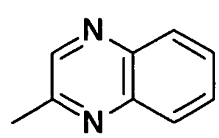 |

Table 21

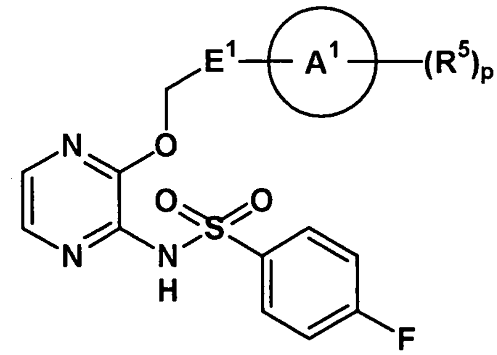

(I－21)

| No. | 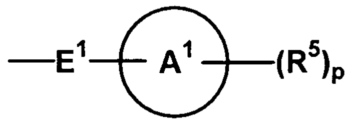 | No. | 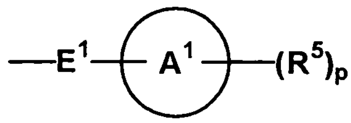 |
|---|---|---|---|
| 1 | 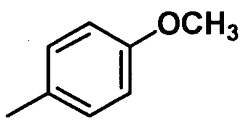 | 8 | 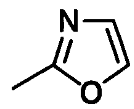 |
| 2 | 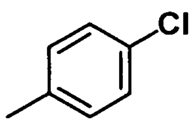 | 9 | 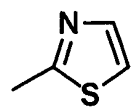 |
| 3 | 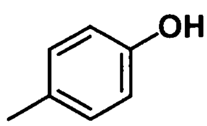 | 10 | 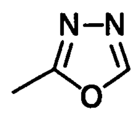 |
| 4 | 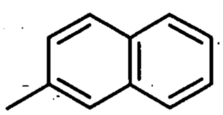 | 11 | 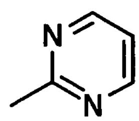 |
| 5 | 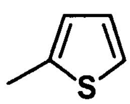 | 12 | 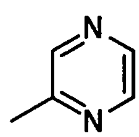 |
| 6 | 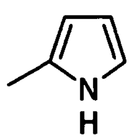 | 13 | 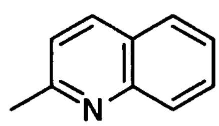 |
| 7 | 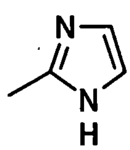 | 14 | 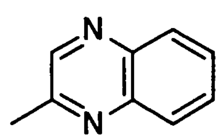 |

Table 22

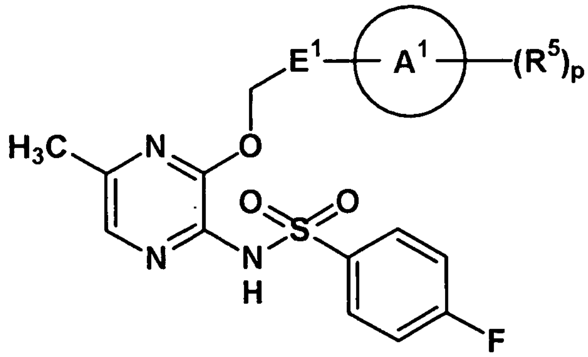

(I－2 2)

| No. | 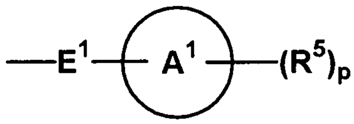 | No. | 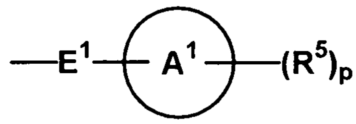 |
|---|---|---|---|
| 1 | 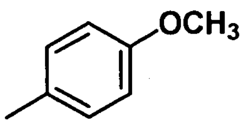 | 8 | 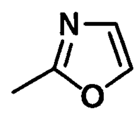 |
| 2 | 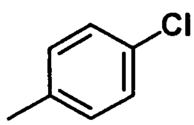 | 9 | 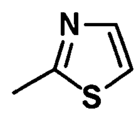 |
| 3 | 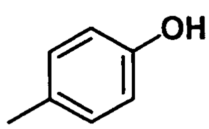 | 10 | 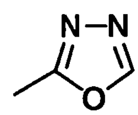 |
| 4 | 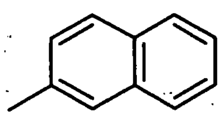 | 11 | 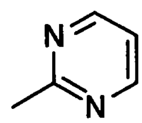 |
| 5 | 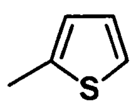 | 12 | 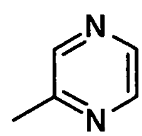 |
| 6 | 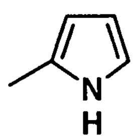 | 13 | 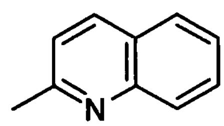 |
| 7 | 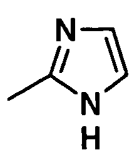 | 14 | 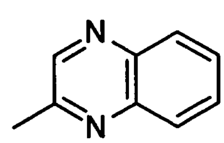 |

Table 23

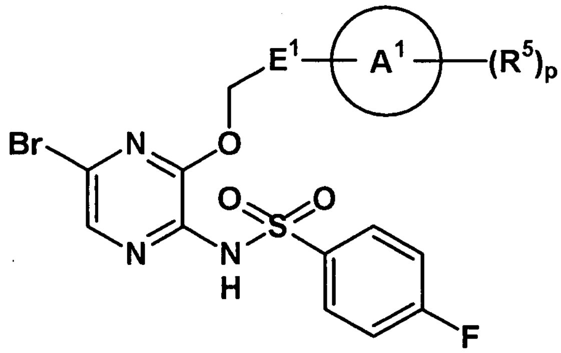

(I－23)

| No. | 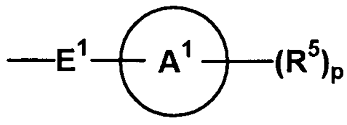 | No. | 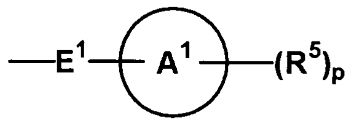 |
|---|---|---|---|
| 1 | 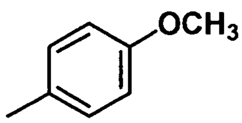 | 8 | 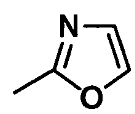 |
| 2 | 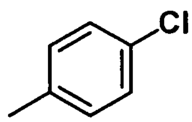 | 9 | 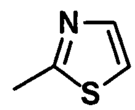 |
| 3 | 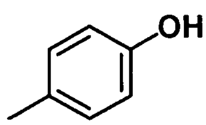 | 10 | 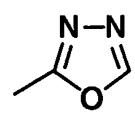 |
| 4 | 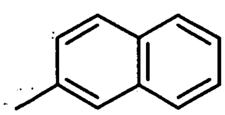 | 11 | 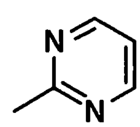 |
| 5 | 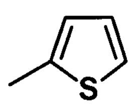 | 12 | 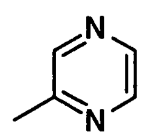 |
| 6 | 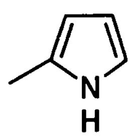 | 13 | 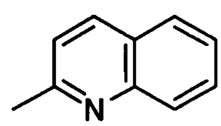 |
| 7 | 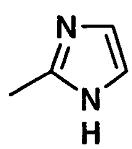 | 14 | 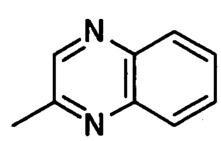 |

Table 24

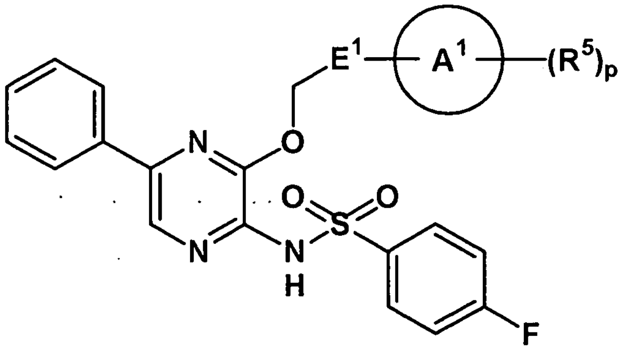

(I－24)

| No. | 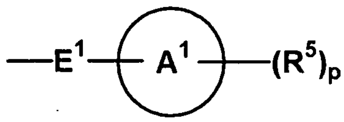 | No. | 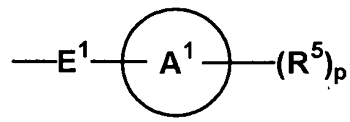 |
|---|---|---|---|
| 1 | 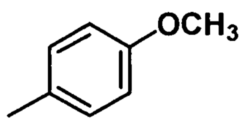 | 8 | 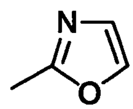 |
| 2 | 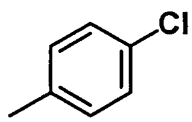 | 9 | 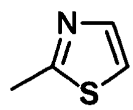 |
| 3 | 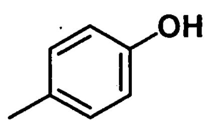 | 10 | 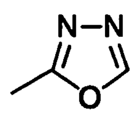 |
| 4 | 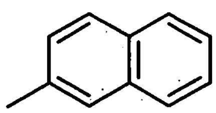 | 11 | 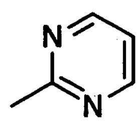 |
| 5 | 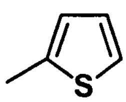 | 12 | 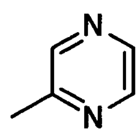 |
| 6 | 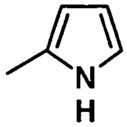 | 13 | 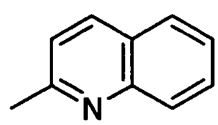 |
| 7 | 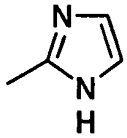 | 14 | 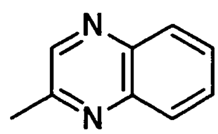 |

Table 25

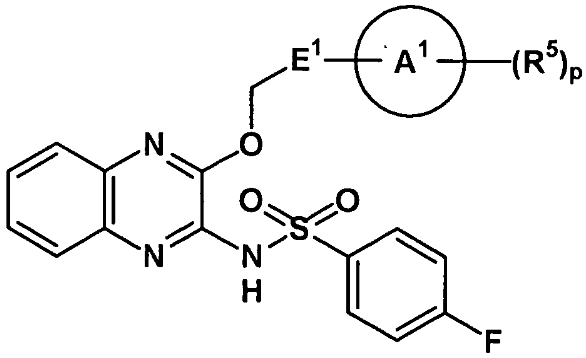

(I－25)

| No. | 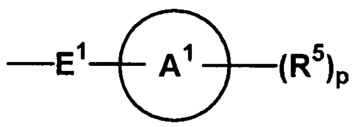 | No. | 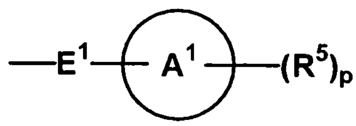 |
|---|---|---|---|
| 1 | 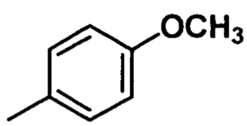 | 8 | 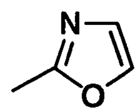 |
| 2 | 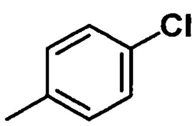 | 9 | 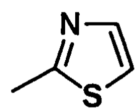 |
| 3 | 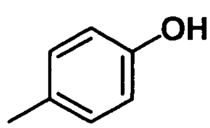 | 10 | 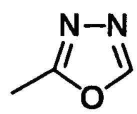 |
| 4 | 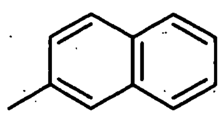 | 11 | 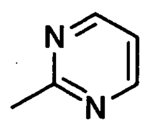 |
| 5 | 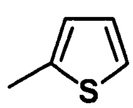 | 12 | 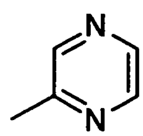 |
| 6 | 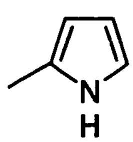 | 13 | 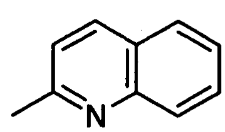 |
| 7 | 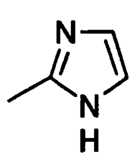 | 14 | 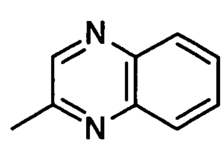 |

Table 26

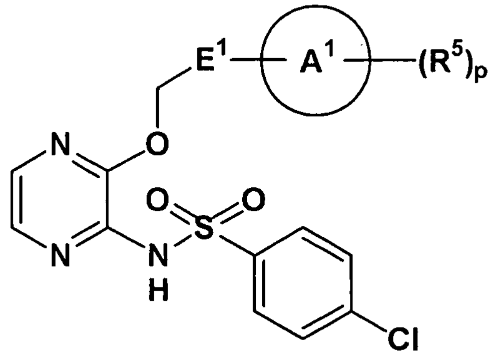

(I－26)

| No. | 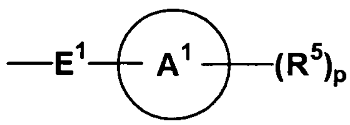 | No. | 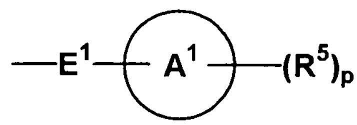 |
|---|---|---|---|
| 1 | 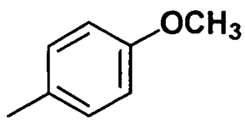 | 8 | 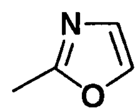 |
| 2 | 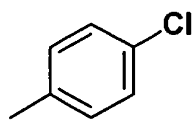 | 9 | 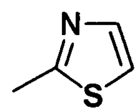 |
| 3 | 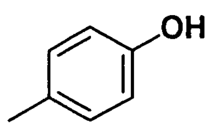 | 10 | 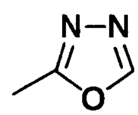 |
| 4 | 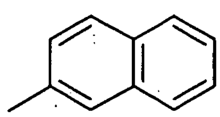 | 11 | 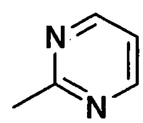 |
| 5 | 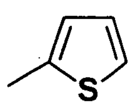 | 12 | 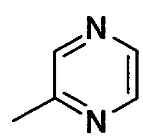 |
| 6 | 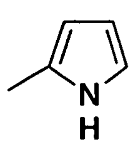 | 13 | 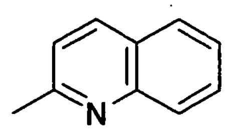 |
| 7 | 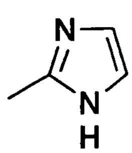 | 14 | 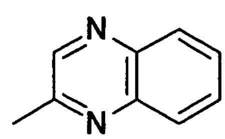 |

Table 27

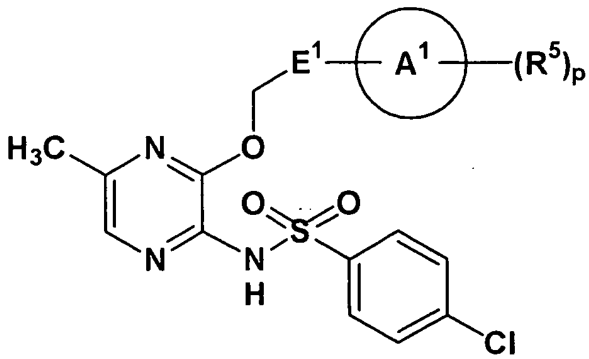

(I－27)

| No. | 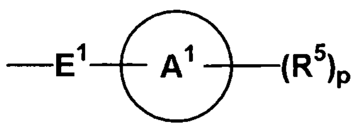 | No. | 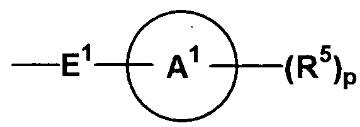 |
|---|---|---|---|
| 1 | 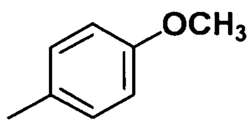 | 8 | 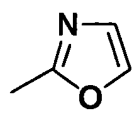 |
| 2 | 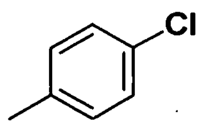 | 9 | 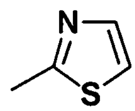 |
| 3 | 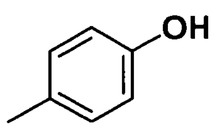 | 10 | 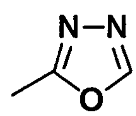 |
| 4 | 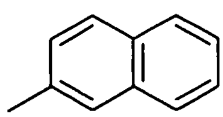 | 11 | 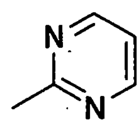 |
| 5 | 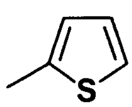 | 12 | 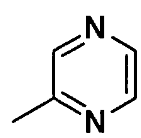 |
| 6 | 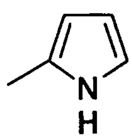 | 13 | 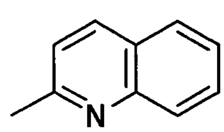 |
| 7 | 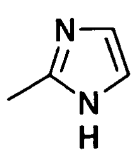 | 14 | 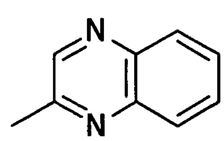 |

Table 28

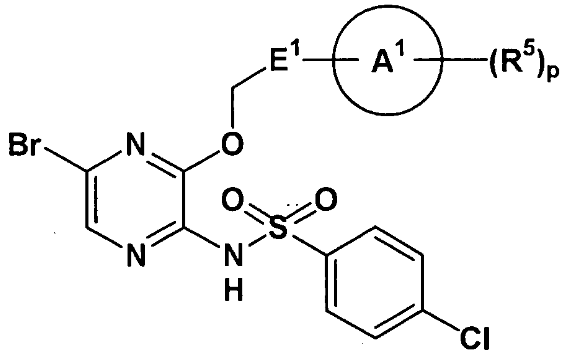

(I－２８)

| No. | 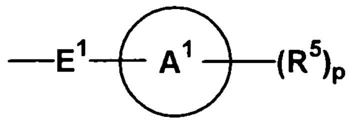 | No. | 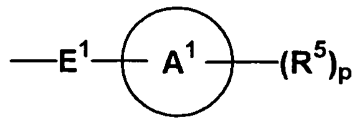 |
|---|---|---|---|
| 1 | 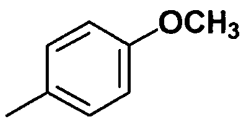 | 8 | 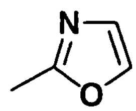 |
| 2 | 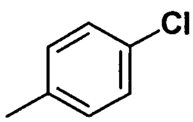 | 9 | 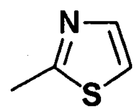 |
| 3 | 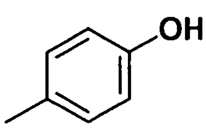 | 10 | 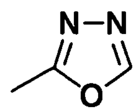 |
| 4 | 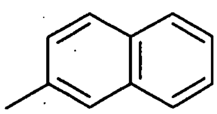 | 11 | 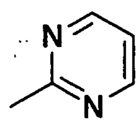 |
| 5 | 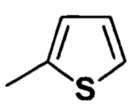 | 12 | 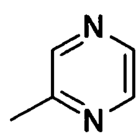 |
| 6 | 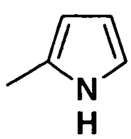 | 13 | 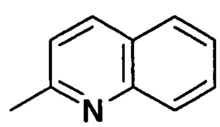 |
| 7 | 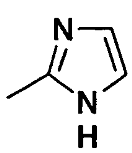 | 14 | 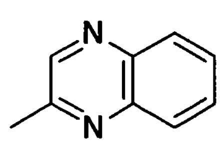 |

Table 29

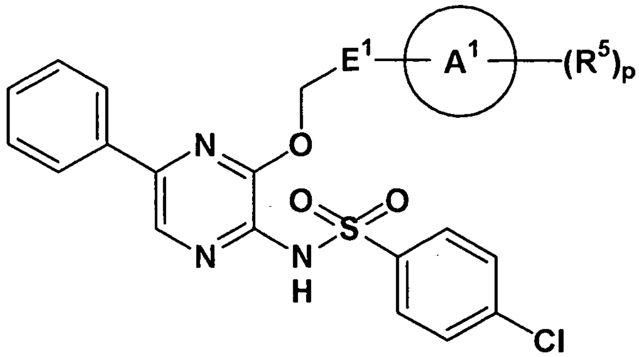

(I－２９)

| No. | 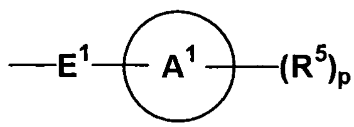 | No. | 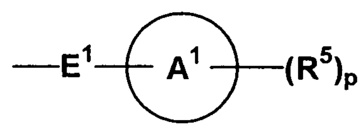 |
|---|---|---|---|
| 1 | 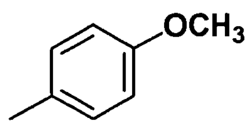 | 8 | 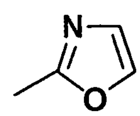 |
| 2 | 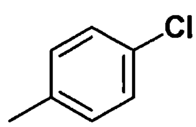 | 9 | 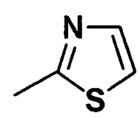 |
| 3 | 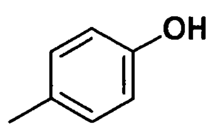 | 10 | 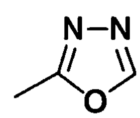 |
| 4 | 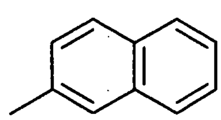 | 11 | 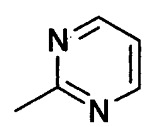 |
| 5 | 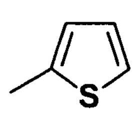 | 12 | 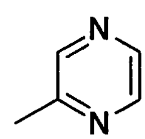 |
| 6 | 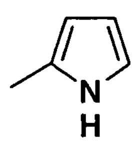 | 13 | 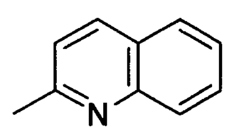 |
| 7 | 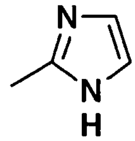 | 14 | 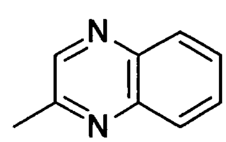 |

Table 30

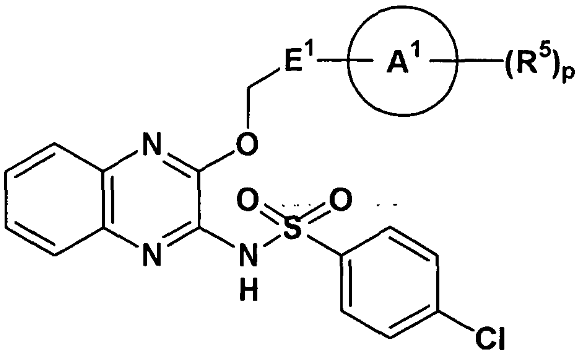

(I－30)

| No. | 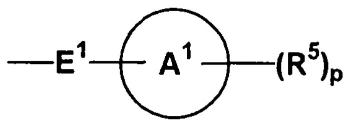 | No. | 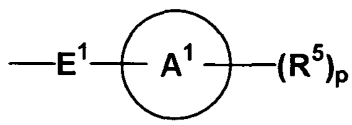 |
|---|---|---|---|
| 1 | 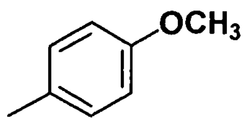 | 8 | 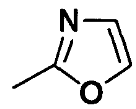 |
| 2 | 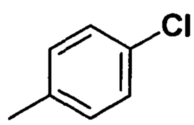 | 9 | 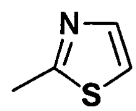 |
| 3 | 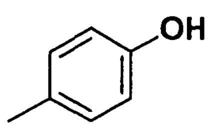 | 10 | 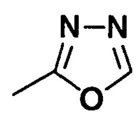 |
| 4 | 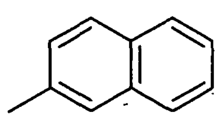 | 11 | 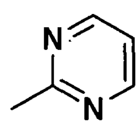 |
| 5 | 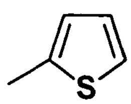 | 12 | 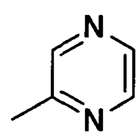 |
| 6 | 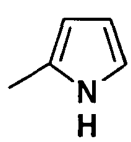 | 13 | 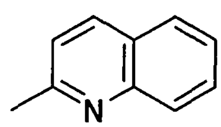 |
| 7 | 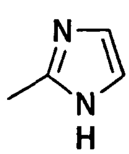 | 14 | 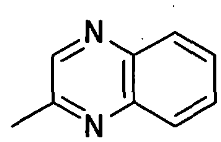 |

Furthermore, in the compounds represented by formula (I-1) to (I-30) as preferable compounds or specific compounds, the substituent represented by

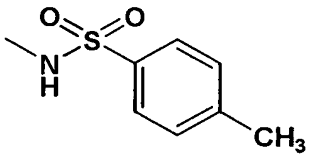 , 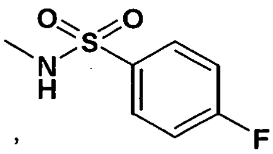 or 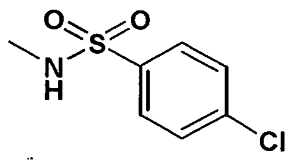

is preferably substituted with (thiophen-2-yl)sulfonylamino, (2,3-dichlorothiophen-5-yl)sulfonylamino, (2,5-dichlorothiophen-3-yl)sulfonylamino, 2,3-dichlorophenylsulfonylamino, 2-methyl-3-chlorophenylsulfonylamino, 2-trifluoromethylphenylsulfonylamino, 2-chlorophenylsulfonylamino, 2-bromophenylsulfonylamino, 2-chloro-4-fluorophenylsulfonylamino, 2,6-dichlorophenylsulfonylamino, 3-bromophenylsulfonylamino, 2,4-difluorophenylsulfonylamino, 2-methylphenylsulfonylamino, 3-chloro-4-methylphenylsulfonylamino, 3-chlorophenylsulfonylamino, 2-fluorophenylsulfonylamino, 2,3,4-trichlorophenylsulfonylamino, 2,4-dichlorophenylsulfonylamino, 2,6-difluorophenylsulfonylamino, 2-cyanophenylsulfonylamino, 2,4,6-trichlorophenylsulfonylamino, phenylsulfonylamino, 3-nitro-4-methylphenylsulfonylamino, 3-nitrophenylsulfonylamino, 4-bromophenylsulfonylamino, 3-methylphenylsulfonylamino, 2,5-difluoro-4-bromophenylsulfonylamino, 3-trifluoromethylphenylsulfonylamino, 2-trifluoromethoxyphenylsulfonylamino, 3-methoxyphenylsulfonylamino, 4-chloro-2,5-dimethylphenylsulfonylamino, 2,4-dichloro-6-methylphenylsulfonylamino, 4-trifluoromethyl-2-chlorophenylsulfonylamino, 2-methyl-4-fluorophenylsulfonylamino, 3-nitro-4-chlorophenylsulfonylamino, 2-methoxycarbonylphenylsulfonylamino, 2-methoxy-5-methylphenylsulfonylamino, 4-ethylphenylsulfonylamino, 2,5-dichlorophenylsulfonylamino, 4-trifluoromethoxysulfonylamino, 2,4,5-trichlorophenylsulfonylamino, 4-(2-propyl) phenylsulfonylamino, 4-(2-methoxyphenyloxy)phenylsulfonylamino, 2-nitro-4-methoxyphenylsulfonylamino, 4-nitrophenylsulfonylamino, 2,5-dimethoxyphenylsulfonylamino, 2-methyl-5-nitrophenylsulfonylamino, 4-butoxyphenylsulfonylamino, 2-methoxy-4-methylphenylsulfonylamino, 2-methoxy-5-butylphenylsulfonylamino, 3,5-dimethylphenylsulfonylamino, 2,3,6-trimethyl-4-methoxyphenylsulfonylamino, 2-methoxy-5-chlorophenylsulfonylamino, 2,4,6-trimethylphenylsulfonylamino, or 4-methoxyphenylsulfonylamino.

Method of producing the compound of the present invention

A compound of formula (I) may be prepared by combining known methods, for Example, a method shown below, methods described in Examples, or methods described in *Comprehensive Organic Transformations: A Guide to Functional Group Preparations*, 2nd Edition (Richard C. Larock, John Wiley & Sons Inc., 1999, or other methods.
Among the compounds of the present invention of formula (I), a compound in which J is bound to ring D via an oxygen atom, i.e., a compound of formula (I-A):

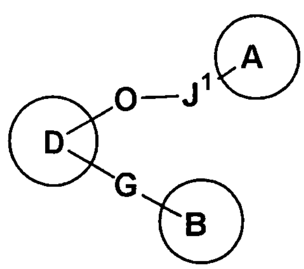

(I-A)

wherein $J^1$ represents a bond or a spacer having 1 to 7 atoms in its main chain, and other symbols have the same meanings as described above; can be prepared by a method of (a-1) or (b-1) shown below.

(a-1): A compound of the present invention of formula (I-A) can be prepared by subjecting a compound of formula (II):

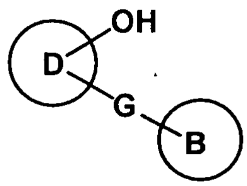

(II)

wherein all symbols have the same meanings as described above;
and a compound represented by formula (III):

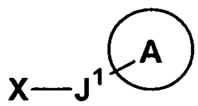

(III)

wherein X represents a leaving group (e.g., halogen, methanesulfonyloxy (OMs), p-toluenesulfonyloxy (OTs), trifluoromethanesulfonyloxy (OTf), *etc.*), and other symbols have the same meanings as described above;
to etherification, and if necessary, to deprotection reaction and/or to cleavage reaction from a resin.

The etherification is carried out by a known method, for Example, by reacting in an organic solvent (*N*,*N*-dimethylformamide, dimethylsulfoxide, chloroform, methylene chloride, diethyl ether, tetrahydrofuran, methyl t-butyl ether, 1,4-dioxane, 1,2-dimethoxyethane, *etc.)* in the presence of a base [alkali metal hydride (sodium hydride, potassium hydride, *etc.*), organometal reagent (*N*-butyl lithium, *etc.*), quaternary ammonium salt (tetrabutylammonium fluoride, *etc.),* or the like] at 0 to 120°C.

The compound of formula (I-A) wherein at least one group has carboxyl, hydroxyl, amino or thiol can be prepared by subjecting the compound in which the respective groups are protected, to deprotection reaction.

A protective group for carboxyl includes, for Example, methyl, an ethyl, an allyl, *t*-butyl, trichloroethyl, benzyl (Bn), phenacyl, and the like.

A protective group for hydroxyl includes, for Example, methyl, trityl, methoxymethyl (MOM), 1-ethoxyethyl (EE), methoxyethoxymethyl (MEM), 2-tetrahydropyranyl (THP), trimethylsilyl (TMS), triethylsilyl (TES), *t*-butyldimethylsilyl (TBDMS), t-butyldiphenylsilyl (TBDPS), acetyl (Ac), pivaloyl, benzoyl, benzyl (Bn), *p*-methoxybenzyl, allyloxycarbonyl (Alloc), 2,2,2-trichloroethoxycarbonyl (Troc), and the like.

A protective group for amino includes, for Example, benzyloxycarbonyl, *t*-butoxycarbonyl, allyloxycarbonyl (Alloc), 1-methyl-1-(4-biphenyl)ethoxycarbonyl (Bpoc), trifluoroacetyl, 9-fluororenylmethoxycarbonyl, benzyl (Bn), *p*-methoxybenzyl, benzyloxymethyl (BOM), 2-(trimethylsilyl)ethoxymethyl (SEM), and the like.

A protective group for thiol includes, for Example, benzyl, methoxybenzyl, methoxymethyl (MOM), 2-tetrahydropyranyl (THP), diphenylmethyl and acetyl (Ac).

A protective group for carboxyl, hydroxyl, amino or thiol is not particularly limited in addition to the above-described groups as long as it can be deprotected easily and selectively. For Example, those described in *Protective Groups in Organic Synthesis* (T. W. Greene, John Wiley & Sons Inc., 1999) may be used.

The deprotection of the protective group for carboxyl, hydroxyl, amino or thiol is well known. For Example, it is

(1) alkaline hydrolysis,
(2) deprotection of a protective group in acidic conditions,
(3) deprotection of a protective group by hydrogenolysis,
(4) deprotection of a protective group containing silyl,
(5) deprotection of a protective group using a metal,
(6) deprotection of a protective group using an organometal, and the like.

In the following, these methods are specifically described:

(1) The deprotection of the protective group by alkaline hydrolysis condition may be carried out, for Example, in an organic solvent (methanol, tetrahydrofuran, 1,4-dioxane, *etc.*) with alkaline metal hydroxide (sodium hydroxide,

potassium hydroxide, lithium hydroxide, *etc.*), alkaline earth metal hydroxide (barium hydroxide, calcium hydroxide, *etc.*), carbonate (sodium carbonate or potassium carbonate, *etc.*), an aqueous solution thereof or a mixture thereof at 0 to 40°C.

(2) The deprotection of the protective group in acidic conditions may be carried out, for Example, in an organic solvent (methylene chloride, chloroform, 1,4-dioxane, ethyl acetate, anisole; *etc.*), organic acid (acetic acid, trifluoroacetic acid, methanesulfonic acid, *p*-toluenesulfonic acid, *etc.*), inorganic acid (hydrochloric acid, sulfuric acid, *etc.*), or a mixture thereof (hydrogen bromide/acetic acid, *etc.*) at 0 to 100°C.

(3) The deprotection of the protective group by hydrogenolysis may be carried out, for Example, in a solvent (ethers (tetrahydrofuran, 1,4-dioxane, 1,2-dimethoxyethane, diethyl ether, *etc.*), alcohols (methanol, ethanol, *etc.*), benzenes (benzene, toluene, *etc.*), ketones (acetone, methylethylketone, *etc.*), nitriles (acetonitrile, *etc.*), amides (*N*,*N*-dimethylformamide, *etc.*), water, ethyl acetate, acetic acid, a mixture of two or more thereof, *etc.*) in the presence of a catalyst (palladium on carbon, palladium black, palladium hydroxide, platinum oxide, Raney nickel, *etc.*) under hydrogen atmosphere at a normal pressure or elevated pressure, or in the presence of ammonium formate at 0 to 200°C.

(4) The deprotection of the protective group for silyl may be carried out, for Example, in an organic solvent (tetrahydrofuran, acetonitrile, *etc.*), with fluoride (tetrabutylammonium fluoride, an aqueous solution of hydrofluoride, hydrofluoride-pyridine complex, *etc.*) at -20 to 40°C.

(5) The deprotection of the protective group a using metal may be carried out, for Example, in an acidic solvent (acetic acid, a pH 4.2 to 7.2 buffer, a mixed solution of the buffer and an organic solvent such as tetrahydrofuran, *etc.*) in the presence of powder zinc, with or without an ultrasonic wave at a temperature of 0 to 40°C.

(6) The deprotection of the protective group using a metal complex may be carried out, for Example, in an organic solvent (methylene chloride, *N*,*N*-dimethylformamide, tetrahydrofuran, ethyl acetate, acetonitrile, 1,4-dioxane, ethanol, *etc.*), water or a mixed solvent thereof in the presence of a trap reagent (tributyltin hydride, triethylsilane, dimedone, morpholine, diethylamine, pyrrolidine, *etc.*), an organic acid (acetic acid, formic acid, 2-ethylhexanic acid, *etc.*) and/or an organic acid salt (sodium 2-ethylhexanate, potassium 2-ethylhexanate, etc)in the presence or absence of a phosphine reagent (triphenylphosphine, *etc.*) using a metal complex (tetrakis(triphenylphosphine) palladium (O), dichlorobis(triphenylphosphine)palladium (II), palladium acetate (II), chlorotris(triphenylphosphine) rhodium (I), *etc.*) at 0 to 40°C.

In addition to the above methods, the deprotection may be carried out by the method described in *Protective Groups in Organic Synthesis* (T. W. Greene, John Wiley & Sons Inc., 1999).

Furthermore, if the compound has a moiety to bind to a resin in the molecule, and the resin is a polystyrene resin, the compound of the present invention can be cleaved from the resin by the following method. The cleavage reaction from the resin may be carried out by a known method, for Example, by reacting in an organic solvent (methylene chloride, 1,2-dichloroethane, toluene, *etc.*), with acid (acetic acid, trifluoroacetic acid, hydrochloric acid, *etc.*) at 0 to 100°C.

As well understood to the skilled persons in the art, the objective compounds of the present invention may be prepared easily by using these deprotection reactions.

Furthermore, if necessary, after those reactions, a procedure of converting the compound into the objective non-toxic salts may be carried out according to a known method.

(b-1): A compound of the present invention of formula (I-A) can be prepared by subjecting a compound of formula (IV):

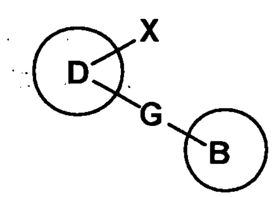

(IV)

wherein all symbols have the same meanings as described above;
and a compound of formula (V):

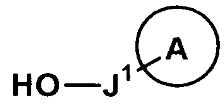

(V)

wherein all symbols have the same meanings as described above;
to the same reaction as those in above-described (a-1), and if necessary, to deprotection reaction and/or to cleavage reaction from a resin. The deprotection of the protective group can be carried out in the same manner as those in the above-described method. Furthermore, if the compound has a moiety to bind to a resin in the molecule, and the resin is a polystyrene resin, the compound of the present invention can be cleaved from the resin in the same manner as those in the above-described method.

Among the compounds of the present invention of formula (I), a compound in which G is bound to ring D via $-NHSO_2-$, i.e., a compound of formula (I-B):

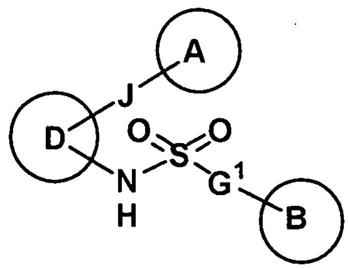

(I-B)

wherein $G^1$ represents a bond or a spacer having 1 or 2 atoms in its main chain, and other symbols have the same meanings as described above;
can be prepared by a method of (a-2) or (b-2) shown below.

(a-2): A compound of the present invention of formula (I-B) can be prepared by subjecting a compound of formula (VI):

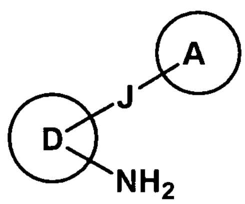

(VI)

wherein all symbols have the same meanings as described above,
and a compound of formula (VII):

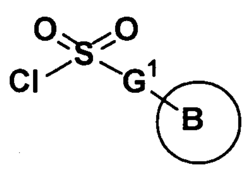

(VII)

wherein all symbols have the same meanings as described above;
to sulfonamidation.

The sulfonamidation may be carried out, for Example, by reacting in an organic solvent (chloroform, methylene chloride, 1,2-dichloroethane, diethyl ether, tetrahydrofuran, *etc.*) in the presence of a base (diisopropylethylamine, pyridine, triethylamine, *N*,*N*-dimethylaniline, *N*,*N*-dimethylaminopyridine, sodium hydride, potassium hydride, *etc.*) at 0 to 40°C.

The compound of formula (I-B) wherein at least one group contains carboxyl, hydroxyl, amino or thiol can be prepared by subjecting the compound in which the respective groups are protected, to deprotection reaction. The deprotection of the protective group can be carried out in the same manner as those in the above-described method. Furthermore, if the compound has a moiety to bind to a resin in the molecule, and the resin is a polystyrene resin, the compound of the present invention can be cleaved from the resin in the same manner as those in the above-described method.

(b-2): A compound of the present invention of formula (I-B) can be prepared by subjecting a compound of formula (VIII):

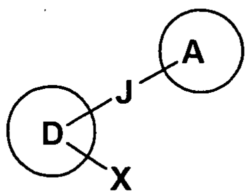

(VIII)

wherein all symbols have the same meanings as described above;
and a compound represented by formula (IX):

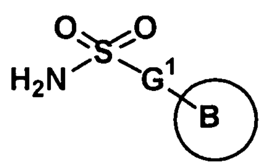

(IX)

wherein all symbols have the same meanings as described above;
to reaction, and if necessary, to deprotection reaction and/or to cleavage reaction from a resin.

This reaction may be carried out by a known method, for Example, by reacting in an organic solvent (*N*,*N*-dimethylformamide, *N*,*N*-dimethylacetamide, dimethylsulfoxide, *etc.*) in the presence or absence of a base (potassium carbonate, cesium carbonate, triethylamine, *N*-butyl lithium, sodium hydride, sodium hydroxide, *etc.*) at 0 to 200°C.

The deprotection of the protective group can be carried out in the same manner as those in the above-described method. Furthermore, if the compound has a moiety to bind to a resin in the molecule, and the resin is a polystyrene resin, the compound of the present invention can be cleaved from the resin in the same manner as those in the above-described method.

Among the compounds of the present invention of formula (I), a compound in which G is bound to ring D via -NHCO-, i.e., a compound of formula (I-C):

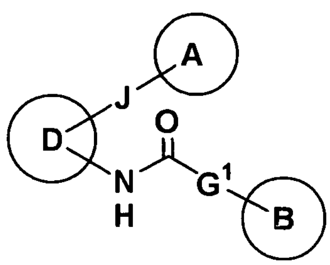

(I-C)

wherein all symbols have the same meanings as described above;
can be prepared by subjecting a compound of formula (VI) and a compound represented by formula (X):

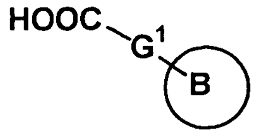 (X)

wherein all symbols have the same meanings as described above;
to amidation, and if necessary, to deprotection reaction and/or to cleavage reaction from a resin.

The amidation is well known, and it includes, for Example,

(1) a method using an acyl halide,
(2) a method using a mixed acid anhydride, and
(3) a method using a condensing agent, and the like.

In the following, these methods are explained in detail.

(1) The method using an acyl halide may be carried out, for Example, by reacting carboxylic acid with acyl halide (e.g., oxalyl chloride, thionyl chloride, *etc.*) in an organic solvent (e.g., chloroform, methylene chloride, diethyl ether, tetrahydrofuran, *etc.*) or without a solvent at -20°C to reflux temperature. And then the obtained acyl halide derivative may be reacted with amine in an inert organic solvent (e.g., chloroform, methylene chloride, diethyl ether, tetrahydrofuran, *etc.*) in the presence of a base (e.g., pyridine, triethyl amine, *N*,*N*-dimethyl aniline, *N*,*N*-dimethylaminopyridine, diisopropylethylamine, *etc.)* at 0 to 40°C. Alternatively, the obtained acyl halide derivative may be reacted with amine in an organic solvent (e.g., 1,4-dioxane, tetrahydrofuran, *etc.*) using an alkaline aqueous solution (e.g., sodium bicarbonate, sodium hydroxide, *etc.*) at 0 to 40°C.
(2) The method using a mixed acid anhydride may be carried out, for Example, by reacting carboxylic acid with acyl halide (e.g., pivaloyl chloride, *p*-toluenesulfonyl chloride, methanesulfonyl chloride, *etc.*) or an acid derivative (e.g., ethyl chloroformate, isobutyl chloroformate, *etc.*) in an organic solvent (e.g., chloroform, methylene chloride, diethyl ether, tetrahydrofuran, *etc.*) or without a solvent in the presence of a base (e.g., pyridine, triethylamine, *N*,*N*-dimethylaniline, *N*,*N*-dimethylaminopyridine, diisopropylethylamine, *etc.*) at 0 to 40°C. And then the obtained mixed acid anhydride derivative may be reacted with amine in an organic solvent (e.g., chloroform, methylene chloride, diethyl ether, tetrahydrofuran, *etc.*) at 0 to 40°C.
(3) The method using a condensing agent may be carried out, for Example, by reacting carboxylic acid with amine in an organic solvent (e.g., chloroform, methylene chloride, *N*,*N*-dimethylformamide, diethyl ether, tetrahydrofuran, *etc.*) or without a solvent in the presence or absence of a base (e.g., pyridine, triethylamine, *N*,*N*-dimethylaniline, *N*,*N*-dimethylaminopyridine, *etc.*), using a condensing agent (e.g., 1,3-dicyclohexyl carbodiimide (DCC), 1-ethyl-3-[3-(dimethylamino)propyl]carbodiimide (EDC), 1,3-diisopropylcarbodiimide (DIC), 1,1'-carbodiimidazole (CDI), 2-chloro-1-methylpyridinium iodide, 1-propanephosphonic acid cyclic anhydride (PPA), *etc.*) in the presence or absence of 1-hydroxybenzotriazole (HOBt) at 0 to 40°C.

The reactions described in (1), (2) and (3) may be carried out under an atmosphere of an inert gas (e.g., argon, nitrogen, *etc.*) on anhydrous condition.

The compound of formula (I-C) in which at least one group contains carboxyl, hydroxyl, amino or thiol can be prepared by subjecting the compound in which the respective groups are protected, to deprotection reaction. The deprotection of the protective group can be carried out in the same manner as those in the above-described method. Furthermore, if the compound has a moiety to bind to a resin in the molecule, and the resin is a polystyrene resin, the compound of the present invention can be cleaved from the resin in the same manner as those in the above-described method.

Among the compounds of the present invention of formula (I), a compound in which J is a bond or bound to ring D via carbon, i.e., a compound of formula (I-D):

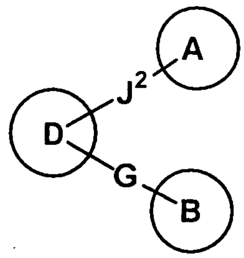

(I-D)

wherein $J^2$ is the same as J, wherein the atoms which bind to the bond or ring D are carbon atoms, and other symbols have the same meanings as described above;
can be prepared by a method of (a-3) or (b-3) shown below.

(a-3): A compound of the present invention of formula (I-D) can be prepared by subjecting a compound of formula (IV) and a compound of formula (XI):

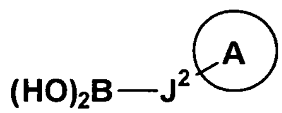

(XI)

wherein all symbols have the same meanings as described above;
to reaction, and if necessary, to deprotection reaction and/or to cleavage reaction from a resin.

The reaction of the compound of formula (IV) and the compound of formula (XI) may be carried out by a known method, for Example, by reacting in an organic solvent (benzene, toluene, *N*,*N*-dimethylformamide, 1,4-dioxane, tetrahydrofuran, methanol, acetonitrile, 1,2-dimethoxyethane, acetone, *etc.*) in the presence of a base (sodium ethylate, sodium hydroxide, potassium hydroxide, triethylamine, sodium carbonate, sodium hydrocarbonate, potassium carbonate, cesium carbonate, thallium carbonate, tripotassium phosphate, cesium fluoride, barium hydroxide, tetrabutylammonium fluoride, *etc.*) or an aqueous solution thereof, or a mixture thereof and a catalyst (tetrakis(triphenylphosphine) palladium ($Pd(PPh_3)_4$), dichlorobis(triphenylphosphine)palladium ($Pd(Cl_2(PPh_3)_2)$, palladium acetate ($Pd(OAc)_2$), palladium black, 1,1'-bis(diphenylphosphinoferrocene)dichloropalladium ($PdCl_2(dppf)_2$), dichlorodiallylpalladium ($PdCl_2(allyl)_2$), phenylbis(triphenylphosphine)palladium iodide ($PhPdI(PPh_3)_2$), *etc.*) at 10 to 120°C.

The deprotection reaction and the cleavage reaction from the resin can be carried out in the same manner as those in the above-described method.

(b-3): A compound of the present invention of formula (I-D) can be prepared by subjecting the compound of formula (XII):

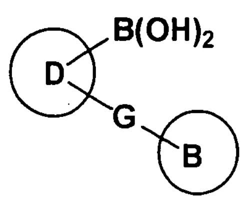

(XII)

wherein all symbols have the same meanings as described above;
and the compound of formula (XIII):

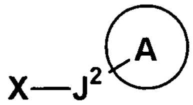

(XIII)

wherein all symbols have the same meanings as described above;
to the same reaction as those above-described for the compound of formula (IV) and the compound of formula (XI), and if necessary, to deprotection reaction and/or to cleavage reaction from a resin.

The deprotection reaction and the cleavage reaction from the resin can be carried out in the same manner as those in the above-described method.

Among the compounds of the present invention of formula (I), a compound in which G is a bond or bound to ring D via carbon, i.e., a compound of formula (I-E):

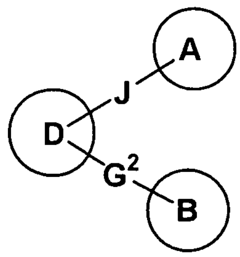

(I-E)

wherein $G^2$ is the same as G, wherein G is a bond or the atom which binds to ring D is carbon, and other symbols have the same meanings as described above;
can be prepared by a method of (a-4) or (b-4) shown below.

(a-4): A compound of the present invention of formula (I-E) can be prepared by subjecting a compound of formula (VIII) and a compound of formula (XIV):

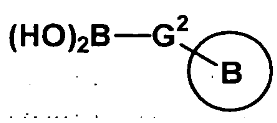

(XIV)

wherein all symbols have the same meanings as described above;
to the same reaction as those above-described for the compound of formula (IV) and the compound of formula (XI), and if necessary, to deprotection reaction and/or to cleavage reaction from a resin.

The deprotection reaction and the cleavage reaction from the resin can be carried out in the same manner as those in the above-described method.

(b-4): A compound of the present invention of formula (I-E) can be prepared by subjecting a compound of formula (XV):

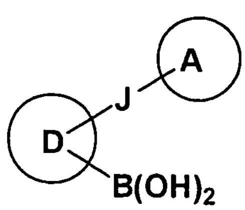

(XV)

wherein all symbols have the same meanings as described above;
and a compound of formula (XVI):

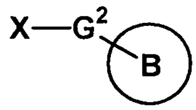

(XVI)

wherein all symbols have the same meanings as described above, to the same reaction as those above-described for the compound of formula (IV) and the compound of formula (XI), and if necessary, to deprotection reaction and/or to cleavage reaction from a resin.

The deprotection reaction and the cleavage reaction from the resin can be carried out in the same manner as those in the above-described method.

Among the compounds of the present invention of formula (I), a compound in which one of substituents in ring D is C1-8, C2-8 alkenyl or C2-8 alkynyl, i.e., a compound of formula (I-F):

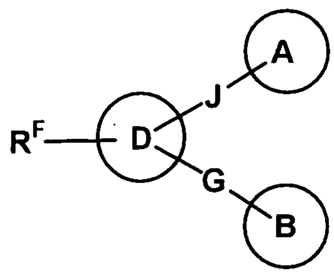

(I-F)

wherein $R^F$ represents C1-8 alkyl, C2-8 alkenyl or C2-8 alkynyl, and other symbols have the same meanings as described above;
can be prepared by subjecting a compound of formula (XVII):

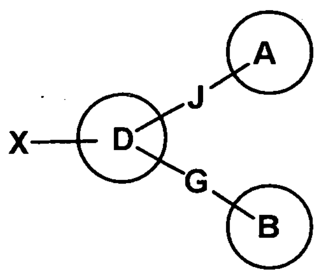

(XVII)

wherein all symbols have the same meanings as described above;
to alkylation reaction, and if necessary, to deprotection reaction and/or to cleavage reaction from a resin.

The alkylation reaction may be carried out by a known method, for Example, by reacting in an organic solvent (tetrahydrofuran, diethyl ether, *etc.*) in the presence of an organometal reagent (methylmagnesium bromide, n-butyl lithium, ethynylmagnesium bromide, *etc.*) and a catalyst ([1,3-bis(diphenylphosphino)propane]dichloro nickel (II) ($NiCl_2$ (dppp)), *etc.*) at 0 to 40°C.

The deprotection reaction and the cleavage reaction from the resin can be carried out in the same manner as those in the above-described method.

Among the compounds of the present invention of formula (I), a compound in which one of substituents in ring D is a cyclic group which may be substituted, i.e., the compound of formula (I-G):

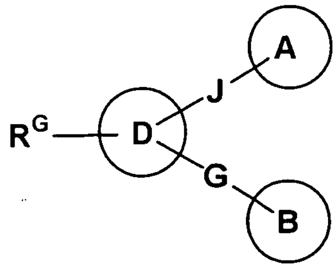

(I-G)

wherein $R^G$ represents a cyclic group which may be substituted, and other symbols have the same meanings as described above;
can be prepared by subjecting a compound of formula (XVII) and the compound of formula (XVIII):

$$R^G—B(OH)_2 \qquad (XVIII)$$

wherein all symbols have the same meanings as described above;
to the same reaction as those above-described for the compound of formula (IV) and the compound of formula (XI), and if necessary, to deprotection reaction and/or to cleavage reaction from a resin.

The deprotection reaction and the cleavage reaction from the resin can be carried out in the same manner as those in the above-described method.

Among the compounds of the present invention of formula (I), a compound in which one of substituents in ring D represents $COOR^{a1}$, and $R^{a1}$ represents a group other than hydrogen, i.e., a compound of formula (I-H):

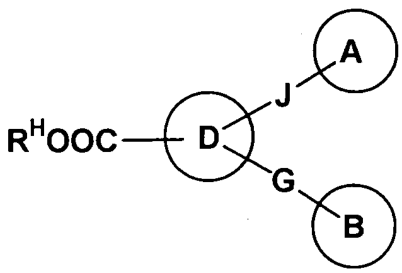

(I-H)

wherein $R^H$ is the same as $R^{a1}$ except hydrogen, and other symbols have the same meanings as described above;
can be prepared by subjecting a compound of formula (XVII) and a compound of formula (XIX)

$$R^H\text{-}OH \qquad (XIX)$$

wherein all symbols have the same meanings as described above;
to reaction under an atmosphere of carbon monoxide gas, and if necessary, to deprotection reaction and/or to cleavage reaction from a resin.

The reaction of the compound of formula (XVII) with the compound of formula (XIX) may be carried out by a known method, for Example, by reacting in an organic solvent (*N*,*N*-dimethylformamide, dimethyl sulfoxide, tetrahydrofuran, 1,2-dimethoxyethane, *etc.*) in the presence of a base (triethylamine, diisopropylethylamine, *N*-methylmorpholine, *etc.*) and a catalyst (tetrakis(triphenylphosphine)palladium ($Pd(PPh_3)_4$), dichlorobis(triphenylphosphine)palladium ($PdCl_2(PPh_3)_2$), palladium acetate ($Pd(OAc)_2$), palladium black, 1,1'-bis(diphenylphosphinoferrocene)dichloropalladium ($PdCl_2(dppf)_2$), dichlorodiallylpalladium ($PdCl_2(allyl)_2$), phenylbis(triphenylphosphine)palladium iodide ($PhPdI(PPh_3)_2$), *etc.*) at 10 to 120°C under an atmosphere of carbon monoxide gas.

The deprotection reaction and the cleavage reaction from the resin can be carried out in the same manner as those in the above-described method.

Among the compounds of the present invention of formula (I), a compound in which one of substituents in ring D is COOH, i.e., a compound of formula (I-I):

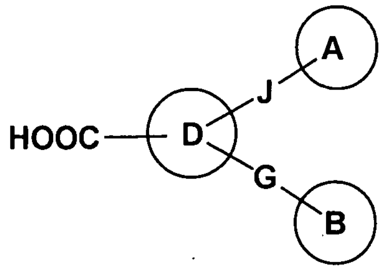

(I-I)

wherein all symbols have the same meanings as described above;
can be prepared by a method of (a-5) or (b-5) shown below.

(a-5): A compound of the present invention of formula (I-I) can be prepared by subjecting the compound of formula (I-H) to deprotection reaction.

The deprotection of the protective group can be carried out in the same manner as those in the above-described method.

(b-5): A compound of the present invention of formula (I-I) can be prepared by subjecting a compound of formula (XVII) and 2-trimethylsilylethanol of formula (XX):

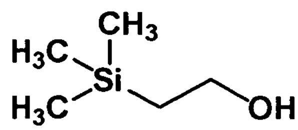

(XX)

to reaction in the presence of carbon monoxide gas, and if necessary, to deprotection reaction and/or to cleavage reaction from a resin.

The reaction between the compound of formula (XVII) and the compound of formula (XX) may be carried out in the same manner as those above-described in the reaction between the compound of formula (XVII) and the compound of formula (XIX).

The deprotection reaction and the cleavage reaction from the resin can be carried out in the same manner as those in the above-described method.

Among the compounds of the present invention of formula (I), a compound in which one of substituents in ring D represents $CONR^{a1}R^{a2}$, i.e., a compound of formula (I-J):

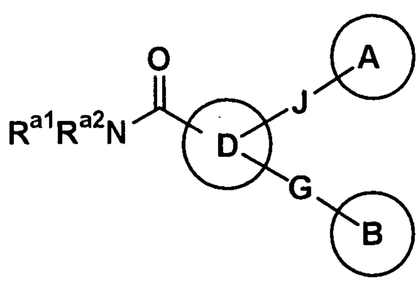

(I-J)

wherein all symbols have the same meanings as described above;
can be prepared by subjecting the compound of formula (I-I) prepared by above-described method and the compound of formula (XXI):

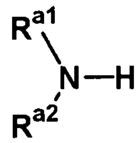

(XXI)

wherein all symbols have the same meanings as described above;
to amidation which is the same reaction used for synthesizing the compound of formula (I-C), and if necessary, to deprotection reaction and/or to cleavage reaction from a resin.

The deprotection reaction and the cleavage reaction from the resin can be carried out in the same manner as those in the above-described method.

Among the compounds of the present invention of formula (I), a compound in which one of substituents in ring D is $CH_2OH$, i.e., a compound of formula (I-K):

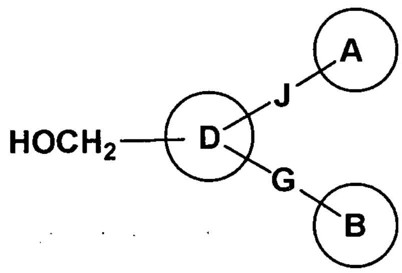

(I-K)

wherein all symbols have the same meanings as described above;
can be prepared by subjecting the compound of formula (I-H) or the compound of formula (I-I) which was prepared by above-described method, to reduction reaction, and if necessary, to deprotection reaction and/or to cleavage reaction from a resin.

The reduction reaction may be carried out by a known method, for Example, by reacting in an organic solvent (tetrahydrofuran, diethyl ether, *etc.*) in the presence of a reducing agent (sodium borohydride, lithium borohydride, aluminum lithium hydride, diisobutyl aluminum hydride, a boran-dimethylsulfide complex, *etc.*) at -20 to 100°C.

The deprotection reaction and the cleavage reaction from the resin can be carried out in the same manner as those in the above-described method.

Among the compounds of the present invention of formula (II), a compound in which G is bound to ring D via $-NHSO_2-$, i.e., a compound of formula (II-B):

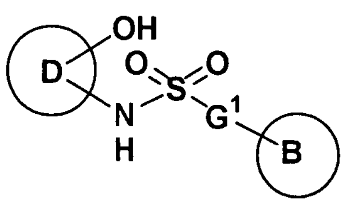

(II-B)

wherein all symbols have the same meanings as described above;
can be prepared by subjecting a compound represented by formula (XXII):

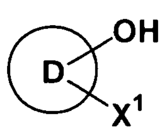

(XXII)

wherein $X^1$ is the same as X, and other symbols have the same meanings as described above;
and the compound of formula (IX) to the same reaction as those above-described in (b-2) and if necessary, to deprotection reaction and/or to cleavage reaction from a resin.

The deprotection reaction and the cleavage reaction from the resin can be carried out in the same manner as those in the above-described method.

Among the compounds of the present invention of formula (IV), a compound in which G is bound to ring D via $-NHSO_2-$, i.e., a compound of formula (IV-B):

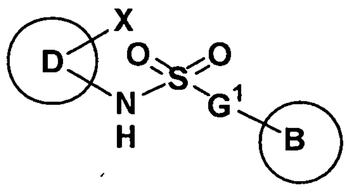

(IV-B)

wherein all symbols have the same meanings as described above;
can be prepared by subjecting a compound represented by formula (XXIII):

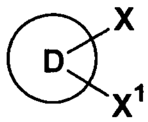

(XXIII)

wherein all symbols have the same meanings as described above;
and the compound of formula (IX) to the same reaction as those above-described in (b-2) and if necessary, to deprotection reaction and/or to cleavage reaction from a resin.

The deprotection reaction and the cleavage reaction from the resin can be carried out in the same manner as those in the above-described method.

Among the compounds of the present invention of formula (XII), a compound in which G is bound to ring D via $-NHSO_2-$, i.e., a compound of formula (XII-B):

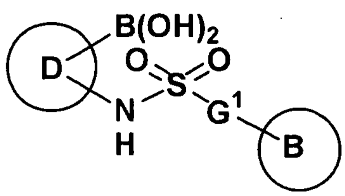

(XII-B)

wherein all symbols have the same meanings as described above;
can be prepared by subjecting a compound of formula (XXIV):

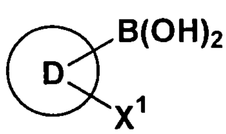

(XXIV)

wherein all symbols have the same meanings as described above;
and the compound of formula (IX) to the same reaction as those above-described in (b-2) and if necessary, to deprotection reaction and/or to cleavage reaction from a resin.

The deprotection reaction and the cleavage reaction from the resin can be carried out in the same manner as those in the above-described method.

Among the compounds of the present invention of formula (VI), a compound in which J is bound to ring D via oxygen, i.e., a compound of formula (VI-B):

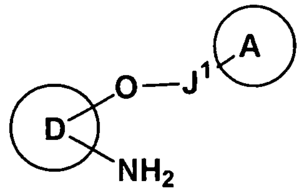

(VI-B)

wherein all symbols have the same meanings as described above;
can be prepared by subjecting a compound of formula (XXV):

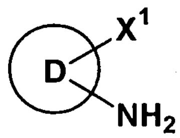

(XXV)

wherein all symbols have the same meanings as described above;
and the compound of formula (V) to the same reaction as those above-described in (a-1) and if necessary, to deprotection reaction and/or to cleavage reaction from a resin.

The deprotection reaction and the cleavage reaction from the resin can be carried out in the same manner as those in the above-described method.

Among the compounds of the present invention of formula (VIII), a compound in which J is bound to ring D via an oxygen atom, i.e., a compound of formula (VIII-B):

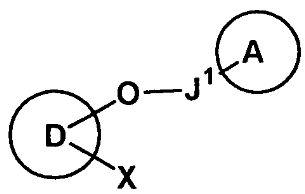

(VIII-B)

wherein all symbols have the same meanings as described above;
can be prepared by subjecting the compound of formula (XXIII) and the compound of formula (V) to the same reaction as those above-described in (a-1) and if necessary, to deprotection reaction and/or to cleavage reaction from a resin.

The deprotection reaction and the cleavage reaction from the resin can be carried out in the same manner as those in the above-described method.

Among the compounds of the present invention of formula (XV), a compound in which J is bound to ring D via an oxygen atom, i.e., a compound of formula (XV-B):

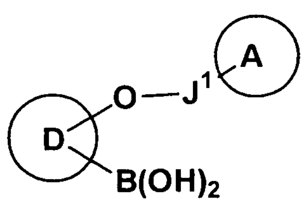

(XV-B)

wherein all symbols have the same meanings as described above;
can be prepared by subjecting the compound of formula (XXIV) and the compound of formula (V) to the same reaction as those above-described in (a-1) and if necessary, to deprotection reaction and/or to cleavage reaction from a resin.

The deprotection reaction and the cleavage reaction from the resin can be carried out in the same manner as those in the above-described method.

Other compounds of formulae (II) to (XXV) used as starting materials or reagents are known per se or may be prepared by known methods, for Example, or methods described in *Comprehensive Organic Transformations: A Guide to Functional Group Preparations,* 2nd Edition (Richard C. Larock, Jolm Wiley & Sons Inc., 1999).

In each reaction in the present specification, as apparent to the skilled persons in the art, the reactions involving heating may be carried out using a water bath, an oil bath, a sand bath or a microwave.
In each reaction in the present specification, a solid-supported reagent which is supported on a high molecular polymer (e.g., polystyrene, polyacrylamide, polypropylene, polyethylene glycol, *etc.*) may be suitably used.
In each reaction in the present specification, the reaction products may be purified by a conventional purification method, for Example, by distillation at a normal or reduced pressure, high performance liquid chromatography using silica gel or magnesium silicate, thin layer chromatography, ion-exchange resin, scavenger resin, column chromatography, washing or recrystallization. The purification may be done after each reaction or after several reactions.
In the reactions using a polystyrene resin in the present specification, the reaction products may be purified by a conventional purification method, for Example, by washing with a solvent (*N*,*N*-dimethylformamide, methylene chloride, methanol, tetrahydrofuran, toluene, acetic acid/toluene, *etc.*) several times.

Pharmacological activities

Pharmacological tests in addition to those described in Experimental Examples include the followings. As shown below, CCR4 antagonistic activity *in vitro* of the compound of the present invention can be demonstrated, and also efficacy *in vivo* can be confirmed.
As a system for screening a CCR4 antagonist, for Example, a system for measuring effects for the transient increase of Ca ions induced by mediation of CCR4 ligands other than MDC, for Example, TARC, *etc.* can be conducted since CCR4 is a seven transmembraneous G protein-coupled type receptor. Furthermore, CCR4 antagonistic activity also can be demonstrated by the method described in WO2002/30357, WO2002/30358 or WO2002/94264, or a modification thereof, and these methods can be also used as a screening method. Furthermore, these publications also disclose experimental methods using animals, and therefore according to the methods or a modification thereof, the efficacy of a CCR4 antagonist *in vivo* model can be confirmed.

Toxicity

The toxicity of the compound of the present invention is very low, and it is believed that the compound is safe enough for pharmaceutical use.

INDUSTRIAL APPLICABILITY

Application to Pharmaceuticals

Since the compound of the present invention of formula (I) has CCR4 antagonistic activity in animals including human, especially human, it is considered that it is useful as a preventive and/or therapeutic agent for diseases associated with CCR4, i.e., CCR4-mediated diseases such as inflammatory and/or allergic diseases [e.g., systemic inflammatory response syndrome (SIRS), anaphylaxis or anaphylaxis-like reaction, allergic vasculitis, organ graft rejection, hepatitis, nephritis, nephrosis, pancreatitis, rhinitis, arthritis, inflammatory ocular diseases (e.g., conjunctivitis, *etc.*), inflammatory bowel diseases (e.g., ulcerative colitis, Crohn's disease, eosinophilic gastroenteropathy, *etc.*), diseases in cerebro and/or circulatory system (e.g., atherosclerosis, thrombosis, ischemic/reperfusion disorders, restenosis, infarction, *etc.*), respiratory diseases (e.g., acute respiratory distress syndrome (ARDS), asthma, allergic bronchopulmonary aspergillosis, *etc.*), dermatic diseases [e.g., dermatitis (e.g., atopic dermatitis, psoriasis, contact dermatitis, eczema, urticaria, pruritis, *etc.*), and the like], autoimmune diseases (e.g., multiple sclerosis, chronic rheumatoid arthritis, systemic erythematosus, Type I diabetes mellitus, glomerular nephritis, Sjogren's syndrome, *etc.*), metabolism and/or endocrinologic diseases (e.g., diabetes mellitus, *etc.*), cancer diseases [for Example, malignant neoplasm (e.g., leukemia, cancer and cancer metastasis, *etc.*), and the like], infections [for Example, viral diseases (e.g., acquired immunodeficiency syndrome, severe acute respiratory syndrome (SARS), and the like], and the like.
Furthermore, the compound of the present invention represented by formula (I) has activity of regulating a TNF$\alpha$ amount in the living body, especially in the blood, i.e., activity of regulating TNF$\alpha$, more specifically, activity of suppressing TNF$\alpha$ production. In addition, the compound of the present invention has activity of inhibiting the effector cell functions (e.g., migration, *etc.*) of expressing CCR4, i.e., inhibitory activity for effector cell functions. Therefore, the compound of the present invention is considered to be useful as a preventive and/or therapeutic agent for diseases which is suggested to be associated with CCR4, and diseases which is suggested to be associated with the effector cells, especially the above-described disease group.
A combination agent obtained by combining the compound of formula (I) or a non-toxic salt thereof with other medicaments may be administered to accomplish the following purposes:

1) to supplement and/or enhance the preventive and/or therapeutic effect of the present compound;
2) to improve the kinetics and/or absorption and reduce the dose of the present compound; and/or
3) to eliminate the side effects of the present compound; and
in addition, (1) to supplement and/or enhance the preventive and/or therapeutic effect of the other medicaments used in combination; (2) to improve the kinetics and/or absorption and reduce the dose of the other medicaments used in combination; and/or (3) to eliminate the side effects of the other medicaments used in combination.

A combination of the compound of formula (I) and other medicaments may be administered in the form of the formulations having these components incorporated in one preparation, or may be administered in separate preparations. In the case where these medicaments are administered in separate preparations, they may be administered simultaneously or at different times. In the latter case, the compound of formula (I) may be administered before the other medicaments. Alternatively, the other medicaments may be administered before the compound of formula (I). The method for the administration of these medicaments are the same or different.
The diseases on which the preventive and/or therapeutic effect of the above mentioned combination preparations works are not specifically limited but may be those for which the preventive and/or therapeutic effect of the compound represented by formula (I) is supplemented and/or enhanced.
The weight ratio of the compound of formula (I) and the other medicaments is not specifically limited.
Any combination of two or more other medicaments may be administered.
Furthermore, the other medicaments for supplementing and/or enhancing the preventive and/or therapeutic effect of the compound of formula (I) include not only those found so far but also those which will be found on the basis of the above mentioned mechanism.
Examples of other drugs for supplementing and/or enhancing the preventive and/or therapeutic effect of the compounds of formula (I) on atopic dermatitis include steroid drugs, nonsteroidal anti-inflammatory drugs (NSAID), immunosuppressants, prostaglandins, antiallergic drugs, mediator release inhibitors, antihistamines, metabolism promoters (forskolin preparations, *etc*.), phosphodiesterase inhibitors, chemokine inhibitors, and the like.
Examples of other drugs for supplementing and/or enhancing the preventive and/or therapeutic effect of the compounds of formula (I) on allergic conjunctivitis include leukotriene receptor antagonists, antihistamines, mediator release inhibitors, nonsteroidal anti-inflammatory drugs, prostaglandins, steroids, nitric oxide synthase inhibitor, chemokine inhibitors, and the like.
Examples of other drugs for supplementing and/or enhancing the preventive and/or therapeutic effect of the compounds of formula (I) on allergic rhinitis include antihistamines, mediator release inhibitors, thromboxane synthase inhibitors, thromboxane $A_2$ receptor antagonists, leukotriene receptor antagonists, steroids, $\alpha$ adrenaline receptor stimulants, xanthine derivatives, anticholinergic agents, prostaglandins, nitric oxide synthase inhibitors, $\beta_2$ adrenaline receptor stimulants, phosphodiesterase inhibitors, chemokine inhibitors, and the like.
Examples of other drugs for supplementing and/or enhancing the preventive and/or therapeutic effect of the compounds of formula (I) on asthma include brondilators ($\beta_2$ adrenaline receptor stimulants, xanthine derivatives, anticholinergic agents, *etc*.), antiinflammatants (steroids, nonsteroidal anti-inflammatory drugs (NSAID), *etc*.), prostaglandins, leukotriene receptor antagonists, phosphodiesterase inhibitors, chemokine inhibitors, oriental drugs, and the like.
Examples of the steroids include, e.g., as drugs for external use, clobetasol propionate, diflorasone acetate, fluocinonide, mometasone furancarboxylate, betamethasone dipropionate, betamethasone butyrate propionate, betamethasone valerate, difluprednate, budesonide, diflucortolone valerate, amcinonide, halcinonide, dexamethasone, dexamethasone propionate, dexamethasone valerate, dexamethasone acetate, hydrocortisone acetate, hydrocortisone butyrate, hydrocortisone butyrate propionate, deprodone propionate, prednisolone valerate acetate, fluocinolone acetonide, beclomethasone propionate, triamcinolone acetonide, flumethasone pivalate, alclomethasone propionate, clobethasone butyrate, prednisolone, beclomethasone propionate, fludroxycortide, and the like.
Examples of drugs for internal use and injections include cortisone acetate, hydrocortisone, hydrocortisone sodium phosphate, hydrocortisone sodium succinate, fludrocortisone acetate, prednisolone, prednisolone acetate, prednisolone sodium succinate, prednisolone butyl acetate, prednisolone sodium phosphate, halopredone acetate, methylprednisolone, methylprednisolone acetate, methylprednisolone sodium succinate, triamcinolone, triamcinolone acetate, triamcinolone acetonide, dexamethasone, dexamethasone acetate, dexamethasone sodium phosphate, dexamethasone palmitate, paramethasone acetate, betamethasone, and the like.
Examples of inhalations include beclomethasone propionate, fluticasone propionate, budesonide, flunisolide, triamcinolone, ST-126P, ciclesonide, dexamethasone palomithionate, mometasone furcate, prasterone sulfonate, deflazacort, methylprednisolone sleptanate, methylprednisolone sodium succinate, and the like.
Examples of the nonsteroidal anti-inflammatory drugs include sasapyrine, sodium salicylate, aspirin, aspirin·dialuminate blend, diflunisal, indomethacin, sprofen, ufenamate, dimethylisopropylazulene, bufexamac, felbinac, diclofenac, tolmetin sodium, clinoril, fenbufen, nabumetone, proglumetacin, indometacin famesil, acemetacin, proglumetacin maleate, amfenac sodium, mofezolac, etodolac, ibuprofen, ibuprofen piconol, naproxen, flurbiprofen, flurbiprofen axetil, ketoprofen; fenoprofen calcium, tiaprofen, oxaprozin, pyranoprofen, loxoprofen sodium, alminoprofen, zaltoprofen, mefenamic acid, aluminum mefenamate, tolfenamic acid, floctafenine, ketophenylbutazone, oxyfenbutazone, piroxicam, tenoxicam, ampiroxicam, napageln ointment, epirizol, tiaramide hydrochloride, tinoridine hydrochloride, emorfazone, sulpirin, migrenin, saridon, sedes G, amipylo N, sorbon, pilin-based cold drugs, acetaminophen, fenacetine, dimethothiazine mesylate, simetride-containing agents, non-pilin-based cold drugs, and the like.

Examples of the immunosuppressants include protopic (FK-506), methotrexate, cyclosporin, ascomycin, leflunomide, bucillamine, salazosulfapyridine, sirolimus, mycophenolate mofetyl, and the like.

Examples of prostaglandins (hereinafter, abbreviated as PG) include PG receptor agonists, PG receptor antagonists, and the like.

Examples of PG receptors include PGE receptors (EP1, EP2, EP3 and EP4), PGD receptors (DP, CRTH2), PGF receptors (FP), PGI receptors (IP), TX receptors (TP), and the like.

Examples of the mediator release inhibitors include tranilast, sodium cromoglycate, amlexanox, repirinast, ibudilast, tazanolast, pemirolast potassium and the like.

Examples of the antihistamines include ketotifen fumarate, mequitazine, azelastine hydrochloride, oxatomide, terfenadine, emedastine fumarate, epinastine hydrochloride, astemizole, ebastine, cetirizine hydrochloride, bepotastine, fexofenadine, loratadine, desloratadine, olopatadine hydrochloride, TAK-427, ZCR-2060, NIP-530, mometasone furoate, mizolastine, BP-294, andolast, auranofin, acrivastine, famotidine, ranitidine, cimetidine, and the like.

Examples of the phosphodiesterase inhibitors include PDE4 inhibitors such as roliplam, cilomilast (trade name: Ariflo), Bay 19-8004, NIK-616, roflumilast (BY-217), cipamfylline (BRL-61063), atizoram (CP-80633), SCH-351591, YM-976, V-11294A, PD-168787, D-4396, IC-485, and the like.

Examples of the leukotriene receptor antagonists include pranlukast hydrate, montelukast, zafirlukast, seratrodast, MCC-847, KCA-757, CS-615, YM-158, L-740515, CP-195494, LM-1484, RS-635, A-93178, S-36496, BIIL-284, ONO-4057, and the like.

Examples of thromboxane $A_2$ receptor antagonists include, for Example, seratrodast, ramatroban, domitroban calcium hydrate, KT-2-962, and the like.

Examples of thromboxane synthase inhibitors include, for Example, ozagrel hydrochloride, imitrodast sodium, and the like.

Examples of xanthine derivatives include, for Example, aminophylline, theophylline, doxophylline, cipamfylline, diprophylline, and the like.

Examples of anticholinergic agents include, for Example, ipratropium bromide, oxitropium bromide, flutropium bromide, cimetropium bromide, temiverine, tiotropium bromide, revatropate (UK-112166), oxybutinin hydrochloride, bethanechol hydrochloride, propiverine hydrochloride, propantheline bromide, methylbenactyzium bromide, butylscopolamine bromide, tolterodine tartrate, trospium chloride, Z-338, UK-112166-04, KRP-197, darifenacin, YM-905, mephenzolate bromide, ipratropium bromide, and the like.

Examples of the $\beta_2$ adrenaline receptor stimulants include fenoterol hydrobromide, salbutamol sulfate, terbutaline sulfate, formoterol fumarate, salmeterol xinafoate, isoprotenol sulfate, orciprenalin sulfate, chloroprenaline sulfate, epinephrine, trimetoquinol hydrochloride, hexoprenaline mesyl sulfate, procaterol hydrochloride, tulobuterol hydrochloride, tulobuterol, pirbuterol hydrochloride, clenbuterol hydrochloride, mabuterol hydrochloride, ritodrine hydrochloride, bambuterol, dopexamine hydrochloride, meradrin tartrate, AR-C68397, levosalbutamol, R,R-formoterol, KUR-1246, KUL-7211, AR-C89855, S-1319, and the like.

Examples of the chemokine inhibitors include endogenous ligands of chemokine receptors or derivatives thereof, and non-peptidic low molecular compounds or antibodies for chemokine receptors.

Examples of the endogenous ligands of chemokine receptors include MIP-1$\alpha$, MIP-1$\beta$, RANTES, SDF-1$\alpha$, SDF-1$\beta$, MCP-1, MCP-2, MCP-4, Eotaxin, MDC, and the like.

Examples of the derivatives of endogenous ligands include AOP-RANTES, Met-SDF-1$\alpha$, Met-SDF-1$\beta$, and the like.

Examples of the antibodies for chemokine receptors include Pro-140, and the like.

Examples of the non-peptidic low molecular compounds include antagonists and agonists for CCR1, CCR2, CCR3, CCR4, CCR5, CXCR1, CXCR2, CXCR3 and CXCR4 receptors.

Examples of the oriental drugs include syouseiryuutou, Ephedrae Herba extracts, Liriope platyphylla extracts, and the like.

The compound of the present invention of formula (I) is safe and low-toxic, thus can be administered to human and mammals other than human (e.g., rat, mouse, rabbit, sheep, swine, bovine, cat, dog, monkey, *etc.*).

For the purpose above described, the compounds of formula (I), pharmacologically acceptable salts thereof, acid addition salts or hydrates thereof, or a combination of the compounds of formula (I) and other medicaments may be normally administered systemically or locally, usually by oral or parenteral administration.

The doses to be administered are determined depending upon, for Example, ages, body weights, symptoms,

the desired therapeutic effects, the route of administration and the duration of the treatment. For the human adult, the doses per person are generally from 1 ng to 100 mg, by oral administration, up to several times per day, and from 0.1 ng to 10 mg, by parenteral administration, up to several times per day, or continuous administration 1 to 24 hours per day from vein.

As mentioned above, the doses to be used depend upon various conditions. Therefore, there are cases in which doses lower than or greater than the ranges specified above may be used.

To administer the compounds in the present invention of formula (I), use is made of solid preparations for internal use and liquid preparations for internal use for oral administration as well as preparations for injections, external preparations, suppositories, eye drops, nasal drops, inhalations and the like for parenteral administration.

Examples of the solid preparations for internal use for oral administration include tablets, pills, capsules, powders, granules and the like. The capsules include hard capsules and soft capsules.

Such a solid preparation for internal use is prepared by a formulation method commonly employed by using one or two or more active substances either as it is or as a mixture with an excipient (lactose, mannitol, glucose, microcrystalline cellulose, starch, *etc.*), a binder (hydroxypropylcellulose, polyvinylpyrrolidone, magnesium metasilicate aluminate, *etc.*), a disintegrating agent (calcium cellulose glycolate, *etc.*), a lubricant (magnesium stearate, *etc.*), a stabilizer and a dissolution aid (glutamic acid, aspartic acid, *etc.*). If necessary, it may be coated with a coating agent (sucrose, gelatin, hydroxypropylcellulose, hydroxypropylmethylcellulose phthalate, *etc.*). It may be coated with two or more layers. Moreover, capsules made of an absorbable material such as gelatin are involved in the scope thereof.

The liquid preparations for internal use for oral administration include pharmaceutically acceptable aqueous solutions, suspensions, emulsions, syrups, elixirs and the like. Such a liquid preparation is prepared by dissolving, suspending or emulsifying one or more active substances in a diluent commonly employed (purified water, ethanol or a mixture thereof, *etc.*). Such liquid forms may also further comprise some additives such as humectants, suspending agents, emulsifying agents, sweetening agents, flavoring agents, aroma, preservatives, buffers and the like.

The dosage forms of the parenteral administration preparations for external use include ointments, gels, creams, fomentations, patches, liniments, atomized agents, inhalations, sprays, aerosols, eye drops, nasal drops and the like. Such a preparation contains one or two or more active substances and is prepared by a well known method or a commonly employed formulation.

Ointments are prepared in accordance with a well known formulation or a commonly employed formulation. For Example, they are prepared by softening or melting one or two or more active substances in a base. The ointment base is selected from well known ones or those commonly employed. For Example, use may be made of one base or a mixture of two or more thereof selected from higher fatty acids or higher fatty acid esters (adipic acid, myristic acid, palmitic acid, stearic acid, oleic acid, adipic acid esters, myristic acid esters, palmitic acid esters, stearic acid esters, oleic acid esters, *etc.*), waxes (beeswax, whale wax, ceresin, *etc.*), surfactants (polyoxyethylene alkyl ether phosphoric acid esters, *etc.),* higher alcohols (cetanol, stearyl alcohol, cetostearyl alcohol, *etc.*), silicone oils (dimethylpolysiloxane, *etc.*), hydrocarbons (hydrophilic vaseline, white vaseline, refined lanolin, liquid paraffin, *etc.*), glycols (ethylene glycol, diethylene glycol, propylene glycol, polyethylene glycol, macrogol, *etc.*), vegetable oils (castor oil, olive oil, sesame oil, turpentine oil, *etc.*), animal oils (mink oil, yolk oil, squalane, squalene, *etc.*), water, absorption promoters and skin irritation inhibitors. The ointments may further contain a humectant, a preservative, a stabilizer, an antioxidant, a flavor, and the like.

Gels are prepared in accordance with a well known formulation or a formulation commonly employed. For Example, they are prepared by melting one or more active substances in a base. The gel base is selected from well known ones or those commonly employed. For Example, use may be made of one base or a mixture of two or more thereof selected from lower alcohols (ethanol, isopropyl alcohol, *etc.*), gelling agents (carboxymethylcellulose, hydroxyethylcellulose, hydroxypropylcellulose, ethylcellulose, *etc.*), neutralizing agents (triethanolamine, diisopropanolamine, *etc.*), surfactants (polyethylene glycol monostearate, *etc.*), gums, water, absorption promoters and skin irritation inhibitors. The gels may further contain a preservative, an antioxidant, a flavor, and the like.

Creams are prepared in accordance with a well known formulation or a formulation commonly employed. For Example, they are prepared by melting or emulsifying one or more active substances in a base. The cream base is selected from well known ones or those commonly employed. For Example, use may be made of one base or a mixture of two or more thereof selected from higher fatty acid esters, lower alcohols, hydrocarbons, polyhydric alcohols (propylene glycol, 1,3-butylene glycol, *etc.*), higher alcohols (2-hexyldecanol, cetanol, *etc.*), emulsifiers (polyoxyethylene alkyl ethers, fatty acid esters, *etc.*), water, absorption promoters and skin irritation inhibitors. The creams may further contain a preservative, an antioxidant, a flavor, and the like.

Fomentations are prepared in accordance with a well known formulation or a formulation commonly employed. For Example, they are prepared by melting one or more active substances in a base, kneading and then applying and spreading the kneaded matter on a substrate. The fomentation base is selected from well known ones or those commonly employed. For Example, use may be made of one base or a mixture of two or more thereof selected from thickeners (polyacrylic acid, polyvinylpyrrolidone, gum acacia, starch, gelatin, methylcellulose, *etc.*), moistening agents

(urea, glycerin, propylene glycol, *etc.*), fillers (kaolin, zinc oxide, talc, calcium, magnesium, *etc.*), water, dissolution aids, tackifiers and skin irritation inhibitors. The fomentations may further contain a preservative, an antioxidant, a flavor, and the like.

Patches are prepared in accordance with a well known formulation or a formulation commonly employed. For Example, they are prepared by melting one or more active substances in a base and then applying and spreading on a substrate. The patch base is selected from well known ones or those commonly employed. For Example, use may be made of one base or a mixture of two or more thereof selected from polymer bases, fats and oils, higher fatty acids, tackifiers and skin irritation inhibitors. The patches may further contain a preservative, an antioxidant, a flavor, and the like.

Liniments are prepared in accordance with a well known formulation or a formulation commonly employed. For Example, they are prepared by dissolving, suspending or emulsifying one or two or more active substances in one or more media selected from water, alcohols (ethanol, polyethylene glycol, *etc.*), higher fatty acids, glycerin, soap, emulsifiers, suspending agents, and the like. The liniments may further contain a preservative, an antioxidant, a flavor, and the like.

Atomized agents, inhalations and sprays may contain, in addition to a diluent commonly employed, a stabilizer such as sodium hydrogen sulfite, a buffering agent for imparting isotonicity, for Example, an isotonic agent such as sodium chloride, sodium citrate or citric acid. Methods for producing a spray are described in detail in, for Example, U.S. Patent No. 2,868,691 and U.S. Patent No. 3,095,355.

The injections for parenteral administration include all forms of injections and also drops, for Example, an intramuscular injection, a subcutaneous injection, an intradermal injection, an intraarterial injection, an intravenous injection, a peritoneal injection, an intrathecal injection, an intravenous drop, and the like.

The injections for parenteral administration include solutions, suspensions, emulsions and solid injections to be dissolved or suspended before use. Such an injection is used by dissolving, suspending or emulsifying one or more active substances in a solvent. The solvent includes, for Example, distilled water for injection, physiological saline, vegetable oils, alcohols such as propylene glycol, polyethylene glycol and ethanol, and mixtures thereof. The injection may further contain a stabilizer, a dissolution aid (glutamic acid, aspartic acid, Polysorbate 80 (registered trademark), *etc.*), a suspending agent, an emulsifier, a soothing agent, a buffer, a preservative, and the like. Such an injection may be produced by sterilizing at the final step or employing an aseptic process. Alternatively, it is also possible that an aseptic solid product such as a freeze-dried product is produced and sterilized or dissolved in aseptic distilled water for injection or another solvent before use.

Eye drops for parenteral administration may be in the form of liquid, suspension, emulsion, liquid dissolved in a solvent in use or ointment.

These eye drops are prepared by any known method. For Example, one or more active substances are dissolved, suspended or emulsified in a solvent. As such a solvent for eye drops, there may be used sterilized purified water, physiological saline and other aqueous solvents or non-aqueous solvents for injection (e.g., vegetable oils, *etc.*), singly or in combination thereof. The eye drops may contain ones selected from an isotonic agent (e.g., sodium chloride, concentrated glycerin, *etc.*), a buffering agent (e.g., sodium phosphate, sodium acetate, *etc.*), a surfactant (e.g., Polysolvate 80 (trade name), Polyoxyl stearate 40, polyoxyethylene-hydrogenated castor oil, *etc.*), a stabilizer (sodium citrate, sodium edetate, *etc.*), a preservative (e.g., benzalconium chloride, paraben, *etc.*), and the like. The eye drops are sterilized at the final step or prepared by an aseptic process. Alternatively, an aseptic solid agent such as freeze-dried product which has previously been prepared may be rendered aseptic or dissolved in aseptic distilled water for injection or other solvent before use.

The inhalations for parenteral administration include aerosols, powders for inhalation and liquids for inhalation. Such inhalations may be dissolved or suspended in water or another adequate medium for use.

The inhalations may be prepared in accordance with a well known method.

For Example, liquid preparations for inhalation may be, if necessary, prepared by appropriately selecting a preservative (benzalkonium chloride, paraben, *etc.*), a colorant, a buffering agent (sodium phosphate, sodium acetate, *etc.*), an isotonic agent (sodium chloride, concentrated glycerin, *etc.*), a thickener (carboxyvinyl polymer, *etc.*), an absorption promoter, and the like.

Powders for inhalation may be prepared, if necessary, by appropriately selecting a lubricant (stearic acid and its salt, *etc.*), a binder (starch, dextrin, *etc.*), an excipient (lactose, cellulose, *etc.*), a colorant, a preservative (benzalkonium chloride, paraben, *etc.*), an absorption promoter, and the like.

When the liquids for inhalation are administered, a sprayer (atomizer, nebulizer) is usually used. When the powders for inhalation are used, an inhalation administration apparatus for powder agents is usually used.

Other compositions for parenteral administration include suppositories and pessaries for vaginal administration which contain one or more active substances, and are prepared in accordance with common formulations.

The compounds of the present invention are designated as follows.

The name of the compounds used in the present specification is designated according to IUPAC regulations,

or using a computer program conducting designation generally according to IUPAC regulations, ACD/Name (registered trademark, version 6.00, Advanced Chemistry Development Inc.).

BEST MODE FOR CARRYING OUT THE INVENTION

The present invention is explained below in detail based on Reference Examples and Examples, but the present invention is not limited thereto.
The solvents in the parentheses show the eluting or developing solvents in chromatographic separations or TLC and the ratios of the solvents used are by volume.
The solvents in the parentheses in NMR show the solvents for measurement.
MS was conducted to detect only positive ions (Pos., 20 V) using an ESI method (an electron spray ionization method) unless otherwise stated.
Measurement conditions for HPLC was conducted as follows unless otherwise stated.

Column used: Xterra (registered trademark) MS $C_{18}$ 5 μm, 4.6 × 50 mm I.D.
Flow rate used: 3 ml/min
Solvents used
Liquid A: an aqueous solution of 0.1% trifluoroacetic acid
Liquid B: a solution of 0.1% trifluoroacetic acid-acetonitrile

After initiating the measurement, for 0.5 minutes, the mixing ratio of Liquid A and Liquid B was fixed at 95/5. Thereafter, for 2.5 minutes, the mixing ratio of Liquid A and Liquid B was changed linearly to 0/100. Then, for 0.5 minute, the mixing ratio of Liquid A and Liquid B was fixed at 0/100. Then, for 0.01 minutes, the mixing ratio of Liquid A and Liquid B was changed linearly to 95/5.

Reference Example 1

2, 6-dibromo-3-(4-methylphenylsulfonylamino)pyrazine

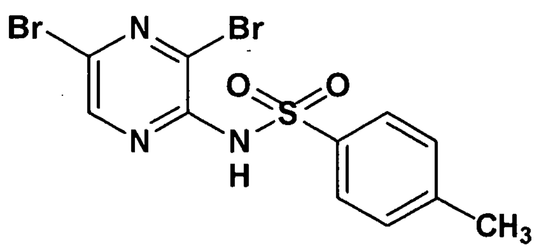

To a solution of 2, 6-dibromo-3-aminopyrazine (2.53 g) in 1, 2-dimethoxyethane (20 mL), 60% sodium hydride (1 g) was added under cooling with ice. The mixture was stirred for 30 minutes at room temperature. To the mixture, p-toluenesulfonyl chloride (1.91 g) was added under cooling with ice. The mixture was stirred for 1.5 hours at 0 °C. 2N hydrochloric acid was added to the reaction mixture, and the mixture was concentrated. The aqueous layer was extracted with ethyl acetate. The extract was washed with a saturated aqueous solution of sodium chloride, dried over anhydrous magnesium sulfate and concentrated. The residue was purified by column chromatography on silica gel (hexane : ethyl acetate = 5 : 1 → 2 : 1) to give the title compound (2.04 g) having the following physical data.
TLC: Rf 0.28(hexane : ethyl acetate = 1 : 1);
NMR ($d_6$-DMSO): δ 8.44(s, 1H), 7.86(d, J=8.1Hz, 2H), 7.38(d, J=8.1Hz, 2H), 2.37(s, 3H).

Example 1

6-bromo-2-(phenylmethyloxy)-3-(4-methylphenylsulfonylamino)pyrazine

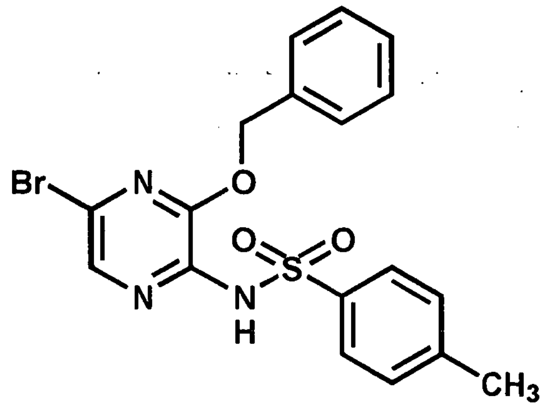

To a solution of benzyl alcohol (0.153 mL) in 1, 4-dioxane (3 mL), 60% sodium hydride (118 mg) was added at room temperature. The mixture was stirred for 30 minutes, and the compound prepared in Reference Example 1 (300 mg) was added to the mixture. The mixture was stirred for 1.5 hours at 65 °C. 1N hydrochloric acid was added to the reaction mixture, and the mixture was concentrated. The aqueous layer was extracted with ethyl acetate. The extract was washed with a saturated aqueous solution of sodium chloride, dried over anhydrous magnesium sulfate and concentrated. The residue was purified by column chromatography on silica gel (hexane : ethyl acetate = 6 : 1) to give the title compound (305 mg) having the following physical data.
TLC: Rf 0.57(hexane : ethyl acetate = 1 : 1);
NMR ($d_6$-DMSO): δ 11.12(s, 1H), 7.92(s, 1H), 7.85(dd, J=6.6, 1.8Hz, 2H), 7.52(dd, J=6.6, 1.8Hz, 2H), 7.43-7.34(m, 5H), 5.36(s, 2H), 2.35(s, 3H).

Example 1(1) - 1(8)

The following compounds were obtained, using the compound prepared in Reference Example 1 and a corresponding alcohol compound in stead of benzyl alcohol, by the same procedure as Example 1.

Example 1(1)

6-bromo-2-((pyridin-3-yl)methyloxy)-3-(4-methylphenylsulfonylamino)pyrazine
TLC: Rf 0.59(chloroform : methanol = 10 : 1);
NMR($d_6$-DMSO): δ 11.15(br, 1H), 8.77(d, J=1.8Hz, 1H), 8.57(dd, J=5.1, 1.8Hz, 1H), 7.97-7.94(m, 2H), 7.84(d, J=8.1Hz, 2H), 7.45(dd, J=7.8, 4.8Hz, 1H), 7.36(d, J=8.1Hz, 2H), 5.39(s, 2H), 2.35(s, 3H).

Example 1(2)

6-bromo-2-((3, 4-dimethoxyphenyl)methyloxy)-3-(4-methylphenylsulfonylamino)pyrazine
TLC: Rf 0.47(hexane : ethyl acetate = 1 : 1);
NMR($d_6$-DMSO): δ 11.06(br, 1H), 7.91(s, 1H), 7.84(d, J=8.4Hz, 2H), 7.35(d, J=8.4Hz, 2H), 7.15(d, J=1.8Hz, 1H), 7.04 (dd, J=8.1, 1.8Hz, 1H), 6.95(d, J=8.1Hz, 1H), 5.28(s, 2H), 3.76(s, 3H), 3.75(s, 3H), 2.35(s, 3H).

Example 1(3)

6-bromo-2-((3-(2-dimethylaminoethyloxy)-4-methoxyphenyl)methyloxy)-3-(4-methylphenylsulfonylamino) pyrazine
TLC: Rf 0.32(chloroform : methanol = 10 : 1);
NMR($d_6$-DMSO): δ 7.67(d, J=8.1Hz, 2H), 7.47(s, 1H), 7.19(d, J=1.8Hz, 1H), 7.16(d, J=8.1Hz, 2H), 7.09(dd, J=8.1, 1.8Hz, 1H), 7.01(d, J=8.1Hz, 1H), 5.13(s, 2H), 4.22(t, J=5.4Hz, 2H), 3.77(s, 3H), 3.24(t, J=5.4Hz, 2H), 2.69(s, 6H), 2.29(s, 3H).

Example 1(4)

6-bromo-2-((3-(2-(morpholin-4-yl)ethyloxy)-4-methoxyphenyl)methyloxy)-3-(4-methylphenylsulfonylamino) pyrazine

TLC: Rf 0.55(chloroform : methanol = 10 : 1);
NMR($d_6$-DMSO): δ 7.80(m, 3H), 7.31(d, J=8.4Hz, 2H), 7.18(d, J=1.8Hz, 1H), 7.05(dd, J=8.1, 1.8Hz, 1H), 6.97(d, J=8.1Hz, 1H), 5.24(s, 2H), 4.11(t, J=5.1Hz, 2H), 3.76(s, 3H), 3.59(m, 4H), 2.82(t, J=5.1Hz, 2H), 2.61(m, 4H), 2.34(s, 3H).

Example 1(5)

6-bromo-2-((3-(2-diethylaminoethyloxy)-4-methoxyphenyl)methyloxy)-3-(4-methylphenylsulfonylamino) pyrazine
TLC: Rf 0.33(chloroform : methanol = 10 : 1);
NMR($d_6$-DMSO): δ 7.67(d, J=7.5Hz, 2H), 7.47(s, 1H), 7.19(brs, 1H), 7.16(d, J=7.5Hz, 2H), 7.09(brd, J=7.2Hz, 1H), 7.01(d, J=7.2Hz, 1H), 5.14(s, 2H), 4.25(t, J=5.1Hz, 2H), 3.78(s, 3H), 3.38(m, 2H), 3.12(q, J=7.2Hz, 4H), 2.29(s, 3H), 1.18(t, J=7.2Hz, 6H).

Example 1(6)

6-bromo-2-((3-(2-diisopropylaminoethyloxy)-4-methoxyphenyl)methyloxy)-3-(4-methylphenylsulfonylamino) pyrazine
TLC: Rf 0.43(chloroform : methanol = 10 : 1);
NMR($d_6$-DMSO): δ 7.68(d, J=8.1Hz, 2H), 7.51(br, 1H), 7.19-7.15(m, 3H), 7.05(d, J=8.1Hz, 1H), 6.98(d, J=8.1Hz, 1H), 5.16(s, 2H), 4.12(m, 2H), 3.76(s, 3H), 3.60-3.30(m, 4H), 2.29(s, 3H), 1.21(m, 12H).

Example 1(7)

6-bromo-2-((3-(2-(N-methyl-N-(2-dimethylaminoethyl)amino)ethyloxy)-4-methoxyphenyl)methyloxy)-3-(4-methylphenylsulfonylamino)pyrazine triethylamine salt
TLC: Rf 0.45(chloroform : methanol: triethylamine = 9 : 1 : 0.5);
NMR($d_6$-DMSO): δ 7.63(d, J=7.8Hz, 2H), 7.36(s, 1H), 7.12(m, 3H), 7.01(d, J=7.8Hz, 1H), 6.95(d, J=7.8Hz, 1H), 5.10 (s, 2H), 4.09(t, J=5.4Hz, 2H), 3.75(s, 3H), 2.95(m, 8H), 2.80(t, J=5.4Hz, 2H), 2.70(t, J=6.0Hz, 2H), 2.59(s, 6H), 2.31 (s, 3H), 2.27(s, 3H), 1.12(t, J=7.5Hz, 9H).

Example 1(8)

6-bromo-2-((3-dimethylaminomethyl-4-methoxyphenyl)methyloxy)-3-(4-methylphenylsulfonylamino)pyrazine
TLC: Rf 0.17(chloroform : methanol = 10 : 1);
NMR($d_6$-DMSO): δ 7.73(d, J=7.8Hz, 2H), 7.65(s, 1H), 7.59(dd, J=8.7, 1.8Hz, 1H), 7.53(d, J=1.8Hz, 1H), 7.24(d, J=7.8Hz, 2H), 7.15(d, J=8.7Hz, 1H), 5.23(s, 2H), 4.18(s, 2H), 3.85(s, 3H), 2.69(s, 6H), 2.31(s, 3H).

Example 2

5, 6-dimethyl-2-((3-(2-dimethylaminoethyloxy)-4-methoxyphenyl)methyloxy) -3-(4-methylphenylsulfonylamino)pyrazine
The title compound having the following physical data was obtained, using 2-bromo-3-amino-5,6-dimethylpyrazine in stead of 2,6-dibromo-3-aminopyrazine, and 3-(2-dimethylaminoethyl)oxy-4-methoxybenzyl alcohol in stead of benzyl alcohol, by the same procedure as a series of reactions of Reference Example 1 → Example 1.
TLC: Rf 0.30(chloroform : methanol = 10 : 1);
NMR($d_6$-DMSO): δ 7.85(d, J=8.4Hz, 2H), 7.31(d, J=8.4Hz, 2H), 7.13(d, J=2.1Hz, 1H), 7.02(dd, J=8.4, 2.1Hz, 1H), 6.93 (d, J=8.4Hz, 1H), 5.21(s, 2H), 4.05(t, J=6.0Hz, 2H), 3.74(s, 3H), 2.72(t, J=6.0Hz, 2H), 2.34(s, 3H), 2.29(s, 6H), 2.24 (s, 3H), 2.18(s, 3H).

Example 2(1) - 2(5)

The following compounds were obtained, using a corresponding pyrazine compound in stead of 2-bromo-3-amino-5,6-dimethylpyrazien and a corresponding alcohol compound in stead of 3-(2-dimethylaminoethyl)oxy-4-methoxybenzyl alcohol, by the same procedure as Example 2.

Example 2(1)

6-methyl-2-((3-(2-(morpholin-4-yl)ethyloxy)-4-methoxyphenyl)methyloxy)-3-(4-methylphenylsulfonylamino) pyrazine
TLC: Rf 0.58(chloroform : methanol = 10 : 1);
NMR ($CD_3OD$): δ 7.85(d, J=8.4Hz, 2H), 7.52(s, 1H), 7.27(d, J=8.4Hz, 2H), 7.13(d, J=1.8Hz, 1H), 7.00(dd, J=8.1, 1.8Hz, 1H), 6.92(d, J=8.1Hz, 1H), 5.33(s, 2H), 4.16(t, J=5.7Hz, 2H), 3.82(s, 3H), 3.69(m, 4H), 2.81(t, J=5.7Hz, 2H), 2.62(m, 4H), 2.37(s, 3H), 2.29(s, 3H).

Example 2(2)

6-methyl-2-((3-(2-diethylaminoethyloxy)-4-methoxyphenyl)methyloxy)-3-(4-methylphenylsulfonylamino) pyrazine
TLC: Rf 0.33(chloroform : methanol = 10 : 1);
NMR($d_6$-DMSO): δ 7.78(d, J=8.1Hz, 2H), 7.50(s, 1H), 7.28(d, J=8.1Hz, 2H), 7.15(s, 1H), 7.04(d, J=8.1Hz, 1H), 6.95 (d, J=8.1,Hz, 1H), 5.23(s, 2H), 4.05(t, J=6.0Hz, 2H), 3.75(s, 3H), 2.93(t, J=6.0Hz, 2H), 2.69(q, J=7.2Hz, 4H), 2.33(s, 3H), 2.23(s, 3H), 1.01(t, J=7.2Hz, 6H).

Example 2(3)

6-methyl-2-((3-(2-diisopropylaminoethyloxy)-4-methoxyphenyl)methyloxy)-3-(4-methylphenylsulfonylamino) pyrazine
TLC: Rf 0.43(chloroform : methanol = 10 : 1);
NMR($d_6$-DMSO): δ 7.79(d, J=8.1Hz, 2H), 7.53(s, 1H), 7.29(d, J=8.1Hz, 2H), 7.12(s, 1H), 7.02(d, J=8.1Hz, 1H), 6.93 (d, J=8.1Hz, 1H), 5.24(s, 2H), 3.89(t, J=6.9Hz, 2H), 3.74(s, 3H), 3.08(m, 2H), 2.86(t, J=6.9Hz, 2H), 2.33(s, 3H), 2.24 (s, 3H), 1.00(d, J=6.3Hz, 12H).

Example 2(4)

6-methyl-2-((3-(2-(N-methyl-N-(2-dimethylaminoethyl)amino)ethyloxy)-4-methoxyphenyl)methyloxy)-3-(4-methylphenylsulfonylamino)pyrazine triethylamine salt
TLC: Rf 0.45(chloroform : methanol : triethylamine = 9 : 1 : 0.5),
NMR($d_6$-DMSO): δ 7.75(d, J=8.1Hz, 2H), 7.43(s, 1H), 7.24(d, J=8.1Hz, 2H), 7.15(s, 1H), 7.03(d, J=8.1Hz, 1H), 6.95 (d, J=8.1Hz, 1H), 5.21(s, 2H), 4.08(t, J=5.1Hz, 2H), 3.75(s, 3H), 2.96(q, J=7.2Hz, 6H), 2.88(t, J=6.0Hz, 2H), 2.79(t, J=5.1Hz, 2H), 2.68(t, J=6.0Hz, 2H), 2.54(s, 6H), 2.31(s, 3H), 2.30(s, 3H), 2.20(s, 3H), 1.13(t, J=7.2Hz, 9H).

Example 2(5)

6-methyl-2-((3-dimethylaminomethyl-4-methoxyphenyl)methyloxy)-3-(4-methylphenylsulfonylamino)pyrazine
TLC: Rf 0.17(chloroform : methanol = 10 : 1);
NMR($d_6$-DMSO): δ 7.79(d, J=8.4Hz, 2H), 7.53(s, 1H), 7.46(m, 2H), 7.30(d, J= 8.4Hz, 2H), 7.04(d, J=9.0Hz, 1H), 5.27 (s, 2H), 3.80(s, 3H), 3.71(s, 2H), 2.37(s, 6H), 2.33(s, 3H), 2.24(s, 3H).

Reference Example 2

2-chloro-3-(4-methylphenylsulfonylamino)pyrazine
To a solution of 2, 3-dichloropyrazine (5 g) and 4-methylbenzenesulfonamide (5.74 g) in dimethylsulfoxide (60 mL), potassium carbonate (13.91 g) was added. The mixture was stirred for 4 hours at 110 °C. The reaction mixture was cooled to room temperature, and water and 2N hydrochloric acid were added to the mixture. The precipitated sold was collected by filtration, and dried to give the title compound (7.67 g) having the following physical data.
TLC: Rf 0.74(chloroform : methanol = 10 : 1);
NMR ($d_6$-DMSO): δ 11.19(br, 1H), 8.22(d, J=2.4Hz, 1H), 8.11(d, J=2.4Hz, 1H), 7.88(d, J=8.4Hz, 2H), 7.38(d, J=8.4Hz, 2H), 2.37(s, 3H).

Example 3

2-(phenylmethyloxy)-3-(4-methylphenylsulfonylamino)pyrazine

The title compound having the following physical data was obtained, using the compound prepared in Reference Example 2 in stead of the compound prepared in Reference Example 1, by the same procedure as a series of reactions of Example 1.
TLC: Rf 0.38(hexane : ethyl acetate = 2 : 1);
NMR ($d_6$-DMSO): δ 10.91(brs, 1H), 7.87(d, J=8.1Hz, 2H), 7.73(m, 2H), 7.50(m, 2H), 7.36(m, 5H), 5.38(s, 2H), 2.35(s, 3H).

Example 3(1) - 3(11)

The following compounds were obtained, using the compound prepared in Reference Example 2 and a corresponding alcohol compound in stead of benzyl alcohol, by the same procedure as a series of reactions of Example 3.

Example 3(1)

2-((pyridin-3-yl)methyloxy)-3-(4-methylphenylsulfonylamino)pyrazine
TLC: Rf 0.43(chloroform : methanol = 10 : 1);
NMR($d_6$-DMSO): δ 10.95(s, 1H), 8.74(d, J=1.8Hz, 1H), 8.54(dd, J=4.8, 1.8Hz, 1H), 7.92(d, J=7.8Hz, 1H), 7.85(d, J=8.4Hz, 2H), 7.75(d, 3.0Hz, 1H), 7.73(d, J=3.0Hz, 1H), 7.41(dd, J=7.8, 4.8Hz, 1H), 7.35(d, J=8.4Hz, 2H), 5.40(s, 2H), 2.34(s, 3H).

Example 3(2)

2-(2-phenoxyethyloxy)-3-(4-methylphenylsulfonylamino)pyrazine
TLC: Rf 0.62(hexane : ethyl acetate = 1 : 1);
NMR($d_6$-DMSO): δ 10.88(s, 1H), 7.86(d, J=8.4Hz, 2H), 7.71(m, 2H), 7.34(d, J=8.4Hz, 2H), 7.29(dd, J=7.8, 7.2Hz, 2H), 6.97(d, J=7.8Hz, 2H), 6.94(t, J=7.2Hz, 1H), 4.60(m, 2H), 4.34(m, 2H), 2.34(s, 3H).

Example 3(3)

2-((pyridin-4-yl)methyloxy)-3-(4-methylphenylsulfonylamino)pyrazine
TLC: Rf 0.35(chloroform : methanol = 10 : 1);
NMR($d_6$-DMSO): δ 11.04(s, 1H), 8.56(d, J=5.7Hz, 2H), 7.88(d, J=8.4Hz, 2H), 7.74(d, J=2.7Hz, 1H), 7.72(d, J=2.7Hz, 1H), 7.49(d, J=5.7Hz, 2H), 7.36(d, J=8.4Hz, 2H), 5.43(s, 2H), 2.35(s, 3H).

Example 3(4)

2-((3-methoxymethyloxyphenyl)methyloxy)-3-(4-methylphenylsulfonylamino)pyrazine
TLC: Rf 0.67(hexane : ethyl acetate = 1 : 1);
NMR($d_6$-DMSO): δ 10.91(s, 1H), 7.86(d, J=8.4Hz, 2H), 7.82-7.64(m, 2H), 7.35(d, J=8.4Hz, 2H), 7.29(t, J=7.8Hz, 1H), 7.17(brs, 1H), 7.11(brd, J=7.8Hz, 1H), 6.98(m, 1H), 5.34(s, 2H), 5.10(s, 2H), 3.36(s, 3H), 2.34(s, 3H).

Example 3(5)

2-(3-aminophenylmethyloxy)-3-(4-methylphenylsulfonylamino)pyrazine
TLC: Rf 0.56(benzene : ethyl acetate = 1 : 1);
NMR($d_6$-DMSO): δ 7.88(d, J=8.1Hz, 2H), 7.74(d, J=3.0Hz, 1H), 7.71(d, J=3.0Hz, 1H), 7.36(d, J=8.1Hz, 2H), 7.01(t, J=7.2Hz, 1H), 6.63-6.61(m, 2H), 6.57(d, J=7.2Hz, 1H), 5.24(s, 2H), 2.36(s, 3H).

Example 3(6)

2-((3-(2-dimethylaminoethyloxy)-4-methoxyphenyl)methyloxy)-3-(4-methylphenylsulfonylamino)pyrazine
TLC: Rf 0.27(chloroform : methanol = 10 : 1);
NMR($d_6$-DMSO): δ 7.78(d, J=8.4Hz, 2H), 7.54(d, J=3.0Hz, 1H), 7.49(d, J=3.0Hz, 1H), 7.26(d, J=8.4Hz, 2H), 7.14(d, J=1.5Hz, 1H), 7.04(dd, J=8.4, 1.5Hz, 1H), 6.96(d, J=8.4Hz, 1H), 5.23(s, 2H), 4.11(t, J=5.4Hz, 2H), 3.75(s, 3H), 2.91 (t, J=5.4Hz, 2H), 2.43(s, 6H), 2.32(s, 3H).

Example 3(7)

2-((3-(2-(morpholin-4-yl)ethyloxy)-4-methoxyphenyl)methyloxy)-3-(4-methylphenylsulfonylamino)pyrazine
TLC: Rf 0.54(chloroform : methanol = 10 : 1);
NMR ($CD_3OD$): δ 7.99(d, J=7.8Hz, 2H), 7.64(d, J=3.0Hz, 1H), 7.62(d, J=3.0Hz, 1H), 7.29(d, J=7.8Hz, 2H), 7.14(d, J=2.1Hz, 1H), 7.02(dd, J=8.4, 2.1Hz, 1H), 6.93(d, J=8.4Hz, 1H), 5.35(s, 2H), 4.16(t, J=5.4Hz, 2H), 3.82(s, 3H), 3.70 (m, 4H), 2.82(t, J=5.4Hz, 2H), 2.64(m, 4H), 2.38(s, 3H).

Example 3(8)

2-((3-(2-diethylaminoethyloxy)-4-methoxyphenyl)methyloxy)-3-(4-methylphenylsulfonylamino)pyrazine
TLC: Rf 0.33(chloroform : methanol = 10 : 1);
NMR($d_6$-DMSO): δ 7.78(d, J=8.1Hz, 2H), 7.53(d, J=2.7Hz, 1H), 7.47(d, J=2.7Hz, 1H), 7.25(d, J=8.1Hz, 2H), 7.14(d, J=1.2Hz, 1H), 7.04(dd, J=8.1, 1.2Hz, 1H), 6.96(d, J=8.1Hz, 1H), 5.23(s, 2H), 4.12(t, J=6.0Hz, 2H), 3.75(s, 3H), 3.08 (t, J=6.0Hz, 2H), 2.84(q, J=6.9Hz, 4H), 2.32(s, 3H), 1.07(t, J=6.9Hz, 6H).

Example 3(9)

2-((3-(2-diisopropylaminoethyloxy)-4-methoxyphenyl)methyloxy)-3-(4-methylphenylsulfonylamino)pyrazine
TLC: Rf 0.43(chloroform : methanol = 10 : 1);
NMR($d_6$-DMSO): δ 7.81(d, J=8.1Hz, 2H), 7.60(m, 2H), 7.29(d, J=8.1Hz, 2H), 7.12(d, J=1.5Hz, 1H), 7.03(dd, J=8.1, 1.5Hz, 1H), 6.95(d, J= 8.1Hz, 1H), 5.26(s, 2H), 3.98(m, 2H), 3.75(s, 3H), 3.36(m, 2H), 3.03(br, 2H), 2.33(s, 3H), 1.08 (d, J=7.2Hz, 12H).

Example 3(10)

2-((3-(2-(N-methyl-N-(2-dimethylaminoethyl)amino)ethyloxy)-4-methoxyphenyl)methyloxy)-3-(4-methylphenylsulfonylamino)pyrazine triethylamine salt
TLC: Rf 0.45(chloroform : methanol : triethylamine = 9 : 1 : 0.5);
NMR($d_6$-DMSO): δ 7.71(d, J=7.8Hz, 2H), 7.39(d, J=2.7Hz, 1H), 7.25(d, J=2.7Hz, 1H), 7.18(d, J=7.8Hz, 2H), 7.12(s, 1H), 7.00(d, J=8.1Hz, 1H), 6.97(d, J=8.1Hz, 1H), 5.18(s, 2H), 4.08(t, J=5.7Hz, 2H), 3.74(s, 3H), 2.97(m, 8H), 2.79(t, J=5.4Hz, 2H), 2.71(t, J=5.7Hz, 2H), 2.60(s, 6H), 2.31(s, 3H), 2.29(s, 3H), 1.14(t, J=7.2Hz, 9H).

Example 3(11)

2-((3-dimethylaminomethyl-4-methoxyphenyl)methyloxy)-3-(4-methylphenylsulfonylamino)pyrazine
TLC: Rf 0.17(chloroform : methanol = 10 : 1);
NMR($d_6$-DMSO): δ 7.79(d, J=7.8Hz, 2H), 7.55(d, J=2.7Hz, 1H), 7.51(d, J=2.7Hz, 1H), 7.47(s, 2H), 7.26(d, J=7.8Hz, 2H), 7.06(d, J=9.3Hz, 1H), 5.26(s, 2H), 3.85(s, 2H), 3.81(s, 3H), 2.46(s, 6H), 2.32(s, 3H).

Example 4

2-((pyridin-3-yl)methyloxy)-3-(4-chlorophenylsulfonylamino)pyrazine
The title compounds having the following physical data was obtained, using 4-chlorobenzenesulfonamide in stead of 4-methylbenzenesulfonamide, and 3-(hydroxymethyl)pyridine in stead of benzyl alcohol, by the same procedure as a series of reactions of Reference Example 2 → Example 1.
TLC: Rf 0.40(hexane : ethyl acetate = 3 : 7);
NMR($d_6$-DMSO): δ 11.17(s, 1H), 8.74(d, J=1.8Hz, 1H), 8.54(dd, J=4.8, 1.8Hz, 1H), 7.97(d, J=8.4Hz, 2H), 7.92(d, J=8.1Hz, 1H), 7.79(d, J=3.0Hz, 1H), 7.74(d, J=3.0Hz, 1H), 7.64(d, J=8.4Hz, 2H), 7.42(dd, J=8.1, 4.8Hz, 1H), 5.41(s, 2H).

Example 4(1) - 4(5)

The compounds having the following physical data were obtained, using a corresponding sulfonamide compound in stead of 4-chlorobenzenesulfonamide, and a corresponding alcohol compound in stead of 3-(hydroxymethyl) pyridine, by the same procedure as a series of reactions of Example 4.

Example 4(1)

2-(2-phenoxyethyloxy)-3-(4-chlorophenylsulfonylamino)pyrazine
TLC: Rf 0.68(hexane : ethyl acetate = 1 : 1);
NMR($d_6$-DMSO): δ 11.11(s, 1H), 7.97(d, J=8.4Hz, 2H), 7.76(m, 1H), 7.72(m, 1H), 7.63(d, J=8.4Hz, 2H), 7.29(dd, J=8.1, 7.2Hz, 2H), 6.97(d, J=8.1Hz, 2H), 6.94(t, J=7.2Hz, 1H), 4.61(m, 2H), 4.34(m, 2H).

Example 4(2)

2-((pyridin-3-yl)methyloxy)-3-(4-fluorophenylsulfonylamino)pyrazine
TLC: Rf 0.2 1 (chloroform : methanol = 10 : 1);
NMR($d_6$-DMSO): δ 11.10(s, 1H), 8.75(d, J=2.1Hz, 1H), 8.55(dd, J=4.5, 2.1Hz, 1H), 8.05(dd, J=7.2, 5.1Hz, 2H), 7.93 (m, 1H), 7.79(d, J=3.0Hz, 1H), 7.75(d, J=3.0Hz, 1H), 7.50-7.35(m, 3H), 5.42(s, 2H).

Example 4(3)

2-(2-phenoxyethyloxy)-3-(4-fluorophenylsulfonylamino)pyrazine
TLC: Rf 0.33(hexane : ethyl acetate = 3 : 1);
NMR($d_6$-DMSO): δ 11.04(s, 1H), 8.04(m, 2H), 7.75(m, 1H), 7.72(m, 1H), 7.39(t, J=8.7Hz, 2H), 7.29(t, J=8.7Hz, 2H), 6.97(d, J=7.5Hz, 2H), 6.92(d, J=7.2Hz, 1H), 4.61(m, 2H), 4.34(m, 2H).

Example 4(4)

2-((pyridin-3-yl)methyloxy)-3-(4-ethylphenylsulfonylamino)pyrazine
TLC: Rf 0.40(chloroform : methanol = 10 : 1);
NMR($d_6$-DMSO): δ 10.95(s, 1H), 8.74(d, J=1.8Hz, 1H), 8.54(dd, J=4.8, 1.8Hz, 1H), 7.93(dt, J=8.1, 1.8Hz, 1H), 7.89(d, J=8.4Hz, 2H), 7.76(d, J=3.0Hz, 1H), 7.74(d, J=3.0Hz, 1H), 7.42(dd, J=8.1, 4.8Hz, 1H), 7.38(d, J=8.4Hz, 2H), 5.40(s, 2H), 2.65(q, J=7.5Hz, 2H), 1.16(t, J=7.5Hz, 3H).

Example 4(5)

2-(phenylmethyloxy)-3-(2-methoxy-4-methylphenylsulfonylamino)pyrazine
TLC: Rf 0.59(hexane : ethyl acetate = 1 : 1);
NMR($CDCl_3$): δ 7.98(d, J=8.1Hz, 1H), 7.96(s, 1H), 7.68(d, J=3.0Hz, 1H), 7.63(d, J=3.0Hz, 1H), 7.44-7.40(m, 5H), 6.86 (d, J=8.1Hz, 1H), 6.66(s, 1H), 5.40(s, 2H), 3.72(s, 3H), 2.36(s, 3H).

Example 5

2-((pyridin-3-yl)methyloxy)-3-(4-methylphenylsulfonylamino)quinoxaline

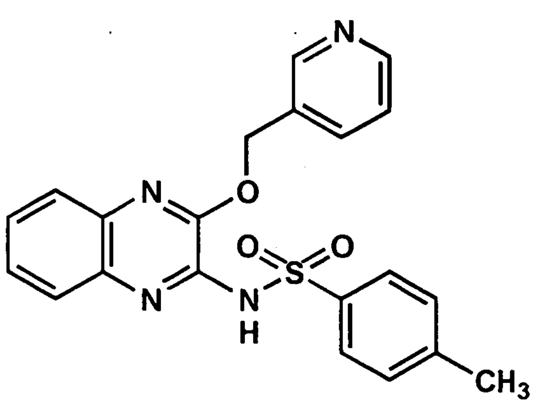

The title compound having the following physical data was obtained, using 2, 3-dichloroquinoxaline in stead of 2, 3-dichloropyrazine, and 3-(hydroxymethyl)pyridine in stead of benzyl alcohol, by the same procedure as a series of reactions of Reference Example 2 → Example 1.

TLC: Rf 0.22(chloroform : methanol = 10 : 1);
NMR($d_6$-DMSO): δ 11.39(brs, 1H), 8.84(d, J=1.8Hz, 1H), 8.58(dd, J=4.5, 1.8Hz, 1H), 8.06(d, J=8.1Hz, 2H), 8.02(m, 1H), 7.80-7.65(m, 2H), 7.58-7.50(m, 2H), 7.46(m, 1H), 7.40(d, J=8.1Hz, 2H), 5.57(s, 2H), 2.35(s, 3H).

Example 5(1) - 5(17)

The following compounds were obtained, using 2, 3-dichloroquinoxaline compound or a corresponding pyrazine compound, a corresponding sulfonamide compound in stead of 4-methylbenzenesulfonamide, and 3-(hydroxymethyl)pyridine in stead of benzyl alcohol, by the same procedure as a series of reactions of Example 5.

Example 5(1)

2-(2-phenoxyethyloxy)-3-(4-methylphenylsulfonylamino)quinoxaline
TLC: Rf 0.71(hexane : ethyl acetate = 1 : 1);
NMR ($d_6$-DMSO): δ 11.30(br, 1H), 8.05(m, 2H), 7.70(m, 2H), 7.50(m, 2H), 7.40-7.28(m, 4H), 7.01-6.95(m, 3H), 4.75 (m, 2H), 4.43(m, 2H), 2.33(s, 3H).

Example 5(2)

2-((pyridin-4-yl)methyloxy)-3-(4-methylphenylsulfonylamino)quinoxaline
TLC: Rf 0.51 (chloroform : methanol = 10 : 1);
NMR ($d_6$-DMSO): δ 11.46(br, 1H), 8.60(m, 2H), 8.06(m, 2H), 7.73(m, 1H), 7.65(m, 1H), 7.59(m, 2H), 7.52(m, 2H), 7.40 (m, 2H), 5.59(s, 2H), 2.34(s, 3H).

Example 5(3)

2-((pyridin-3-yl)methyloxy)-3-(4-chlorophenylsulfonylamino)quinoxaline
TLC: Rf 0.15(chloroform : methanol = 10 : 1);
NMR($d_6$-DMSO): δ 12.00-11.00(br, 1H), 8.82(d, J=1.8Hz, 1H), 8.56(dd, J=4.8, 1.8Hz, 1H), 8.14(d, J=8.4Hz, 2H), 8.01 (dt, J=6.0, 1.8Hz, 1H), 7.72(m, 2H), 7.67(d, J=8.4Hz, 2H), 7.53(m, 2H), 7.44(m, 1H), 5.55(s, 2H).

Example 5(4)

2-(2-phenoxyethyloxy)-3-(4-chlorophenylsulfonylamino)quinoxaline
TLC: Rf 0.83(hexane : ethyl acetate = 1 : 1);
NMR($d_6$-DMSO): δ 11.50(brs, 1H), 8.20-8.10(m, 2H), 7.80-7.70 (m, 2H), 7.67(d, J=7.8Hz, 2H), 7.52(m, 2H), 7.29(t, J=7.8Hz, 2H), 6.99(d, J=7.8Hz, 2H), 6.94(t, J=7.2Hz, 1H), 4.75(m, 2H), 4.42(m, 2H).

Example 5(5)

2-((pyridin-4-yl)methyloxy)-3-(4-chlorophenylsulfonylamino)quinoxaline
TLC: Rf 0.12(chloroform : methanol = 10 : 1);
NMR ($d_6$-DMSO): δ 8.58(m, 2H), 8.13(d, J=8.4Hz, 2H), 7.70-7.57 (m, 4H), 7.55(d, J=5.7Hz, 2H), 7.45(m, 2H), 5.55(s, 2H).

Example 5(6)

2-((pyridin-3-yl)methyloxy)-3-(4-fluorophenylsulfonylamino)quinoxaline
TLC: Rf 0.15 (chloroform : methanol = 10 : 1);
NMR($d_6$-DMSO): δ 11.50(brs, 1H), 8.82(d, J=1.8Hz, 1H), 8.56 (dd, J=4.5, 1.8Hz, 1H), 8.21(m, 2H), 8.01(dt, J=7.8, 1.8Hz, 1H), 7.78-7.66(m, 2H), 7.52(m, 2H), 7.45(m, 1H), 7.41(m, 2H), 5.55(s, 2H).

Example 5(7)

2-(2-phenoxyethyloxy)-3-(4-fluorophenylsulfonylamino)quinoxaline
TLC: Rf 0.71 (hexane : ethyl acetate = 1 : 1);
NMR($d_6$-DMSO): δ 11.41(brs, 1H), 8.22(m, 2H), 7.73(m, 1H), 7.67(m, 1H), 7.52(m, 2H), 7.43(t, J=8.7Hz, 2H), 7.30(dd, J=8.7, 7.5Hz, 2H), 7.00(d, J=7.8Hz, 2H), 6.94(t, J=7.5Hz, 1H), 4.75(m, 2H), 4.42(m, 2H).

Example 5(8)

2-((pyridin-4-yl)methyloxy)-3-(4-fluorophenylsulfonylamino)quinoxaline
TLC: Rf 0.10(chloroform : methanol = 10 : 1);
NMR ($d_6$-DMSO): δ 8.59(m, 2H), 8.24(m, 2H), 7.75(m, 1H), 7.65(m, 1H), 7.58(m, 2H), 7.51(m, 2H), 7.44(m, 2H), 5.57 (s, 2H).

Example 5(9)

2-((pyridin-3-yl)methyloxy)-3-(4-ethylphenylsulfonylamino)quinoxaline
TLC: Rf 0.18(chloroform : methanol = 10 : 1);
NMR($d_6$-DMSO): δ 11.40(brs, 1H), 8.82(d, J=1.5Hz, 1H), 8.56(dd, J=4.8, 1.5Hz, 1H), 8.06(d, J=8.4Hz, 2H), 8.01(m, 1H), 7.80-7.65(m, 2H), 7.60-7.48(m, 2H), 7.44(m, 1H), 7.42(d, J=8.4Hz, 2H), 5.55(s, 2H), 2.63(q, J=7.2Hz, 2H), 1.14 (t, J=7.2Hz, 3H).

Example 5(10)

2-(2-phenoxyethyloxy)-3-(4-ethylphenylsulfonylamino)quinoxaline
TLC: Rf 0.76(hexane : ethyl acetate = 1 : 1);
NMR($d_6$-DMSO): δ 11.28(s, 1H), 8.07(d, J=8.4Hz, 2H), 7.71(m, 1H), 7.66(m, 1H), 7.51(m, 2H), 7.41(d, J=8.4Hz, 2H), 7.30(dd, J=8.1, 7.2Hz, 2H), 6.99(d, J=8.1Hz, 2H), 6.94(t, J= 7.2Hz, 1H), 4.74(m, 2H), 4.42(m, 2H), 2.63(q, J=7.5Hz, 2H), 1.14(t, J=7.5Hz, 3H).

Example 5(11)

2-((pyridin-4-yl)methyloxy)-3-(4-ethylphenylsulfonylamino)quinoxaline
TLC: Rf 0.10(chloroform : methanol = 10 : 1);
NMR($d_6$-DMSO): δ 8.59(m, 2H), 8.08(d, J=8.1Hz, 2H), 7.74(m, 1H), 7.65(m, 1H), 7.59(d, J=5.7Hz, 2H), 7.51(m, 2H), 7.43(d, J=8.1Hz, 2H), 5.58(s, 2H), 2.64(q, J=7.5Hz, 2H), 1.15(t, J=7.5Hz, 3H).

Example 5(12)

2-(phenylmethyloxy)-3-(4-methylphenylsulfonylamino)quinoxaline
TLC: Rf 0.71(hexane : ethyl acetate = 1 : 1);
NMR ($d_6$-DMSO): δ 11.27(s, 1H), 8.04(d, J=7.8Hz, 2H), 7.68(m, 2H), 7.58(d, J=6.9Hz, 2H), 7.51(m, 2H), 7.44-7.28(m, 5H), 5.52(s, 2H), 2.33(s, 3H).

Example 5(13)

2-((pyridin-3-yl)methyloxy)-3-(4-propylphenylsulfonylamino)quinoxaline
TLC: Rf 0.49(chloroform : methanol = 10 : 1);
NMR($d_6$-DMSO): δ 11.36(brs, 1H), 8.83(d, J=1.5Hz, 1H), 8.56(dd, J=4.5, 1.5Hz, 1H), 8.06(d, J=8.4Hz, 2H), 8.01(d, J=4.5Hz, 1H), 7.69(m, 2H), 7.52(m, 2H), 7.44(m, 1H), 7.40(d, J=8.4Hz, 2H), 5.55(s, 2H), 2.59(t, J=7.5Hz, 2H), 1.55(q, J=7.5Hz, 2H), 0.84(t, J=7.5Hz, 3H).

Example 5(14)

6-phenyl-2-((pyridin-3-yl)methyloxy)-3-(4-methylphenylsulfonylamino)pyrazine
TLC: Rf 0.30(hexane : ethyl acetate = 1 : 2);
NMR($d_6$-DMSO): δ 11.05(brs, 1H), 8.82(d, J=2.1Hz, 1H), 8.54(dd, J=3.9, 2.1Hz, 1H), 8.37(s, 1H), 7.99(dd, J=3.9,1.8Hz, 3H), 7.89(d, J=8.1Hz, 2H), 7.45-7.36(m, 6H), 5.56(s, 2H), 2.35(s, 3H).

Example 5(15)

5-phenyl-2-((pyridin-3-yl)methyloxy)-3-(4-methylphenylsulfonylamino)pyrazine
TLC: Rf 0.29(hexane : ethyl acetate = 1 : 2);
NMR ($d_6$-DMSO): δ 11.14(brs, 1H), 8.79(m, 1H), 8.55(m, 1H), 8.35(s, 1H), 7.96(m, 4H), 7.77(d, J=6.9Hz, 2H), 7.46-7.36 (m, 5H), 5.47(s, 2H), 2.33(s, 3H).

Example 5(16)

5-phenyl-2-(2-(N-phenyl-N-methylamino)ethyloxy)-3-(4-methylphenylsulfonylamino)pyrazine TLC: Rf 0.41 (hexane : ethyl acetate = 1 : 1);
NMR($d_6$-DMSO): δ 10.91(brs, 1H), 8.30(s, 1H), 7.93(d, J=8.4Hz, 2H), 7.76(d, J=8.4Hz, 2H), 7.40(m, 5H), 7.16(t, J=8.4Hz, 2H), 6.77(d, J=8.4Hz, 2H), 6.61(t, J=8.4Hz, 1H), 4.50(t, J=6.0Hz, 2H), 3.78(t, J=6.0Hz, 2H), 2.97(s, 3H), 2.34 (s, 3H).

Example 5(17)

6-phenyl-2-(2-(N-methyl-N-phenylamino)ethyloxy)-3-(4-methylphenylsulfonylamino)pyrazine

NMR ($CDCl_3$): δ 8.21(s, 1H), 8.07(d, J=7.8Hz, 2H), 7.75(d, J=7.8Hz, 2H), 7.48-7.29(m, 11H), 4.49(br, 2H), 4.00(br, 2H), 3.31(br, 3H), 2.41(s, 3H).

Reference Example 3

2-bromo-6-methyl-3-(4-methylphenylsulfonylamino)pyrazine
The title compound having the following physical data was obtained, using 2-bromo-3-amino-6-methylpyrazine in stead of 2, 6-dibromo-3-aminopyrazine, by the same procedure as a series of reactions of Reference Example 1.
TLC: Rf 0.61(hexane : ethyl acetate = 1 : 1);
NMR ($d_6$-DMSO): δ 10.82(br, 1H), 8.13(s, 1H), 7.85(d, J=8.1Hz, 2H), 7.37(d, J=8.1Hz, 2H), 2.36(s, 3H), 2.35(s, 3H).

Reference Example 4

2-bromo-6-methyl-3-(N-(4-methylphenylsulfonyl)-N-(2-trimethylsilylethyl)amino)pyrazine
To a solution of the compound prepared in Reference Example 3 (1 g) and 2-(trimethylsilyl)ethanol (0.628 mL) in methylene chloride (25 mL), 1.73 mol/g polymer support triphenylphosphine (2.53 g, catalog number: 800380, Argonaut Technologies) and diethyl azodicarboxylate (1.99 mL, 40% toluene solution) were added at 0 °C. The mixture was stirred for 2 hours at 0 °C and overnight at room temperature. The reaction mixture was filtered. The filtrate was concentrated. The residue was purified by column chromatography on silica gel (hexane : ethyl acetate = 4 : 1 → 3 : 1) to give the title compound (320 mg) having the following physical data.
TLC: Rf 0.58(hexane : ethyl acetate = 2 : 1);
NMR($CDCl_3$): δ 7.92(d, J=8.4Hz, 2H), 7.30(d, J=8.4Hz, 2H), 7.15(s, 1H), 3.69(m, 2H), 2.43(s, 3H), 2.34(s, 3H), 0.67 (m, 2H), -0.07(s, 9H).

Reference Example 5

6-methyl-2-((3, 4-dimethoxyphenyl)methyloxy)-3-(N-(4-methylphenylsulfonyl) -N-(2-trimethylsilylethyl)amino)pyrazine
The title compound having the following physical data was obtained, using the compound prepared in Reference Example 4, and 3,4-dimethoxybenzyl alcohol in stead of benzyl alcohol, by the same procedure as a series of reactions of Example 1.
TLC: Rf 0.56(hexane : ethyl acetate = 1 : 1).

Example 6

6-methyl-2-((3, 4-dimethoxyphenyl)methyloxy)-3-(4-methylphenylsulfonylamino)pyrazine
The compound prepared in Reference Example 5 was dissolved into excessive amounts of 1N tetrabutylammonium fluoride. The mixture was stirred for 1 hour at room temperature and concentrated. The residue was purified by column chromatography on silica gel (hexane : ethyl acetate = 3 : 1 → 2 : 1) to give the title compound (88 mg) having the following physical data. TLC: Rf 0.45(hexane : ethyl acetate = 1 : 1);
NMR($d_6$-DMSO): δ 10.58(br, 1H), 7.82(d, J=8.1Hz, 2H), 7.61(s, 1H), 7.32(d, J=8.1Hz, 2H), 7.13(d, J=1.5Hz, 1H), 7.03 (dd, J=8.1, 1.5Hz, 1H), 6.94(d, J=8.1Hz, 1H), 5.27(s, 2H), 3.75(s, 3H), 3.74(s, 3H), 2.34(s, 3H), 2.27(s, 3H).

Example 6(1)

6-methyl-2-((3-(2-dimethylaminoethyloxy)-4-methoxyphenyl)methyloxy)-3-(4-methylphenylsulfonylamino)pyrazine

The title compound having the following physical data was obtained, using 3-(2-dimetylaminoethyl)oxy-4-methoxybenzyl alcohol in stead of 3, 4-dimethoxybenzyl alcohol, by the same procedure as a series of reactions of Reference Example 5 → Example 6.
TLC: Rf 0.36(chloroform : methanol = 10 : 1);
NMR($d_6$-DMSO): δ 7.78(d, J=8.1Hz, 2H), 7.52(s, 1H), 7.28(d, J=8.1Hz, 2H), 7.15(d, J=1.8Hz, 1H), 7.04(dd, J=8.1, 1.8Hz, 1H), 6.95(d, J=8.1Hz, 1H), 5.23(s, 2H), 4.06(t, J=5.1Hz, 2H), 3.75(s, 3H), 2.76(t, J=5.1Hz, 2H), 2.33(s, 3H), 2.32(s, 6H), 2.23(s, 3H).

Reference Example 6

6-bromo-2-((3,4-dimethoxyphenyl)methyloxy)-3-aminopyrazine

The title compound having the following physical data was obtained, using 2,6-dibromo-3-aminopyrazine and 3,4-dimethoxybenzyl alcohol in stead of benzyl alcohol, by the same procedure as a series of reactions of Example 1.
TLC: Rf 0.35(hexane : ethyl acetate = 1 : 1);
NMR($d_6$-DMSO): δ 7.60(s, 1H), 7.13(d, J=1.8Hz, 1H), 7.02(dd, J=8.4, 1.8Hz, 1H), 6.93(d, J=8.4Hz, 1H), 6.49(br, 2H), 3.76(s, 3H), 3.74(s, 3H).

Example 7

6-bromo-2-((3,4-dimethoxyphenyl)methyloxy)-3-(4-chlorophenylsulfonylamino)pyrazine

The title compound having the following physical data was obtained, using the compound prepared in Reference Example 6 and 4-chlorobenzenesulfonyl chloride in stead of 4-methylbenzenesulfonyl chloride, by the same procedure as a series of reactions of Reference
Example 1.
TLC: Rf 0.50(benzene : ethyl acetate = 3 : 1);
NMR($d_6$-DMSO): δ 11.25(br, 1H), 7.92(m, 3H), 7.63(d, J=9.0Hz, 2H), 7.14(d, J=1.8Hz, 1H), 7.04(dd, J=8.1, 1.8Hz, 1H), 6.95(d, J=8.1Hz, 1H), 5.28(s, 2H), 3.75(s, 3H), 3.74(s, 3H).

Example 7(1)-7(4)

The following compounds were obtained, using the compound prepared in Reference Example 6 and a corresponding sulfonyl chloride compound in stead of 4-chlorobenzenesulfonyl chloride, by the same procedure as a series of reactions of Example 7.

Example 7(1)

6-bromo-2-((3,4-dimethoxyphenyl)methyloxy)-3-(phenylsulfonylamino)pyrazine
TLC: Rf 0.52(benzene : ethyl acetate = 3 : 1);
NMR($d_6$-DMSO): δ 11.15(br, 1H), 7.97-7.91(m, 3H), 7.64-7.53(m, 3H), 7.15(d, J=2.1Hz, 1H), 7.05(dd, J=8.4, 2.1Hz, 1H), 6.95(d, J=8.4Hz, 1H), 5.28(s, 2H), 3.76(s, 3H), 3.75(s, 3H).

Example 7(2)

6-bromo-2-((3,4-dimethoxyphenyl)methyloxy)-3-(4-fluorophenylsulfonylamino)pyrazine
TLC: Rf 0.48(benzene : ethyl acetate = 3 : 1);
NMR($d_6$-DMSO): δ 11.18(br, 1H), 8.01(m, 2H), 7.92(s, 1H), 7.40(t, J=8.7Hz, 2H), 7.14(d, J=2.4Hz, 1H), 7.05(dd, J=8.1, 2.4Hz, 1H), 6.96(d, J=8.4Hz, 1H), 5.28(s, 2H), 3.76(s, 3H), 3.75(s, 3H).

Example 7(3)

6-bromo-2-((3,4-dimethoxyphenyl)methyloxy)-3-(2-methylphenylsulfonylamino)pyrazine
TLC: Rf 0.60(benzene : ethyl acetate = 3 : 1);
NMR($d_6$-DMSO): δ 11.10(br, 1H), 7.93(s, 1H), 7.75(m, 2H), 7.44(d, J=5.1Hz, 2H), 7.15(d, J=1.8Hz, 1H), 7.05(dd, J=8.4, 1.8Hz, 1H), 6.95(d, J= 8.4Hz, 1H), 5.28(s, 2H), 3.76(s, 3H), 3.75(s, 3H), 2.36(s, 3H).

Example 7(4)

6-bromo-2-((3,4-dimethoxyphenyl)methyloxy)-3-(3-methylphenylsulfonylamino)pyrazine
TLC: Rf 0.60(benzene : ethyl acetate = 3 : 1);
NMR($d_6$-DMSO): δ 11.32(br, 1H), 7.92(m, 1H), 7.83(s, 1H), 7.50(dt, J=1.2, 7.5Hz, 1H), 7.35(m, 2H), 7.15(d, J= 1.2Hz, 1H), 7.03(dd, J=8.1, 1.8Hz, 1H), 6.95(d, J= 8.1Hz, 1H), 5.30(s, 2H), 3.76(s, 3H), 3.75(s, 3H), 2.57(s, 3H).

Example 8

6-(4-methylphenyl)-2-((3,4-dimethoxyphenyl)methyloxy)-3-(4-methylphenylsulfonylamino)pyrazine
To a solution of the compound prepared in Example 1 (2) (70 mg), 4-methylphenylboric acid (38 mg) and potassium carbonate (60 mg) in 1,2-dimethoxyethane (1 mL) and water (0.5 mL), dichlorobis(triphenylphosphine)palladium (II) (4.9 mg, 5% N-methylpyrrolidone solution) was added. The mixture was stirred for 2 hours at 80 °C. 2N hydrochloric acid was added to the reaction mixture, and the mixture was extracted with chloroform. The extract was washed with a saturated aqueous solution of sodium chloride, dried over anhydrous magnesium sulfate and concentrated. The residue was purified by column chromatography on silica gel (hexane : ethyl acetate = 5 : 1 → 3 : 1) to give the title compound (48 mg) having the following physical data.
TLC: Rf 0.46(hexane : ethyl acetate = 1 : 1);
NMR($d_6$-DMSO): δ 10.88(br, 1H), 8.31(s, 1H), 7.90(d, J=8.4Hz, 2H), 7.88(d, J=8.4Hz, 2H), 7.36(d, J=8.1Hz, 2H), 7.26 (d, J=8.1Hz, 2H), 7.20(d, J=1.8Hz, 1H), 7.08(dd, J=8.1, 1.8Hz, 1H), 6.94(d, J=8.1Hz, 1H), 5.43(s, 2H), 3.73(s, 3H), 3.72(s, 3H), 2.35(s, 3H), 2.33(s, 3H).

Example 8(1) - 8(3)

The following compounds were obtained, using a corresponding boric acid compound in stead of 4-methylphenylboric acid, by the same procedure as a series of reactions of Example 8.

Example 8(1)

6-(3-aminophenyl)-2-((3,4-dimethoxyphenyl)methyloxy)-3-(4-methylphenylsulfonylamino)pyrazine
TLC: Rf 0.31 (hexane : ethyl acetate = 1 : 2);
NMR($d_6$-DMSO): δ 8.17(s, 1H), 7.87(d, J=8.1Hz, 2H), 7.35(d, J=7.8Hz, 2H), 7.23(d, J=9.3Hz, 2H), 7.10(m, 3H), 6.94 (d, J=8.1Hz, 1H), 6.58(dt, J=7.5, 2.1Hz, 1H), 5.42(s, 2H), 3.73(s, 6H), 2.35(s, 3H).

Example 8(2)

6-(3-formylphenyl)-2-((3,4-dimethoxyphenyl)methyloxy)-3-(4-methylphenylsulfonylamino)pyrazine
TLC: Rf 0.36(hexane : ethyl acetate = 1 : 1);
NMR($d_6$-DMSO): δ 11.04(br, 1H), 10.07(s, 1H), 8.53(s, 1H), 8.44(s, 1H), 8.33(d, J=7.8Hz, 1H), 7.90(m, 3H), 7.68(t, J=7.8Hz, 1H), 7.36(d, J=7.8Hz, 2H), 7.20(d, J=1.8Hz, 1H), 7.12(dd, J=8.1, 1.8Hz, 1H), 6.94(d, J=8.4Hz, 1H), 5.46(s, 2H), 3.72(s, 6H), 2.35(s, 3H).

Example 8(3)

6-(3-methoxyphenyl)-2-((3,4-dimethoxyphenyl)methyloxy)-3-(4-methylphenylsulfonylamino)pyrazine
TLC: Rf 0.40(hexane : ethyl acetate = 1 : 1);
NMR($d_6$-DMSO): δ 10.95(br, 1H), 8.35(s, 1H), 7.84(d, J=8.1Hz, 2H), 7.57(d, J=8.1Hz, 1H), 7.51(m, 1H), 7.35(m, 3H), 7.19(d, J=1.8Hz, 1H), 7.08(dd, J=7.8, 1.8Hz, 1H), 6.94(m, 2H), 5.43(s, 2H), 3.79(s, 3H), 3.72(s, 6H), 2.34(s, 3H).

Example 9

2-((3-dimethylaminophenyl)methyloxy)-3-(4-methylphenylsulfonylamino)pyrazine
To a solution of the compound prepared in Example 3(5) (367 mg) in methylene chloride (8 mL), 37% aqueous solution of formaldehyde (297 μL) was added at room temperature. Triacetoxy sodium borohydride (839 mg) was added to the mixture at 0 °C. The mixture was stirred for 4 hours at room temperature. Water was to the reaction mixture, and the mixture was extracted with ethyl acetate. The extract was dried over anhydrous magnesium sulfate and concentrated. The residue was purified by column chromatography on silica gel (hexane : ethyl acetate = 2 : 1) to give the title compound (328 mg) having the following physical data.

TLC: Rf 0.65(hexane : ethyl acetate = 1 : 1);
NMR($d_6$-DMSO): δ 10.90(s, 1H), 7.88(d, J=8.1Hz, 2H), 7.78 - 7.66(m, 2H), 7.36(d, J=8.1Hz, 2H), 7.17(t, J=8.4Hz, 1H), 6.84(brs, 1H), 6.77(brd, J=7.8Hz, 1H), 6.68(m, 1H), 5.33(s, 2H), 2.89(s, 6H), 2.36(s, 3H).

Example 9(1)

6-(3-dimethylaminophenyl)-2-((3,4-dimethoxyphenyl)methyloxy)-3-(4-methylphenylsulfonylamino)pyrazine

The title compound having the following physical data was obtained, using the compound prepared in Example 8(1) in stead of the compound prepared in Example 3(5) by the same procedure as a series of reactions of Example 9.
TLC: Rf 0.47(hexane : ethyl acetate = 1 : 1);
NMR($d_6$-DMSO): δ 10.90(br, 1H), 8.30(s, 1H), 7.88(d, J=8.4Hz, 2H), 7.36(d, J=8.4Hz, 2H), 7.28-7.18(m, 4H), 7.07(dd, J=8.1, 1.2Hz, 1H), 6.92(d, J=8.1Hz, 1H), 6.74(m, 1H), 5.44(s, 2H), 3.72(s, 6H), 2.93(s, 6H), 2.35(s, 3H).

Example 10

2-((3-acetylaminophenyl)methyloxy)-3-(4-methylphenylsulfonylamino)pyrazine

To a solution of the compound prepared in Example 3(5) (150 mg) in methylene chloride (6 mL), pyridine (131 μL) and anhydrous acetic acid (76 μL) were added. The mixture was stirred for 2 hours at room temperature. Ethyl acetate was added to the reaction mixture. The mixture was washed with 2N hydrochloric acid, dried over anhydrous magnesium sulfate and concentrated. The residue was purified by column chromatography on silica gel (hexane : ethyl acetate = 1 : 2) to give the title compound (153 mg) having the following physical data.
TLC: Rf 0.22(hexane : ethyl acetate = 1 : 2);
NMR($d_6$-DMSO): δ 10.92(s, 1H), 9.98(s, 1H), 7.88(d, J=8.4Hz, 2H), 7.80-7.68(m, 2H), 7.63(s, 1H), 7.56(d, J=7.5Hz, 1H), 7.36(d, J=8.4Hz, 2H), 7.30(t, J=7.5Hz, 1H), 7.17(d, J=7. 5Hz, 1H), 5.37(s, 2H), 2.36(s, 3H), 2.04(s, 3H).

Example 11

6-bromo-2-((3-(2-dimethylaminoethyloxy)-4-methoxyphenyl)methyloxy)-3-(4-methylphenylsulfonylamino) pyrazine sodium salt

To a solution of the compound prepared in Example 1(3) (120 mg) in ethanol (2 mL), 1N aqueous solution of sodium hydroxide (0.217 mL) was added. The mixture was stirred for 1 hour at 80 °C. The reaction mixture was concentrated to give the title compound (94 mg) having the following physical data.
TLC: Rf 0.32(chloroform : methanol = 10 : 1);
NMR($d_6$-DMSO): δ 7.62(d, J=8.4Hz, 2H), 7.33(s, 1H), 7.10(m, 3H), 7.00(dd, J=8.4, 1.8Hz, 1H), 6.93(d, J=8.4Hz, 1H), 5.08(s, 2H), 4.02(t, J=6.0Hz, 2H), 3.74(s, 3H), 2.61(t, J=6.0Hz, 2H), 2.27(s, 3H), 2.20(s, 6H).

Example 12

6-bromo-2-((pyridin-1-oxide-3-yl)methyloxy)-3-(4-methylphenylsulfonylamino)pyrazine

To a solution of the compound prepared in Example 1(1) (500 mg) in chloroform (5 mL), 70% m-chloroperbenzoic acid (340 mg) was added. The mixture was stirred for 2 hours at room temperature. The reaction mixture was diluted with ethyl acetate. The diluted solution was washed with a saturated solution of sodium chloride, dried over anhydrous magnesium sulfate and concentrated. The residue was purified by column chromatography on silica gel (hexane : ethyl acetate = 1 : 1 → 1 : 2 → 0 : 1 → ethyl acetate : methanol = 5 : 1 → 3 : 1) to give the title compound (317 mg) having the following physical data.
TLC: Rf 0.54(chloroform ; methanol = 10 : 1);
NMR($d_6$-DMSO): δ 11.28(br, 1H), 8.58(s, 1H), 8.19(m, 1H), 7.89(s, 1H), 7.83(d, J=8.1Hz, 2H), 7.46(m, 2H), 7.34(d, J=8.1Hz, 2H), 5.32(s, 2H), 2.35(s, 3H).

Example 13

6-bromo-2-((1-methylpyridinium-3-yl)methyloxy)-3-(4-methylphenylsulfonylamino)pyrazine chloride

To a solution of the compound prepared in Example 1(1) (300 mg) in acetone (5 mL), methyl iodide (64 μL) was added. The mixture was stirred overnight at room temperature. The reaction mixture was concentrated. A solution of the residue in methanol was though chlorine ion-exchange resin (preliminary washing: methanol x2, water x2, methanol x2) to give the title compound (288 mg) having the following physical data.
NMR($d_6$-DMSO): δ 11.30(br, 1H), 9.38(s, 1H), 9.02(d, J=6.0Hz, 1H), 8.75(d, J=6.0Hz, 1H), 8.20(dd, J=8.1, 6.0Hz, 1H), 8.00(s, 1H), 7.89(d, J=8.1Hz, 2H), 7.36(d, J=8.1Hz, 2H), 5.58(s, 2H), 4.41(s, 3H), 2.35(s, 3H).

Reference Example 7

2-chloro-3-(3-methylphenylsulfonylamino)quinoxaline

The title compound having the following physical data was obtained, using 2,3-dichloroquinoxaline in stead of 2,3-dichloropyrazine, and 3-methylbenzenesulfonamide in stead of 4-methylbenzenesulfonamide, by the same procedure as a series of reactions of Reference
Example 2.
TLC: Rf 0.40(hexane : ethyl acetate = 1 : 1);
NMR($d_6$-DMSO): δ 8.12 (m, 2H), 7.87 (m, 2H), 7.71 (t, J=7.8Hz, 1H), 7.61 (t, J=7.8Hz, 1H), 7.42 (m, 2H), 2.45 (s, 3H).

Reference Example 8

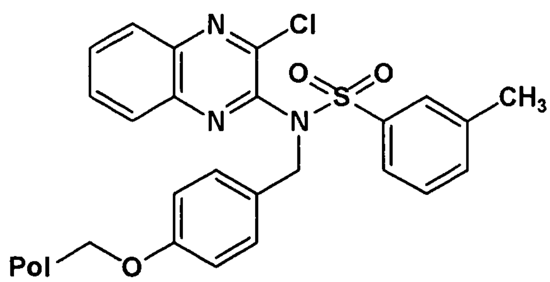

(1)

wherein Pol is 1% divinylbenzene copolymer polystyrene resin.

Wang resin (Watanabe Chemical Co., Ltd., 1% divinylbenzene copolymer polystyrene, 100-200 mesh, catalog number: A00110, 0.82 mmol/g, 4.0g) was suspended in anhydrous tetrahydrofuran (40 mL). Under an atmosphere of argon, the compound prepared in Example 7 (1.82 g), triphenylphosphine (1.29 g) and 40% solution of diethyl azodicarboxylate in toluene (2.24 mL), successively, were added to the suspension at -78 °C. The mixture was stirred for 16 hours at room temperature. The reaction mixture was filtered. The obtained resin was washed with tetrahydrofuran (40 mL) for three times, methanol (40 mL) for two times and methylene chloride (40 mL) for four times, successively, and dried to give the title compound (5.36 g). Reference Example 9

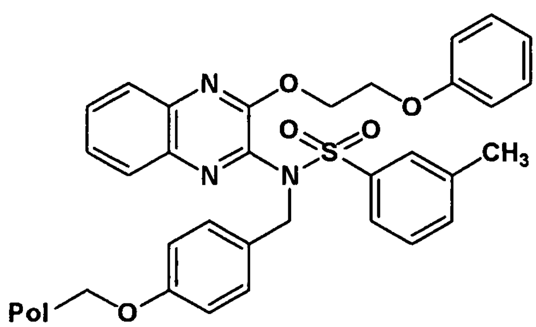

(2)

Under an atmosphere of argon, to a suspension of the compound prepared in Reference Example 8 (750 mg) in anhydrous tetrahydrofuran (6 mL), 2-phenoxyethanol (1.15 mL) and 1.0M tetrabutylammonium fluoride in tetrahydrofuran solution (2.3 mL) were successively added at room temperature. The mixture was stirred for 24 hours at 60 °C. The reaction mixture was cooled to room temperature, and filtered. The obtained resin was washed with tetrahydrofuran (10 mL) for three times and methylene chloride (10 mL) for three times, successively, and dried to give the title compound (2) (834 mg).

Example 14

2-(2-phenoxyethyloxy)-3-(3-methylphenylsulfonylamino)quinoxaline

A suspension of the compound (2) prepared in Reference Example 9 (834 mg) in 50% solution of trifluoroacetic

acid in 1,2-dichloroethane (10 mL) was stirred for 2 hours at room temperature. The reaction mixture was filtered. The obtained resin was washed with 50% solution of trifluoroacetic acid in 1,2-dichloroethane (10 mL) for three times. The filtrate and the washings were concentrated to give the title compound (168 mg) having the following physical data.
TLC: Rf 0.56(hexane : ethyl acetate = 3 : 2);
NMR ($d_6$-DMSO): δ 11.34 (br, 1H),-8.10-7.85 (m, 2H), 7.84-7.62 (m, 2H), 7.60-7.40 (m, 4H), 7.32 (t, J=8.1Hz, 2H), 7.02 (d, J=8.1Hz, 2H), 6.97 (t, J=8.1Hz, 1H), 4.77 (m, 2H), 4.45 (m, 2H), 2.41 (s, 3H);
HPLC maintenance time (minutes): 4.18;
Mass data: 893 $(2M+Na)^+$, 436 $(M+H)^+$.

Example 14(1) - 14(210)

The following compounds were obtained, using 2,3-dichloroquinoxaline, a corresponding sulfonamide compound and a corresponding alcohol compound, by the same procedure as a series of reactions of Reference Example 7 → Reference Example 8 → Reference Example 9 → Example 14.

Example 14(1)

2-((pyridin-3-yl)methyloxy)-3-(4-methylphenylsulfonylamino)quinoxaline
HPLC maintenance time (minutes): 3.34;
Mass data: 813 $(2M+H)^+$, 407 $(M+H)^+$.

Example 14(2)

2-((pyridin-4-yl)methyloxy)-3-(4-methylphenylsulfonylamino)quinoxaline
HPLC maintenance time (minutes): 3.37;
Mass data: 835 $(2M+Na)^+$, 407 $(M+H)^+$.

Example 14(3)

2-(2-(pyridin-2-yl)ethyloxy)-3-(2-methylphenylsulfonylamino)quinoxaline
HPLC maintenance time (minutes): 3.33;
Mass data: 863 $(2M+Na)^+$, 421 $(M+H)^+$.

Example 14(4)

2-(3-(pyridin-3-yl)propyloxy)-3-(4-methoxyphenylsulfonylamino)quinoxaline
HPLC maintenance time (minutes): 3.36;
Mass data: 923 $(2M+Na)^+$, 451 $(M+H)^+$.

Example 14(5)

2-(2-(N-methyl-N-benzylamino)ethyloxy)-3-(4-methylphenylsulfonylamino)quinoxaline
HPLC maintenance time (minutes): 3.50;
Mass data: 463 $(M+H)^+$, 242.

Example 14(6)

2-(2-(N-methyl-N-phenylamino)ethyloxy)-3-(4-methylphenylsulfonylamino)quinoxaline
HPLC maintenance time (minutes): 3.64;
Mass data: 449 $(M+H)^+$.

Example 14(7)

2-(2-(3,5-dimethylpyrazol-1-yl)ethyloxy)-3-(4-methylphenylsulfonylamino)quinoxaline
HPLC maintenance time (minutes): 3.61;
Mass data: 438 $(M+H)^+$.

Example 14(8)

2-(2-phenoxyethyloxy)-3-(4-methylphenylsulfonylamino)quinoxaline
HPLC maintenance time (minutes): 4.15;
Mass data: 893 $(2M+Na)^+$, 436 $(M+H)^+$.

Example 14(9)

2-(3-phenylpropyloxy)-3-(4-methylphenylsulfonylamino)quinoxaline
HPLC maintenance time (minutes): 4.31;
Mass data: 889 $(2M+Na)^+$, 434 $(M+H)^+$.

Example 14(10)

2-(4-phenylbutyloxy)-3-(4-methylphenylsulfonylamino)quinoxaline
HPLC maintenance time (minutes): 4.40;
Mass data: 917 $(2M+Na)^+$, 448 $(M+H)^+$.

Example 14(11)

2-(2-(thiophen-2-yl)ethyloxy)-3-(4-methylphenylsulfonylamino)quinoxaline
HPLC maintenance time (minutes): 4.17;
Mass data: 873 $(2M+Na)^+$, 426 $(M+H)^+$.

Example 14(12)

2-(3-(pyridin-3-yl)propyloxy)-3-(4-methylphenylsulfonylamino)quinoxaline
HPLC maintenance time (minutes): 3.40;
Mass data: 435 $(M+H)^+$.

Example 14(13)

2-(3-benzyloxypropyloxy)-3-(4-methylphenylsulfonylamino)quinoxaline
HPLC maintenance time (minutes): 4.23;
Mass data: 464 $(M+H)^+$.

Example 14(14)

2-(2-(pyridin-2-yl)ethyloxy)-3-(4-methylphenylsulfonylamino)quinoxaline
HPLC maintenance time (minutes): 3.34;
Mass data: 421 $(M+H)^+$.

Example 14(15)

2-(3-(pyridin-2-yl)propyloxy)-3-(4-methylphenylsulfonylamino)quinoxaline
HPLC maintenance time (minutes): 3.40;
Mass data: 435 $(M+H)^+$.

Example 14(16)

2-(2-(pyridin-4-yl)ethyloxy)-3-(4-methylphenylsulfonylamino)quinoxaline
HPLC maintenance time (minutes): 3.36;
Mass data: 421 $(M+H)^+$.

Example 14(17)

2-((tetrahydrofuran-2-yl)methyloxy)-3-(4-methylphenylsulfonylamino)quinoxaline
HPLC maintenance time (minutes): 3.93;

Mass data: 821 $(2M+Na)^+$, 400 $(M+H)^+$.

Example 14(18)

2-(cyclohexylmethyloxy)-3-(4-methylphenylsulfonylamino)quinoxaline
HPLC maintenance time (minutes): 4.47;
Mass data: 845 $(2M+Na)^+$, 412 $(M+H)^+$.

Example 14(19)

2-(2-(piperidin-1-yl)ethyloxy)-3-(4-methylphenylsulfonylamino)quinoxaline
HPLC maintenance time (minutes): 3.37;
Mass data: 427 $(M+H)^+$.

Example 14(20)

2-(2-cyclopentylethyloxy)-3-(4-methylphenylsulfonylamino)quinoxaline
HPLC maintenance time (minutes): 4.48;
Mass data: 845 $(2M+Na)^+$, 412 $(M+H)^+$.

Example 14(21)

2-(2-(morpholin-4-yl)ethyloxy)-3-(4-methylphenylsulfonylamino)quinoxaline
HPLC maintenance time (minutes): 3.30;
Mass data: 429 $(M+H)^+$.

Example 14(22)

2-(2-(pyrazol-1-yl)ethyloxy)-3-(4-methylphenylsulfonylamino)quinoxaline
HPLC maintenance time (minutes): 3.75;
Mass data: 410 $(M+H)^+$.

Example 14(23)

2-(2-cyclopropylethyloxy)-3-(4-methylphenylsulfonylamino)quinoxaline
HPLC maintenance time (minutes): 4.20;
Mass data: 789 $(2M+Na)^+$, 384 $(M+H)^+$.

Example 14(24)

2-((pyridin-2-yl)methyloxy)-3-(4-methylphenylsulfonylamino)quinoxaline
HPLC maintenance time (minutes): 3.40;
Mass data: 407 $(M+H)^+$, 242.

Example 14(25)

2-(2-cyclohexylethyloxy)-3-(4-methylphenylsulfonylamino)quinoxaline
HPLC maintenance time (minutes): 4.55;
Mass data: 873 $(2M+Na)^+$, 426 $(M+H)^+$.

Example 14(26)

2-(3-(piperidin-1-yl)propyloxy)-3-(4-methylphenylsulfonylamino)quinoxaline
HPLC maintenance time (minutes): 3.42;
Mass data: 441 $(M+H)^+$.

Example 14(27)

2-(cyclopentylmethyloxy)-3-(4-methylphenylsulfonylamino)quinoxaline
HPLC maintenance time (minutes): 4.35;
Mass data: 817 $(2M+Na)^+$, 398 $(M+H)^+$.

Example 14(28)

2-(2-phenylethyloxy)-3-(4-methylphenylsulfonylamino)quinoxaline
HPLC maintenance time (minutes): 4.21;
Mass data: 420 $(M+H)^+$.

Example 14(29)

2-((pyridin-2-yl)methyloxy)-3-(phenylsulfonylamino)quinoxaline
HPLC maintenance time (minutes): 3.33;
Mass data: 393 $(M+H)^+$, 302.

Example 14(30)

2-((pyridin-2-yl)methyloxy)-3-(2-methylphenylsulfonylamino)quinoxaline
HPLC maintenance time (minutes): 3.38;
Mass data: 407 $(M+H)^+$, 316.

Example 14(31)

2-((pyridin-2-yl)methyloxy)-3-(3-methylphenylsulfonylamino)quinoxaline
HPLC maintenance time (minutes): 3.42;
Mass data: 407 $(M+H)^+$.

Example 14(32)

2-((pyridin-2-yl)methyloxy)-3-(4-methoxyphenylsulfonylamino)quinoxaline
HPLC maintenance time (minutes): 3.36;
Mass data: 423 $(M+H)^+$.

Example 14(33)

2-(2-cyclohexylethyloxy)-3-(phenylsulfonylamino)quinoxaline
HPLC maintenance time (minutes): 4.48;
Mass data: 412 $(M+H)^+$.

Example 14(34)

2-(2-cyclohexylethyloxy)-3-(2-methylphenylsulfonylamino)quinoxaline
HPLC maintenance time (minutes): 4.57;
Mass data: 426 $(M+H)^+$.

Example 14(35)

2-(2-cyclohexylethyloxy)-3-(3-methylphenylsulfonylamino)quinoxaline
HPLC maintenance time (minutes): 4.59;
Mass data: 426 $(M+H)^+$.

Example 14(36)

2-(2-cyclohexylethyloxy)-3-(4-methoxyphenylsulfonylamino)quinoxaline
HPLC maintenance time (minutes): 4.50;

Mass data: 442 $(M+H)^+$.

Example 14(37)

2-(3-(piperidin-1-yl)propyloxy)-3-(phenylsulfonylamino)quinoxaline
HPLC maintenance time (minutes): 3.33;
Mass data: 427 $(M+H)^+$.

Example 14(38)

2-(3-(piperidin-1-yl)propyloxy)-3-(2-methylphenylsulfonylamino)quinoxaline
HPLC maintenance time (minutes): 3.40;
Mass data: 441 $(M+H)^+$.

Example 14(39)

2-(3-(piperidin-1-yl)propyloxy)-3-(3-methylphenylsulfonylamino)quinoxaline
HPLC maintenance time (minutes): 3.42;
Mass data: 441 $(M+H)^+$.

Example 14(40)

2-(3-(piperidin-1-yl)propyloxy)-3-(4-methoxyphenylsulfonylamino)quinoxaline
HPLC maintenance time (minutes): 3.36;
Mass data: 457 $(M+H)^+$.

Example 14(41)

2-(cyclopentylmethyloxy)-3-(phenylsulfonylamino)quinoxaline
HPLC maintenance time (minutes): 4.26;
Mass data: 789 $(2M+Na)^+$, 384 $(M+H)^+$.

Example 14(42)

2-(cyclopentylmethyloxy)-3-(2-methylphenylsulfonylamino)quinoxaline
HPLC maintenance time (minutes): 4.35;
Mass data: 817 $(2M+Na)^+$, 398 $(M+H)^+$.

Example 14(43)

2-(cyclopentylmethyloxy)-3-(3-methylphenylsulfonylamino)quinoxaline
HPLC maintenance time (minutes): 4.35;
Mass data: 817 $(2M+Na)^+$, 398 $(M+H)^+$.

Example 14(44)

2-(cyclopentylmethyloxy)-3-(4-methoxyphenylsulfonylamino)quinoxaline
HPLC maintenance time (minutes): 4.28;
Mass data: 849 $(2M+Na)^+$, 414 $(M+H)^+$.

Example 14(45)

2-(2-pneylethyloxy)-3-(phenylsulfonylamino)quinoxaline
HPLC maintenance time (minutes): 4.13;
Mass data: 833 $(2M+Na)^+$, 406 $(M+H)^+$.

Example 14(46)

2-(2-phenylethyloxy)-3-(2-methylphenylsulfonylamino)quinoxaline
HPLC maintenance time (minutes): 4.22;
Mass data: 861 $(2M+Na)^+$, 420 $(M+H)^+$.

Example 14(47)

2-(2-phenylethyloxy)-3-(3-methylphenylsulfonylamino)quinoxaline
HPLC maintenance time (minutes): 4.23;
Mass data: 861 $(2M+Na)^+$, 420 $(M+H)^+$.

Example 14(48)

2-(2-phenylethyloxy)-3-(4-methoxyphenylsulfonylamino)quinoxaline
HPLC maintenance time (minutes): 4.15;
Mass data: 893 $(2M+Na)^+$, 436 $(M+H)^+$.

Example 14(49)

2-(2-(N-methyl-N-benzylamino)ethyloxy)-3-(2-methylphenylsulfonylamino)quinoxaline
HPLC maintenance time (minutes): 3.49;
Mass data: 463 $(M+H)^+$.

Example 14(50)

2-(2-(N-methyl-N-benzylamino)ethyloxy)-3-(3-methylphenylsulfonylamino)quinoxaline
HPLC maintenance time (minutes): 3.49;
Mass data: 463 $(M+H)^+$.

Example 14(51)

2-(2-(N-methyl-N-benzylamino)ethyloxy)-3-(phenylsulfonylamino)quinoxaline
HPLC maintenance time (minutes): 3.40;
Mass data: 449 $(M+H)^+$.

Example 14(52)

2-(2-(N-methyl-N-benzylamino)ethyloxy)-3-(4-methoxyphenylsulfonylamino)quinoxaline
HPLC maintenance time (minutes): 3.42;
Mass data: 479 $(M+H)^+$.

Example 14(53)

2-(2-(N-methyl-N-phenylamino)ethyloxy)-3-(2-methylphenylsulfonylamino)quinoxaline
HPLC maintenance time (minutes): 3.67;
Mass data: 449 $(M+H)^+$.

Example 14(54)

2-(2-(N-methyl-N-phenylamino)ethyloxy)-3-(3-methylphenylsulfonylamino)quinoxaline
HPLC maintenance time (minutes): 3.67;
Mass data: 449 $(M+H)^+$.

Example 14(55)

2-(2-(N-methyl-N-phenylamino)ethyloxy)-3-(phenylsulfonylamino)quinoxaline
HPLC maintenance time (minutes): 3.58;

Mass data: 435 $(M+H)^+$.

Example 14(56)

2-(2-(N-methyl-N-phenylamino)ethyloxy)-3-(4-methoxyphenylsulfonylamino)quinoxaline
HPLC maintenance time (minutes): 3.62;
Mass data: 465 $(M+H)^+$.

Example 14(57)

2-(2-(3,5-dimethylpyrazol-1-yl)ethyloxy)-3-(2-methylphenylsulfonylamino)quinoxaline
HPLC maintenance time (minutes): 3.60;
Mass data: 897 $(2M+Na)^+$, 438 $(M+H)^+$.

Example 14(58)

2-(2-(3,5-dimethylpyrazol-1-yl)ethyloxy)-3-(3-methylphenylsulfonylamino)quinoxaline
HPLC maintenance time (minutes): 3.64;
Mass data: 897 $(2M+Na)^+$, 438 $(M+H)^+$.

Example 14(59)

2-(2-(3,5-dimethylpyrazol-1-yl)ethyloxy)-3-(phenylsulfonylamino)quinoxaline
HPLC maintenance time (minutes): 3.51;
Mass data: 869 $(2M+Na)^+$, 424 $(M+H)^+$.

Example 14(60)

2-(2-(3,5-dimethylpyrazol-1-yl)ethyloxy)-3-(4-methoxyphenylsulfonylamino)quinoxaline
HPLC maintenance time (minutes): 3.55;
Mass data: 929 $(2M+Na)^+$, 454 $(M+H)^+$.

Example 14(61)

2-(2-phenoxyethyloxy)-3-(2-methylphenylsulfonylamino)quinoxaline
HPLC maintenance time (minutes): 4.15;
Mass data: 893 $(2M+Na)^+$, 436 $(M+H)^+$.

Example 14(62)

2-(3-benzyloxypropyloxy)-3-(3-methylphenylsulfonylamino)quinoxaline
HPLC maintenance time (minutes): 4.22;
Mass data: 949 $(2M+Na)^+$, 464 $(M+H)^+$.

Example 14(63)

2-(2-phenoxyethyloxy)-3-(phenylsulfonylamino)quinoxaline
HPLC maintenance time (minutes): 4.06;
Mass data: 865 $(2M+Na)^+$, 422 $(M+H)^+$.

Example 14(64)

2-(2-phenoxyethyloxy)-3-(4-methoxyphenylsulfonylamino)quinoxaline
HPLC maintenance time (minutes): 4.08;
Mass data: 925 $(2M+Na)^+$, 452 $(M+H)^+$.

Example 14(65)

2-(3-phenylpropyloxy)-3-(2-methylphenylsulfonylamino)quinoxaline
HPLC maintenance time (minutes): 4.32;
Mass data: 889 $(2M+Na)^+$, 434 $(M+H)^+$, 120.

Example 14(66)

2-(3-phenylpropyloxy)-3-(3-methylphenylsulfonylamino)quinoxaline
HPLC maintenance time (minutes): 4.32;
Mass data: 889 $(2M+Na)^+$, 434 $(M+H)^+$, 120.

Example 14(67)

2-(3-phenylpropyloxy)-3-(phenylsulfonylamino)quinoxaline
HPLC maintenance time (minutes): 4.22;
Mass data: 861 $(2M+Na)^+$, 420 $(M+H)^+$, 120.

Example 14(68)

2-(3-phenylpropyloxy)-3-(4-methoxyphenylsulfonylamino)quinoxaline
HPLC maintenance time (minutes): 4.24;
Mass data: 921 $(2M+Na)^+$, 450 $(M+H)^+$, 120.

Example 14(69)

2-(4-phenylbutyloxy)-3-(2-methylphenylsulfonylamino)quinoxaline HPLC maintenance time (minutes): 4.39;
Mass data: 917 $(2M+Na)^+$, 448 $(M+H)^+$.

Example 14(70)

2-(4-phenylbutyloxy)-3-(3-methylphenylsulfonylamino)quinoxaline
HPLC maintenance time (minutes): 4.41;
Mass data: 917 $(2M+Na)^+$, 448 $(M+H)^+$.

Example 14(71)

2-(4-phenylbutyloxy)-3-(phenylsulfonylamino)quinoxaline
HPLC maintenance time (minutes): 4.32;
Mass data: 889 $(2M+Na)^+$, 434 $(M+H)^+$.

Example 14(72)

2-(4-phenylbutyloxy)-3-(4-methoxyphenylsulfonylamino)quinoxaline
HPLC maintenance time (minutes): 4.32;
Mass data: 949 $(2M+Na)^+$, 464 $(M+H)^+$.

Example 14(73)

2-(2-(thiophen-2-yl)ethyloxy)-3-(2-methylphenylsulfonylamino)quinoxaline
HPLC maintenance time (minutes): 4.17;
Mass data: 873 $(2M+Na)^+$, 426 $(M+H)^+$.

Example 14(74)

2-(2-(thiophen-2-yl)ethyloxy)-3-(3-methylphenylsulfonylamino)quinoxaline
HPLC maintenance time (minutes): 4.17;
Mass data: 873 $(2M+Na)^+$, 426 $(M+H)^+$.

Example 14(75)

2-(2-(thiophen-2-yl)ethyloxy)-3-(phenylsulfonylamino)quinoxaline
HPLC maintenance time (minutes): 4.09;
Mass data: 845 $(2M+Na)^+$, 412 $(M+H)^+$.

Example 14(76)

2-(2-(thiophen-2-yl)ethyloxy)-3-(4-methoxyphenylsulfonylamino)quinoxaline
HPLC maintenance time (minutes): 4.10;
Mass data: 905 $(2M+Na)^+$, 442 $(M+H)^+$.

Example 14(77)

2-(3-(pyridin-3-yl)propyloxy)-3-(2-methylphenylsulfonylamino)quinoxaline
HPLC maintenance time (minutes): 3.38;
Mass data: 891 $(2M+Na)^+$, 435 $(M+H)^+$.

Example 14(78)

2-(3-(pyridin-3-yl)propyloxy)-3-(3-methylphenylsulfonylamino)quinoxaline
HPLC maintenance time (minutes): 3.40;
Mass data: 891 $(2M+Na)^+$, 435 $(M+H)^+$.

Example 14(79)

2-(3-(pyridin-3-yl)propyloxy)-3-(phenylsulfonylamino)quinoxaline
HPLC maintenance time (minutes): 3.33;
Mass data: 863 $(2M+Na)^+$, 421 $(M+H)^+$.

Example 14(80)

2-(3-benzyloxypropyloxy)-3-(2-methylphenylsulfonylamino)quinoxaline
HPLC maintenance time (minutes): 4.22;
Mass data: 949 $(2M+Na)^+$, 464 $(M+H)^+$.

Example 14(81)

2-(3-benzyloxypropyloxy)-3-(phenylsulfonylamino)quinoxaline
HPLC maintenance time (minutes): 4.13;
Mass data: 921 $(2M+Na)^+$, 450 $(M+H)^+$.

Example 14(82)

2-(3-(benzyloxy)propyloxy)-3-(4-methoxyphenylsulfonylamino)quinoxaline
HPLC maintenance time (minutes): 4.13;
Mass data: 981 $(2M+Na)^+$, 480 $(M+H)^+$.

Example 14(83)

2-(2-(pyridin-2-yl)ethyloxy)-3-(2-methylphenylsulfonylamino)quinoxaline
HPLC maintenance time (minutes): 3.34;
Mass data: 863 $(2M+Na)^+$, 421 $(M+H)^+$.

Example 14(84)

2-(2-(pyridin-2-yl)ethyloxy)-3-(phenylsulfonylamino)quinoxaline
HPLC maintenance time (minutes): 3.27;

Mass data: 835 $(2M+Na)^+$, 407 $(M+H)^+$.

Example 14(85)

2-(2-(pyridin-2-yl)ethyloxy)-3-(4-methoxyphenylsulfonylamino)quinoxaline
HPLC maintenance time (minutes): 3.31;
Mass data: 895 $(2M+Na)^+$, 437 $(M+H)^+$.

Example 14(86)

2-(3-(pyridin-2-yl)propyloxy)-3-(2-methylphenylsulfonylamino)quinoxaline
HPLC maintenance time (minutes): 3.36; Mass data: 891 $(2M+Na)^+$, 435 $(M+H)^+$.

Example 14(87)

2-(3-(pyridin-2-yl)propyloxy)-3-(3-methylphenylsulfonylamino)quinoxaline
HPLC maintenance time (minutes): 3.38;
Mass data: 891 $(2M+Na)^+$, 435 $(M+H)^+$.

Example 14(88)

2-(3-(pyridin-2-yl)propyloxy)-3-(phenylsulfonylamino)quinoxaline
HPLC maintenance time (minutes): 3.31;
Mass data: 863 $(2M+Na)^+$, 421 $(M+H)^+$.

Example 14(89)

2-(3-(pyridin-2-yl)propyloxy)-3-(4-methoxyphenylsulfonylamino)quinoxaline
HPLC maintenance time (minutes): 3.33;
Mass data: 923 $(2M+Na)^+$, 451 $(M+H)^+$, 332.

Example 14(90)

2-(2-(pyridin-4-yl)ethyloxy)-3-(2-methylphenylsulfonylamino)quinoxaline
HPLC maintenance time (minutes): 3.33;
Mass data: 841 $(2M+H)^+$, 421 $(M+H)^+$.

Example 14(91)

2-(2-(pyridin-4-yl)ethyloxy)-3-(3-methylphenylsulfonylamino)quinoxaline
HPLC maintenance time (minutes): 3.34;
Mass data: 841 $(2M+H)^+$, 421 $(M+H)^+$.

Example 14(92)

2-(2-(pyridin-4-yl)ethyloxy)-3-(phenylsulfonylamino)quinoxaline
HPLC maintenance time (minutes): 3.27;
Mass data: 813 $(2M+H)^+$, 407 $(M+H)^+$.

Example 14(93)

2-(2-(pyridin-4-yl)ethyloxy)-3-(4-methoxyphenylsulfonylamino)quinoxaline
HPLC maintenance time (minutes): 3.29;
Mass data: 873 $(2M+H)^+$, 437 $(M+H)^+$.

Example 14(94)

2-((tetrahydrofuran-2-yl)methyloxy)-3-(2-methylphenylsulfonylamino)quinoxaline

HPLC maintenance time (minutes): 3.93;
Mass data: 821 $(2M+Na)^+$, 400 $(M+H)^+$.

Example 14(95)

2-((tetrahydrofuran-2-yl)methyloxy)-3-(3-methylphenylsulfonylamino)quinoxaline
HPLC maintenance time (minutes): 3.93;
Mass data: 821 $(2M+Na)^+$, 400 $(M+H)^+$.

Example 14(96)

2-((tetrahydrofuran-2-yl)methyloxy)-3-(phenylsulfonylamino)quinoxaline
HPLC maintenance time (minutes): 3.82;
Mass data: 793 $(2M+Na)^+$, 386 $(M+H)^+$.

Example 14(97)

2-((tetrahydrofuran-2-yl)methyloxy)-3-(4-methoxyphenylsulfonylamino)quinoxaline
HPLC maintenance time (minutes): 3.84;
Mass data: 853 $(2M+Na)^+$, 416 $(M+H)^+$.

Example 14(98)

2-(cyclohexylmethyloxy)-3-(2-methylphenylsulfonylamino)quinoxaline
HPLC maintenance time (minutes): 4.50;
Mass data: 845 $(2M+Na)^+$, 412 $(M+H)^+$.

Example 14(99)

2-((cyclohexyl)methyloxy)-3-(3-methylphenylsulfonylamino)quinoxaline
HPLC maintenance time (minutes): 4.48;
Mass data: 845 $(2M+Na)^+$, 412 $(M+H)^+$.

Example 14(100)

2-(cyclohexylmethyloxy)-3-(phenylsulfonylamino)quinoxaline
HPLC maintenance time (minutes): 4.37;
Mass data: 817 $(2M+Na)^+$, 398 $(M+H)^+$.

Example 14(101)

2-(cyclohexylmethyloxy)-3-(4-methoxyphenylsulfonylamino)quinoxaline
HPLC maintenance time (minutes): 4.39;
Mass data: 877 $(2M+Na)^+$, 428 $(M+H)^+$.

Example 14(102)

2-(2-(piperidin-1-yl)ethyloxy)-3-(2-methylphenylsulfonylamino)quinoxaline
HPLC maintenance time (minutes): 3.35;
Mass data: 427 $(M+H)^+$.

Example 14(103)

2-(2-(piperidin-1-yl)ethyloxy)-3-(3-methylphenylsulfonylamino)quinoxaline
HPLC maintenance time (minutes): 3.36;
Mass data: 427 $(M+H)^+$.

Example 14(104)

2-(2-(piperidin-1-yl)ethyloxy)-3-(phenylsulfonylamino)quinoxaline
HPLC maintenance time (minutes): 3.29;
Mass data: 413 $(M+H)^+$.

Example 14(105)

2-(2-(piperidin-1-yl)ethyloxy)-3-(4-methoxyphenylsulfonylamino)quinoxaline
HPLC maintenance time (minutes): 3.33;
Mass data: 443 $(M+H)^+$.

Example 14(106)

2-(2-cyclopentylethyloxy)-3-(2-methylphenylsulfonylamino)quinoxaline
HPLC maintenance time (minutes): 4.46;
Mass data: 845 $(2M+Na)^+$, 412 $(M+H)^+$.

Example 14(107)

2-(2-cyclopentylethyloxy)-3-(3-methylphenylsulfonylamino)quinoxaline
HPLC maintenance time (minutes): 4.46;
Mass data: 845 $(2M+Na)^+$, 412 $(M+H)^+$.

Example 14(108)

2-(2-cyclopentylethyloxy)-3-(phenylsulfonylamino)quinoxaluie
HPLC maintenance time (minutes): 4.37;
Mass data: 817 $(2M+Na)^+$, 398 $(M+H)^+$.

Example 14(109)

2-(2-cyclopentylethyloxy)-3-(4-methoxyphenylsulfonylamino)quinoxaline
HPLC maintenance time (minutes): 4.39;
Mass data: 877 $(2M+Na)^+$, 428 $(M+H)^+$.

Example 14(110)

2-(2-(morpholin-4-yl)ethyloxy)-3-(2-methylphenylsulfonylainino)quinoxaline
HPLC maintenance time (minutes): 3.27;
Mass data: 879 $(2M+Na)^+$, 429 $(M+H)^+$.

Example 14(111)

2-(2-(morpholin-4-yl)ethyloxy)-3-(3-methylphenylsulfonylamino)quinoxaline
HPLC maintenance time (minutes): 3.29;
Mass data: 879 $(2M+Na)^+$, 429 $(M+H)^+$.

Example 14(112)

2-(2-(morpholin-4-yl)ethyloxy)-3-(phenylsulfonylamino)quinoxaline
HPLC maintenance time (minutes): 3.22;
Mass data: 415 $(M+H)^+$.

Example 14(113)

2-(2-(morpholin-4-yl)ethyloxy)-3-(4-methoxyphenylsulfonylamino)quinoxaline HPLC maintenance time (minutes): 3.25;

Mass data: 911 $(2M+Na)^+$, 445 $(M+H)^+$.

Example 14(114)

2-(2-(pyrazol-1-yl)ethyloxy)-3-(2-methylphenylsulfonylamino)quinoxaline
HPLC maintenance time (minutes): 3.77;
Mass data: 841 $(2M+Na)^+$, 410 $(M+H)^+$.

Example 14(115)

2-(2-(pyrazol-1-yl)ethyloxy)-3-(3-methylphenylsulfonylamino)quinoxaline
HPLC maintenance time (minutes): 3.77;
Mass data: 841 $(2M+Na)^+$, 410 $(M+H)^+$.

Example 14(116)

2-(2-(pyrazol-1-yl)ethyloxy)-3-(phenylsulfonylamino)quinoxaline
HPLC maintenance time (minutes): 3.67;
Mass data: 813 $(2M+Na)^+$, 396 $(M+H)^+$.

Example 14(117)

2-(2-(pyrazol-1-yl)ethyloxy)-3-(4-methoxyphenylsulfonylamino)quinoxaline
HPLC maintenance time (minutes): 3.71;
Mass data: 873 $(2M+Na)^+$, 426 $(M+H)^+$.

Example 14(118)

2-(2-cyclopropylethyloxy)-3-(2-methylphenylsulfonylamino)quinoxaline
HPLC maintenance time (minutes): 4.21;
Mass data: 789 $(2M+Na)^+$, 384 $(M+H)^+$.

Example 14(119)

2-(2-cyclopropylethyloxy)-3-(3-methylphenylsulfonylamino)quinoxaline
HPLC maintenance time (minutes): 4.21;
Mass data: 789 $(2M+Na)^+$, 384 $(M+H)^+$.

Example 14(120)

2-(2-cyclopropylethyloxy)-3-(phenylsulfonylamino)quinoxaline
HPLC maintenance time (minutes): 4.10;
Mass data: 761 $(2M+Na)^+$, 370 $(M+H)^+$.

Example 14(121)

2-(2-cyclopropylethyloxy)-3-(4-methoxyphenylsulfonylamino)quinoxaline
HPLC maintenance time (minutes): 4.11;
Mass data: 821 $(2M+Na)^+$, 400 $(M+H)^+$.

Example 14(122)

2-((pyridin-3-yl)methyloxy)-3-(2-methylphenylsulfonylamino)quinoxaline
HPLC maintenance time (minutes): 3.33;
Mass data: 813 $(2M+H)^+$, 407 $(M+H)^+$.

Example 14(123)

2-((pyridin-3-yl)methyloxy)-3-(3-methylphenylsulfonylamino)quinoxaline
HPLC maintenance time (minutes): 3.33;
Mass data: 813 $(2M+H)^+$, 407 $(M+H)^+$.

Example 14(124)

2-((pyridin-3-yl)methyloxy)-3-(phenylsulfonylamino)quinoxaline
HPLC maintenance time (minutes): 3.27;
Mass data: 785 $(2M+H)^+$, 393 $(M+H)^+$.

Example 14(125)

2-((pyridin-3-yl)methyloxy)-3-(4-methoxyphenylsulfonylamino)quinoxaline
HPLC maintenance time (minutes): 3.29;
Mass data: 845 $(2M+H)^+$, 423 $(M+H)^+$.

Example 14(126)

2-((pyridin-4-yl)methyloxy)-3-(2-methylphenylsulfonylamino)quinoxaline
HPLC maintenance time (minutes): 3.31;
Mass data: 813 $(2M+H)^+$, 407 $(M+H)^+$.

Example 14(127)

2-((pyridin-4-yl)methyloxy)-3-(3-methylphenylsulfonylamino)quinoxaline
HPLC maintenance time (minutes): 3.33;
Mass data: 813 $(2M+H)^+$, 407 $(M+H)^+$.

Example 14(128)

2-((pyridin-4-yl)methyloxy)-3-(phenylsulfonylamino)quinoxaline
HPLC maintenance time (minutes): 3.25;
Mass data: 785 $(2M+H)^+$, 393 $(M+H)^+$.

Example 14(129)

2-((pyridin-4-yl)methyloxy)-3-(4-methoxyphenylsulfonylamino)quinoxaline
HPLC maintenance time (minutes): 3.29;
Mass data: 845 $(2M+H)^+$, 423 $(M+H)^+$.

Example 14(130)

2-(2-(N-methyl-N-benzylamino)ethyloxy)-3-(4-cyanophenylsulfonylamino)quinoxaline
HPLC maintenance time (minutes): 3.40;
Mass data: 474 $(M+H)^+$.

Example 14(131)

2-(2-(N-methyl-N-benzylamino)ethyloxy)-3-(4-bromoplienylsulfonylamino)quinoxaline
HPLC maintenance time (minutes): 3.55;
Mass data: 529 $(M+H)^+$.

Example 14(132)

2-(2-(N-methyl-N-phenylamino)ethyloxy)-3-(4-cyanophenylsulfonylamino)quinoxaline
HPLC maintenance time (minutes): 3.62;

Mass data: 460 $(M+H)^+$.

Example 14(133)

2-(2-(N-methyl-N-phenylamino)ethyloxy)-3-(4-bromophenylsulfonylamino)quinoxaline
HPLC maintenance time (minutes): 3.78;
Mass data: 513 $(M+H)^+$.

Example 14(134)

2-(2-phenoxyethyloxy)-3-(4-cyanophenylsulfonylamino)quinoxaline
HPLC maintenance time (minutes): 4.04;
Mass data: 469 $(M+Na)^+$, 447 $(M+H)^+$.

Example 14(135)

2-(2-phenoxyethyloxy)-3-(4-bromophenylsulfonylamino)quinoxaline
HPLC maintenance time (minutes): 4.26;
Mass data: 522 $(M+Na)^+$, 500 $(M+H)^+$.

Example 14(136)

2-(3-phenylpropyloxy)-3-(4-cyanophenylsulfonylamino)quinoxaline
HPLC maintenance time (minutes): 4.21;
Mass data: 467 $(M+Na)^+$, 445 $(M+H)^+$.

Example 14(137)

2-(3-phenylpropyloxy)-3-(4-bromophenylsulfonylamino)quinoxaline
HPLC maintenance time (minutes): 4.43;
Mass data: 520 $(M+Na)^+$, 498 $(M+H)^+$.

Example 14(138)

2-(4-phenylbutyloxy)-3-(4-cyanophenylsulfonylamino)quinoxaline
HPLC maintenance time (minutes): 4.28;
Mass data: 481 $(M+Na)^+$, 459 $(M+H)^+$.

Example 14(139)

2-(4-phenylbutyloxy)-3-(4-bromophenylsulfonylamino)quinoxaline
HPLC maintenance time (minutes): 4.52;
Mass data: 534 $(M+Na)^+$, 512 $(M+H)^+$.

Example 14(140)

2-(3-(pyridin-3-yl)propyloxy)-3-(4-cyanophenylsulfonylamino)quinoxaline
HPLC maintenance time (minutes): 3.33;
Mass data: 446 $(M+H)^+$.

Example 14(141)

2-(3-(pyridin-3-yl)propyloxy)-3-(4-bromophenylsulfonylamino)quinoxaline
HPLC maintenance time (minutes): 3.47;
Mass data: 499 $(M+H)^+$.

Example 14(142)

2-(3-benzyloxypropyloxy)-3-(4-cyanophenylsulfonylamino)quinoxaline
HPLC maintenance time (minutes): 4.11;
Mass data: 497 $(M+Na)^+$, 475 $(M+H)^+$.

Example 14(143)

2-(3-benzyloxypropyloxy)-3-(4-bromophenylsulfonylamino)quinoxaline
HPLC maintenance time (minutes): 4.34;
Mass data: 550 $(M+Na)^+$, 528 $(M+H)^+$.

Example 14(144)

2-(2-(pyridin-2-yl)ethyloxy)-3-(4-cyanophenylsulfonylamino)quinoxaline
HPLC maintenance time (minutes): 3.27;
Mass data: 432 $(M+H)^+$.

Example 14(145)

2-(2-(pyridin-2-yl)ethyloxy)-3-(4-bromophenylsulfonylamino)quinoxaline
HPLC maintenance time (minutes): 3.42;
Mass data: 485 $(M+H)^+$.

Example 14(146)

2-(3-(pyridin-2-yl)propyloxy)-3-(4-cyanophenylsulfonylamino)quinoxaline
HPLC maintenance time (minutes): 3.31;
Mass data: 446 $(M+H)^+$.

Example 14(147)

2-(3-(pyridin-2-yl)propyloxy)-3-(4-bromophenylsulfonylamino)quinoxaline
HPLC maintenance time (minutes): 3.47;
Mass data: 499 $(M+H)^+$.

Example 14(148)

2-(2-(pyridin-4-yl)ethyloxy)-3-(4-cyanophenylsulfonylamino)quinoxaline
HPLC maintenance time (minutes): 3.27;
Mass data: 432 $(M+H)^+$.

Example 14(149)

2-((tetrahydrofuran-2-yl)methyloxy)-3-(4-cyanophenylsulfonylamino)quinoxaline
HPLC maintenance time (minutes): 3.82;
Mass data: 433 $(M+Na)^+$, 411 $(M+H)^+$.

Example 14(150)

2-((tetrahydrofuran-2-yl)methyloxy)-3-(4-bromophenylsulfonylamino)quinoxaline
HPLC maintenance time (minutes): 4.06;
Mass data: 486 $(M+Na)^+$, 464 $(M+H)^+$.

Example 14(151)

2-(cyclohexylmethyloxy)-3-(4-cyanophenylsulfonylamino)quinoxaline
HPLC maintenance time (minutes): 4.35;

Mass data: 423 $(M+H)^+$.

Example 14(152)

2-(cyclohexylmethyloxy)-3-(4-bromophenylsulfonylamino)quinoxaline HPLC maintenance time (minutes): 4.59; Mass data: 476 $(M+H)^+$.

Example 14(153)

2-(2-(piperidin-1-yl)ethyloxy)-3-(4-cyanophenylsulfonylamino)quinoxaline HPLC maintenance time (minutes): 3.29; Mass data: 438 $(M+H)^+$.

Example 14(154)

2-(2-(piperidin-1-yl)ethyloxy)-3-(4-bromophenylsulfonylamino)quinoxaline HPLC maintenance time (minutes): 3.44; Mass data: 491 $(M+H)^+$.

Example 14(155)

2-(2-cyclopentylethyloxy)-3-(4-cyanophenylsulfonylamino)quinoxaline HPLC maintenance time (minutes): 4.33; Mass data: 423 $(M+H)^+$.

Example 14(156)

2-(2-cyclopentylethyloxy)-3-(4-bromophenylsulfonylamino)quinoxaline HPLC maintenance time (minutes): 4.59; Mass data: 476 $(M+H)^+$.

Example 14(157)

2-(2-(morpholin-4-yl)ethyloxy)-3-(4-cyanophenylsulfonylamino)quinoxaline HPLC maintenance time (minutes): 3.22; Mass data: 440 $(M+H)^+$.

Example 14(158)

2-(2-(morpholin-4-yl)ethyloxy)-3-(4-bromophenylsulfonylamino)quinoxaline HPLC maintenance time (minutes): 3.36; Mass data: 493 $(M+H)^+$.

Example 14(159)

2-(2-cyclopropylethyloxy)-3-(4-cyanophenylsulfonylamino)quinoxaline HPLC maintenance time (minutes): 4.08; Mass data: 395 $(M+H)^+$.

Example 14(160)

2-(2-cyclopropylethyloxy)-3-(4-bromophenylsulfonylamino)quinoxaline HPLC maintenance time (minutes): 4.33; Mass data: 448 $(M+H)^+$.

Example 14(161)

2-((pyridin-2-yl)methyloxy)-3-(4-cyanophenylsulfonylamino)quinoxaline HPLC maintenance time (minutes): 3.34;
Mass data: 418 $(M+H)^+$.

Example 14(162)

2-((pyridin-2-yl)methyloxy)-3-(4-bromophenylsulfonylamino)quinoxaline HPLC maintenance time (minutes): 3.53;
Mass data: 471 $(M+H)^+$.

Example 14(163)

2-(2-cyclohexylethyloxy)-3-(4-cyanophenylsulfonylamino)quinoxaline HPLC maintenance time (minutes): 4.45;
Mass data: 437 $(M+H)^+$.

Example 14(164)

2-(2-cyclohexylethyloxy)-3-(4-bromophenylsulfonylamino)quinoxaline HPLC maintenance time (minutes): 4.72;
Mass data: 490 $(M+H)^+$.

Example 14(165)

2-(3-(piperidin-1-yl)propyloxy)-3-(4-cyanophenylsulfonylamino)quinoxaline HPLC maintenance time (minutes): 3.33;
Mass data: 452 $(M+H)^+$.

Example 14(166)

2-(3-(piperidin-1-yl)propyloxy)-3-(4-bromophenylsulfonylanuno)quinoxaline HPLC maintenance time (minutes): 3.49;
Mass data: 505 $(M+H)^+$.

Example 14(167)

2-(2-phenylethyloxy)-3-(4-cyanophenylsulfonylamino)quinoxaline HPLC maintenance time (minutes): 4.10;
Mass data: 453 $(M+Na)^+$, 431 $(M+H)^+$.

Example 14(168)

2-(2-phenylethyloxy)-3-(4-bromophenylsulfonylamino)quinoxaline HPLC maintenance time (minutes): 4.34;
Mass data: 506 $(M+Na)^+$, 484 $(M+H)^+$.

Example 14(169)

2-((pyridin-3-yl)methyloxy)-3-(4-cyanophenylsulfonylamino)quinoxaline HPLC maintenance time (minutes): 3.25;
Mass data: 418 $(M+H)^+$.

Example 14(170)

2-((pyridin-3-yl)methyloxy)-3-(4-bromophenylsulfonylamino)quinoxaline HPLC maintenance time (minutes): 3.40;

Mass data: 471 $(M+H)^+$.

Example 14(171)

2-((pyridin-4-yl)methyloxy)-3-(4-cyanophenylsulfonylamino)quinoxaline
HPLC maintenance time (minutes): 3.25;
Mass data: 418 $(M+H)^+$.

Example 14(172)

2-((pyridin-4-yl)methyloxy)-3-(4-bromophenylsulfonylamino)quinoxaline
HPLC maintenance time (minutes): 3.40;
Mass data: 471 $(M+H)^+$.

Example 14(173)

2-(2-(N-methyl-N-phenylamino)ethyloxy)-3-(4-fluorophenylsulfonylamino)quinoxaline
HPLC maintenance time (minutes): 3.80;
Mass data: 453 $(M+H)^+$.

Example 14(174)

2-(2-phenoxyethyloxy)-3-(4-fluorophenylsulfonylamino)quinoxaline
HPLC maintenance time (minutes): 4.28;
Mass data: 901 $(2M+Na)^+$, 440 $(M+H)^+$.

Example 14(175)

2-(2-(thiophen-2-yl)ethyloxy)-3-(4-fluorophenylsulfonylamino)pyrazine
HPLC maintenance time (minutes): 4.32;
Mass data: 881 $(2M+Na)^+$, 430 $(M+H)^+$.

Example 14(176)

2-(cyclopentylmethyloxy)-3-(4-fluorophenylsulfonylamino)quinoxalme
HPLC maintenance time (minutes): 4.50;
Mass data: 825 $(2M+Na)^+$, 402 $(M+H)^+$.

Example 14(177)

2-(2-phenylethyloxy)-3-(4-fluorophenylsulfonylamino)quinoxaline
HPLC maintenance time (minutes): 4.35;
Mass data: 869 $(2M+Na)^+$, 424 $(M+H)^+$.

Example 14(178)

2-((pyridin-3-yl)methyloxy)-3-(4-fluorophenylsulfonylamino)quinoxaline
HPLC maintenance time (minutes): 3.44;
Mass data: 821 $(2M+H)^+$, 411 $(M+H)^+$.

Example 14(179)

2-((pyridin-4-yl)methyloxy)-3-(4-fluorophenylsulfonylamino)quinoxaline
HPLC maintenance time (minutes): 3.44;
Mass data: 411 $(M+H)^+$.

Example 14(180)

2-(2-(N-methyl-N-phenylamino)ethyloxy)-3-(4-chlorophenylsulfonylamino)quinoxaline
HPLC maintenance time (minutes): 3.91;
Mass data: 469 $(M+H)^+$.

Example 14(181)

2-(2-phenoxyethyloxy)-3-(4-chlorophenylsulfonylamino)quinoxaline
HPLC maintenance time (minutes): 4.41;
Mass data: 933 $(2M+Na)^+$, 456 $(M+H)^+$.

Example 14(182)

2-((pyridin-2-yl)methyloxy)-3-(4-chlorophenylsulfonylamino)quinoxaline
HPLC maintenance time (minutes): 3.64;
Mass data: 875 $(2M+Na)^+$, 427 $(M+H)^+$.

Example 14(183)

2-(cyclopentylmethyloxy)-3-(4-chlorophenylsulfonylamino)quinoxaline
HPLC maintenance time (minutes): 4.63;
Mass data: 857 $(2M+Na)^+$, 418 $(M+H)^+$.

Example 14(184)

2-(2-phenylethyloxy)-3-(4-chlorophenylsulfonylamino)quinoxaline
HPLC maintenance time (minutes): 4.48;
Mass data: 901 $(2M+Na)^+$, 440 $(M+H)^+$.

Example 14(185)

2-((pyridin-3-yl)methyloxy)-3-(4-chlorophenylsulfonylamino)quinoxaline
HPLC maintenance time (minutes): 3.55;
Mass data: 427 $(M+H)^+$.

Example 14(186)

2-((pyridin-4-yl)methyloxy)-3-(4-chlorophenylsulfonylamino)quinoxaline
HPLC maintenance time (minutes): 3.55;
Mass data: 427 $(M+H)^+$.

Example 14(187)

2-(2-(N-methyl-N-phenylamino)ethyloxy)-3-(4-ethylphenylsulfonylamino)quinoxaline
HPLC maintenance time (minutes): 3.93;
Mass data: 463 $(M+H)^+$.

Example 14(188)

2-(2-phenoxyethyloxy)-3-(4-ethylphenylsulfonylamino)quinoxaline
HPLC maintenance time (minutes): 4.43;
Mass data: 921 $(2M+Na)^+$, 450 $(M+H)^+$.

Example 14(189)

2-(2-(thiophen-2-yl)ethyloxy)-3-(4-ethylphenylsulfonylamino)pyrazine
HPLC maintenance time (minutes): 4.46;

Mass data: 901 $(2M+Na)^+$, 440 $(M+H)^+$.

Example 14(190)

2-((pyridin-2-yl)methyloxy)-3-(4-ethylphenylsulfonylamino)quinoxaline
HPLC maintenance time (minutes): 3.66;
Mass data: 863 $(2M+Na)^+$, 421 $(M+H)^+$.

Example 14(191)

2-(cyclopentylmethyloxy)-3-(4-ethylphenylsulfonylamino)quinoxaline
HPLC maintenance time (minutes): 4.65;
Mass data: 845 $(2M+Na)^+$, 412 $(M+H)^+$.

Example 14(192)

2-(2-phenylethyloxy)-3-(4-ethylphenylsulfonylamino)quinoxaline
HPLC maintenance time (minutes): 4.50;
Mass data: 889 $(2M+Na)^+$, 434 $(M+H)^+$.

Example 14(193)

2-((pyridin-3-yl)methyloxy)-3-(4-ethylphenylsulfonylamino)quinoxaline
HPLC maintenance time (minutes): 3.58;
Mass data: 841 $(2M+H)^+$, 421 $(M+H)^+$.

Example 14(194)

2-((pyridin-4-yl)methyloxy)-3-(4-ethylphenylsulfonylamino)quinoxaline
HPLC maintenance time (minutes): 3.56;
Mass data: 841 $(2M+H)^+$, 421 $(M+H)^+$.

Example 14(195)

2-(2-(N-methyl-N-phenylamino)ethyloxy)-3-(4-propylphenylsulfonylamino)quinoxaline
HPLC maintenance time (minutes): 4.04;
Mass data: 477 $(M+H)^+$.

Example 14(196)

2-(2-phenoxyethyloxy)-3-(4-propylphenylsulfonylamino)quinoxaline
HPLC maintenance time (minutes): 4.52;
Mass data: 949 $(2M+Na)^+$, 464 $(M+H)^+$.

Example 14(197)

2-(2-(thiophen-2-yl)ethyloxy)-3-(4-propylphenylsulfonylamino)pyrazine
HPLC maintenance time (minutes): 4.55;
Mass data: 929 $(2M+Na)^+$, 454 $(M+H)^+$.

Example 14(198)

2-((pyridin-2-yl)methyloxy)-3-(4-propylphenylsulfonylamino)quinoxaline
HPLC maintenance time (minutes): 3.77;
Mass data: 891 $(2M+Na)^+$, 435 $(M+H)^+$.

Example 14(199)

2-(cyclopentylmethyloxy)-3-(4-propylphenylsulfonylamino)quinoxaline
HPLC maintenance time (minutes): 4.76;
Mass data: 873 $(2M+Na)^+$, 426 $(M+H)^+$.

Example 14(200)

2-(2-phenylethyloxy)-3-(4-propylphenylsulfonylamino)quinoxaline
HPLC maintenance time (minutes): 4.61;
Mass data: 917 $(2M+Na)^+$, 448 $(M+H)^+$.

Example 14(201)

2-((pyridin-3-yl)methyloxy)-3-(4-propylphenylsulfonylamino)quinoxaline
HPLC maintenance time (minutes): 3.66;
Mass data: 869 $(2M+H)^+$, 435 $(M+H)^+$.

Example 14(202)

2-((pyridin-4-yl)methyloxy)-3-(4-propylphenylsulfonylamino)quinoxaline
HPLC maintenance time (minutes): 3.67;
Mass data: 869 $(2M+H)^+$, 435 $(M+H)^+$.

Example 14(203)

2-(2-(N-methyl-N-phenylamino)ethyloxy)-3-(4-(1-methylethyl)phenylsulfonylamino)quinoxaline
HPLC maintenance time (minutes): 4.04;
Mass data: 975 $(M+Na)^+$, 477 $(M+H)^+$.

Example 14(204)

2-(2-phenoxyethyloxy)-3-(4-(1-methylethyl)phenylsulfonylamino)quinoxaline
HPLC maintenance time (minutes): 4.48;
Mass data: 949 $(2M+H)^+$, 464 $(M+H)^+$.

Example 14(205)

2-(2-(thiophen-2-yl)ethyloxy)-3-(4-(1-methylethyl)phenylsulfonylamino)pyrazine
HPLC maintenance time (minutes): 4.54;
Mass data: 929 $(2M+Na)^+$, 454 $(M+H)^+$.

Example 14(206)

2-((pyridin-2-yl)methyloxy)-3-(4-(1-methylethyl)phenylsulfonylamino)quinoxaline
HPLC maintenance time (minutes): 3.75;
Mass data: 891 $(2M+Na)^+$, 435 $(M+H)^+$.

Example 14(207)

2-(cyclopentylmethyloxy)-3-(4-(1-methylethyl)phenylsulfonylamino)quinoxaline
HPLC maintenance time (minutes): 4.74;
Mass data: 874 $(2M+Na)^+$, 426 $(M+H)^+$.

Example 14(208)

2-(2-phenylethyloxy)-3-(4-(1-methylethyl)phenylsulfonylamino)quinoxaline
HPLC maintenance time (minutes): 4.59;

Mass data: 917 (2M+Na)+, 448 (M+H)+.

Example 14(209)

2-((pyridin-3-yl)methyloxy)-3-(4-(1-methylethyl)phenylsulfonylamino)quinoxaline
HPLC maintenance time (minutes): 3.66;
Mass data: 869 (2M+H)+, 435 (M+H)+.

Example 14(210)

2-((pyridin-4-yl)methyloxy)-3-(4-(1-methylethyl)phenylsulfonylamino)quinoxaline
HPLC maintenance time (minutes): 3.66;
Mass data: 891 (2M+Na)+, 435 (M+H)+.

Reference Example 10

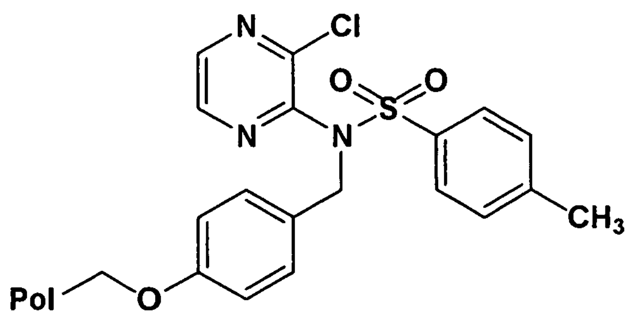

(3)

The compound (3) was obtained, using a compound prepared in Reference Example 2 in stead of a compound prepared in Reference Example 7 by the same procedure as a series of reactions of Reference Example 8.

Example 15

2-(2-phenoxyethyloxy)-3-(4-methylphenylsulfonylamino)pyrazine

Under an atmosphere of argon, to a suspension of the compound (3) prepared in Reference Example 10 (100 mg) in anhydrous 1,4-dioxane (2 mL), 2-phenoxyethanol (0.214 mL) and 16M n-butyl lithium-hexane solution (0.267 mL) were successively added at room temperature. The mixture was stirred foe 16 hours at 100 °C. The reaction mixture was cooled to room temperature and filtered. The obtained resin was washed with tetrahydrofuran (2mL) for two times, methanol (2 mL) for four times and methylene chloride (2 mL) for five times. The title compound (26 mg) having the following physical data was obtained by the same procedure as a series of reactions of Example 14.
TLC: Rf 0.89(chloroform : methanol = 10 : 1);
NMR($d_6$-DMSO): δ 10.88 (s, 1H), 7.86 (d, J=8.4Hz, 2H), 7.71 (m, 2H), 7.34 (d, J=8.4Hz, 2H), 7.29 (dd, J=7.8, 7.2Hz, 2H), 6.97 (d, J=7.8Hz, 2H), 6.94 (t, J=7.2Hz, 1H), 4.60 (m, 2H), 4.34 (m, 2H), 2.34 (s, 3H);
HPLC maintenance time (minutes): 3.89;
Mass data: 771 (2M+H)+, 386 (M+H)+.

Example 15(1) - 15(63)

The following compounds were obtained, using 2,3-dichloropyrazine, a corresponding sulfonamide compound and a corresponding alcohol compound, by the same procedure as a series of reactions of Reference Example 2 → Reference Example 10 →Example 15.

Example 15(1)

2-(2-(N-methyl-N-benzylamino)ethyloxy)-3-(4-methylphenylsulfonylamino)pyrazine
HPLC maintenance time (minutes): 3.33;

Mass data: 413 $(M+H)^+$.

Example 15(2)

2-(2-(N-methyl-N-phenylamino)ethyloxy)-3-(4-methylphenylsulfonylamino)pyrazine
HPLC maintenance time (minutes): 3.33;
Mass data: 819 $(2M+Na)^+$, 399 $(M+H)^+$.

Example 15(3)

2-(2-(3,5-dimethylpyrazol-1-yl)ethyloxy)-3-(4-methylphenylsulfonylamino)pyrazine
HPLC maintenance time (minutes): 3.29;
Mass data: 797 $(2M+Na)^+$, 388 $(M+H)^+$.

Example 15(4)

2-(3-benzyloxypropyloxy)-3-(4-methylphenylsulfonylamino)pyrazine
HPLC maintenance time (minutes): 3.98;
Mass data: 414 $(M+H)^+$.

Example 15(5)

2-(3-phenylpropyloxy)-3-(4-methylphenylsulfonylamino)pyrazine
HPLC maintenance time (minutes): 4.08;
Mass data: 789 $(2M+Na)^+$, 384 $(M+H)^+$.

Example 15(6)

2-(4-phenylbutyloxy)-3-(4-methylphenylsulfonylamino)pyrazine
HPLC maintenance time (minutes): 4.16;
Mass data: 817 $(2M+Na)^+$, 398 $(M+H)^+$.

Example 15(7)

2-(2-(thiophen-2-yl)ethyloxy)-3-(4-methylphenylsulfonylamino)pyrazine
HPLC maintenance time (minutes): 3.92;
Mass data: 773 $(2M+Na)^+$, 376 $(M+H)^+$.

Example 15(8)

2-(3-(pyridin-3-yl)propyloxy)-3-(4-methylphenylsulfonylamino)pyrazine
HPLC maintenance time (minutes): 3.21;
Mass data: 769 $(2M+H)^+$, 385 $(M+H)^+$.

Example 15(9)

2-(3-(pyridin-2-yl)propyloxy)-3-(4-methylphenylsulfonylamino)pyrazine
HPLC maintenance time (minutes): 3.20;
Mass data: 385 $(M+H)^+$.

Example 15(10)

2-((tetrahydrofuran-2-yl)methyloxy)-3-(4-methylphenylsulfonylamino)pyrazine
HPLC maintenance time (minutes): 3.60;
Mass data: 721 $(2M+Na)^+$, 350 $(M+H)^+$.

Example 15(11)

2-(cyclohexylmethyloxy)-3-(4-methylphenylsulfonylamino)pyrazine
HPLC maintenance time (minutes): 4.22;
Mass data: 745 $(2M+Na)^+$, 362 $(M+H)^+$.

Example 15(12)

2-(2-(piperidin-1-yl)ethyloxy)-3-(4-methylphenylsulfonylamino)pyrazine
HPLC maintenance time (minutes): 3.19;
Mass data: 377 $(M+H)^+$.

Example 15(13)

2-(2-cyclopentylethyloxy)-3-(4-methylphenylsulfonylamino)pyrazine
HPLC maintenance time (minutes): 4.20;
Mass data: 745 $(2M+Na)^+$, 362 $(M+H)^+$.

Example 15(14)

2-(2-(morpholin-4-yl)ethyloxy)-3-(4-methylphenylsulfonylamino)pyrazine
HPLC maintenance time (minutes): 3.11;
Mass data: 379 $(M+H)^+$.

Example 15(15)

2-(2-(pyrazol-1-yl)ethyloxy)-3-(4-methylphenylsulfonylamino)pyrazine
HPLC maintenance time (minutes): 3.46;
Mass data: 741 $(2M+Na)^+$, 360 $(M+H)^+$.

Example 15(16)

2-(2-cyclopropylethyloxy)-3-(4-methylphenylsulfonylamino)pyrazine
HPLC maintenance time (minutes): 3.91;
Mass data: 689 $(2M+Na)^+$, 334 $(M+H)^+$.

Example 15(17)

2-((pyridin-2-yl)methyloxy)-3-(4-methylphenylsulfonylamino)pyrazine
HPLC maintenance time (minutes): 3.16;
Mass data: 735 $(2M+Na)^+$, 357 $(M+H)^+$.

Example 15(18)

2-(2-cyclohexylethyloxy)-3-(4-methylphenylsulfonylamino)pyrazine
HPLC maintenance time (minutes): 4.32;
Mass data: 773 $(2M+Na)^+$, 376 $(M+H)^+$.

Example 15(19)

2-(3-(piperidin-1-yl)propyloxy)-3-(4-methylphenylsulfonylamino)pyrazine
HPLC maintenance time (minutes): 3.22;
Mass data: 391 $(M+H)^+$.

Example 15(20)

2-(cyclopentylmethyloxy)-3-(4-methylphenylsulfonylamino)pyrazine
HPLC maintenance time (minutes): 4.07;

Mass data: 717 $(2M+Na)^+$, 348 $(M+H)^+$.

Example 15(21)

2-(2-phenylethyloxy)-3-(4-methylphenylsulfonylamino)pyrazine
HPLC maintenance time (minutes): 3.96;
Mass data: 761 $(2M+Na)^+$, 370 $(M+H)^+$.

Example 15(22)

2-((pyridin-3-yl)methyloxy)-3-(4-methylphenylsulfonylamino)pyrazine
HPLC maintenance time (minutes): 3.15;
Mass data: 713 $(2M+H)^+$, 357 $(M+H)^+$.

Example 15(23)

2-((pyridin-4-yl)methyloxy)-3-(4-methylphenylsulfonylamino)pyrazine
HPLC maintenance time (minutes): 3.14;
Mass data: 713 $(2M+H)^+$, 357 $(M+H)^+$.

Example 15(24)

2-(2-(N-methyl-N-phenylamino)ethyloxy)-3-(4-fluorophenylsulfonylamino)pyrazine
HPLC maintenance time (minutes): 3.49;
Mass data: 403 $(M+H)^+$.

Example 15(25)

2-(2-phenoxyethyloxy)-3-(4-fluorophenylsulfonylamino)pyrazine
HPLC maintenance time (minutes): 3.99;
Mass data: 801 $(2M+Na)^+$, 390 $(M+H)^+$.

Example 15(26)

2-(cyclopentylmethyloxy)-3-(4-fluorophenylsulfonylamino)pyrazine
HPLC maintenance time (minutes): 4.17;
Mass data: 725 $(2M+Na)^+$, 352 $(M+H)^+$, 270.

Example 15(27)

2-(2-phenylethyloxy)-3-(4-fluorophenylsulfonylamino)pyrazine
HPLC maintenance time (minutes): 4.06;
Mass data: 769 $(2M+Na)^+$, 374 $(M+H)^+$.

Example 15(28)

2-((pyridin-3-yl)methyloxy)-3-(4-fluorophenylsulfonylamino)pyrazine
HPLC maintenance time (minutes): 3.19;
Mass data: 361 $(M+H)^+$.

Example 15(29)

2-((pyridin-4-yl)methyloxy)-3-(4-fluorophenylsulfonylamino)pyrazine
HPLC maintenance time (minutes): 3.19;
Mass data: 361 $(M+H)^+$.

Example 15(30)

2-(2-(N-methyl-N-phenylamino)ethyloxy)-3-(4-chlorophenylsulfonylamino)pyrazine
HPLC maintenance time (minutes): 3.58;
Mass data: 419 $(M+H)^+$.

Example 15(31)

2-(2-phenoxyethyloxy)-3-(4-chlorophenylsulfonylamino)pyrazine
HPLC maintenance time (minutes): 4.12;
Mass data: 406 $(M+H)^+$.

Example 15(32)

2-(2-(thiophen-2-yl)ethyloxy)-3-(4-chlorophenylsulfonylamino)pyrazine
HPLC maintenance time (minutes): 4.13;
Mass data: 396 $(M+H)^+$.

Example 15(33)

2-(cyclopentylmethyloxy)-3-(4-chlorophenylsulfonylamino)pyrazine
HPLC maintenance time (minutes): 4.30;
Mass data: 757 $(2M+Na)^+$, 368 $(M+H)^+$.

Example 15(34)

2-(2-phenylethyloxy)-3-(4-chlorophenylsulfonylamino)pyrazine
HPLC maintenance time (minutes): 4.19;
Mass data: 801 $(2M+Na)^+$, 390 $(M+H)^+$.

Example 15(35)

2-((pyridin-3-yl)methyloxy)-3-(4-chlorophenylsulfonylamino)pyrazine
HPLC maintenance time (minutes): 3.30;
Mass data: 753 $(2M+H)^+$, 377 $(M+H)^+$.

Example 15(36)

2-((pyridin-4-yl)methyloxy)-3-(4-chlorophenylsulfonylamino)pyrazine
HPLC maintenance time (minutes): 3.30;
Mass data: 377 $(M+H)^+$.

Example 15(37)

2-(2-(N-methyl-N-phenylamino)ethyloxy)-3-(4-bromophenylsulfonylamino)pyrazine
HPLC maintenance time (minutes): 3.64;
Mass data: 463 $(M+H)^+$.

Example 15(38)

2-(2-phenoxyethyloxy)-3-(4-bromophenylsulfonylamino)pyrazine
HPLC maintenance time (minutes): 4.16;
Mass data: 450 $(M+H)^+$.

Example 15(39)

2-(2-(thiophen-2-yl)ethyloxy)-3-(4-bromophenylsulfonylamino)pyrazine
HPLC maintenance time (minutes): 4.17;

Mass data: 440 $(M+H)^+$.

Example 15(40)

2-((pyridin-2-yl)methyloxy)-3-(4-bromophenylsulfonylamino)pyrazine
HPLC maintenance time (minutes): 3.37;
Mass data: 421 $(M+H)^+$.

Example 15(41)

2-(cyclopentylmethyloxy)-3-(4-bromophenylsulfonylamino)pyrazine
HPLC maintenance time (minutes): 4.33;
Mass data: 845 $(2M+Na)^+$, 412 $(M+H)^+$.

Example 15(42)

2-(2-phenylethyloxy)-3-(4-bromophenylsulfonylamino)pyrazine
HPLC maintenance time (minutes): 4.21;
Mass data: 434 $(M+H)^+$.

Example 15(43)

2-((pyridin-3-yl)methyloxy)-3-(4-bromophenylsulfonylamino)pyrazine
HPLC maintenance time (minutes): 3.33;
Mass data: 421 $(M+H)^+$.

Example 15(44)

2-((pyridin-4-yl)methyloxy)-3-(4-bromophenylsulfonylamino)pyrazine
HPLC maintenance time (minutes): 3.33;
Mass data: 421 $(M+H)^+$.

Example 15(45)

2-(2-(N-methyl-N-phenylamino)ethyloxy)-3-(4-ethylphenylsulfonylamino)pyrazine
HPLC maintenance time (minutes): 3.62;
Mass data: 413 $(M+H)^+$.

Example 15(46)

2-(2-phenoxyethyloxy)-3-(4-ethylphenylsulfonylamino)pyrazine
HPLC maintenance time (minutes): 4.15;
Mass data: 821 $(2M+Na)^+$, 400 $(M+H)^+$.

Example 15(47)

2-(2-(thiophen-2-yl)ethyloxy)-3-(4-ethylphenylsulfonylamino)pyrazine
HPLC maintenance time (minutes): 4.15;
Mass data: 801 $(2M+Na)^+$, 390 $(M+H)^+$.

Example 15(48)

2-((pyridin-2-yl)methyloxy)-3-(4-ethylphenylsulfonylamino)pyrazine
HPLC maintenance time (minutes): 3.38;
Mass data: 763 $(2M+Na)^+$, 371 $(M+H)^+$.

Example 15(49)

2-(cyclopentylmethyloxy)-3-(4-ethylphenylsulfonylamino)pyrazine
HPLC maintenance time (minutes): 4.33;
Mass data: 745 $(2M+Na)^+$, 362 $(M+H)^+$, 280.

Example 15(50)

2-(2-phenylethyloxy)-3-(4-ethylphenylsulfonylamino)pyrazine
HPLC maintenance time (minutes): 4.21;
Mass data: 789 $(2M+Na)^+$, 384 $(M+H)^+$.

Example 15(51)

2-((pyridin-3-yl)methyloxy)-3-(4-ethylphenylsulfonylamino)pyrazine
HPLC maintenance time (minutes): 3.34;
Mass data: 741 $(2M+H)^+$, 371 $(M+H)^+$.

Example 15(52)

2-((pyridin-4-yl)methyloxy)-3-(4-ethylphenylsulfonylamino)pyrazine
HPLC maintenance time (minutes): 3.33;
Mass data: 763 $(2M+Na)^+$, 371 $(M+H)^+$.

Example 15(53)

2-(2-(N-methyl-N-phenylamino)ethyloxy)-3-(4-propylphenylsulfonylamino)pyrazine
HPLC maintenance time (minutes): 3.77;
Mass data: 875 $(2M+Na)^+$, 427 $(M+H)^+$.

Example 15(54)

2-(2-phenoxyethyloxy)-3-(4-propylphenylsulfonylamino)pyrazine
HPLC maintenance time (minutes): 4.25;
Mass data: 849 $(2M+Na)^+$, 414 $(M+H)^+$.

Example 15(55)

2-(2-(thiophen-2-yl)ethyloxy)-3-(4-propylphenylsulfonylamino)pyrazine
HPLC maintenance time (minutes): 4.28;
Mass data: 829 $(2M+Na)^+$, 404 $(M+H)^+$.

Example 15(56)

2-((pyridin-2-yl)methyloxy)-3-(4-propylphenylsulfonylamino)pyrazine
HPLC maintenance time (minutes): 3.49;
Mass data: 791 $(2M+Na)^+$, 385 $(M+H)^+$.

Example 15(57)

2-(2-phenoxyethyloxy)-3-(4-(1-methylethyl)phenylsulfonylamino)pyrazine
HPLC maintenance time (minutes): 4.22;
Mass data: 849 $(2M+Na)^+$, 414 $(M+H)^+$.

Example 15(58)

2-(2-(thiophen-2-yl)ethyloxy)-3-(4-(1-methylethyl)phenylsulfonylamino)pyrazine
HPLC maintenance time (minutes): 4.26;

Mass data: 829 (2M+Na)+, 404 (M+H)+.

Example 15(59)

2-((pyridin-2-yl)methyloxy)-3-(4-(1-methylethyl)phenylsulfonylamino)pyrazine
HPLC maintenance time (minutes): 3.49;
Mass data: 791 (2M+Na)+, 385 (M+H)+.

Example 15(60)

2-(cyclopentylmethyloxy)-3-(4-(1-methylethyl)phenylsulfonylamino)pyrazine
HPLC maintenance time (minutes): 4.41;
Mass data: 773 (2M+Na)+, 376 (M+H)+.

Example 15(61)

2-(2-phenylethyloxy)-3-(4-ethylphenylsulfonylamino)pyrazine
HPLC maintenance time (minutes): 4.30;
Mass data: 817 (2M+Na)+, 398 (M+H)+.

Example 15(62)

2-((pyridin-3 -yl)methyloxy)-3-(4-(1-methylethyl)phenylsulfonylamino)pyrazine
HPLC maintenance time (minutes): 3.44;
Mass data: 769 (2M+H)+, 385 (M+H)+.

Example 15(63)

2-((pyridin-4-yl)methyloxy)-3-(4-(1-methylethyl)phenylsulfonylamino)pyrazine
HPLC maintenance time (minutes): 3.42;
Mass data: 791 (2M+Na)+, 385 (M+H)+.

Reference Example 11

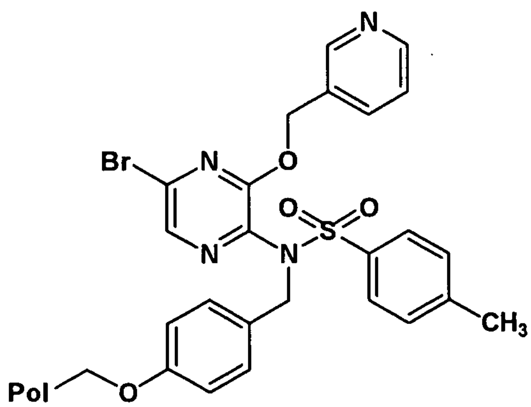

(4)

The compound (4) was obtained, using the compound prepared in Example 1(1) in stead of the compound prepared in Reference Example 7, by the same procedure as a series of reactions of Reference Example 8.

Example 16

6-phenyl-2-((pyridin-3-yl)methyloxy)-3-(4-methylphenylsulfonylamino)pyrazine
Under an atmosphere of argon, to a suspension of the compound (4) prepared in Reference Example 11 (50 mg)

in 1,2-dimethoxyethane (1 mL), phenylboric acid (28 mg), 2N aqueous solution of sodium carbonate (0.30 mL) and dichlorobis(triphenylphosphine)palladium (II) (4.3 mg), successively, were added at room temperature. The mixture was stirred for 6 hours at 90 °C. The reaction mixture was cooled to room temperature, and filtered. The obtained resin was washed with a mixture of 1,2-dimethoxyethane and water (2 mL) for two times, water (2 mL) for two times, a mixture of 1,2-dimethoxyethane and water (2 mL) for three times, a mixture of 0.2N hydrochloric acid and tetrahydrofuran (1 : 2)(2mL) for three times, a mixture of water and tetrahydrofuran (1 : 2) (2 mL) for three times, tetrahydrofuran (2 mL) for three times, and 1,2-dichloroethane (2 mL) for three times. The title compound (11 mg) having the following physical data was obtained by the same procedure as a series of reactions of Example 14.
TLC: Rf 0.70(chloroform : methanol = 10 : 1);
NMR($d_6$-DMSO): δ 11.05 (brs, 1H), 8.82 (d, J=2.1Hz, 1H), 8.54 (dd, J=3.9, 2.1Hz, 1H), 8.37 (s, 1H), 7.99 (dd, J=3.9, 1.8Hz, 3H), 7.89 (d, J=8.1Hz, 2H), 7.45-7.36 (m, 6H), 5.56 (s, 2H), 2.35 (s, 3H);
HPLC maintenance time (minutes): 3.49;
Mass condition: ESI (Pos., 40 V);
Mass data: 433 $(M+H)^+$.

Example 16(1) - 16(60)

The following compounds were obtained, using a corresponding boric acid compound in stead of phenylboric acid, by the same procedure as a series of reactions of Example 16.

Example 16(1)

6-(3-nitrophenyl)-2-((pyridin-3-yl)methyloxy)-3-(4-methylphenylsulfonylamino)pyrazine
HPLC maintenance time (minutes): 3.49;
Mass data: 955 $(2M+H)^+$, 478 $(M+H)^+$.

Example 16(2)

6-(2,4-dichlorophenyl)-2-((pyridin-3-yl)methyloxy)-3-(4-methylphenylsulfonylamino)pyrazine
HPLC maintenance time (minutes): 3.71;
Mass data: 501 $(M+H)^+$.

Example 16(3)

6-(naphthalen-1-yl)-2-((pyridin-3-yl)methyloxy)-3-(4-methylphenylsulfonylamino)pyrazine
HPLC maintenance time (minutes): 3.62;
Mass data: 965 $(2M+H)^+$, 483 $(M+H)^+$.

Example 16(4)

6-(4-fluorophenyl)-2-((pyridin-3-yl)methyloxy)-3-(4-methylphenylsulfonylamino)pyrazine
HPLC maintenance time (minutes): 3.53;
Mass data: 901 $(2M+H)^+$, 451 $(M+H)^+$.

Example 16(5)

6-(4-chlorophenyl)-2-((pyridin-3-yl)methyloxy)-3-(4-methylphenylsulfonylamino)pyrazine
HPLC maintenance time (minutes): 3.62;
Mass data: 933 $(2M+H)^+$, 467 $(M+H)^+$.

Example 16(6)

6-(4-methylphenyl)-2-((pyridin-3-yl)methyloxy)-3-(4-methylphenylsulfonylamino)pyrazine
HPLC maintenance time (minutes): 3.58;
Mass data: 893 $(2M+H)^+$, 447 $(M+H)^+$.

Example 16(7)

6-(4-methoxyphenyl)-2-((pyridin-3-yl)methyloxy)-3-(4-methylphenylsulfonylamino)pyrazine
HPLC maintenance time (minutes): 3.49;
Mass data: 925 $(2M+H)^+$, 463 $(M+H)^+$.

Example 16(8)

6-(3-methylphenyl)-2-((pyridin-3-yl)methyloxy)-3-(4-methylphenylsulfonylamino)pyrazine
HPLC maintenance time (minutes): 3.58;
Mass data: 893 $(2M+H)^+$, 447 $(M+H)^+$.

Example 16(9)

6-(3-chloro-4-fluorophenyl)-2-((pyridin-3-yl)methyloxy)-3-(4-methylphenylsulfonylamino)pyrazine
HPLC maintenance time (minutes): 3.64;
Mass data: 969 $(2M+H)^+$, 485 $(M+H)^+$.

Example 16(10)

6-(3,5-bis(trifluoromethyl)phenyl)-2-((pyridin-3-yl)methyloxy)-3-(4-methylphenylsulfonylamino)pyrazine
HPLC maintenance time (minutes): 3.80;
Mass data: 569 $(M+H)^+$.

Example 16(11)

6-(3,5-dichloromethylphenyl)-2-((pyridin-3-yl)methyloxy)-3-(4-methylphenylsulfonylamino)pyrazine
HPLC maintenance time (minutes): 3.75;
Mass data: 501 $(M+H)^+$.

Example 16(12)

6-(4-phenylphenyl)-2-((pyridin-3-yl)methyloxy)-3-(4-methylphenylsulfonylamino)pyrazine
HPLC maintenance time (minutes): 3.77;
Mass data: 509 $(M+H)^+$.

Example 16(13)

6-(4-methylthiophenyl)-2-((pyridin-3-yl)methyloxy)-3-(4-methylphenylsulfonylamino)pyrazine
HPLC maintenance time (minutes): 3.60;
Mass data: 957 $(2M+H)^+$, 479 $(M+H)^+$.

Example 16(14)

6-(2-methylphenyl)-2-((pyridin-3-yl)methyloxy)-3-(4-methylphenylsulfonylamino)pyrazine
HPLC maintenance time (minutes): 3.38;
Mass data: 893 $(2M+H)^+$, 447 $(M+H)^+$.

Example 16(15)

6-(phenanthren-9-yl)-2-((pyridin-3-yl)methyloxy)-3-(4-methylphenylsulfonylamino)pyrazine
HPLC maintenance time (minutes): 3.80;
Mass data: 533 $(M+H)^+$.

Example 16(16)

6-(3-aminophenyl)-2-((pyridin-3-yl)methyloxy)-3-(4-methylphenylsulfonylamino)pyrazine
HPLC maintenance time (minutes): 3.09;

Mass data: 895 $(2M+H)^+$, 448 $(M+H)^+$.

Example 16(17)

6-(4-carboxyphenyl)-2-((pyridin-3-yl)methyloxy)-3-(4-methylphenylsulfonylamino)pyrazine HPLC maintenance time (minutes): 3.33;
Mass data: 953 $(2M+H)^+$, 477 $(M+H)^+$.

Example 16(18)

6-(2-formylphenyl)-2-((pyridin-3-yl)methyloxy)-3-(4-methylphenylsulfonylamino)pyrazine HPLC maintenance time (minutes): 3.42;
Mass data: 921 $(2M+H)^+$, 461 $(M+H)^+$.

Example 16(19)

6-(3-trifluoromethylphenyl)-2-((pyridin-3-yl)methyloxy)-3-(4-methylphenylsulfonylamino)pyrazine HPLC maintenance time (minutes): 3.66;
Mass data: 501 $(M+H)^+$.

Example 16(20)

6-(4-trifluoromethylphenyl)-2-((pyridin-3-yl)methyloxy)-3-(4-methylphenylsulfonylamino)pyrazine HPLC maintenance time (minutes): 3.67;
Mass data: 501 $(M+H)^+$.

Example 16(21)

6-(3-formylphenyl)-2-((pyridin-3-yl)methyloxy)-3-(4-methylphenylsulfonylamino)pyrazine HPLC maintenance time (minutes): 3.40;
Mass data: 921 $(2M+H)^+$, 461 $(M+H)^+$.

Example 16(22)

6-(3-methoxyphenyl)-2-((pyridin-3-yl)methyloxy)-3-(4-methylphenylsulfonylamino)pyrazine HPLC maintenance time (minutes): 3.38;
Mass data: 925 $(2M+H)^+$, 463 $(M+H)^+$.

Example 16(23)

6-(3-nitro-4-methylphenyl)-2-((pyridin-3-yl)methyloxy)-3-(4-methylphenylsulfonylamino)pyrazine HPLC maintenance time (minutes): 3.56;
Mass data: 983 $(2M+H)^+$, 492 $(M+H)^+$.

Example 16(24)

6-(3-acetylaminophenyl)-2-((pyridin-3-yl)methyloxy)-3-(4-methylphenylsulfonylamino)pyrazine HPLC maintenance time (minutes): 3.31;
Mass data: 979 $(2M+H)^+$, 490 $(M+H)^+$.

Example 16(25)

6-(3-fluorophenyl)-2-((pyridin-3-yl)methyloxy)-3-(4-methylphenylsulfonylamino)pyrazine HPLC maintenance time (minutes): 3.53;
Mass data: 901 $(2M+H)^+$, 451 $(M+H)^+$.

Example 16(26)

6-(2-methoxyphenyl)-2-((pyridin-3-yl)methyloxy)-3-(4-methylphenyisulfonylamino)pyrazine, HPLC maintenance time (minutes): 3.51;
Mass data: 925 $(2M+H)^+$, 463 $(M+H)^+$.

Example 16(27)

6-(naphthalen-2-yl)-2-((pyridin-3-yl)methyloxy)-3-(4-methylphenylsulfonylamino)pyrazine
HPLC maintenance time (minutes): 3.66;
Mass data: 965 $(2M+H)^+$, 483 $(M+H)^+$.

Example 16(28)

6-(2-trifluoromethylphenyl)-2-((pyridin-3-yl)methyloxy)-3-(4-methylphenylsulfonylamino)pyrazine
HPLC maintenance time (minutes): 3.58;
Mass data: 501 $(M+H)^+$.

Example 16(29)

6-(3-ethoxyphenyl)-2-((pyridin-3-yl)methyloxy)-3-(4-methylphenylsulfonylamino)pyrazine
HPLC maintenance time (minutes): 3.60;
Mass data: 953 $(2M+H)^+$, 477 $(M+H)^+$.

Example 16(30)

6-(2-chlorophenyl)-2-((pyridin-3-yl)methyloxy)-3-(4-methylphenylsulfonylamino)pyrazine
HPLC maintenance time (minutes): 3.56;
Mass data: 933 $(2M+H)^+$, 467 $(M+H)^+$.

Example 16(31)

6-(2-fluorophenyl)-2-((pyridin-3-yl)methyloxy)-3-(4-methylphenylsulfonylamino)pyrazine
HPLC maintenance time (minutes): 3.51;
Mass data: 901 $(2M+H)^+$, 451 $(M+H)^+$.

Example 16(32)

6-(4-ethylphenyl)-2-((pyridin-3-yl)methyloxy)-3-(4-methylphenylsulfonylamino)pyrazine
HPLC maintenance time (minutes): 3.67;
Mass data: 921 $(2M+H)^+$, 461 $(M+H)^+$.

Example 16(33)

6-(3,4-dimethylphenyl)-2-((pyridin-3-yl)methyloxy)-3-(4-methylphenylsulfonylamino)pyrazine
HPLC maintenance time (minutes): 3.66;
Mass data: 921 $(2M+H)^+$, 461 $(M+H)^+$.

Example 16(34)

6-(1,3-dioxaindan-5-yl)-2-((pyridin-3-yl)methyloxy)-3-(4-methylphenylsulfonylamino)pyrazine
HPLC maintenance time (minutes): 3.49;
Mass data: 953 $(2M+H)^+$, 477 $(M+H)^+$.

Example 16(35)

6-(4-(1,1-dimethylethyl)phenyl)-2-((pyridin-3-yl)methyloxy)-3-(4-methylphenylsulfonylamino)pyrazine
HPLC maintenance time (minutes): 3.82;

Mass data: 977 $(2M+H)^+$, 489 $(M+H)^+$.

Example 16(36)

6-(3,4-dimethoxyphenyl)-2-((pyridin-3-yl)methyloxy)-3-(4-methylphenylsulfonylamino)pyrazine
HPLC maintenance time (minutes): 3.42;
Mass data: 985 $(2M+H)^+$, 493 $(M+H)^+$.

Example 16(37)

6-(2,4-dimethoxyphenyl)-2-((pyridin-3-yl)methyloxy)-3-(4-methylphenylsulfonylamino)pyrazine
HPLC maintenance time (minutes): 3.53;
Mass data: 985 $(2M+H)^+$, 493 $(M+H)^+$.

Example 16(38)

6-(4-(1-methylethyl)phenyl)-2-((pyridin-3-yl)methyloxy)-3-(4-methylphenylsulfonylamino)pyrazine
HPLC maintenance time (minutes): 3.75;
Mass data: 949 $(2M+H)^+$, 475 $(M+H)^+$.

Example 16(39)

6-(4-hydroxyphenyl)-2-((pyridin-3-yl)methyloxy)-3-(4-methylphenylsulfonylamino)pyrazine
HPLC maintenance time (minutes): 3.31;
Mass data: 897 $(2M+H)^+$, 449 $(M+H)^+$.

Example 16(40)

6-(3-(1-methylethyl)phenyl)-2-((pyridin-3-yl)methyloxy)-3-(4-methylphenylsulfonylamino)pyrazine
HPLC maintenance time (minutes): 3.73;
Mass data: 949 $(2M+H)^+$, 475 $(M+H)^+$.

Example 16(41)

6-(4-methylcarbonylphenyl)-2-((pyridin-3-yl)methyloxy)-3-(4-methylphenylsulfonylamino)pyrazine
HPLC maintenance time (minutes): 3.42;
Mass data: 949 $(2M+H)^+$, 475 $(M+H)^+$.

Example 16(42)

6-(3-methylcarbonylphenyl)-2-((pyridin-3-yl)methyloxy)-3-(4-methylphenylsulfonylamino)pyrazine
HPLC maintenance time (minutes): 3.44;
Mass data: 949 $(2M+H)^+$, 475 $(M+H)^+$.

Example 16(43)

6-(3,4,5-trimethoxyphenyl)-2-((pyridin-3-yl)methyloxy)-3-(4-methylphenylsulfonylamino)pyrazine
HPLC maintenance time (minutes): 3.45;
Mass data: 523 $(M+H)^+$.

Example 16(44)

6-(2,3-dichlorophenyl)-2-((pyridin-3-yl)methyloxy)-3-(4-methylphenylsulfonylamino)pyrazine
HPLC maintenance time (minutes): 3.66;
Mass data: 501 $(M+H)^+$.

Example 16(45)

6-(4-(2-carboxyethenyl)phenyl)-2-((pyridin-3-yl)methyloxy)-3-(4-methylphenylsulfonylamino)pyrazine
HPLC maintenance time (minutes): 3.36;
Mass data: 503 $(M+H)^+$.

Example 16(46)

6-(4-benzyloxyphenyl)-2-((pyridin-3-yl)methyloxy)-3-(4-methylphenylsulfonylamino)pyrazine
HPLC maintenance time (minutes): 3.78;
Mass data: 539 $(M+H)^+$.

Example 16(47)

6-(4-phenyl-3-fluorophenyl)-2-((pyridin-3-yl)methyloxy)-3-(4-methylphenylsulfonylamino)pyrazine
HPLC maintenance time (minutes): 3.80;
Mass data: 527 $(M+H)^+$.

Example 16(48)

6-(3-(2-carboxyethenyl)phenyl)-2-((pyridin-3-yl)methyloxy)-3-(4-methylphenylsulfonylamino)pyrazine
HPLC maintenance time (minutes): 3.40;
Mass data: 503 $(M+H)^+$.

Example 16(49)

6-(4-(2-nitroethenyl)phenyl)-2-((pyridin-3-yl)methyloxy)-3-(4-methylphenylsulfonylamino)pyrazine
HPLC maintenance time (minutes): 3.40;
Mass data: 504 $(M+H)^+$.

Example 16(50)

6-(3-phenylphenyl)-2-((pyridin-3-yl)methyloxy)-3-(4-methylphenylsulfonylamino)pyrazine
HPLC maintenance time (minutes): 3.77;
Mass data: 509 $(M+H)^+$.

Example 16(51)

6-(4-(2-carboxyethyl)phenyl)-2-((pyridin-3-yl)methyloxy)-3-(4-methylphenylsulfonylamino)pyrazine
HPLC maintenance time (minutes): 3.38;
Mass data: 505 $(M+H)^+$.

Example 16(52)

6-(3,5-difluorophenyl)-2-((pyridin-3-yl)methyloxy)-3-(4-methylphenylsulfonylamino)pyrazine
HPLC maintenance time (minutes): 3.58;
Mass data: 937 $(2M+H)^+$, 469 $(M+H)^+$.

Example 16(53)

6-(4-ethylthiophenyl)-2-((pyridin-3-yl)methyloxy)-3-(4-methylphenylsulfonylamino)pyrazine
HPLC maintenance time (minutes): 3.67;
Mass data: 985 $(2M+H)^+$, 493 $(M+H)^+$.

Example 16(54)

6-(2-methoxy-5-(1-methylethyl)phenyl)-2-((pyridin-3-yl)methyloxy)-3-(4-methylphenylsulfonylamino)pyrazine

HPLC maintenance time (minutes): 3.73;
Mass data: 505 $(M+H)^+$.

Example 16(55)

6-(2-methylthiophenyl)-2-((pyridin-3-yl)methyloxy)-3-(4-methylphenylsulfonylamino)pyrazine
HPLC maintenance time (minutes): 3.55;
Mass data: 957 $(2M+H)^+$, 479 $(M+H)^+$.

Example 16(56)

6-(2,4-difluorophenyl)-2-((pyridin-3-yl)methyloxy)-3-(4-methylphenylsulfonylamino)pyrazine
HPLC maintenance time (minutes): 3.58;
Mass data: 937 $(2M+H)^+$, 469 $(M+H)^+$.

Example 16(57)

6-(2-methylcarbonylphenyl)-2-((pyridin-3-yl)methyloxy)-3-(4-methylphenylsulfonylamino)pyrazine
HPLC maintenance time (minutes): 3.40;
Mass data: 949 $(2M+H)^+$, 475 $(M+H)^+$.

Example 16(58)

6-(3-carboxyphenyl)-2-((pyridin-3-yl)methyloxy)-3-(4-methylphenylsulfonylamino)pyrazine
HPLC maintenance time (minutes): 3.34;
Mass data: 953 $(2M+H)^+$, 477 $(M+H)^+$.

Example 16(59)

6-(4-dimethylaminophenyl)-2-((pyridin-3-yl)methyloxy)-3-(4-methylphenylsulfonylamino)pyrazine
HPLC maintenance time (minutes): 3.21;
Mass data: 951 $(2M+H)^+$, 476 $(M+H)^+$.

Example 16(60)

6-(4-(thiophen-2-yl)phenyl)-2-((pyridin-3-yl)methyloxy)-3-(4-methylphenylsulfonylamino)pyrazine
HPLC maintenance time (minutes): 3.38;
Mass data: 871 $(2M+H)^+$, 439 $(M+H)^+$.

Example 17

6-bromo-2-((3,4-dimethoxyphenyl)methyloxy)-3-(4-methylphenylsulfonylamino)pyrazine
To a suspension of 60% sodium hydride (12 mg) in 1,4-dioxane (1.0 mL), a solution of 3,4-dimethoxybenzyl alcohol (25 mg) in 1,4-dioxane (1 mL) was dropped at room temperature. The mixture was stirred for 30 minutes at room temperature. A solution of the compound prepared in Reference Example 1 (41 mg) in 1,4-dioxane (1 mL) was added to the reaction mixture at room temperature. The mixture was stirred for 3 hours at 100 °C. The reaction mixture was cooled to room temperature and concentrated. After the residue was washed with diisopropyl ether (5 mL), 1N hydrochloric acid (1 mL) was added. The mixture was extracted with chloroform (3 mL) for two times. The extract was concentrated to give the title compound (26 mg) having the following physical data.
TLC: Rf 0.47 (hexane : ethyl acetate = 1 : 1);
NMR ($d_6$-DMSO): δ 11.06 (br, 1H), 7.91 (s, 1H), 7.84 (d, J=8.4Hz, 2H), 7.35 (d, J=8.4Hz, 2H), 7.15 (d, J=1.8Hz, 1H), 7.04 (dd, J=8.1, 1.8Hz, 1H), 6.95 (d, J=8.1Hz, 1H), 5.28 (s, 2H), 3.76 (s, 3H), 3.75 (s, 3H), 2.35 (s, 3H);
HPLC maintenance time (minutes): 4.10;
Mass data: 494 $(M+H)^+$.

Example 17(1) - 17(63)

The following compounds were obtained, using a corresponding alcohol compound in stead of 3,4-dimethoxybenzyl alcohol, by the same procedure as a series of reactions of Example 17.

Example 17(1)

2-((3-methylphenyl)methyloxy)-3-(4-methylphenylsulfonylamino)pyrazine
HPLC maintenance time (minutes): 4.08;
Mass data: 761 $(2M+Na)^+$, 370 $(M+H)^+$.

Example 17(2)

2-((3-chlorophenyl)methyloxy)-3-(4-methylphenylsulfonylamino)pyrazine
HPLC maintenance time (minutes): 4.09;
Mass data: 390 $(M+H)^+$.

Example 17(3)

2-((3-methoxyphenyl)methyloxy)-3-(4-methylphenylsulfonylamino)pyrazine
HPLC maintenance time (minutes): 3.98;
Mass data: 793 $(2M+Na)^+$, 386 $(M+H)^+$.

Example 17(4)

6-bromo-2-((3-methylphenyl)methyloxy)-3-(4-methylphenylsulfonylamino)pyrazine
HPLC maintenance time (minutes): 4.34;
Mass data: 448 $(M+H)^+$.

Example 17(5)

6-bromo-2-((3-chlorophenyl)methyloxy)-3-(4-methylphenylsulfonylamino)pyrazine
HPLC maintenance time (minutes): 4.34;
Mass data: 468 $(M+H)^+$.

Example 17(6)

6-bromo-2-((3-methoxyphenyl)methyloxy)-3-(4-methylphenylsulfonylamino)pyrazine
HPLC maintenance time (minutes): 4.22;
Mass data: 466 $(M+H)^+$.

Example 17(7)

6-bromo-2-((3-trifluoromethylphenyl)methyloxy)-3-(4-methylphenylsulfonylamino)pyrazine
HPLC maintenance time (minutes): 4.34;
Mass data: 502 $(M+H)^+$.

Example 17(8)

2-((2-methoxyphenyl)methyloxy)-3-(4-methylphenylsulfonylamino)pyrazine
HPLC maintenance time (minutes): 3.99;
Mass data: 793 $(2M+Na)^+$, 386 $(M+H)^+$;
NMR ($d_6$-DMSO): $\delta$ 10.88 (s, 1H), 7.88 (d, J=8.4Hz, 2H), 7.76 (d, J=9.0Hz, 2H), 7.49 (d, J=6.6Hz, 1H), 7.38-7.31 (m, 3H), 7.05 (d, J=7.8Hz, 1H), 6.97 (td, J=7.8, 0.9Hz, 1H), 5.38 (s, 2H), 3.01 (s, 3H), 2.36 (s, 3H).

Example 17(9)

2-(1-phenylethyloxy)-3-(4-methylphenylsulfonylamino)pyrazine
HPLC maintenance time (minutes): 4.04;
Mass data: 761 $(2M+Na)^+$, 370 $(M+H)^+$;
NMR ($d_6$-DMSO): $\delta$ 10.94 (s, 1H), 7.90 (d, J=8.4Hz, 2H), 7.66 (s, 2H), 7.48 (d, J=7.2Hz, 2H), 7.39-7.22 (m, 5H9, 6.12

(q, J=6.3Hz, 1H), 2.36 (s, 3H), 1.59 (d, J=6.3Hz, 3H).

Example 17(10)

2-((furan-2-yl)methyloxy)-3-(4-methylphenylsulfonylamino)pyrazine
HPLC maintenance time (minutes): 3.82;
Mass data: 713 $(2M+Na)^+$, 346 $(M+H)^+$;
NMR ($d_6$-DMSO): δ 10.88 (s, 1H), 7.86 (d, J=8.1Hz, 2H), 7.80-7.73 (m, 3H), 7.36 (d, J=8.1Hz, 2H), 6.63 (d, J=3.0Hz, 1H), 6.51-6.49 (m, 1H), 5.35 (s, 2H), 2.36 (s, 3H).

Example 17(11)

2-((2-methylphenyl)methyloxy)-3-(4-methylphenylsulfonylamino)pyrazine
HPLC maintenance time (minutes): 4.06;
Mass data: 761 $(2M+Na)^+$, 370 $(M+H)^+$.

Example 17(12)

2-((2-chlorophenyl)methyloxy)-3-(4-methylphenylsulfonylamino)pyrazine
HPLC maintenance time (minutes): 4.10;
Mass data: 390 $(M+H)^+$.

Example 17(13)

6-bromo-2-((2-phenylphenyl)methyloxy)-3-(4-methylphenylsulfonylamino)pyrazine
HPLC maintenance time (minutes): 4.45;
Mass data: 510 $(M+H)^+$.

Example 17(14)

2-((2-trifluoromethylphenyl)methyloxy)-3-(4-methylphenylsulfonylamino)pyrazine
HPLC maintenance time (minutes): 4.11;
Mass data: 869 $(2M+Na)^+$, 424 $(M+H)^+$.

Example 17(15)

2-((naphthalen-1-yl)methyloxy)-3-(4-methylphenylsulfonylamino)pyrazine
HPLC maintenance time (minutes): 4.15;
Mass data: 733 $(2M+Na)^+$, 406 $(M+H)^+$.

Example 17(16)

2-((naphthalen-2-yl)methyloxy)-3-(4-methylphenylsulfonylamino)pyrazine
HPLC maintenance time (minutes): 4.17;
Mass data: 833 $(2M+Na)^+$, 406 $(M+H)^+$.

Example 17(17)

2-((2,3-dimethoxyphenyl)methyloxy)-3-(4-methylphenylsulfonylamino)pyrazine
HPLC maintenance time (minutes): 3.95;
Mass data: 853 $(2M+Na)^+$, 416 $(M+H)^+$.

Example 17(18)

2-((2,5-dimethoxyphenyl)methyloxy)-3-(4-methylphenylsulfonylamino)pyrazine
HPLC maintenance time (minutes): 3.99;
Mass data: 853 $(2M+Na)^+$, 416 $(M+H)^+$.

Example 17(19)

2-((3,5-dimethoxyphenyl)methyloxy)-3-(4-methylphenylsulfonylamino)pyrazine
HPLC maintenance time (minutes): 3.97;
Mass data: 853 $(2M+Na)^+$, 416 $(M+H)^+$.

Example 17(20)

2-((2,3-dichlorophenyl)methyloxy)-3-(4-methylphenylsulfonylamino)pyrazine
HPLC maintenance time (minutes): 4.21;
Mass data: 424 $(M+H)^+$.

Example 17(21)

2-((2,4-dichlorophenyl)methyloxy)-3-(4-methylphenylsulfonylamino)pyrazine
HPLC maintenance time (minutes): 4.24;
Mass data: 424 $(M+H)^+$.

Example 17(22)

2-((2,5-dichlorophenyl)methyloxy)-3-(4-methylphenylsulfonylamino)pyrazine
HPLC maintenance time (minutes): 4.21;
Mass data: 424 $(M+H)^+$.

Example 17(23)

2-((2,6-dichlorophenyl)methyloxy)-3-(4-methylphenylsulfonylamino)pyrazine
HPLC maintenance time (minutes): 4.19;
Mass data: 424 $(M+H)^+$.

Example 17(24)

2-((3,4-dichlorophenyl)methyloxy)-3-(4-methylphenylsulfonylamino)pyrazine
HPLC maintenance time (minutes): 4.21;
Mass data: 424 $(M+H)^+$.

Example 17(25)

2-((3,5-dichlorophenyl)methyloxy)-3-(4-methylphenylsulfonylamino)pyrazine
HPLC maintenance time (minutes): 4.24;
Mass data: 424 $(M+H)^+$.

Example 17(26)

2-((4-ethylphenyl)methyloxy)-3-(4-methylphenylsulfonylamino)pyrazine
HPLC maintenance time (minutes): 4.17;
Mass data: 789 $(2M+Na)^+$, 384 $(M+H)^+$.

Example 17(27)

2-((4-(1-methylethyl)phenyl)methyloxy)-3-(4-methylphenylsulfonylamino)pyrazine
HPLC maintenance time (minutes): 4.26;
Mass data: 817 $(2M+Na)^+$, 398 $(M+H)^+$.

Example 17(28)

2-((4-(1,1-dimethylethyl)phenyl)methyloxy)-3-(4-methylphenylsulfonylamino)pyrazine
HPLC maintenance time (minutes): 4.35;

Mass data: 845 $(2M+Na)^+$, 412 $(M+H)^+$.

Example 17(29)

2-((4-phenylphenyl)methyloxy)-3-(4-methylphenylsulfonylamino)pyrazine
HPLC maintenance time (minutes): 4.26;
Mass data: 885 $(2M+Na)^+$, 432 $(M+H)^+$.

Example 17(30)

2-((3-phenoxyphenyl)methyloxy)-3-(4-methylphenylsulfonylamino)pyrazine
HPLC maintenance time (minutes): 4.26;
Mass data: 917 $(2M+Na)^+$, 448 $(M+H)^+$.

Example 17(31)

2-((2-phenylphenyl)methyloxy)-3-(4-methylphenylsulfonylamino)pyrazine
HPLC maintenance time (minutes): 4.00;
Mass data: 885 $(2M+Na)^+$, 432 $(M+H)^+$.

Example 17(32)

2-((1,3-dioxaindan-4-yl)methyloxy)-3-(4-methylphenylsulfonylamino)pyrazine
HPLC maintenance time (minutes): 3.95;
Mass data: 821 $(2M+Na)^+$, 400 $(M+H)^+$.

Example 17(33)

2-((1,3-dioxaindan-5-yl)methyloxy)-3-(4-methylphenylsulfonylamino)pyrazine
HPLC maintenance time (minutes): 3.93;
Mass data: 821 $(2M+Na)^+$, 400 $(M+H)^+$.

Example 17(34)

2-((3-trifluoromethylphenyl)methyloxy)-3-(4-methylphenylsulfonylamino)pyrazine
HPLC maintenance time (minutes): 4.13;
Mass data: 869 $(2M+Na)^+$, 424 $(M+H)^+$.

Example 17(35)

2-((4-methylphenyl)methyloxy)-3-(4-methylphenylsulfonylamino)pyrazine
HPLC maintenance time (minutes): 4.08;
Mass data: 761 $(2M+Na)^+$, 370 $(M+H)^+$.

Example 17(36)

2-((4-chlorophenyl)methyloxy)-3-(4-methylphenylsulfonylamino)pyrazine
HPLC maintenance time (minutes): 4.10;
Mass data: 801 $(2M+Na)^+$, 390 $(M+H)^+$.

Example 17(37)

2-((4-methoxyphenyl)methyloxy)-3-(4-methylphenylsulfonylamino)pyrazine
HPLC maintenance time (minutes): 3.97;
Mass data: 793 $(2M+Na)^+$, 386 $(M+H)^+$.

Example 17(38)

2-((4-trifluoromethylphenyl)methyloxy)-3-(4-methylphenylsulfonylamino)pyrazine
HPLC maintenance time (minutes): 4.13;
Mass data: 869 $(2M+Na)^+$, 424 $(M+H)^+$.

Example 17(39)

6-bromo-2-((4-methylphenyl)methyloxy)-3-(4-methylphenylsulfonylamino)pyrazine
HPLC maintenance time (minutes): 4.32;
Mass data: 448 $(M+H)^+$.

Example 17(40)

6-bromo-2-((2-methylphenyl)methyloxy)-3-(4-methylphenylsulfonylamino)pyrazine
HPLC maintenance time (minutes): 4.30;
Mass data: 448 $(M+H)^+$.

Example 17(41)

6-bromo-2-((2-chlorophenyl)methyloxy)-3-(4-methylphenylsulfonylamino)pyrazine
HPLC maintenance time (minutes): 4.32;
Mass data: 468 $(M+H)^+$.

Example 17(42)

6-bromo-2-((2-methoxyphenyl)methyloxy)-3-(4-methylphenylsulfonylamino)pyrazine
HPLC maintenance time (minutes): 4.26;
Mass data: 464 $(M+H)^+$.

Example 17(43)

6-bromo-2-((2-trifluoromethylphenyl)methyloxy)-3-(4-methylphenylsulfonylamino)pyrazine
HPLC maintenance time (minutes): 4.34;
Mass data: 502 $(M+H)^+$.

Example 17(44)

6-bromo-2-((naphthalen-1-yl)methyloxy)-3-(4-methylphenylsulfonylamino)pyrazine
HPLC maintenance time (minutes): 4.37;
Mass data: 482 $(M+H)^+$.

Example 17(45)

6-bromo-2-((2,3-dimethoxyphenyl)methyloxy)-3-(4-methylphenylsulfonylamino)pyrazine
HPLC maintenance time (minutes): 4.21;
Mass data: 494 $(M+H)^+$.

Example 17(46)

6-bromo-2-((4-ethylphenyl)methyloxy)-3-(4-methylphenylsulfonylamino)pyrazine
HPLC maintenance time (minutes): 4.43;
Mass data: 462 $(M+H)^+$.

Example 17(47)

6-bromo-2-((4-(1-methylethyl)phenyl)methyloxy)-3-(4-methylphenylsulfonylamino)pyrazine
HPLC maintenance time (minutes): 4.50;

Mass data: 476 $(M+H)^+$.

Example 17(48)

6-bromo-2-((4-(1,1-dimethylethyl)phenyl)methyloxy)-3-(4-methylphenylsulfonylamino)pyrazine HPLC maintenance time (minutes): 4.55;
Mass data: 490 $(M+H)^+$.

Example 17(49)

6-bromo-2-((1,3-dioxaindan-4-yl)methyloxy)-3-(4-methylphenylsulfonylamino)pyrazine HPLC maintenance time (minutes): 4.19;
Mass data: 478 $(M+H)^+$.

Example 17(50)

6-bromo-2-((1,3-dioxaindan-5-yl)methyloxy)-3-(4-methylphenylsulfonylamino)pyrazine HPLC maintenance time (minutes): 4.17;
Mass data: 478 $(M+H)^+$.

Example 17(51)

6-bromo-2-((4-chlorophenyl)methyloxy)-3-(4-methylphenylsulfonylamino)pyrazine HPLC maintenance time (minutes): 4.34;
Mass data: 468 $(M+H)^+$.

Example 17(52)

6-bromo-2-((4-methoxyphenyl)methyloxy)-3-(4-methylphenylsulfonylamino)pyrazine HPLC maintenance time (minutes): 4.22;
Mass data: 464 $(M+H)^+$.

Example 17(53)

6-bromo-2-((4-trifluoromethylphenyl)methyloxy)-3-(4-methylphenylsulfonylamino)pyrazine HPLC maintenance time (minutes): 4.35;
Mass data: 502 $(M+H)^+$.

Example 17(54)

6-bromo-2-((naphthalen-2-yl)methyloxy)-3-(4-methylphenylsulfonylamino)pyrazine HPLC maintenance time (minutes): 4.41;
Mass data: 484 $(M+H)^+$.

Example 17(55)

6-bromo-2-((2,5-dimethoxyphenyl)methyloxy)-3-(4-methylphenylsulfonylamino)pyrazine HPLC maintenance time (minutes): 4.22;
Mass data: 494 $(M+H)^+$.

Example 17(56)

6-bromo-2-((3,5-dimethoxyphenyl)methyloxy)-3-(4-methylphenylsulfonylamino)pyrazine HPLC maintenance time (minutes): 4.22;
Mass data: 494 $(M+H)^+$.

Example 17(57)

6-bromo-2-((2,3-dichlorophenyl)methyloxy)-3-(4-methylphenylsulfonylamino)pyrazine
HPLC maintenance time (minutes): 4.43;
Mass data: 502 $(M+H)^+$.

Example 17(58)

6-bromo-2-((2,4-dichlorophenyl)methyloxy)-3-(4-methylphenylsulfonylamino)pyrazine
HPLC maintenance time (minutes): 4.45;
Mass data: 502 $(M+H)^+$.

Example 17(59)

6-bromo-2-((2,5-dichlorophenyl)methyloxy)-3-(4-methylphenylsulfonylamino)pyrazine
HPLC maintenance time (minutes): 4.43;
Mass data: 502 $(M+H)^+$.

Example 17(60)

6-bromo-2-((2,6-dichlorophenyl)methyloxy)-3-(4-methylphenylsulfonylamino)pyrazine
HPLC maintenance time (minutes): 4.45;
Mass data: 502 $(M+H)^+$.

Example 17(61)

6-bromo-2-((3,4-dichlorophenyl)methyloxy)-3-(4-methylphenylsulfonylamino)pyrazine
HPLC maintenance time (minutes): 4.43;
Mass data: 502 $(M+H)^+$.

Example 17(62)

6-bromo-2-((4-phenylphenyl)methyloxy)-3-(4-methylphenylsulfonylamino)pyrazine
HPLC maintenance time (minutes): 4.50;
Mass data: 510 $(M+H)^+$.

Example 17(63)

6-bromo-2-((3-phenoxyphenyl)methyloxy)-3-(4-methylphenylsulfonylamino)pyrazine
HPLC maintenance time (minutes): 4.46;
Mass data: 526 $(M+H)^+$.

Reference Example 12

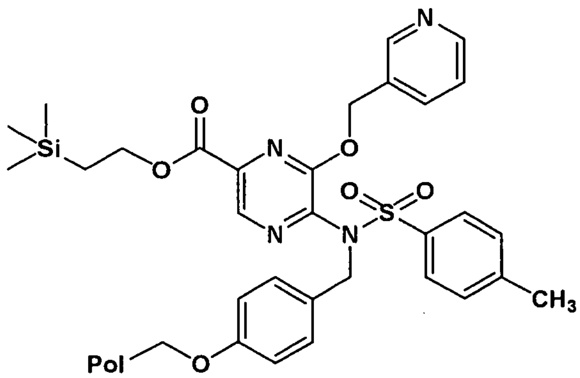

(5)

To a suspension of the compound (4) prepared in Reference Example 11 (1.0 g) in dimethylsulfoxide (10 mL), triethylamine (0.948 mL), 2-(trimethylsilyl)ethanol (0.487 mL) and dichlorobis(triphenylphosphine)palladium (II) (96 mg), successively, were added at room temperature. Under an atmosphere of carbon monoxide gas, the mixture was stirred for 24 hours at 80 °C. The reaction mixture was cooled to room temperature and filtered. The obtained resin was washed with dimethylsulfoxide (30 mL) for three times, tetrahydrofuran (30 mL) for three times and methylene chloride (30 mL) for three times, and dried to give the compound (5) (1.03 g). Reference Example 13

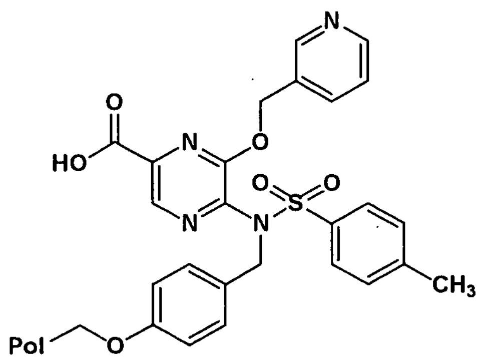

(6)

To a suspension of the compound (5) prepared in Reference Example 12 (500 mg) in tetrahydrofuran (5 mL), 1M tetrabutylammonium fluoride in tetrahydrofuran solution (5 mL) was added to room temperature. The mixture was stirred for 1 hour at room temperature. The reaction mixture was filtered. The obtained resin was washed with tetrahydrofuran (20 mL) for three times, a mixture of 0.2N hydrochloric acid and tetrahydrofuran (1:2)(20 mL) for three times, a mixture of water and tetrahydrofuran (1:2)(20 mL) for three times, tetrahydrofuran (20 mL) for three times and methylene chloride (20 mL) for three times, and dried to give the compound (6) (490 mg).

Example 18

6-(perhydroazepin-1-yl)carbonyl-2-((pyridin-3-yl)methyloxy)-3-(4-methylphenylsulfonylamino)pyrazine

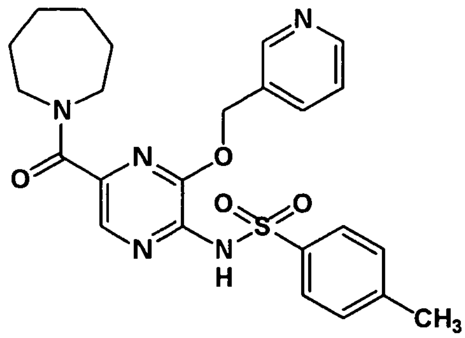

To a suspension of the compound (6) prepared in Reference Example 13 (490 mg) in N,N-dimethylformamide (5 mL), homopiperidine (0.186 mL), N,N-diisopropylethylamine (0.575 mL) and hexafluorophosphoric acid benzotriazol-1-yl-1-oxy-tris(pyrrolidino)phosphonium (859 mg) were successively added. The mixture was stirred for 4 hours at room temperature. The reaction mixture was filtered. To a suspension of the obtained resin in N,N-dimethylformamide (5 mL), homopiperidine (0.186 mL), N,N-diisopropylethylamine (0.575 mL), hexafluorophosphoric acid benzotriazol-1-yl-1-oxy-tris(pyrrolidino)phosphonium (859 mg) was successively added. The mixture was stirred for 16 hours at room temperature and the reaction mixture was filtered. The obtained resin was washed with N,N-dimethylformamide (20 mL) for five times and methylene chloride (20 mL) for five times. The title compound (167 mg) having the following physical data was obtained by the same procedure as a series of reactions of Example 14, using 50% solution of trifluoroacetic acid in methylene chloride in stead of 50% solution of trifluoroacetic acid in 1,2-dichloroethane.
TLC: Rf 0.66(chloroform : methanol = 10 : 1);
NMR($d_6$-DMSO): $\delta$ 8.57 (br, 1H), 8.49 (d, J=3.9Hz,, 1H), 7.65 (d, J=7.8Hz, 2H), 7.34 (m, 3H), 6.92 (d, J=7.8Hz, 2H), 5.26 (s, 2H), 3.49 (m, 4H), 2.37 (s, 3H), 1.60 (m, 4H), 1.41 (m, 4H);
HPLC maintenance time (minutes): 3.22;
Mass data: 963 $(2M+H)^+$, 482 $(M+H)^+$.

Example 18(1) - 18(77)

The following compounds were obtained, using a corresponding amine compound in stead of homopiperidine, by the same procedure as a series of reactions of Example 18.

Example 18(1)

6-cyclobutylaminocarbonyl-2-((pyridin-3-yl)methyloxy)-3-(4-methylphenylsulfonylamino)pyrazine
HPLC maintenance time (minutes): 3.26;
Mass data: 907 $(2M+H)^+$, 454 $(M+H)^+$.

Example 18(2)

6-cyclopentylaminocarbonyl-2-((pyridin-3-yl)methyloxy)-3-(4-methylphenylsulfonylamino)pyrazine
HPLC maintenance time (minutes): 3.33;
Mass data: 935 $(2M+H)^+$, 468 $(M+H)^+$.

Example 18(3)

6-cyclohexylaminocarbonyl-2-((pyridin-3-yl)methyloxy)-3-(4-methylphenylsulfonylamino)pyrazine
HPLC maintenance time (minutes): 3.40;
Mass data: 963 $(2M+H)^+$, 482 $(M+H)^+$.

Example 18(4)

6-(5-methylisooxazol-3-yl)aminocarbonyl-2-((pyridin-3-yl)methyloxy)-3-(4-methylphenylsulfonylamino)pyrazine
HPLC maintenance time (minutes): 3.36;

Mass data: 961 $(2M+H)^+$, 481 $(M+H)^+$.

Example 18(5)

6-(pyrrolidin-1-yl)carbonyl-2-((pyridin-3-yl)methyloxy)-3-(4-methylphenylsulfonylamino)pyrazine
HPLC maintenance time (minutes): 3.18;
Mass data: 907 $(2M+H)^+$, 454 $(M+H)^+$.

Example 18(6)

6-(3-hydroxypyrrolidin-1-yl)carbonyl-2-((pyridin-3-yl)methyloxy)-3-(4-methylphenylsulfonylamino)pyrazine
HPLC maintenance time (minutes): 3.07;
Mass data: 939 $(2M+H)^+$, 470 $(M+H)^+$.

Example 18(7)

6-(1,3-thiazolin-2-yl)aminocarbonyl-2-((pyridin-3-yl)methyloxy)-3-(4-methylphenylsulfonylamino)pyrazine
HPLC maintenance time (minutes): 3.14;
Mass data: 969 $(2M+H)^+$, 485 $(M+H)^+$.

Example 18(8)

6-((tetrahydrofuran-2-yl)methyl)aminocarbonyl-2-((pyridin-3-yl)methyloxy)-3-(4-methylphenylsulfonylamino) pyrazine
HPLC maintenance time (minutes): 3.20;
Mass data: 967 $(2M+H)^+$, 484 $(M+H)^+$.

Example 18(9)

6-(4-methylpiperazin-1-yl)carbonyl-2-((pyridin-3-yl)methyloxy)-3-(4-methylphenylsulfonylamino)pyrazine
HPLC maintenance time (minutes): 3.01;
Mass data: 965 $(2M+H)^+$, 483 $(M+H)^+$.

Example 18(10)

6-(morpholin-4-yl)carbonyl-2-((pyridin-3-yl)methyloxy)-3-(4-methylphenylsulfonylamino)pyrazine
HPLC maintenance time (minutes): 3.14;
Mass data: 939 $(2M+H)^+$, 470 $(M+H)^+$.

Example 18(11)

6-(thiomorpholin-4-yl)carbonyl-2-((pyridin-3-yl)methyloxy)-3-(4-methylphenylsulfonylamino)pyrazine
HPLC maintenance time (minutes): 3.25;
Mass data: 971 $(2M+H)^+$, 486 $(M+H)^+$.

Example 18(12)

6-(pyridin-3-yl)aminocarbonyl-2-((pyridin-3-yl)methyloxy)-3-(4-methylphenylsulfonylamino)pyrazine
HPLC maintenance time (minutes): 3.09;
Mass data: 953 $(2M+H)^+$, 477 $(M+H)^+$.

Example 18(13)

6-(pyridin-4-yl)aminocarbonyl-2-((pyridin-3-yl)methyloxy)-3-(4-methylphenylsulfonylamino)pyrazine
HPLC maintenance time (minutes): 3.10;
Mass data: 477 $(M+H)^+$.

Example 18(14)

6-(1,1-dimethylethyl)aminocarbonyl-2-((pyridin-3-yl)methyloxy)-3-(4-methylphenylsulfonylamino)pyrazine
HPLC maintenance time (minutes): 3.32;
Mass data: 456 $(M+H)^+$.

Example 18(15)

6-(1,2-dimethylpropyl)aminocarbonyl-2-((pyridin-3-yl)methyloxy)-3-(4-methylphenylsulfonylamino)pyrazine
HPLC maintenance time (minutes): 3.36;
Mass data: 939 $(2M+H)^+$, 470 $(M+H)^+$.

Example 18(16)

6-(1-methyl-2-methoxyethyl)aminocarbonyl-2-((pyridin-3-yl)methyloxy)-3-(4-methylphenylsulfonylamino) pyrazine
HPLC maintenance time (minutes): 3.20;
Mass data: 943 $(2M+H)^+$, 472 $(M+H)^+$.

Example 18(17)

6-(1,3-dimethylbutyl)aminocarbonyl-2-((pyridin-3-yl)methyloxy)-3-(4-methylphenylsulfonylamino)pyrazine
HPLC maintenance time (minutes): 3.47;
Mass data: 967 $(2M+H)^+$, 484 $(M+H)^+$.

Example 18(18)

6-(1-methylpropyl)aminocarbonyl-2-((pyridin-3-yl)methyloxy)-3-(4-methylphenylsulfonylamino)pyrazine
HPLC maintenance time (minutes): 3.31;
Mass data: 911 $(2M+H)^+$, 456 $(M+H)^+$.

Example 18(19)

6-(1-ethylpropyl)aminocarbonyl-2-((pyridin-3-yl)methyloxy)-3-(4-methylphenylsulfonylamino)pyrazine
HPLC maintenance time (minutes): 3.36;
Mass data: 939 $(2M+H)^+$, 470 $(M+H)^+$.

Example 18(20)

6-(1-methylbutyl)aminocarbonyl-2-((pyridin-3-yl)methyloxy)-3-(4-methylphenylsulfonylamino)pyrazine
HPLC maintenance time (minutes): 3.40;
Mass data: 939 $(2M+H)^+$, 470 $(M+H)^+$.

Example 18(21)

6-(2,2-dimethylpropyl)aminocarbonyl-2-((pyridin-3-yl)methyloxy)-3-(4-methylphenylsulfonylamino)pyrazine
HPLC maintenance time (minutes): 3.36;
Mass data: 939 $(2M+H)^+$, 470 $(M+H)^+$.

Example 18(22)

6-(2-hydroxypropyl)aminocarbonyl-2-((pyridin-3-yl)methyloxy)-3-(4-methylphenylsulfonylamino)pyrazine
HPLC maintenance time (minutes): 3.09;
Mass data: 915 $(2M+H)^+$, 458 $(M+H)^+$.

Example 18(23)

6-(2-methylpropyl)aminocarbonyl-2-((pyridin-3-yl)methyloxy)-3-(4-methylphenylsulfonylamino)pyrazine

HPLC maintenance time (minutes): 3.29;
Mass data: 911 $(2M+H)^+$, 456 $(M+H)^+$.

Example 18(24)

6-(2-fluoroethyl)aminocarbonyl-2-((pyridin-3-yl)methyloxy)-3-(4-methylphenylsulfonylamino)pyrazine
HPLC maintenance time (minutes): 3.16;
Mass data: 891 $(2M+H)^+$, 446 $(M+H)^+$.

Example 18(25)

6-(2-acetylaminoethyl)aminocarbonyl-2-((pyridin-3-yl)methyloxy)-3-(4-methylphenylsulfonylamino)pyrazine
HPLC maintenance time (minutes): 3.09;
Mass data: 969 $(2M+H)^+$, 485 $(M+H)^+$.

Example 18(26)

6-(2-methoxyethyl)aminocarbonyl-2-((pyridin-3-yl)methyloxy)-3-(4-methylphenylsulfonylamino)pyrazine
HPLC maintenance time (minutes): 3.16;
Mass data: 915 $(2M+H)^+$, 458 $(M+H)^+$.

Example 18(27)

6-pentylaminocarbonyl-2-((pyridin-3-yl)methyloxy)-3-(4-methylphenylsulfonylamino)pyrazine
HPLC maintenance time (minutes): 3.42;
Mass data: 939 $(2M+H)^+$, 470 $(M+H)^+$.

Example 18(28)

6-dimethylaminocarbonyl-2-((pyridin-3-yl)methyloxy)-3-(4-methylphenylsulfonylamino)pyrazine
HPLC maintenance time (minutes): 3.12;
Mass data: 855 $(2M+H)^+$, 428 $(M+H)^+$.

Example 18(29)

6-diethylaminocarbonyl-2-((pyridin-3-yl)methyloxy)-3-(4-methylphenylsulfonylamino)pyrazine
HPLC maintenance time (minutes): 3.25;
Mass data: 911 $(2M+H)^+$, 456 $(M+H)^+$.

Example 18(30)

6-(N-methyl-N-propylamino)carbonyl-2-((pyridin-3-yl)methyloxy)-3-(4-methylphenylsulfonylamino)pyrazine
HPLC maintenance time (minutes): 3.27;
Mass data: 911 $(2M+H)^+$, 456 $(M+H)^+$.

Example 18(31)

6-dipropylaminocarbonyl-2-((pyridin-3-yl)methyloxy)-3-(4-methylphenylsulfonylamino)pyrazine
HPLC maintenance time (minutes): 3.41;
Mass data: 967 $(2M+H)^+$, 484 $(M+H)^+$.

Example 18(32)

6-butylaminocarbonyl-2-((pyridin-3-yl)methyloxy)-3-(4-methylphenylsulfonylamino)pyrazine
HPLC maintenance time (minutes): 3.32;
Mass data: 911 $(2M+H)^+$, 456 $(M+H)^+$.

Example 18(33)

6-ethylaminocarbonyl-2-((pyridin-3-yl)methyloxy)-3-(4-methylphenylsulfonylamino)pyrazine
HPLC maintenance time (minutes): 3.16;
Mass data: 855 $(2M+H)^+$, 428 $(M+H)^+$.

Example 18(34)

6-(3-hydroxypiperidin-1-yl)carbonyl-2-((pyridin-3-yl)methyloxy)-3-(4-methylphenylsulfonylamino)pyrazine
HPLC maintenance time (minutes): 3.09;
Mass data: 967 $(2M+H)^+$, 484 $(M+H)^+$.

Example 18(35)

6-(N-methyl-N-(2-methylpropyl)amino)carbonyl-2-((pyridin-3-yl)methyloxy)-3-(4-methylphenylsulfonylamino)pyrazine
HPLC maintenance time (minutes): 3.31;
Mass data: 939 $(2M+H)^+$, 470 $(M+H)^+$.

Example 18(36)

6-(N-methyl-N-pentylamino)carbonyl-2-((pyridin-3-yl)methyloxy)-3-(4-methylphenylsulfonylamino)pyrazine
HPLC maintenance time (minutes): 3.44;
Mass data: 967 $(2M+H)^+$, 484 $(M+H)^+$.

Example 18(37)

6-(N-methyl-N-(1-methylethyl)amino)carbonyl-2-((pyridin-3-yl)methyloxy)-3-(4-methylphenylsulfonylamino) pyrazine
HPLC maintenance time (minutes): 3.25;
Mass data: 911 $(2M+H)^+$, 456 $(M+H)^+$.

Example 18(38)

6-(1-methylethyl)aminocarbonyl-2-((pyridin-3-yl)methyloxy)-3-(4-methylphenylsulfonylamino)pyrazine
HPLC maintenance time (minutes): 3.23;
Mass data: 883 $(2M+H)^+$, 442 $(M+H)^+$.

Example 18(39)

6-(pyrazol-3-yl)aminocarbonyl-2-((pyridin-3-yl)methyloxy)-3-(4-methylphenylsulfonylamino)pyrazine
HPLC maintenance time (minutes): 3.18;
Mass data: 931 $(2M+H)^+$, 466 $(M+H)^+$.

Example 18(40)

6-(1,2,3,6-tetrahydropyridin-1-yl)carbonyl-2-((pyridin-3-yl)methyloxy)-3-(4-methylphenylsulfonylamino)pyrazine
HPLC maintenance time (minutes): 3.27;
Mass data: 931 $(2M+H)^+$, 466 $(M+H)^+$.

Example 18(41)

6-(pyrimidin-4-yl)aminocarbonyl-2-((pyridin-3-yl)methyloxy)-3-(4-methylphenylsulfonylamino)pyrazine
HPLC maintenance time (minutes): 3.22;
Mass data: 933 $(2M+H)^+$, 478 $(M+H)^+$.

Example 18(42)

6-((1R)-1-hydroxymethyl-2-methylpropyl)aminocarbonyl-2-((pyridin-3-yl)methyloxy)-3-(4-methylphenylsulfonylamino)pyrazine
HPLC maintenance time (minutes): 3.22;
Mass data: 971 $(2M+H)^+$, 486 $(M+H)^+$.

Example 18(43)

6-((1R)-1-methyl-2-hydroxyethyl)aminocarbonyl-2-((pyridin-3-yl)methyloxy)-3-(4-methylphenylsulfonylamino)pyrazine
HPLC maintenance time (minutes): 3.11;
Mass data: 915 $(2M+H)^+$, 458 $(M+H)^+$.

Example 18(44)

6-(N-ethyl-N-(2-hydroxyethylamino)carbonyl-2-((pyridin-3-yl)methyloxy)-3-(4-methylphenylsulfonylamino) pyrazine
HPLC maintenance time (minutes): 3.01;
Mass data: 943 $(2M+H)^+$, 472 $(M+H)^+$.

Example 18(45)

6-(3-pyrrolin-1-yl)carbonyl-2-((pyridin-3-yl)methyloxy)-3-(4-methylphenylsulfonylamino)pyrazine
HPLC maintenance time (minutes): 3.18;
Mass data: 903 $(2M+H)^+$, 452 $(M+H)^+$.

Example 18(46)

6-(2-hydroxymethylpyrrolidin-1-yl)carbonyl-2-((pyridin-3-yl)methyloxy)-3-(4-methylphenylsulfonylamino) pyrazine
HPLC maintenance time (minutes): 3.11;
Mass data: 967 $(2M+H)^+$, 484 $(M+H)^+$.

Example 18(47)

6-(2-methylpiperidin-1-yl)carbonyl-2-((pyridin-3-yl)methyloxy)-3-(4-methylphenylsulfonylamino)pyrazine
HPLC maintenance time (minutes): 3.33;
Mass data: 963 $(2M+H)^+$, 482 $(M+H)^+$.

Example 18(48)

6-(3-methylpiperidin-1-yl)carbonyl-2-((pyridin-3-yl)methyloxy)-3-(4-methylphenylsulfonylamino)pyrazine
HPLC maintenance time (minutes): 3.33;
Mass data: 963 $(2M+H)^+$, 482 $(M+H)^+$.

Example 18(49)

6-(4-methylpiperidin-1-yl)carbonyl-2-((pyridin-3-yl)methyloxy)-3-(4-methylphenylsulfonylamino)pyrazine
HPLC maintenance time (minutes): 3.36;
Mass data: 963 $(2M+H)^+$, 482 $(M+H)^+$.

Example 18(50)

6-(1,1-dimethylpropyl)aminocarbonyl-2-((pyridin-3-yl)methyloxy)-3-(4-methylphenylsulfonylamino)pyrazine
HPLC maintenance time (minutes): 3.39;
Mass data: 939 $(2M+H)^+$, 470 $(M+H)^+$.

Example 18(51)

6-(1-hydroxymethylpropylamino)carbonyl-2-((pyridin-3-yl)methyloxy)-3-(4-methylphenylsulfonylamino)pyrazine
HPLC maintenance time (minutes): 3.16;
Mass data: 943 $(2M+H)^+$, 472 $(M+H)^+$.

Example 18(52)

6-(2-methylbutylamino)carbonyl-2-((pyridin-3-yl)methyloxy)-3-(4-methylphenylsulfonylamino)pyrazine
HPLC maintenance time (minutes): 3.38;
Mass data: 939 $(2M+H)^+$, 470 $(M+H)^+$.

Example 18(53)

6-(2-dimethylaminoethyl)aminocarbonyl-2-((pyridin-3-yl)methyloxy)-3-(4-methylphenylsulfonylamino)pyrazine
HPLC maintenance time (minutes): 3.00;
Mass data: 941 $(2M+H)^+$, 471 $(M+H)^+$.

Example 18(54)

6-(2-hydroxyethyl)aminocarbonyl-2-((pyridin-3-yl)methyloxy)-3-(4-methylphenylsulfonylamino)pyrazine
HPLC maintenance time (minutes): 3.07;
Mass data: 887 $(2M+H)^+$, 444 $(M+H)^+$.

Example 18(55)

6-(2-propynyl)aminocarbonyl-2-((pyridin-3-yl)methyloxy)-3-(4-methylphenylsulfonylamino)pyrazine
HPLC maintenance time (minutes): 3.22;
Mass data: 875 $(2M+H)^+$, 438 $(M+H)^+$.

Example 18(56)

6-(2-propenyl)aminocarbonyl-2-((pyridin-3-yl)methyloxy)-3-(4-methylphenylsulfonylamino)pyrazine
HPLC maintenance time (minutes): 3.22;
Mass data: 879 $(2M+H)^+$, 440 $(M+H)^+$.

Example 18(57)

6-(3-methylbutyl)aminocarbonyl-2-((pyridin-3-yl)methyloxy)-3-(4-methylphenylsulfonylamino)pyrazine
HPLC maintenance time (minutes): 3.40;
Mass data: 939 $(2M+H)^+$, 470 $(M+H)^+$.

Example 18(58)

6-(3-dimethylaminopropyl)aminocarbonyl-2-((pyridin-3-yl)methyloxy)-3-(4-methylphenylsulfonylamino)pyrazine
HPLC maintenance time (minutes): 3.01;
Mass data: 969 $(2M+H)^+$, 485 $(M+H)^+$.

Example 18(59)

6-(3-ethoxypropyl)aminocarbonyl-2-((pyridin-3-yl)methyloxy)-3-(4-methylphenylsulfonylamino)pyrazine
HPLC maintenance time (minutes): 3.23;
Mass data: 971 $(2M+H)^+$, 486 $(M+H)^+$.

Example 18(60)

6-hexylaminocarbonyl-2-((pyridin-3-yl)methyloxy)-3-(4-methylphenylsulfonylamino)pyrazine
HPLC maintenance time (minutes): 3.51;
Mass data: 967 $(2M+H)^+$, 484 $(M+H)^+$.

Example 18(61)

6-(N,N-bis(2-propenyl)amino)carbonyl-2-((pyridin-3-yl)methyloxy)-3-(4-methylphenylsulfonylamino)pyrazine
HPLC maintenance time (minutes): 3.35;
Mass data: 959 $(2M+H)^+$, 480 $(M+H)^+$.

Example 18(62)

6-(N-methyl-N-butylamino)carbonyl-2-((pyridin-3-yl)methyloxy)-3-(4-methylphenylsulfonylamino)pyrazine
HPLC maintenance time (minutes): 3.34;
Mass data: 939 $(2M+H)^+$, 470 $(M+H)^+$.

Example 18(63)

6-(N-ethyl-N-butylamino)carbonyl-2-((pyridin-3-yl)methyloxy)-3-(4-methylphenylsulfonylamino)pyrazine
HPLC maintenance time (minutes): 3.42;
Mass data: 967 $(2M+H)^+$, 484 $(M+H)^+$.

Example 18(64)

6-(N-methyl-N-hydroxyamino)carbonyl-2-((pyridin-3-yl)methyloxy)-3-(4-methylphenylsulfonylamino)pyrazine
HPLC maintenance time (minutes): 3.11;
Mass data: 859 $(2M+H)^+$, 430 $(M+H)^+$.

Example 18(65)

6-(3-methoxypropyl)aminocarbonyl-2-((pyridin-3-yl)methyloxy)-3-(4-methylphenylsulfonylamino)pyrazine
HPLC maintenance time (minutes): 3.16;
Mass data: 943 $(2M+H)^+$, 472 $(M+H)^+$.

Example 18(66)

6-(N-methyl-N-(2-dimethylaminoethylamino)carbonyl-2-((pyridin-3-yl)methyloxy)-3-(4-methylphenylsulfonylamino)pyrazine
HPLC maintenance time (minutes): 3.00;
Mass data: 969 $(2M+H)^+$, 485 $(M+H)^+$.

Example 18(67)

6-(N-ethyl-N-(1-methylethylamino)carbonyl-2-((pyridin-3-yl)methyloxy)-3-(4-methylphenylsulfonylamino) pyrazine
HPLC maintenance time (minutes): 3.31;
Mass data: 939 $(2M+H)^+$, 470 $(M+H)^+$.

Example 18(68)

6-(N-(1-hydroxyethyl)-N-(1-methylethyl)amino)carbonyl-2-((pyridin-3-yl)methyloxy)-3-(4-methylphenylsulfonylamino)pyrazine
HPLC maintenance time (minutes): 3.05;
Mass data: 971 $(2M+H)^+$, 486 $(M+H)^+$.

Example 18(69)

6-((1S)-1-hydroxymethyl-2-methylpropyl)aminocarbonyl-2-((pyridin-3-yl)methyloxy)-3-(4-methylphenylsulfonylamino)pyrazine
HPLC maintenance time (minutes): 3.20;
Mass data: 971 $(2M+H)^+$, 486 $(M+H)^+$.

Example 18(70)

6-((2R)-2-hydroxymethylpyrrolidin-1-yl)carbonyl-2-((pyridin-3-yl)methyloxy)-3-(4-methylphenylsulfonylamino)pyrazine
HPLC maintenance time (minutes): 3.12;
Mass data: 967 $(2M+H)^+$, 484 $(M+H)^+$.

Example 18(71)

6-((2R)-2-hydroxypropyl)aminocarbonyl-2-((pyridin-3-yl)methyloxy)-3-(4-methylphenylsulfonylamino)pyrazine
HPLC maintenance time (minutes): 3.11;
Mass data: 915 $(2M+H)^+$, 458 $(M+H)^+$.

Example 18(72)

6-((2S)-2-hydroxypropyl)aminocarbonyl-2-((pyridin-3-yl)methyloxy)-3-(4-methylphenylsulfonylamino)pyrazine
HPLC maintenance time (minutes): 3.11;
Mass data: 915 $(2M+H)^+$, 458 $(M+H)^+$.

Example 18(73)

6-(N-ethyl-N-methylamino)carbonyl-2-((pyridin-3-yl)methyloxy)-3-(4-methylphenylsulfonylamino)pyrazine
HPLC maintenance time (minutes): 3.18;
Mass data: 883 $(2M+H)^+$, 442 $(M+H)^+$.

Example 18(74)

6-(2,2,2-trifluoroethyl)aminocarbonyl-2-((pyridin-3-yl)methyloxy)-3-(4-methylphenylsulfonylamino)pyrazine
HPLC maintenance time (minutes): 3.33;
Mass data: 963 $(2M+H)^+$, 482 $(M+H)^+$.

Example 18(75)

6-(N-(2-hydroxyethyl)-N-propylamino)carbonyl-2-((pyridin-3-yl)methyloxy)-3-(4-methylphenylsulfonylamino) pyrazine
HPLC maintenance time (minutes): 3.05;
Mass data: 971 $(2M+H)^+$, 486 $(M+H)^+$.

Example 18(76)

6-(N-methyl-N-(2-methoxyethyl)amino)carbonyl-2-((pyridin-3-yl)methyloxy)-3-(4-methylphenylsulfonylamino)pyrazine
HPLC maintenance time (minutes): 3.16;
Mass data: 943 $(2M+H)^+$, 472 $(M+H)^+$.

Example 18(77)

6-benzylaminocarbonyl-2-((pyridin-3-yl)methyloxy)-3-(4-methylphenylsulfonylamino)pyrazine
HPLC maintenance time (minutes): 3.38;

Mass data: 979 $(2M+H)^+$, 490 $(M+H)^+$;
NMR ($d_6$-DMSO): δ 11.60-11.20 (br, 1H), 9.10 (t, J=6.3Hz, 1H), 8.80 (s, 1H), 8.56 (d, J=3.6Hz, 1H), 8.24 (s, 1H), 7.98 (dt, J=6.9, 1.8Hz, 1H), 7.89 (d, J=8.4Hz, 2H), 7.45-7.08 (m, 8H), 5.61 (s, 2H), 4.48 (d, J=6.3Hz, 2H), 2.35 (s, 3H).

Reference Example 14

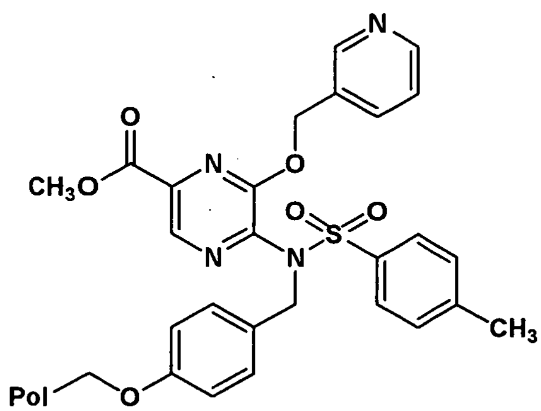

(7)

The compound (7) was obtained, using methanol in stead of 2-(trimethylsilyl)ethanol, by the same procedure as a series of reactions of Reference Example 12.

Example 19

6-hydroxymethyl-2-((pyridin-3-yl)methyloxy)-3-(4-methylphenylsulfonylamino)pyrazine

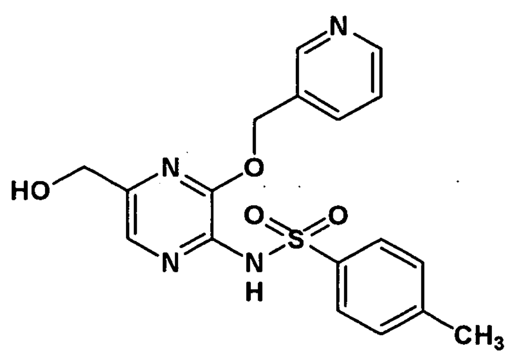

The compound (7) prepared in Reference Example 14 (400 mg) was added to 2M lithium borohydride in tetrahydrofuran solution (2 mL) at room temperature. The mixture was stirred for 2 hours at room temperature. The reaction mixture was filtered. The obtained resin was washed with methanol (3mL) for five times, tetrahydrofuran (3 mL) for five times and dichloroethane (3 mL) for five times. The title compound (41 mg) having the following physical data was obtained by the same procedure as a series of reactions of Example 14.
HPLC maintenance time (minutes): 3.07;
Mass data: 773 $(2M+H)^+$, 387 $(M+H)^+$.

Example 20

6-methyl-2-((pyridin-3-yl)methyloxy)-3-(4-methylphenylsulfonylamino)pyrazine

To a suspension of the compound (4) prepared in Reference Example 11 (300 mg) in anhydrous tetrahydrofuran (5 mL), [1,3-bis(diphenylphosphino)propane]dichloro nickel (II) (109 mg) was added at room temperature. The mixture was cooled to 0 °C, and 0.93M methyl magnesium bromide in tetrahydrofuran solution (1.11 mL) was added to the mixture. The mixture was stirred for 6 hours at room temperature. The reaction mixture was filtered. The obtained resin

was washed with methanol (5 mL) for five times and tetrahydrofuran (5 mL) for five times and 1,2-dichloroethane (3 mL) for five times. The title compound (100 mg) having the following physical data was obtained by the same procedure as a series of reactions of Example 14. NMR($D_2O$ : $CD_3CN$=55 : 45): δ 8.50 (s, 1H), 8.40 (d, J=5.0Hz, 1H), 7.89 (d, J=7.5Hz, 1H), 7.70 (d, J=8.0Hz, 2H), 7.46 (s, 1H), 7.35 (dd, J=7.5, 5.0Hz, 1H), 7.22 (d, J=8.0Hz, 2H), 5.29 (s, 2H), 2.26 (s, 3H), 2.19 (s, 3H);
HPLC maintenance time (minutes): 3.23;
Mass data: 741 $(2M+H)^+$, 371 $(M+H)^+$.

Reference Example 15

2-((3-(methoxymethyloxy)phenyl)methyloxy)-3-(N-(4-methylphenylsulfonyl)-N-(2-trimethylsilylethyl)amino) pyrazine

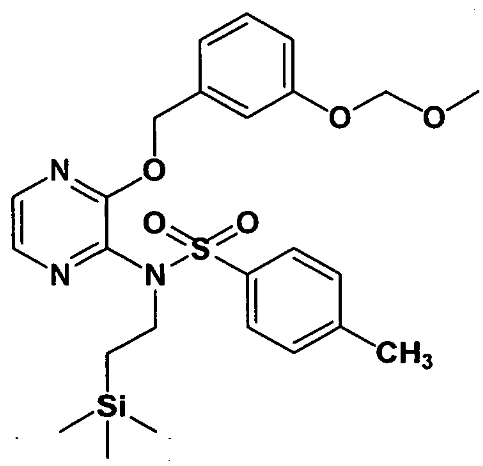

The title compound (843 mg) having the following physical data was obtained, using the compound prepared in Example 3(4) (1.3 mg), by the same procedure as a series of reactions of Reference Example 4.
TLC: Rf 0.75(hexane : ethyl acetate = 1 : 1);
NMR($d_6$-DMSO): δ 8.25 (d, J=2.7Hz, 1H), 8.16 (d, J=2.7Hz, 1H), 7.69 (d, J=8.1Hz, 2H), 7.35 (d, J=8.1Hz, 2H), 7.30 (t, J=7.5Hz, 1H), 7.11 (s, 1H), 7.06 (d, J=7.5Hz, 1H), 6.99 (d, J=7.5Hz, 1H), 5.40 (s, 2H), 5.19 (s, 2H), 3.57-3.50 (m, 2H), 3.36 (s, 3H), 2.38 (s, 3H), 0.50-0.43 (m, 2H), -0.14 (s, 9H).

Reference Example 16

2-((3-hydroxyphenyl)methyloxy)-3-(N-(4-methylphenylsulfonyl)-N-(2-trimethylsilylethyl)amino)pyrazine
To a solution of the compound prepared in Reference Example 15 (820 mg) in acetic acid (5.5 mL), water (5.5 mL) was added. The mixture was stirred for 3 hours at 90 °C. The reaction mixture was cooled to room temperature and concentrated. The residue was purified by column chromatography on silica gel (hexane : ethyl acetate = 2 : 1) to give the title compound (756 mg) having the following physical data.
TLC: Rf 0.34(hexane : ethyl acetate = 1 : 1);
NMR($CDCl_3$): δ 8.25 (d, J=2.7Hz, 1H), 8.14 (d, J=2.7Hz, 1H), 7.69 (d, J=8.4Hz, 2H), 7.36 (d, J=8.4Hz, 2H), 7.15 (t, J=8.1Hz, 1H), 6.85-6.82 (m, 2H), 6.75-6.72 (m, 1H), 5.34 (s, 2H), 3.57-3.49 (m, 2H), 2.39 (s, 3H), 0.50-0.40 (m, 2H), -0.14 (s, 9H).

Example 21

2-((3-ethoxyphenyl)methyloxy)-3-(4-methylphenylsulfonylamino)pyrazine
To a solution of the compound prepared in Reference Example 16 (30 mg) in tetrahydrofuran (2 mL), ethanol (0.011 mL), polymer support triphenylphosphine (1.34 mmol/g, 143mg) and 40% diethyl azodicarboxylate in toluene solution (0.087 mL) were successively added at 0 °C. The mixture was stirred for 18 hours at room temperature. The reaction mixture was filtered. The obtained resin was washed with tetrahydrofuran (1 mL). 5N aqueous solution of sodium hydroxide (1 mL) was added to the mixture of the filtrate and the washings, and the mixture was stirred for 30 minutes. After water (1 mL) was added to the reaction mixture, the mixture was extracted with chloroform (3 mL) for

two times. The extract was dried over magnesium sulfate and concentrated. 1.0M tetrabutylammonium fluoride in tetrahydrofuran solution (0.106 mL) was added to a solution of the obtained residue (23 mg) in tetrahydrofuran (1.5 mL). The mixture was stirred for 17 hours at room temperature. Water (1.5 mL) was added to the reaction mixture, and the mixture was extracted with chloroform (3 mL) for two times. The extract was dried over magnesium sulfate and concentrated to give the title compound (17 mg) having the following physical data.
TLC: Rf 0.73(hexane : ethyl acetate = 1 : 1);
NMR($D_2O$ : $CD_3CN$=40 : 60, 50 °C): δ 7.80 (d, J=8.5Hz, 2H), 7.59 (m, 2H), 7.28 (d, J=8.5Hz, 2H), 7.23 (t, J=8.0Hz, 1H), 6.93 (m, 2H), 6.83 (d, J=8.0Hz, 1H), 5.30 (s, 2H), 3.99 (q, J=7.0Hz, 2H), 2.32 (s, 3H), 1.29 (t, J=7.0Hz, 3H);
HPLC maintenance time (minutes): 4.06;
Mass data: 799 $(2M+H)^+$, 400 $(M+H)^+$.

Example 21(1)-21(7)

The following compounds were obtained, using a corresponding alcohol compound in stead of ethanol, by the same procedure as a series of reactions of Example 21.

Example 21(1)

2-((3-(1-methylethoxy)phenyl)methyloxy)-3-(4-methylphenylsulfonylamino)pyrazine
HPLC maintenance time (minutes): 4.45;
Mass data: 827 $(2M+H)^+$, 428 $(M+H)^+$.

Example 21(2)

2-((3-(2-methylpropyloxy)phenyl)methyloxy)-3-(4-methylphenylsulfonylamino)pyrazine
HPLC maintenance time (minutes): 4.32;
Mass data: 855 $(2M+H)^+$, 428 $(M+H)^+$.

Example 21(3)

2-((3-butyloxyphenyl)methyloxy)-3-(4-methylphenylsulfonylamino)pyrazine
HPLC maintenance time (minutes): 4.32;
Mass data: 855 $(2M+H)^+$, 428 $(M+H)^+$.

Example 21(4)

2-((3-(4-methoxybutyloxy)phenyl)methyloxy)-3-(4-methylphenylsulfonylamino)pyrazine
HPLC maintenance time (minutes): 4.10;
Mass data: 915 $(2M+H)^+$, 458 $(M+H)^+$.

Example 21(5)

2-((3-(3-dimethylaminopropyloxy)phenyl)methyloxy)-3-(4-methylphenylsulfonylamino)pyrazine
HPLC maintenance time (minutes): 3.42;
Mass data: 457 $(M+H)^+$.

Example 21(6)

2-((3-(tetrahydropyran-4-yloxy)phenyl)methyloxy)-3-(4-methylphenylsulfonylamino)pyrazine
HPLC maintenance time (minutes): 3.95;
Mass data: 911 $(2M+H)^+$, 456 $(M+H)^+$.

Example 21(7)

2-((3-(2-(piperidin-1-yl)ethyloxy)phenyl)methyloxy)-3-(4-methylphenylsulfonylamino)pyrazine
HPLC maintenance time (minutes): 3.47;
Mass data: 483 $(M+H)^+$.

Example 22

6-carboxy-2-((pyridin-3-yl)methyloxy)-3-(4-methylphenylsulfonylamino)pyrazine

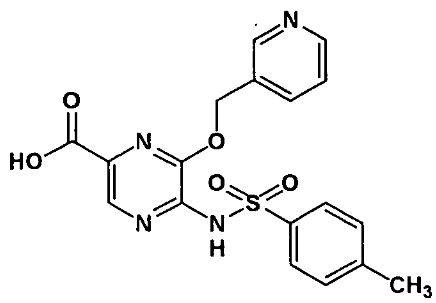

The compound (6) prepared in Reference Example 13 (110 mg) was added to 1M tetrabutylammonium fluoride in tetrahydrofuran solution (1 mL). The mixture was stirred for 1 hour at room temperature. The reaction mixture was filtered. The obtained resin was washed with tetrahydrofuran (3 mL) for five times and 1,2-dichloroethane (3 mL) for five times. The title compound (25 mg) having the following physical data was obtained by the same procedure as a series of reactions of Example 14.
TLC: Rf0.37(chloroform : methanol: acetic acid = 9 : 1 : 0.5);
HPLC maintenance time (minutes): 3.14;
Mass data: 801 $(2M+H)^+$, 401 $(M+H)^+$;
NMR ($d_6$-DMSO): δ 8.72 (s, 1H), 8.51 (d, J=3.3Hz, 1H), 8.03 (s, 1H), 7.94 (d, J=7.2Hz, 1H), 7.67 (d, J=8.1Hz, 2H), 7.38 (dd, J=7.5, 4.8Hz, 1H), 7.11 (d, J=8.1Hz, 2H), 5.29 (s, 2H), 2.27 (s, 3H).

Example 23

6-methoxylcarbonyl-2-((pyridin-3-yl)methyloxy)-3-(4-methylphenylsulfonylamino)pyrazine
The title compound (120 mg) was obtained, using the compound (7) prepared in Reference Example 14 and 25% trifluoroacetic acid in methylene chloride solution in stead of 50% trifluoroacetic acid in 1,2-dichloroethane solution, by the same procedure as a series of reactions of Example 14.
TLC: Rf 0.66(chloroform : methanol : acetic acid = 9 : 1 : 0.5);
HPLC maintenance time (minutes): 3.23;
Mass data: 829 $(2M+H)^+$, 415 $(M+H)^+$;
NMR ($CDCl_3$): δ 8.71 (d, J=1.5Hz, 1H), 8.62 (dd, J=4.8, 1.5Hz, 1H), 8.52 (s, 1H), 8.02 (d, J=8.4Hz, 2H), 7.84 (dt, J=8.4, 1.8Hz, 1H), 7.37-7.26 (m, 3H), 5.48 (s, 2H), 3.94 (s, 3H), 2.41 (s, 3H).

Example 24(1) - 24(114)

The following compounds were obtained, using a corresponding benzyl alcohol in stead of 3,4-dimethoxybenzyl alcohol, and a corresponding sulfonyl chloride in stead of 4-chlorobenzensulfonyl chloride, by the same procedure as a series of reactions of Reference Example 6 → Example 7.

Example 24(1)

N-[3-(benzyloxy)-5-bromo-2-pyrazinyl]-2,3-dichlorobenzenesulfonamide
HPLC maintenance time (minutes): 4.26;
Mass data: 492, 490, 488 $(M+H)^+$.

Example 24(2)

N-[3-(benzyloxy)-5-bromo-2-pyrazinyl]-3-chloro-2-methylbenzenesulfonamide
HPLC maintenance time (minutes): 4.32;
Mass data: 472, 470, 468 $(M+H)^+$.

Example 24(3)

N-[3-(benzyloxy)-5-bromo-2-pyrazinyl]-2-(trifluoromethyl)benzenesulfonamide
HPLC maintenance time (minutes): 4.17;
Mass data: 490, 488 $(M+H)^+$.

Example 24(4)

N-[3-(benzyloxy)-5-bromo-2-pyrazinyl]-2-chlorobenzenesulfonamide
HPLC maintenance time (minutes): 4.11;
Mass data: 458, 456, 454 $(M+H)^+$.

Example 24(5)

N-[3-(benzyloxy)-5-bromo-2-pyrazinyl]-2-bromobenzenesulfonamide
HPLC maintenance time (minutes): 4.15;
Mass data: 502, 500, 498 $(M+H)^+$.

Example 24(6)

N-[3-(benzyloxy)-5-bromo-2-pyrazinyl]-2-chloro-4-fluorobenzenesulfonamide
HPLC maintenance time (minutes): 4.17;
Mass data: 476, 474, 472 $(M+H)^+$.

Example 24(7)

N-[3-(benzyloxy)-5-bromo-2-pyrazinyl]-2,5-dichloro-4-nitro-3-thiophenesulfonamide
HPLC maintenance time (minutes): 4.28;
Mass data: 543, 541, 539 $(M+H)^+$, 317, 214.

Example 24(8)

N-[3-(benzyloxy)-5-bromo-2-pyrazinyl]-2,6-dichlorobenzenesulfonamide
HPLC maintenance time (minutes): 4.21;
Mass data: 492, 490, 488 $(M+H)^+$.

Example 24(9)

N-[3-(benzyloxy)-5-bromo-2-pyrazinyl]-3-bromobenzenesulfonamide
HPLC maintenance time (minutes): 4.28;
Mass data: 502, 500, 498 $(M+H)^+$.

Example 24(10)

N-[3-(benzyloxy)-5-bromo-2-pyrazinyl]-2,4-difluorobenzenesulfonamide
HPLC maintenance time (minutes): 4.12;
Mass data: 458, 456 $(M+H)^+$.

Example 24(11)

N-{5-bromo-3-[(6-methoxy-3-pyridinyl)methoxy]-2-pyrazinyl}-4-methylbenzenesulfonamide
TLC: Rf 0.35 (hexane : ethyl acetate = 2 : 1);
NMR($CDCl_3$): $\delta$ 2.41 (s, 3H), 3.96 (s, 3H), 5.31 (s, 2H), 6.79 (d, J=8.5Hz, 1H), 7.29 (d, J=8.5Hz, 2H), 7.49 (s, 1H), 7.66 (dd, J=8.5, 2.5Hz, 1H), 7.86 (s, 1H), 7.98 (d, J=8.5Hz, 2H), 8.24 (d, J=2.5Hz, 1H);
Mass data: 931 $(2M+H)^+$, 467, 465 $(M+H)^+$.

Example 24(12)

N-[3-(benzyloxy)-5-bromo-2-pyrazinyl]-2-methylbenzenesulfonamide
HPLC maintenance time (minutes): 4.15;
Mass data: 436, 434 $(M+H)^+$.

Example 24(13)

N-{5-bromo-3-[(2-methoxy-3-pyridinyl)methoxy]-2-pyrazinyl}-4-methylbenzenesulfonamide
TLC: Rf 0.35 (hexane : ethyl acetate = 2 : 1);
NMR($CDCl_3$): δ 2.42 (s, 3H), 3.99 (s, 3H), 5.37 (s, 2H), 6.92 (dd, J=7.0, 5.0Hz, 1H), 7.30 (d, J=8.5Hz, 2H), 7.57 (s, 1H) 7.64 (dd, J=7.0, 2.0Hz, 1H), 7.85 (s, 1H), 7.99 (d, J=8.5Hz, 2H), 8.20 (dd, J=5.0, 2.0Hz, 1H);
Mass data: 467, 465 $(M+H)^+$.

Example 24(14)

N-[3-(benzyloxy)-5-bromo-2-pyrazinyl]-3-chloro-4-methylbenzenesulfonamide
HPLC maintenance time (minutes): 4.30;
Mass data: 472, 470, 468 $(M+H)^+$.

Example 24(15)

N-[3-(benzyloxy)-5-bromo-2-pyrazinyl]-3-chlorobenzenesulfonamide
HPLC maintenance time (minutes): 4.19;
Mass data: 458, 456, 454 $(M+H)^+$.

Example 24(16)

N-[3-(benzyloxy)-5-bromo-2-pyrazinyl]-2-fluorobenzenesulfonamide
HPLC maintenance time (minutes): 4.04;
Mass data: 440, 438 $(M+H)^+$.

Example 24(17)

N-[3-(benzyloxy)-5-bromo-2-pyrazinyl]-2,3,4-trichlorobenzenesulfonamide
HPLC maintenance time (minutes): 4.44;
Mass data: 528, 526, 524, 522 $(M+H)^+$.

Example 24(18)

N-[3-(benzyloxy)-5-bromo-2-pyrazinyl]-2,4-dichlorobenzenesulfonamide
HPLC maintenance time (minutes): 4.30;
Mass data: 492, 490, 488 $(M+H)^+$.

Example 24(19)

N-[3-(benzyloxy)-5-bromo-2-pyrazinyl]-2,6-difluorobenzenesulfonamide
HPLC maintenance time (minutes): 4.00;
Mass data: 458, 456 $(M+H)^+$.

Example 24(20)

N-[3-(benzyloxy)-5-bromo-2-pyrazinyl]-2-cyanobenzenesulfonamide
HPLC maintenance time (minutes): 4.00;
Mass data: 447, 445 $(M+H)^+$.

Example 24(21)

N-[3-(benzyloxy)-5-bromo-2-pyrazinyl]-4-fluorobenzenesulfonamide
HPLC maintenance time (minutes): 4.10;
Mass data: 440, 438 $(M+H)^+$.

Example 24(22)

N-[3-(benzyloxy)-5-bromo-2-pyrazinyl]-2,4,6-trichlorobenzenesulfonamide
HPLC maintenance time (minutes): 4.39;
Mass data: 528, 526, 524, 522 $(M+H)^+$.

Example 24(23)

N-[3-(benzyloxy)-5-bromo-2-pyrazinyl]-2-thiophenesulfonamide
TLC: Rf 0.27 (toluene : ethyl acetate = 15 :1);
NMR($CDCl_3$): $\delta$ 5.39 (s, 2H), 7.08 (dd, J=5.0, 4.0Hz, 1H), 7.42 (m, 5H), 7.61 (s, 1H), 7.63 (dd, J=5.0, 1.5Hz, 1H), 7.91 (dd, J=4.0, 1.5Hz, 1H), 7.94 (s, 1H);
Mass data: (APCI, Pos. 40V) 428, 426 $(M+H)^+$, 384, 382, 281, 279.

Example 24(24)

N-[3-(benzyloxy)-5-bromo-2-pyrazinyl]benzenesulfonamide
HPLC maintenance time (minutes): 4.06;
Mass data: 422, 420 $(M+H)^+$.

Example 24(25)

N-[3-(benzyloxy)-5-bromo-2-pyrazinyl]-4-methyl-3-nitrobenzenesulfonamide
HPLC maintenance time (minutes): 4.15;
Mass data: 481, 479 $(M+H)^+$.

Example 24(26)

N-[3-(benzyloxy)-5-bromo-2-pyrazinyl]-3-nitrobenzenesulfonamide
HPLC maintenance time (minutes): 4.13;
Mass data: 467, 465 $(M+H)^+$.

Example 24(27)

N-[3-(benzyloxy)-5-bromo-2-pyrazinyl]-2,5-dichloro-3-thiophenesulfonamide
TLC: Rf 0.49 (toluene : ethyl acetate = 6: 1);
NMR($CDCl_3$): $\delta$ 5.42 (s, 2H), 7.33 (s, 1H), 7.43 (m, 5H), 7.71 (s, 1H), 7.87 (s, 1H);
Mass data: (FAB, Pos.) 500, 498, 496, 494 $(M+H)^+$.

Example 24(28)

N-[3-(benzyloxy)-5-bromo-2-pyrazinyl]-4-bromobenzenesulfonamide
HPLC maintenance time (minutes): 4.26;
Mass data: 502, 500, 498 $(M+H)^+$.

Example 24(29)

N-[3-(benzyloxy)-5-bromo-2-pyrazinyl]-3-methylbenzenesulfonamide
HPLC maintenance time (minutes): 4.13;
Mass data: 436, 434 $(M+H)^+$.

Example 24(30)

N-[3-(benzyloxy)-5-bromo-2-pyrazinyl]-4-bromo-2,5-difluorobenzenesulfonamide
HPLC maintenance time (minutes): 4.26;
Mass data: 538, 536, 534 $(M+H)^+$.

Example 24(31)

N-[3-(benzyloxy)-5-bromo-2-pyrazinyl]-3-(trifluoromethyl)benzenesulfonamide
HPLC maintenance time (minutes): 4.24;
Mass data: 490, 488 $(M+H)^+$.

Example 24(32)

N-[3-(benzyloxy)-5-bromo-2-pyrazinyl]-4-ethylbenzenesulfonamide
HPLC maintenance time (minutes): 4.22;
Mass data: 450, 448 $(M+H)^+$.

Example 24(33)

N-[3-(benzyloxy)-5-bromo-2-pyrazinyl]-1-benzothiophen-3-sulfonamide
HPLC maintenance time (minutes): 4.22;
Mass data: 478, 476 $(M+H)^+$, 214.

Example 24(34)

N-[3-(benzyloxy)-5-bromo-2-pyrazinyl]-2-(trifluoromethoxy)benzenesulfonamide
HPLC maintenance time (minutes): 4.21;
Mass data: 506, 504 $(M+H)^+$.

Example 24(35)

N-[3-(benzyloxy)-5-bromo-2-pyrazinyl]-3-methoxybenzenesulfonamide
HPLC maintenance time (minutes): 4.08;
Mass data: 452, 450 $(M+H)^+$.

Example 24(36)

N-[3-(benzyloxy)-5-bromo-2-pyrazinyl]-4-(trifluoromethyl)benzenesulfonamide
TLC: Rf 0.42 (hexane : ethyl acetate = 4 : 1);
NMR($d_6$-DMSO): δ 5.37 (s, 2H), 7.38 (m, 3H), 7.52 (m, 2H), 7.93 (s, 1H), 7.96 (d, J=8.0Hz, 2H), 8.16 (d, J=8.0Hz, 2H), 11.42 (s, 1H);
Mass data: 490, 488 $(M+H)^+$.

Example 24(37)

N-[3-(benzyloxy)-5-bromo-2-pyrazinyl]-2,4-dichloro-6-methylbenzenesulfonamide
HPLC maintenance time (minutes): 4.54;
Mass data: 506, 504, 502 $(M+H)^+$.

Example 24(38)

N-[3-(benzyloxy)-5-bromo-2-pyrazinyl]-2-chloro-4-(trifluoromethyl)benzenesulfonamide
HPLC maintenance time (minutes): 4.37;
Mass data: 526, 524, 522 $(M+H)^+$.

Example 24(39)

N-[3-(benzyloxy)-5-bromo-2-pyrazinyl]-4,5-dichloro-2-thiophenesulfonamide
TLC: Rf 0.24 (hexane : ethyl acetate = 4 : 1);
NMR($CDCl_3$): δ 5.40 (s, 2H), 7.42 (m, 5H), 7.65 (s, 1H), 7.68 (s, 1H), 7.98 (s, 1H);
Mass data: (FAB, Pos.) 500, 498, 496, 494 $(M+H)^+$.

Example 24(40)

N-[3-(benzyloxy)-5-bromo-2-pyrazinyl]-5-fluoro-2-methylbenzenesulfonamide
HPLC maintenance time (minutes): 4.19;
Mass data: 454, 452 $(M+H)^+$.

Example 24(41)

N-[3-(benzyloxy)-5-bromo-2-pyrazinyl]-4-chloro-3-nitrobenzenesulfonamide
HPLC maintenance time (minutes): 4.21;
Mass data: 503, 501, 499 $(M+H)^+$.

Example 24(42)

2-({[3-(benzyloxy)-5-bromo-2-pyrazinyl]amino}sulfonyl)benzoic acid methyl ester
HPLC maintenance time (minutes): 4.11;
Mass data: 480, 478 $(M+H)^+$.

Example 24(43)

N-[3-(benzyloxy)-5-bromo-2-pyrazinyl]-1-benzothiophen-2-sulfonamide
HPLC maintenance time (minutes): 4.24;
Mass data: 478, 476 $(M+H)^+$, 214.

Example 24(44)

N-[3-(benzyloxy)-5-bromo-2-pyrazinyl]-2,4,6-trimethylbenzenesulfonamide
HPLC maintenance time (minutes): 4.39;
Mass data: 464, 462 $(M+H)^+$.

Example 24(45)

N-[3-(benzyloxy)-5-bromo-2-pyrazuiyl]-2,5-dichlorobenzenesulfonamide
HPLC maintenance time (minutes): 4.28;
Mass data: 492, 490, 488 $(M+H)^+$.

Example 24(46)

N-[3-(benzyloxy)-5-bromo-2-pyrazinyl]-4-(trifluoromethoxy)benzenesulfonamide
HPLC maintenance time (minutes): 4.26;
Mass data: 506, 504 $(M+H)^+$.

Example 24(47)

N-[3-(benzyloxy)-5-bromo-2-pyrazinyl]-2,4,5-trichlorobenzenesulfonamide
HPLC maintenance time (minutes): 4.48;
Mass data: 528, 526, 524, 522 $(M+H)^+$.

Example 24(48)

N-[3-(benzyloxy)-5-bromo-2-pyrazinyl]-5-methyl-2-(trifluoromethyl)-3-furansulfonamide

HPLC maintenance time (minutes): 4.30;
Mass data: 494, 492 $(M+H)^+$, 214, 158.

Example 24(49)

N-[3-(benzyloxy)-5-bromo-2-pyrazinyl]-2-methoxy-4-methylbenzenesulfonamide
HPLC maintenance time (minutes): 4.08;
Mass data: 466, 464 $(M+H)^+$.

Example 24(50)

N-[3-(benzyloxy)-5-bromo-2-pyrazinyl]-4-methoxy-2-nitrobenzene.sulfonamide
HPLC maintenance time (minutes): 4.11;
Mass data: 497, 495 $(M+H)^+$.

Example 24(51)

N-[3-(benzyloxy)-5-bromo-2-pyrazinyl]-4-nitrobenzenesulfonamide
HPLC maintenance time (minutes): 4.10;
Mass data: 467, 465 $(M+H)^+$.

Example 24(52)

N-[3-(benzyloxy)-5-bromo-2-pyrazinyl]-4-chloro-2,5-dimethylbenzenesulfonamide
HPLC maintenance time (minutes): 4.44;
Mass data: 486, 484, 482 $(M+H)^+$.

Example 24(53)

N-[3-(benzyloxy)-5-bromo-2-pyrazinyl]-2,5-dimethyl-3-thiophenesulfonamide
HPLC maintenance time (minutes): 4.22;
Mass data: 456, 454 $(M+H)^+$, 265, 214.

Example 24(54)

N-[3-(benzyloxy)-5-bromo-2-pyrazinyl]-3-biphenylsulfonamide
HPLC maintenance time (minutes): 4.37;
Mass data: 498, 496 $(M+H)^+$.

Example 24(55)

N-[3-(benzyloxy)-5-bromo-2-pyrazinyl]-2-methyl-5-nitrobenzenesulfonamide
HPLC maintenance time (minutes): 4.15;
Mass data: 481, 479 $(M+H)^+$.

Example 24(56)

N-[3-(benzyloxy)-5-bromo-2-pyrazinyl]-5-bromo-2-methoxybenzenesulfonamide
HPLC maintenance time (minutes): 4.19;
Mass data: 532, 530, 528 $(M+H)^+$.

Example 24(57)

N-[3-(benzyloxy)-5-bromo-2-pyrazinyl]-3,5-dimethylbenzenesulfonamide
HPLC maintenance time (minutes): 4.26;
Mass data: 450, 448 $(M+H)^+$.

Example 24(58)

N-[3-(benzyloxy)-5-bromo-2-pyrazinyl]-5-chloro-2-methoxybenzenesulfonamide
HPLC maintenance time (minutes): 4.19;
Mass data: 488, 486, 484 $(M+H)^+$.

Example 24(59)

N-[3-(benzyloxy)-5-bromo-2-pyrazinyl]-4-methoxybenzenesulfonamide
TLC: Rf 0.25 (hexane : ethyl acetate = 4 : 1);
NMR($d_6$-DMSO): δ 3.81 (s, 3H), 5.36 (s, 2H), 7.07 (d, J=9.0Hz, 2H), 7.38 (m, 3H), 7.53 (m, 2H), 7.90 (d, J=8.5Hz, 2H), 7.93 (s, 1H), 11.02 (s, 1H);
Mass data: 452, 450 $(M+H)^+$.

Example 24(60)

4-acetyl-N-[3-(benzyloxy)-5-bromo-2-pyrazinyl]benzenesulfonamide
HPLC maintenance time (minutes): 4.02;
Mass data: 464, 462 $(M+H)^+$.

Example 24(61)

N-[3-(benzyloxy)-5-bromo-2-pyrazinyl]-4-phenoxybenzenesulfonamide
HPLC maintenance time (minutes): 4.39;
Mass data: 514, 512 $(M+H)^+$.

Example 24(62)

3-[4-({[3-(benzyloxy)-5-bromo-2-pyrazinyl]amino}sulfonyl)phenyl]propanoic acid methyl ester
HPLC maintenance time (minutes): 4.06;
Mass data: 508, 506 $(M+H)^+$.

Example 24(63)

N-[3-(benzyloxy)-5-bromo-2-pyrazinyl]-3-cyanobenzenesulfonamide
HPLC maintenance time (minutes): 4.04;
Mass data: 447, 445 $(M+H)^+$.

Example 24(64)

N-[3-(benzyloxy)-5-bromo-2-pyrazinyl]-4-(difluoromethoxy)benzenesulfonamide
HPLC maintenance time (minutes): 4.10;
Mass data: 488, 486 $(M+H)^+$.

Example 24(65)

N-[3-(benzyloxy)-5-bromo-2-pyrazinyl]-4-isopropylbenzenesulfonamide
HPLC maintenance time (minutes): 4.32;
Mass data: 464, 462 $(M+H)^+$.

Example 24(66)

N-[3-(benzyloxy)-5-bromo-2-pyrazinyl]-5-(dimethylamino)-1-naphthalenesulfonamide
HPLC maintenance time (minutes): 3.95;
Mass data: 515, 513 $(M+H)^+$.

Example 24(67)

N-[3-(benzyloxy)-5-bromo-2-pyrazinyl]-5-fluoro-3-methyl-1-benzothiophen-2-sulfonamide HPLC maintenance time (minutes): 4.28;
Mass data: 510, 508 $(M+H)^+$, 317, 214.

Example 24(68)

N-[3-(benzyloxy)-5-bromo-2-pyrazinyl]-4-cyanobenzenesulfonamide
TLC: Rf 0.18 (hexane : ethyl acetate = 4 : 1);
NMR($d_6$-DMSO): δ 5.36 (s, 2H), 7.39 (m, 3H), 7.52 (m, 2H), 7.93 (s, 1H), 8.05 (d, J=8.5Hz, 2H), 8.10 (d, J=8.5Hz, 2H), 11.55 (s, 1H);
Mass data: 447, 445 $(M+H)^+$, 214.

Example 24(69)

N-[3-(benzyloxy)-5-bromo-2-pyrazinyl]-2-naphthalenesulfonamide
HPLC maintenance time (minutes): 4.24;
Mass data: 472, 470 $(M+H)^+$.

Example 24(70)

N-[3-(benzyloxy)-5-bromo-2-pyrazinyl]-4-(1,1-dimethylpropyl)benzenesulfonamide
HPLC maintenance time (minutes): 4.48;
Mass data: 492, 490 $(M+H)^+$.

Example 24(71)

N-[3-(benzyloxy)-5-bromo-2-pyrazinyl]-5-chloro-3-methyl-1-benzothiophen-2-sulfonamide HPLC maintenance time (minutes): 4.43;
Mass data: 528, 526, 524 $(M+H)^+$, 317, 214, 158.

Example 24(72)

N-[3-(benzyloxy)-5-bromo-2-pyrazinyl]-2,3,5,6-tetramethylbenzenesulfonamide
HPLC maintenance time (minutes): 4.46;
Mass data: 478, 476 $(M+H)^+$.

Example 24(73)

N-[3-(benzyloxy)-5-bromo-2-pyrazinyl]-2,5-dimethylbenzenesulfonamide
HPLC maintenance time (minutes): 4.24;
Mass data: 450, 448 $(M+H)^+$.

Example 24(74)

N-[3-(benzyloxy)-5-bromo-2-pyrazinyl]-4-butoxybenzenesulfonamide
HPLC maintenance time (minutes): 4.44;
Mass data: 494, 492 $(M+H)^+$.

Example 24(75)

N-[3-(benzyloxy)-5-bromo-2-pyrazinyl]-5-(5-chloro-1,2,4-thiadiazol-3-yl)-2-thiophenesulfonamide
HPLC maintenance time (minutes): 4.35;
Mass data: 548, 546, 544 $(M+H)^+$, 317, 214, 158.

Example 24(76)

N-[3-(benzyloxy)-5-bromo-2-pyrazinyl]-4-tert-butylbenzenesulfonamide
HPLC maintenance time (minutes): 4.41;
Mass data: 478, 476 $(M+H)^+$.

Example 24(77)

5-({[3-(benzyloxy)-5-bromo-2-pyrazinyl]amino}sulfonyl)-4-methyl-2-thiophenecarboxylic acid methyl ester
HPLC maintenance time (minutes): 4.13;
Mass data: 500, 498 $(M+H)^+$, 214.

Example 24(78)

N-[3-(benzyloxy)-5-bromo-2-pyrazinyl]-5-(5-isoxazolyl)-2-thiophenesulfonamide
HPLC maintenance time (minutes): 4.08;
Mass data: 495, 493 $(M+H)^+$, 214.

Example 24(79)

N-[3-(benzyloxy)-5-bromo-2-pyrazinyl]-2,3,4,5,6-pentamethylbenzenesulfonamide
HPLC maintenance time (minutes): 4.50;
Mass data: 492, 490 $(M+H)^+$.

Example 24(80)

N-[3-(benzyloxy)-5-bromo-2-pyrazinyl]-5-[2-(methylthio)-4-pyrimidinyl]-2-thiophenesulfonamide
HPLC maintenance time (minutes): 4.24;
Mass data: 552, 550 $(M+H)^+$, 214, 158.

Example 24(81)

N-[3-(benzyloxy)-5-bromo-2-pyrazinyl]-4-(methylsulfonyl)benzenesulfonamide
HPLC maintenance time (minutes): 3.86;
Mass data: 500, 498 $(M+H)^+$.

Example 24(82)

N-[3-(benzyloxy)-5-bromo-2-pyrazinyl]-4-methoxy-2,3,6-trimethylbenzenesulfonamide
HPLC maintenance time (minutes): 4.39;
Mass data: 494,492 $(M+H)^+$.

Example 24(83)

N-[3-(benzyloxy)-5-bromo-2-pyrazinyl]-6-(dimethylamino)-2-naphthalenesulfonamide
HPLC maintenance time (minutes): 4.17;
Mass data: 515, 513 $(M+H)^+$.

Example 24(84)

N-[3-(benzyloxy)-5-bromo-2-pyrazinyl]-4-(2-methoxyphenoxy)benzenesulfonamide
HPLC maintenance time (minutes): 4.28;
Mass data: 544, 542 $(M+H)^+$.

Example 24(85)

N-[3-(benzyloxy)-5-bromo-2-pyrazinyl]-2-methoxy-5-methylbenzenesulfonamide
HPLC maintenance time (minutes): 4.12;

Mass data: 466, 464 $(M+H)^+$.

Example 24(86)

N-[3-(benzyloxy)-5-bromo-2-pyrazinyl]-3,4-dimethoxybenzenesulfonamide
HPLC maintenance time (minutes): 3.97;
Mass data: 482, 480 $(M+H)^+$.

Example 24(87)

N-[3-(benzyloxy)-5-bromo-2-pyrazinyl]-2,5-dimethoxybenzenesulfonamide
HPLC maintenance time (minutes): 4.02;
Mass data: 482, 480 $(M+H)^+$.

Example 24(88)

N-[3-(benzyloxy)-5-bromo-2-pyrazinyl]-5-(2-pyridinyl)-2-thiophenesulfonamide
HPLC maintenance time (minutes): 4.08;
Mass data: 505, 503 $(M+H)^+$, 317, 214.

Example 24(89)

N-[3-(benzyloxy)-5-bromo-2-pyrazinyl]-5-chloro-1,3-dimethyl-1H-pyrazol-4-sulfonamide
HPLC maintenance time (minutes): 3.93;
Mass data: 476, 474, 472 $(M+H)^+$, 317,214.

Example 24(90)

N-[3-(benzyloxy)-5-bromo-2-pyrazinyl]-5-(3-isoxazolyl)-2-thiophenesulfonamide
HPLC maintenance time (minutes): 4.06;
Mass data: 495, 493 $(M+H)^+$, 214.

Example 24(91)

N-[3-(benzyloxy)-5-bromo-2-pyrazinyl]-6-chloroimidazo[2,1-b][1,3]thiazole-5-sulfonamide
HPLC maintenance time (minutes): 4.04;
Mass data: 504, 502, 500 $(M+H)^+$, 317, 214.

Example 24(92)

N-[3-(benzyloxy)-5-bromo-2-pyrazinyl]-5-(2-methyl-1,3-thiazol-4-yl)-2-thiophenesulfonamide
HPLC maintenance time (minutes): 4.17;
Mass data: 525, 523 $(M+H)^+$, 214.

Example 24(93)

N-[3-(benzyloxy)-5-bromo-2-pyrazinyl]-5-methyl-1-phenylphenyl-1H-pyrazol-4-sulfonamide
HPLC maintenance time (minutes): 4.10;
Mass data: 502, 500 $(M+H)^+$, 214.

Example 24(94)

N-[3-(benzyloxy)-5-bromo-2-pyrazinyl]-5-(1,3-oxazol-5-yl)-2-thiophenesulfonamide
HPLC maintenance time (minutes): 3.95;
Mass data: 495, 493 $(M+H)^+$, 386, 384, 214.

Example 24(95)

4-({[3-(benzyloxy)-5-bromo-2-pyrazinyl]amino}sulfonyl)-3,5-dimethyl-1H-pyrrole-2-carboxylic acid ethyl ester
HPLC maintenance time (minutes): 4.06;
Mass data: 511, 509 $(M+H)^+$, 416, 414, 231, 214.

Example 24(96)

N-[3-(benzyloxy)-5-bromo-2-pyrazinyl]-2,4-dimethyl-1,3-thiazole-5-sulfonamide
HPLC maintenance time (minutes): 3.95;
Mass data: 457, 455 $(M+H)^+$, 214.

Example 24(97)

N-[3-(benzyloxy)-5-bromo-2-pyrazinyl]-3,5-dichlorobenzenesulfonamide
HPLC maintenance time (minutes): 4.41;
Mass data: 492, 490, 488 $(M+H)^+$, 372, 370, 279, 214, 158.

Example 24(98)

N-[3-(benzyloxy)-5-bromo-2-pyrazinyl]-3,4-dichlorobenzenesulfonamide
HPLC maintenance time (minutes): 4.37;
Mass data: 492, 490, 488 $(M+H)^+$, 317, 214, 158.

Example 24(99)

N-[3-(benzyloxy)-5-bromo-2-pyrazinyl]-3-chloro-4-fluorobenzenesulfonamide
HPLC maintenance time (minutes): 4.22;
Mass data: 476, 474, 472 $(M+H)^+$, 317, 214.

Example 24(100)

N-[3-(benzyloxy)-5-bromo-2-pyrazinyl]-3-fluorobenzenesulfonamide
HPLC maintenance time (minutes): 4.11;
Mass data: 440, 438 $(M+H)^+$, 279, 214.

Example 24(101)

N-[3-(benzyloxy)-5-bromo-2-pyrazinyl]-3,4-difluorobenzenesulfonamide
HPLC maintenance time (minutes): 4.13;
Mass data: 458, 456 $(M+H)^+$, 317, 214.

Example 24(102)

N-[3-(benzyloxy)-5-bromo-2-pyrazinyl]-2,3,4,5,6-pentafluorobenzenesulfonamide
HPLC maintenance time (minutes): 4.21;
Mass data: 512, 510 $(M+H)^+$, 317, 214.

Example 24(103)

N-[3-(benzyloxy)-5-bromo-2-pyrazinyl]-4-methoxy-3,5-dinitrobenzenesulfonamide
HPLC maintenance time (minutes): 4.17;
Mass data: 542, 540 $(M+H)^+$, 317, 214.

Example 24(104)

N-[3-(benzyloxy)-5-bromo-2-pyrazinyl]-4-propylbenzenesulfonamide
HPLC maintenance time (minutes): 4.37;

Mass data: 464, 462 $(M+H)^+$, 279, 214.

Example 24(105)

N-[3-(benzyloxy)-5-bromo-2-pyrazinyl]-4-butylbenzenesulfonamide
HPLC maintenance time (minutes): 4.43;
Mass data: 478, 476 $(M+H)^+$, 317, 214.

Example 24(106)

N-[3-(benzyloxy)-5-bromo-2-pyrazinyl]-4-isopropoxybenzenesulfonamide
HPLC maintenance time (minutes): 4.24;
Mass data: 480, 478 $(M+H)^+$, 214.

Example 24(107)

N-[3-(benzyloxy)-5-bromo-2-pyrazinyl]-3-(difluoromethoxy)benzenesulfonamide
HPLC maintenance time (minutes): 4.11;
Mass data: 488, 486 $(M+H)^+$, 214.

Example 24(108)

N-[3-(benzyloxy)-5-bromo-2-pyrazinyl]-3,5-bis(trifluoromethyl)benzenesulfonamide
HPLC maintenance time (minutes): 4.37;
Mass data: 558, 556 $(M+H)^+$, 317, 214, 158.

Example 24(109)

N-[3-(benzyloxy)-5-bromo-2-pyrazinyl]-5-bromo-2-thiophenesulfonamide
HPLC maintenance time (minutes): 4.22;
Mass data: 508, 506, 504 $(M+H)^+$, 317, 214, 158.

Example 24(110)

N-[3-(benzyloxy)-5-bromo-2-pyrazinyl]-5-chloro-2-thiophenesulfonamide
HPLC maintenance time (minutes): 4.24;
Mass data: 464, 462, 460 $(M+H)^+$, 317, 214, 158.

Example 24(111)

N-[3-(benzyloxy)-5-bromo-2-pyrazinyl]-3,5-dimethyl-4-isoxazolesulfonamide
HPLC maintenance time (minutes): 4.06;
Mass data: 441, 439 $(M+H)^+$, 317, 214.

Example 24(112)

4-({[3-(benzyloxy)-5-bromo-2-pyrazinyl]amino}sulfonyl)-5-(4-chlorophenyl)-2-methyl-3-furancarboxlic acid ethyl ester
HPLC maintenance time (minutes): 4.63;
Mass data: 610, 608, 606 $(M+H)^+$, 214.

Example 24(113)

5-({[3-(benzyloxy)-5-bromo-2-pyrazinyl]amino}sulfonyl)-2-methyl-3-furancarboxylic acid methyl ester
HPLC maintenance time (minutes): 4.06;
Mass data: 484, 482 $(M+H)^+$, 317, 214.

Example 24(114)

N-[3-(benzyloxy)-5-bromo-2-pyrazinyl]-3-thiophenesulfonamide
HPLC maintenance time (minutes): 3.99;
Mass data: 428, 426 $(M+H)^+$, 214.
Biological Examples:

It was demonstrated, for Example, by the following experiments that the compound of the present invention of formula (I) has CCR4 antagonistic activity, inhibitory activity for effector cell functions and TNFα-regulating activity, and shows efficacy in animal disease models.
All the procedures were conducted by conventionally used method on the basis of basic biological methods. Furthermore, the measuring method of the present invention was modified to improve accuracy and/or sensitivity of the measurement for evaluating the compound of the present invention. The experimental method is explained in detail below.

Biological Example 1: Activity on Ca ion increase stimulated by MDC

1-1: Isolation of human CCR4 gene

Human bone marrow cell cDNA was prepared using Marathon cDNA amplification kit (manufactured by Clontech). PCR primers hCCR4XbaI-F1 (SEQ ID NO:1) and hCCR4XbaI-R1 (SEQ ID NO:2) were designed based on the sequence of GenBankX85740.
Using the human bone marrow cell cDNA as the template and using Ex Taq (manufactured by Takara), a PCR reaction (2 minutes at 95°C → [30 seconds at 95°C, 45 seconds at 60°C, 1 minute at 72°C] × 35 times) was carried out. The thus amplified PCR product was subjected to 1% agarose gel electrophoresis, purified using QIAquick Gel Extraction Kit (manufactured by QUIAGEN) and then digested with a restriction enzyme *Xba*1. The digested fragments were ligated to an expression vector pEF-BOS-bsr (*Nucleic acid Research,* 1990, Vol. 18, No. 17, p.5322) using DNA Ligation Kit Ver. 2 (manufactured by Takara) and transformed into *Escherichia coli* DH5a. By preparing the resulting plasmid pEF-BOS-bsr/hCCR4, its DNA sequence was identified.

1-2: Culturing of CHO cell

CHO-dhfr(-) was cultured using Ham's F-12 (containing fetal bovine serum (10%), penicillin (100 U/mL) and streptomycin (100 μg/ml)). Also, the transduced cell was cultured by adding blasticidin (5 μg/mL) to the above medium.

1-3: Transduction into CHO cell

The plasmid pEF-BOS-bsr/hCCR4 was transduced into the CHO-dhfr(-) cell using a DMRIE-C reagent (manufactured by Gibco BRL). After 48 hours, the medium was replaced with a medium containing 5 μg/ml blasticidin to carry out the selection, thereby establishing a stably overexpressing cell.

1-4: Inhibition test on activity of MDC mediated by CCR4 (activity of MDC to induce transient increase of Ca ions).

The thus established human CCR4 stably overexpressing CHO cell (CCR4/CHO cell) was suspended in Ham's F-12 medium and FBS (10%) and dispensed into a 96-well plate at $3.0 \times 10^4$ cells/well. One day after culturing at 37°C, the culture supernatant was discarded, and Ham's F-12 medium (containing Fura-2AM (5 μM), Probenecid (2.5 mM) and HEPES (20 mM; pH 7.4)) was dispensed at 80 μL/well to carry out incubation for 1 hour at 37°C under shaded condition. After washing twice with 1 × Hanks/HEPES (20 mM; pH 7.4) solution, the same solution was dispensed at 100 μL/well. Each of the test compounds was added to the thus Fura-2AM-incorporated CCR4/CHO cell, and 3 minutes thereafter, a recombinant human MDC (manufactured by PeproTach) diluted with 1 × Hanks/HEPES (20 mM; pH 7.4) solution was added thereto to a final concentration of 10 nM. Transient increase in the intracellular $Ca^{2+}$ concentration induced by the human MDC was measured using a $Ca^{2+}$ detector for 96-well (manufactured by Hamamatsu Photonics), and inhibition ratio (%) of the test compound was calculated by the following equation:

$$\text{Inhibition ratio} = [(Ec - Ea)/Ec] \times 100$$

Ec: measured value of $Ca^{2+}$ transient increase by MDC; and

Ea: measured value of $Ca^{2+}$ transient increase by MDC when a test compound was added

Inhibition ratio for each concentration of the compound was calculated, and the value ($IC_{50}$ value) indicating 50% inhibition ratio was determined from the inhibition curve.
As a result, the compounds of the invention showed an inhibition ratio of 50% or more at 10 μM. For Example, the compound of Example 1(1) showed an $IC_{50}$ value of 0.13 μM, and the compound of Example 1(2) showed an $IC_{50}$ value of 0.016 μM.

Biological Example 2: Activity on cell migration stimulated by MDC

2-1: Inhibition tests on MDC-induced T cell strain (CCRF-CEM cell) migration

A medium (0.3 mL) containing or free of MDC (20 nM, manufactured by PeproTech) was added to the lower chamber of 24-transwell plate, and further a medium (0.3 mL) containing a test compound solution of 2-fold concentration (containing 0.02% dimethylsulfoxide (DMSO)) or a medium (0.3 mL) containing DMSO only was added thereto. To the upper chamber, CCRF-CEM cell suspension prepared in $1 \times 10^6$ cells/50μl and the test compound solution of 2-fold concentration (0.05 mL) were added. The test was initiated by superimpose the upper chamber on the lower chamber. The chambers were incubated in a carbon gas incubator (5% $CO_2$, 95% humidity) kept at 37°C for 4 hours, and the cells remaining in the upper chamber were sucked up and removed. Then, 0.1 ml of a buffer (phosphate-buffered saline (PBS) containing ethylenediamine tetra-acetate sodium (EDTA) (20 μM) was added thereto, and subjected to reaction at 4°C for 30 minutes. The transwell plate was centrifuged (1000 r.p.m.) for 5 minutes, and the incubation liquid (600 μL) in the lower chamber was collected, and the cell number was counted using Flow Cytometer (manufactured by Becton·Dickinson).
Migration inhibitory activity by the compound of the present invention was calculated by the following equation:

$$\text{Inhibition ratio (\%)} = [(A-B)/A] \times 100$$

A: Control value of the well to which the medium containing DMSO and not containing test compound was added; and
B: Value of the well to which the medium containing DMSO and the test compound was added

Inhibition ratio for each concentration of the compound was calculated, and the value ($IC_{50}$ value) indicating 50% inhibition ratio was determined from the inhibition curve.
As results, the compound of the present invention showed an inhibition ratio of 50% or more at 10 μM. For Example, the compound of Example 6(1) showed an $IC_{50}$ value of 0.01 μM.

Biological Example 3: Mouse asthma model

3-1: Mouse OVA-induced asthma model

Ovalbumin (OVA, 0.2 mg/mL) and Alum (8 mg/mL) prepared in physiological saline were intraperitoneally administered (500 μL) to the mouse (male, C57BL/6) on Day 1 (test starting day) and Day 8 (1 week thereafter), to sensitize the mouse. On Days 15 to 21, the mouse was taken to an inhalation chamber (W: 240 mm × L: 240 mm × H: 120 mm), and a 2% OVA solution was sprayed with an ultrasonic wave type nebulizer (NE-U12, manufactured by Omron) for 20 minutes, to thus conduct induction. The compound of the present invention suspended in a medium, was administered orally at 30 minutes before OVA sensitization on Day 8 and at 30 minutes before OVA induction on Days 15 to 21. To the control group was administered only the medium. Three hours after OVA inhalation on Day 21, the mouse was exsanguinated, the catheter tube was inserted into the trachea, and the lung was washed with heparin-containing physiological saline (10 U/ml), to give a bronchoalveolar lavage fluid (BALF). Leukocyte number in BALF was counted using hemocyte counter (SF-3000, manufactured by Sysmex).
As results, the compound of the present invention, for Example, the compound of Example 1(3) suppressed leukocyte infiltration into the lung at a dose of 30 mg/kg.

Biological Example 4: Mouse dermatitis model

4-1: Mouse DTH model

The mouse (male, Balb/c) was shaved on the abdomen with hair clippers, and to the abdomen was applied

ethanol solution (100 μL) of 7% (w/v) 2,4,6-trinitrochlrobenzene (TNCB), to sensitize the mouse. Seven days after sensitization, a 1% (w/v) TNCB solution in olive oil (20 μL) was applied to the auricle of the mouse (both sides of the right ear), to conduct induction. The present compound dissolved in a medium, was applied to both sides of the right ear (20 μL) 2 hours before applying TNCB. To the control groups, was applied only the medium. Right after the administration of the compound and 24 hours after TNCB application, the thickness of the mouse auricle was measured with Dialthickness gauge (manufactured by Ozaki Seisakusho), which was used as indicator for efficacy in mouse DTH model.

As results, the compound of the present invention, for Example, the compound of Example 6(1) suppressed the auricle swelling at a concentration of 5%.

4-2: Mouse dermatitis model to which hapten is applied

To the auricle (both sides of the right ear) of the mouse (male, Balb/c), 1% (w/v) TNCB solution (acetone:olive oil = 4:1) (20 μL) was applied to conduct first sensitization. Seven days after sensitization, 1% (w/v) TNCB (acetone: olive oil = 4:1) (20 μL) was applied to the mouse auricle, to conduct induction (Day 0). Furthermore, on Days 2, 4, 6, 8, 10, 12, 14 and 16, the same procedure as on Day 0 was repeated. The compound of the present invention was dissolved in a medium, applied to both sides of the right ear (20 μL) 2 hours before applying TNCB. To the control groups, was applied only the medium. Right after the administration of the compound and 24 hours after TNCB application, the thickness of the mouse auricle was measured with Dialthickness gauge (manufactured by Ozaki Seisakusho), which was used as indicator for efficacy in mouse dermatitis model to which hapten was continuously applied.

As results, the compound of the present invention, for Example, the compound of Example 1(3) suppressed the auricle swelling at a concentration of 5%.

Experimental Example 5: Mouse TNFα production model

5-1: Mouse TNFα production model by LPS stimulation

The present compound suspended in a medium, was orally applied to a mouse (male, C57BL/6), and after 0.5 hour, lipipolysaccharide (LPS, 055:B5, Sigma) was peritoneally administered to the mouse at a dose of 60 mg/kg. To the control groups was orally applied only the medium. Sixty minutes after LPS treatment, heparin-added blood collection was conducted from the abdominal vena cava under ether anesthesia, and centrifuged (12,000 r.p.m.) at 4°C for 3 minutes, to give the plasma. The thus obtained plasma sample was stored at -80°C before use. TNFα in the plasma was quantified using an ELISA kit (R&D systems).

As results, the compound of the present invention, for Example, the compound of Example 1(1) suppressed TNFα production by LPS stimulation at a dose of 100 mg/kg.

Formulation Example 1:

The following components were admixed in conventional method and punched out to obtain 100 tablets each containing 50 mg of active ingredient.

| | |
|---|---|
| · 6-bromo-2-((3, 4-dimethoxyphenyl)methyloxy)-3-(4-methylphenylsulfonylamino)pyrazine | 5.0 g |
| · Carboxymethylcellulose calcium (disintegrating agent) | ····· 0.2 g |
| · Magnesium stearate (lubricating agent) | 0.1 g |
| · Microcrystalline cellulose | 4.7 g |

Formulation Example 2:

The following components were admixed in conventional method. The solution was sterilized in conventional manner, placed 5 ml portions into ampoules and freeze-dried to obtain 100 ampoules each containing 20 mg of the active ingredient.

| | |
|---|---|
| · 6-bromo-2-((3, 4-dimethoxyphenyl)methyloxy)-3-(4-methylphenylsulfonylamino)pyrazine | 2.0 g |
| · mannitol | 20 g |
| · distilled water | 500 ml |

## Claims

1. A compound of formula (I):

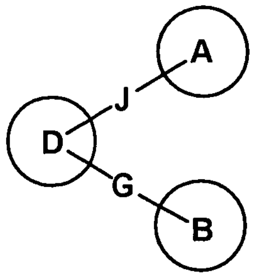

(I)

wherein ring A, ring B, and ring D each independently represents a cyclic group which may be substituted;
J represents a bond or a spacer having 1 to 8 atoms in its main chain; and
G represents a bond or a spacer having 1 to 4 atoms in its main chain;
or a salt thereof.

**2.** The compound according to claim 1, wherein

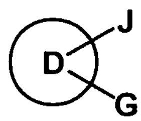

is

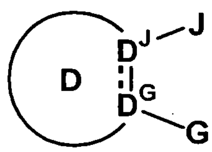

wherein $D^J$ and $D^G$ each independently represents a carbon atom or a nitrogen atom; and
---- represents a single bond or a double bond, and
when ---- represents a double bond, $D^J$ and $D^G$ each represents a carbon atom.

**3.** The compound according to claim 2, wherein ring D is a carbocyclic ring which may be substituted.

**4.** The compound according to claim 2, wherein ring D is a heterocyclic ring which may be substituted.

**5.** The compound according to claim 4, wherein the heterocyclic ring is a 3- to 15-membered monocyclic, bicyclic or tricyclic heterocyclic ring having 1 to 4 nitrogen atoms, 1 or 2 oxygen atoms and/or 1 or 2 sulfur atoms as a hetero atom(s).

**6.** The compound according to claim 2, wherein

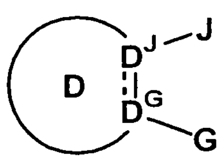

is

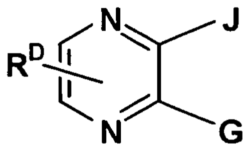 or 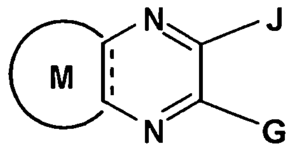

wherein $R^D$ represents a substituent of ring D; and M represents a 3- to 11-membered monocyclic or bicyclic cyclic group which may be substituted.

**7.** The compound according to claim 6, wherein

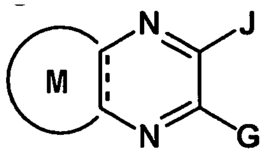

is

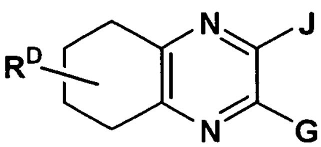, 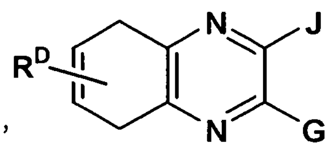, 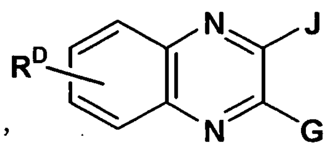、

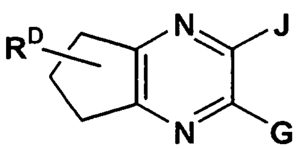, 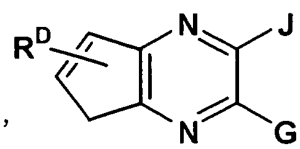 or 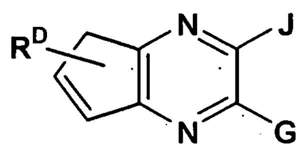

wherein $R^D$ has the same meaning as described in claim 6.

**8.** The compound according to claim 1, wherein ring A is a carbocyclic ring which may be substituted.

**9.** The compound according to claim 1, wherein ring A is a heterocyclic ring which may be substituted.

**10.** The compound according to claim 8, wherein the carbocyclic ring is a C3-15 monocyclic, bicyclic or tricyclic carbocyclic ring.

**11.** The compound according to claim 9, wherein the heterocyclic ring is a 3- to 15-membered monocyclic, bicyclic or tricyclic heterocyclic ring having 1 to 4 nitrogen atoms, 1 or 2 oxygen atoms and/or 1 or 2 sulfur atoms as a hetero atom(s).

**12.** The compound according to claim 10, wherein the carbocyclic ring is a benzene ring or a naphthalene ring.

**13.** The compound according to claim 11 wherein the heterocyclic ring is a pyridine ring, a pyrazole ring, a dioxaindane ring or a benzodioxane ring.

**14.** The compound according to claim 1, wherein ring B is a carbocyclic ring which may be substituted.

**15.** The compound according to claim 1, wherein ring B is a heterocyclic ring which may be substituted.

**16.** The compound according to claim 14, wherein the carbocyclic ring is a C3-15 monocyclic, bicyclic or tricyclic carbocyclic ring.

**17.** The compound according to claim 15, wherein the heterocyclic ring is a 3- to 15-membered monocyclic, bicyclic or tricyclic heterocyclic ring having 1 to 4 nitrogen atoms, 1 or 2 oxygen atoms and/or 1 or 2 sulfur atoms as a hetero atom(s).

**18.** The compound according to claim 16, wherein the carbocyclic ring is a C3-8 monocyclic carbocyclic ring.

**19.** The compound according to claim 17, wherein the heterocyclic ring is a 3- to 8-membered monocyclic heterocyclic ring having 1 to 4 nitrogen atoms, 1 or 2 oxygen atoms and/or 1 or 2 sulfur atoms as a hetero atom(s).

**20.** The compound according to claim 18, wherein the carbocyclic ring is a benzene ring.

**21.** The compound according to claim 19, wherein the heterocyclic ring is a pyridine ring or a thiophene ring.

**22.** The compound according to claim 1, wherein J is a spacer having 1 to 8 atoms in its main chain and containing at least one oxygen atom.

**23.** The compound according to claim 22, wherein the oxygen atom binds to ring D.

**24.** The compound according to claim 22, wherein J is

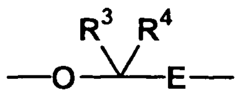

wherein $R^3$ and $R^4$ each independently represents hydrogen or C 1-8 alkyl; and E represents a bond or a spacer having 1 to 6 atoms in its main chain.

**25.** The compound according to claim 24, wherein $R^3$ and $R^4$ each independently represents hydrogen or methyl.

**26.** The compound according to claim 24, wherein E is a bond,

**27.** The compound according to claim 24, wherein E is a spacer having 1 to 6 atoms in its main chain.

**28.** The compound according to claim 27, wherein E is C1-4 alkylene or C1-3 alkyleneoxy.

**29.** The compound according to claim 28, wherein E is methylene or methylenoxy.

**30.** The compound according to claim 1, wherein G is a spacer having 1 to 4 atoms in its main chain and containing at least one nitrogen atom.

**31.** The compound according to claim 30, wherein G is $-NR^{T1}-$, $-NR^{T1}-SO_2-$, $-NR^{T1}-CO-$, $-NR^{T1}-CO-NR^{T2}-$, $-NR^{T1}-SO_2-NR^{T2}-$, $-NR^{T1}-COO-$, $-NR^{T1}-O-$, $-NR^{T1}-NR^{T2}-$, $-NR^{T1}-W-$, $-SO_2-NR^{T1}-$, $-CO-NR^{T1}-$, $-OCO-NR^{T1}-$, $-O-NR^{T1}-$ or $W-NR^{T1}-$, wherein W represents a bivalent C1-3 aliphatic hydrocarbon group which may be substituted; $R^{T1}$ and $R^{T2}$ each independently represents hydrogen, C1-8 alkyl which may be substituted, C2-8 alkenyl which may be substituted, C2-8 alkynyl which may be substituted or a 3- to 8-membered cyclic group which may be substituted.

**32.** The compound according to claim 31, wherein G is $-NH-SO_2-$.

**33.** The compound according to claim 1, wherein the compound is a compound of formula (A):

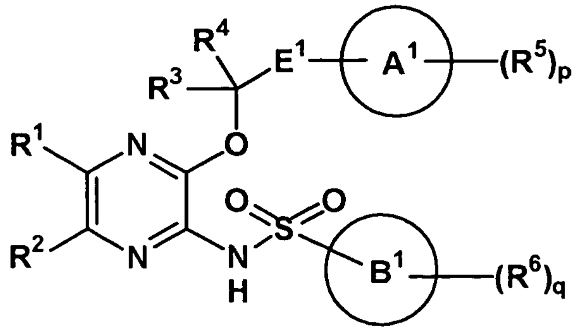

(A)

wherein $R^1$ and $R^2$ each independently represents (1) hydrogen, (2) C1-8 alkyl, (3) C2-8 alkenyl, (4) C2-8 alkynyl, (5) halogen, (6) cyano, (7) nitro, (8) $-CONR^7R^8$, (9) $-COOR^9$, (10) Cyc1 or (11) C1-8 alkyl substituted with 1 to 5 groups selected from (a) $-CONR^7R^8$, (b) $-COOR^9$, (c) $-OR^{10}$, (d) $-NR^{11}R^{12}$, (e) halogen, and (f) Cyc1; or

$R^1$ and $R^2$ are taken together to represent C3-4 alkylene, $-CH=CH-CH_2-$, $-CH_2-CH=CH-$, -CH=CH-CH=CH- or $-CH=CH-CH_2-CH_2-$, wherein the carbocyclic ring to be formed may be substituted with C1-8 alkyl, C2-8 alkenyl, C2-8 alkynyl, C1-8 alkoxy, halogen, cyano, nitro or hydroxyl, wherein $R^7$ and $R^8$ each independently represents (1) hydrogen, (2) C1-8 alkyl, (3) C2-8 alkenyl, (4) C2-8 alkynyl, (5) Cyc2, (6) $-OR^{13}$ or (7) C1-8alkyl, C2-8 alkenyl or C2-8 alkynyl substituted with 1 to 5 groups selected from (a) $-OR^{13}$, (b) $-NR^{14}R^{15}$, (c) $-NR^{16}COR^{17}$, (d) halogen, (e) $CF_3$, and (f) Cyc2; or

$R^7$ and $R^8$ are taken together with the adjacent nitrogen atom to represent a 3- to 8-membered monocyclic heterocyclic ring having at least one nitrogen atom as a hetero atom(s) and 0 to 3 nitrogen atoms, 0 to 1 oxygen atom and/or 0 to 1 sulfur atom as an other hetero atom(s), wherein the heterocyclic ring may be substituted with (a) C1-8 alkyl, (b) halogen, (c) hydroxyl, or (d) C1-8 alkyl substituted with hydroxyl;

$R^{13}$ to $R^{17}$ each independently represents (1) hydrogen, (2) C1-8 alkyl, (3) C2-8 alkenyl, (4) C2-8 alkynyl, (5) Cyc1, or (6) C1-8 alkyl, C2-8 alkenyl or C2-8 alkynyl substituted with Cyc1;

$R^9$ to $R^{12}$ each independently represents (1) hydrogen, (2) C1-8 alkyl, (3) C2-8 alkenyl, (4) C2-8 alkynyl, (5) Cyc1, or (6) C1-8 alkyl, C2-8 alkenyl or C2-8 alkynyl substituted with Cyc1;

Cyc1 represents a C3-15 monocyclic, bicyclic or tricyclic carbocyclic ring or a 3- to 15-membered monocyclic, bicyclic or tricyclic heterocyclic ring having 1 to 4 nitrogen atoms, 1 or 2 oxygen atoms and/or 1 or 2 sulfur atoms as a hetero atom(s), wherein Cyc1 may be substituted with 1 to 5 of $R^{18}$;

$R^{18}$ represents (1) C1-8 alkyl, (2) C2-8 alkenyl, (3) C2-8 alkynyl, (4) halogen, (5) cyano, (6) nitro, (7) trifluoromethyl, (8) trifluoromethoxy, (9) $-OR^{19}$, (10) $-SR^{20}$, (11) $-NR^{21}R^{22}$, (12) $-COR^{23}$, (13) - $COOR^{24}$, (14) $-NR^{25}COR^{26}$, (15) $-CONR^{27}R^{28}$, (16) Cyc2, or (17) C1-8 alkyl, C2-8alkenyl or C2-8 alkynyl substituted with 1 to 5 groups selected from (a) halogen, (b) cyano, (c) nitro, (d) trifluoromethyl, (e) trifluoromethoxy, (f) $-OR^{19}$, (g) $-SR^{20}$, (h) $-NR^{21}R^{22}$, (i) $-COR^{23}$, (j) $-COOR^{24}$, (k) $-NR^{25}COR^{26}$, (1) $-CONR^{27}R^{28}$, and (m) Cyc2;

$R^{19}$ to $R^{28}$ each independently represents (1) hydrogen, (2) C1-8 alkyl, (3) C2-8 alkenyl, (4) C2-8 alkynyl, (5) Cyc2, or (6) C1-8 alkyl, C2-8 alkenyl or C2-8 alkynyl substituted with Cyc2;

Cyc2 represents a C3-8 monocyclic carbocyclic ring or a 3- to 8-membered monocyclic heterocyclic ring having 1 to 4 nitrogen atoms, 1 or 2 oxygen atoms and/or 1 or 2 sulfur atoms as a hetero atom(s), wherein Cyc2 may be substituted with 1 to 5 of $R^{29}$;

$R^{29}$ represents (1) C1-8 alkyl, (2) C2-8 alkenyl, (3) C2-8 alkynyl, (4) halogen, (5) cyano, (6) nitro, (7) hydroxyl, (8) trifluoromethyl, (9) trifluoromethoxy, or (10) $-OR^{100}$;

$R^{100}$ represents C1-8 alkyl.;

$R^3$ and $R^4$ each independently represents hydrogen or C1-8 alkyl;

E' represents a bond or C1-6 alkylene, wherein a carbon atom in the alkylene group may be substituted with oxygen, sulfur, or $-NR^{30}-$;

$R^{30}$ represents (1) C1-8 alkyl, (2) C2-8 alkenyl, (3) C2-8 alkynyl, (4) phenyl, or (5) C1-8 alkyl substituted with phenyl;

ring $A^1$ represents a C3-15 monocyclic, bicyclic or tricyclic carbocyclic ring or a 3- to 15-membered monocyclic, bicyclic or tricyclic heterocyclic ring having 1 to 4 nitrogen atoms, 1 or 2 oxygen atoms and/or 1 or 2 sulfur atoms as a hetero atom(s);

$R^5$ represents (1) C1-8 alkyl, (2) C2-8 alkenyl, (3) C2-8 alkynyl, (4) halogen, (5) cyano, (6) nitro, (7) trifluoromethyl, (8) trifluoromethoxy, (9) $-OR^{31}$, (10) $-NR^{32}R^{33}$, (11) $-NR^{34}COR^{35}$, (12) Cyc3, or (13) C1-8 alkyl, C2-8 alkenyl or C2-8 alkynyl substituted with 1 to 5 groups selected from (a) halogen, (b) cyano, (c) nitro, (d) trifluoromethyl, (e) trifluoromethoxy, (f) $-OR^{31}$, (g) $-NR^{32}COR^{33}$, (h) $-NR^{34}COR^{35}$, and (i) Cyc3;

$R^{31}$ to $R^{35}$ each independently represents (1) hydrogen, (2) C1-8 alkyl, (3) C2-8alkenyl, (4) C2-8 alkynyl, (5) Cyc3, or (6) C1-8 alkyl, C2-8 alkenyl or C2-8 alkynyl substituted with 1 to 5 groups selected from (a) Cyc3, (b) -$OR^{36}$ and (c) -$NR^{37}R^{38}$;
$R^{36}$ to $R^{38}$ each independently represents (1) hydrogen, (2) C1-8 alkyl, (3) -$OR^{39}$, or (4) -$NR^{40}R^{41}$;
$R^{39}$ to $R^{41}$ each independently represents hydrogen or C 1-8 alkyl;
Cyc3 represents a C3-8 monocyclic carbocyclic ring or a 3- to 8-membered monocyclic heterocyclic ring having 1 to 4 nitrogen atoms, 1 or 2 oxygen atoms and/or 1 or 2 sulfur atoms as a hetero atom(s);
ring $B^1$ represents a C3-15 monocyclic, bicyclic or tricyclic carbocyclic ring or a 3- to 15-membered monocyclic, bicyclic or tricyclic heterocyclic ring having 1 to 4 nitrogen atoms, 1 or 2 oxygen atoms and/or 1 or 2 sulfur atoms as a hetero atom(s);
$R^6$ represents (1) C1-8 alkyl, (2) C2-8 alkenyl, (3) C2-8 alkynyl, (4) halogen, (5) cyano, (6) nitro, (7) trifluoromethyl, (8) trifluoromethoxy, (9) -$OR^{42}$, (10) -$NR^{43}R^{44}$, (11) -$SR^{101}$, (12) -$SO_2R^{102}$, (13) -$COR^{103}$, (14) -$COOR^{104}$, (15) Cyc2, or (16) C1-8 alkyl, C2-8 alkenyl or C2-8 alkynyl substituted with 1 to 5 groups selected from (a) -$COOR^{104}$, (b) -$NR^{105}COR^{106}$ , and (c) Cyc2;
$R^{42}$ to $R^{44}$ and $R^{101}$ to $R^{106}$ each independently represents (1) hydrogen, (2) C1-8 alkyl, (3) Cyc2, or (4) -$COR^{107}$, or (5) C1-8 alkyl substituted with 1 to 5 halogen atoms;
$R^{107}$ represents C1-8 alkyl; and
p and q each independently represents 0 or an integer of 1 to 5.

**34.** A prodrug for the compound according to claim 1.

**35.** A pharmaceutical composition which comprises the compound of formula (I):

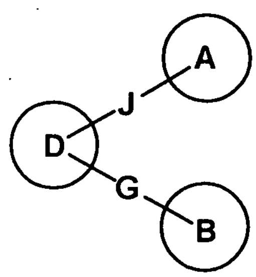

(I)

wherein ring A, ring B, and ring D each independently represents a cyclic group which may be substituted; J represents a bond or a spacer having 1 to 8 atoms in its main chain; and G represents a bond or a spacer having 1 to 4 atoms in its main chain;
or a salt thereof.

**36.** The pharmaceutical composition according to claim 35, which is a chemokine receptor antagonist.

**37.** The pharmaceutical composition according to claim 36, wherein the chemokine receptor is CCR4.

**38.** The pharmaceutical composition according to claim 37, which is a preventive and/or therapeutic agent for CCR4-mediated diseases.

**39.** The pharmaceutical composition according to claim 38, wherein the CCR4-mediated diseases are inflammatory and/or allergic diseases, metabolism and/or endocrine system diseases, cancer diseases or infections.

**40.** The pharmaceutical composition according to claim 39, wherein the CCR4-mediated diseases are inflammatory and/or allergic diseases.

**41.** The pharmaceutical composition according to claim 40, wherein the inflammatory and/or allergic diseases are respiratory diseases or dermatosis.

**42.** The pharmaceutical composition according to claim 41, wherein the respiratory diseases are asthma.

**43.** The pharmaceutical composition according to claim 41, wherein the dermatosis is atopic dermatitis.

**44.** A method for preventing and/or treating CCR4-mediated diseases in a mammal, which comprises administering to a mammal an effective amount of the compound according to claim 1 or a salt thereof.

**45.** Use of the compound according to claim 1 or a salt thereof for the manufacture of a preventive and/or therapeutic agent for CCR4-mediated diseases.

**46.** A pharmaceutical composition which comprises: a preventive and/or therapeutic agent for CCR4-mediated diseases, which comprises the compound according to claim 1 or a salt thereof as an active ingredient; and one or at least two medicaments selected from a bronchodilator drug, a steroid drug, a non-steroidal antiinflammatory drug, a leukotriene receptor antagonist, a phosphodiesterase inhibitor, an immunosuppressant, an anti-allergic drug, a mediator-release inhibitor, an antihistamine drug, a metabolism promoter and/or a chemokine inhibitor.

**47.** The pharmaceutical composition according to claim 35, which is an inhibitor of effector cell function.

**48.** The pharmaceutical composition according to claim 47, which is an inhibitor of cell migration function.

**49.** The pharmaceutical composition according to claim 35, which is a TNF$\alpha$ regulator.

## INTERNATIONAL SEARCH REPORT

International application No.
PCT/JP03/08654

A. CLASSIFICATION OF SUBJECT MATTER
Int.Cl[7] C07D241/22, A61K31/4965, 31/497, 31/498, 31/506, A61P1/04, 1/16, 1/18, 3/10, 9/10, 11/00, 11/06, 13/12, 17/06, 19/02, 25/00, 27/02, 27/16, 29/00, 31/12, 31/18, 35/00, 35/02, 37/00,
According to International Patent Classification (IPC) or to both national classification and IPC

B. FIELDS SEARCHED
Minimum documentation searched (classification system followed by classification symbols)
Int.Cl[7] C07D241/22, A61K31/4965, 31/497, 31/498, 31/506, C07D401/12,403/12, 405/12, 409/12, 413/12, 417/12, A61K31/55

Documentation searched other than minimum documentation to the extent that such documents are included in the fields searched

Electronic data base consulted during the international search (name of data base and, where practicable, search terms used)
CA(STN), REGISTRY(STN), WPIDS(STN)

C. DOCUMENTS CONSIDERED TO BE RELEVANT

| Category* | Citation of document, with indication, where appropriate, of the relevant passages | Relevant to claim No. |
|---|---|---|
| X<br><br><br><br>A | JP 5-345759 A (Kaken Pharmaceutical Co., Ltd.), 27 December, 1993 (27.12.93), Claims; Par. No. [0080]; examples 5, 6 & EP 529365 A | 1-3,8-12, 14-20,22-25, 27-32,34-43, 45-49<br>33 |
| X | JP 4-257578 A (Bayer AG.), 11 September, 1992 (11.09.92), Claims; Par. No. [0182], example & EP 473024 A | 1,4,5,8-28, 30-32,34-43, 45-49 |
| X | WO 01/45694 A (SMITHKLINE BEECHAM CORP.), 28 June, 2001 (28.06.01), Pages 10, 17, 31 & JP 2003-518057 A | 1,3,8-12, 14-20,22-25, 27-32,34-43, 45-49 |

[X] Further documents are listed in the continuation of Box C. [ ] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 05 August, 2003 (05.08.03) | 19 August, 2003 (19.08.03) |
| Name and mailing address of the ISA/<br>Japanese Patent Office | Authorized officer |
| Facsimile No. | Telephone No. |

Form PCT/ISA/210 (second sheet) (July 1998)

INTERNATIONAL SEARCH REPORT

International application No.
PCT/JP03/08654

C (Continuation). DOCUMENTS CONSIDERED TO BE RELEVANT

| Category* | Citation of document, with indication, where appropriate, of the relevant passages | Relevant to claim No. |
|---|---|---|
| X | WO 02/20437 A (MENARINI RICERCHE),<br>14 March, 2002 (14.03.02),<br>Claims; examples 14, 15; page 33<br>& AU 2002-10495 A | 1-12,14-20,<br>35-43,45-49 |
| P,X | WO 03/16254 A (Ono Pharmaceutical Co., Ltd.),<br>27 February, 2003 (27.02.03),<br>Claims<br>(Family: none) | 1-43,45-49 |

Form PCT/ISA/210 (continuation of second sheet) (July 1998)

**INTERNATIONAL SEARCH REPORT**

International application No.
PCT/JP03/08654

**Box I Observations where certain claims were found unsearchable (Continuation of item 2 of first sheet)**

This international search report has not been established in respect of certain claims under Article 17(2)(a) for the following reasons:

1. [X] Claims Nos.: 44
because they relate to subject matter not required to be searched by this Authority, namely:
Claim 44 pertains to a method for treatment of the human body by therapy.

2. [X] Claims Nos.: 1-32, 34-43, and 45-49
because they relate to parts of the international application that do not comply with the prescribed requirements to such an extent that no meaningful international search can be carried out, specifically:
(See extra sheet)

3. [ ] Claims Nos.:
because they are dependent claims and are not drafted in accordance with the second and third sentences of Rule 6.4(a).

**Box II Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.

2. [ ] As all searchable claims could be searched without effort justifying an additional fee, this Authority did not invite payment of any additional fee.

3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest** [ ] The additional search fees were accompanied by the applicant's protest.
[ ] No protest accompanied the payment of additional search fees.

Form PCT/ISA/210 (continuation of first sheet (1)) (July 1998)

## INTERNATIONAL SEARCH REPORT

International application No.
PCT/JP03/08654

Continuation of A. CLASSIFICATION OF SUBJECT MATTER
(International Patent Classification (IPC))

Int.Cl[7] 37/06, 37/08, 43/00, 17/00, C07D401/12, 403/12, 405/12, 409/12, 413/12, 417/12, A61K31/55

(According to International Patent Classification (IPC) or to both national classification and IPC)

Continuation of Box No.I-2 of continuation of first sheet(1)

The subject matters of claims 1-32, 34-43, and 45-49 are a compound represented by the general formula (I), a medicinal composition containing the compound as an active ingredient, etc. However, the compound involves an extremely wide range of various compounds. It is hence difficult to make a complete search for all of them. On the other hand, the compounds which are supported by the description in the meaning of Article 6 of the PCT and are disclosed in the description in the meaning of Article 5 of the PCT are limited to an extremely small part of the compounds.

Consequently, claims 1-32, 34-43, and 45-49 and the description do not comply with the given requirements to such a degree that a meaningful international search can be made.

The term "prodrug" used in the claims is unclear as to what structure is implied, even when the statements in the description are investigated. This term hence makes the scope of the compounds and medicines of the invention unclear.

In this international search report, a search with respect to claims 1-32, 34-43, and 45-49 was hence made for the compounds specified in the description through prior art documents within the range of a reasonable burden.

Form PCT/ISA/210 (extra sheet) (July 1998)

SEQUENCE LISTING

<110> ONO PHARMACEUTICAL CO., LTD.

<120> CCR4 Antagonist and pharmaceutical use thereof

<130> ONF-4645PCT

<150> PCT/JP03/08654
<151> 2003-07-08

<150> JP 2002-200879
<151> 2002-07-10

<160> 2

<210> 1
<211> 35
<212> DNA
<213> Artificial Sequence

<220>
<223> Description of Artificial Sequence: primer hCCR4XbaI-F1

<400> 1
ctagtctaga gacctgcctt gaggagcctg tagag 35

<210> 2
<211> 35
<212> DNA
<213> Artificial Sequence

<220>
<223> Description of Artificial Sequence: primer hCCR4XbaI-R1

<400> 2
ctagtctaga gttcattgac tctgcatttc accat 35